(19) Europäisches Patentamt
European Patent Office
Office européen des brevets

(11) EP 4 663 634 A1

# (12) EUROPEAN PATENT APPLICATION

published in accordance with Art. 153(4) EPC

(43) Date of publication:
**17.12.2025 Bulletin 2025/51**

(21) Application number: **24753228.6**

(22) Date of filing: **01.02.2024**

(51) International Patent Classification (IPC):
***C07D 401/14*** (2006.01) ***C09K 11/06*** (2006.01)
***H10K 50/00*** (2023.01) ***H10K 50/10*** (2023.01)
***H10K 50/11*** (2023.01) ***H10K 59/00*** (2023.01)
***H10K 59/10*** (2023.01) ***H10K 85/60*** (2023.01)
***H10K 101/20*** (2023.01)

(52) Cooperative Patent Classification (CPC):
**C07D 401/14; C09K 11/06; H10K 50/00; H10K 50/10; H10K 50/11; H10K 59/00; H10K 59/10; H10K 85/60;** H10K 2101/20

(86) International application number:
**PCT/JP2024/003317**

(87) International publication number:
**WO 2024/166785 (15.08.2024 Gazette 2024/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.02.2023 JP 2023019500**

(71) Applicant: **Kyulux, Inc.**
**Fukuoka-shi, Fukuoka 819-0388 (JP)**

(72) Inventors:
- **CHO, Yong Joo**
  **Fukuoka-shi, Fukuoka 819-0388 (JP)**
- **SHIMAMURA, Naomi**
  **Fukuoka-shi, Fukuoka 819-0388 (JP)**
- **HIGA, Takuya**
  **Fukuoka-shi, Fukuoka 819-0388 (JP)**
- **MORIMOTO, Kei**
  **Fukuoka-shi, Fukuoka 819-0388 (JP)**
- **KODAMA, Yuka**
  **Fukuoka-shi, Fukuoka 819-0388 (JP)**
- **KANAHARA, Kousei**
  **Fukuoka-shi, Fukuoka 819-0388 (JP)**
- **SUZUKI, Yoshitake**
  **Fukuoka-shi, Fukuoka 819-0388 (JP)**
- **OZAWA, Hiroaki**
  **Fukuoka-shi, Fukuoka 819-0388 (JP)**

(74) Representative: **Zimmermann & Partner Patentanwälte mbB**
**P.O. Box 330 920**
**80069 München (DE)**

## (54) COMPOUND, LIGHT-EMITTING MATERIAL, AND LIGHT-EMITTING ELEMENT

(57) An organic light emitting device uses a compound of the following general formula. $X^1$ to $X^3$ represent N or C(R); R represents H, D or a substituent; $X^4$ represents N or C($R^1$), $X^5$ represents N or C($R^2$), $X^6$ represents N or C($R^3$), and only one of $X^4$ to $X^6$ represents N; $R^1$ to $R^5$ represent H, D, a cyano group, an alkyl group, an aryl group or a donor group, and one or more represent a cyano group and one or more represent a donor group; one or more of $Ar^1$ and $Ar^2$ represent a heteroaryl group bonding via the N; and $L^1$ represents a single bond or a linking group.

EP 4 663 634 A1

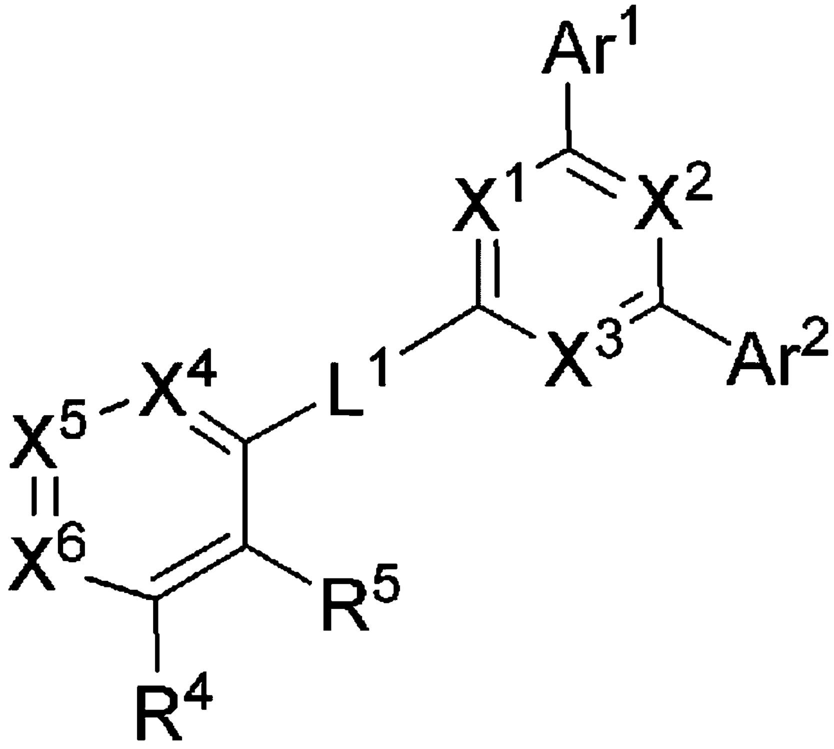

## Description

### Technical Field

The present invention relates to a compound useful as a light emitting material, and a light emitting device using the compound.

### Background Art

Studies for enhancing the light emission efficiency of organic light emitting devices such as organic electroluminescent devices (organic EL devices) are being made actively. In particular, various kinds of efforts have been made for enhancing the light emission efficiency by newly developing and combining an electron transporting material, a hole transporting material, and a light emitting material to constitute an organic electroluminescent device. Among them, there are seen some reports relating to an organic electroluminescent device that utilizes a delayed fluorescent material.

A delayed fluorescent material is a material which, in an excited state, after having undergone reverse intersystem crossing from an excited triplet state to an excited singlet state, emits fluorescence when returning back from the excited singlet state to a ground state thereof. Fluorescence through the route is observed later than fluorescence from the excited singlet state directly occurring from the ground state (ordinary fluorescence), and is therefore referred to as delayed fluorescence. Here, for example, in the case where a light-emitting compound is excited through carrier injection thereinto, the occurring probability of the excited singlet state to the excited triplet state is statistically 25%/75%, and therefore improvement of light emission efficiency by the fluorescence alone from the directly occurring excited singlet state is limited. On the other hand, in a delayed fluorescent material, not only the excited singlet state thereof but also the excited triplet state can be utilized for fluorescent emission through the route via the above-mentioned reverse intersystem crossing, and therefore as compared with an ordinary fluorescent material, a delayed fluorescent material can realize a higher light emission efficiency.

Since such a principle has been clarified, various studies have led to the discovery of various delayed fluorescent materials. Among them, many compounds in which an aromatic ring is substituted with a donor group and an acceptor group are included. For example, a compound having the following skeleton, in which a pyridine ring is substituted with a carbazol-9-yl group as a donor group and a substituted triazinyl group as an acceptor group, has been proposed (see PTL 1).

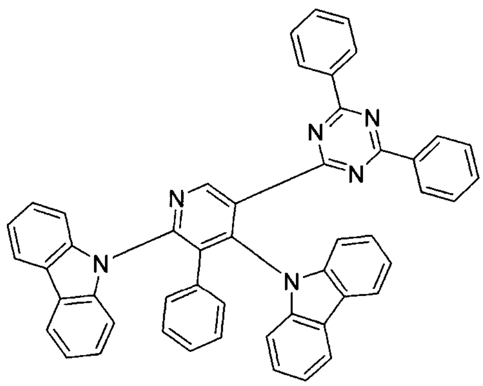

### Citation List

### Patent Literature

PTL 1:WO2021/157600

### Summary of Invention

### Technical Problem

Even when a material emits delayed fluorescence, one having extremely good characteristics and having no problem in practical use has not been provided. For example, PTL 1 describes that the above-mentioned delayed fluorescent material has a short delayed fluorescence lifetime and shows excellent light emission characteristics, but it is

desirable to provide a delayed fluorescent material having an even shorter delayed fluorescence lifetime. Since a long delayed fluorescence lifetime leads to deterioration of the element and a decrease in light emission efficiency at high luminance, in order to implement an organic electroluminescent device having a long lifetime, it is necessary to develop a delayed fluorescent material having a shorter delayed fluorescence lifetime. However, the improvement of delayed fluorescent materials is in the stage of trial and error, and it is not easy to generalize the chemical structure of useful light emitting materials.

Under such circumstances, the present inventors have conducted research for the purpose of providing a compound more useful as a delayed fluorescent material for a light emitting device. Then, the present inventors have conducted intensive studies for the purpose of deriving and generalizing a general formula of a compound more useful as a delayed fluorescent material.

Solution to Problem

As a result of intensive studies for achieving the above object, the present inventors have found that a compound having a structure that satisfies a specific requirement is useful as a light emitting material. The present invention has been proposed based on these findings, and specifically has the following configuration.

[1] A compound represented by the following general formula (1):

General Formula (1)

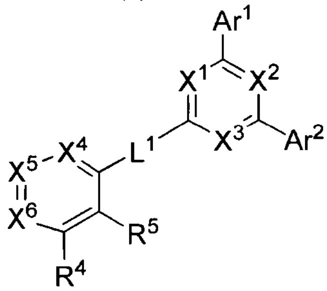

[In the general formula (1), $X^1$ to $X^3$ each independently represent N or C(R), and at least one of $X^1$ to $X^3$ represents N. R represents a hydrogen atom, a deuterium atom or a substituent. $Ar^1$ and $Ar^2$ each independently represent a substituted or unsubstituted aryl group or a substituted or unsubstituted heteroaryl group containing a nitrogen atom as a ring skeleton-constituting atom, and at least one of $Ar^1$ and $Ar^2$ represents a substituted or unsubstituted heteroaryl group bonding via the nitrogen atom. $L^1$ represents a single bond or a divalent linking group. $X^4$ represents N or C($R^1$), $X^5$ represents N or C($R^2$), and $X^6$ represents N or C($R^3$), but only one of $X^4$ to $X^6$ represents N, $R^1$ to $R^5$ each independently represent a hydrogen atom, a deuterium atom, a cyano group, a substituted or unsubstituted alkyl group, a substituted or unsubstituted aryl group, or a donor group. One or more of $R^1$ to $R^5$ represent a donor group, zero to two of $R^1$ to $R^5$ represent a hydrogen atom or a deuterium atom, and zero or one of $R^1$ to $R^5$ represents a substituted or unsubstituted aryl group.]

[2] The compound according to [1], in which $X^5$ represents N.

[3] The compound according to [1], in which $X^6$ represents N.

[4] The compound according to any one of [1] to [3], in which only one of $R^1$ to $R^5$ represents a substituted or unsubstituted aryl group.

[5] The compound according to [4], in which $R^4$ represents a substituted or unsubstituted aryl group.

[6] The compound according to any one of [1] to [5], in which one of $R^1$ to $R^5$ represents a donor group.

[7] The compound according to any one of [1] to [5], in which two of $R^1$ to $R^5$ represent a donor group.

[8] The compound according to any one of [1] to [5], in which three of $R^1$ to $R^5$ represent a donor group.

[9] The compound according to any one of [1] to [8], in which the donor group is a substituted or unsubstituted carbazol-9-yl group.

[10] The compound according to [2] or any one of [4] to [9] citing [2], in which $R^3$ to $R^5$ each independently represent a substituted or unsubstituted aryl group, or a donor group.

[11] The compound according to [3] or any one of [4] to [9] citing [3], in which $R^2$, $R^4$ and $R^5$ each independently represent a substituted or unsubstituted aryl group, or a donor group.

[12] The compound according to any one of [1] to [11], in which $X^1$ to $X^3$ represent N.

[13] The compound according to any one of [1] to [12], in which $Ar^1$ represents a substituted or unsubstituted carbazol-9-yl group, and $Ar^2$ represents a substituted or unsubstituted aryl group.

[14] The compound according to any one of [1] to [12], in which $Ar^1$ and $Ar^2$ each independently represent a substituted

or unsubstituted carbazol-9-yl group.

[15] The compound according to any one of [1] to [14], in which $L^1$ represents a single bond.

[16] The compound according to [2] or any one of [4] to [15] citing [2], in which $R^1$ represents a hydrogen atom.

[17] The compound according to any one of [1] to [16], in which the compound has at least one deuterium atom.

[18] A light emitting material including: the compound according to any one of [1] to [17].

[19] A delayed fluorescent substance including: the compound according to any one of [1] to [17].

[20] A film including: the compound according to any one of [1] to [17].

[21] An organic semiconductor device including: the compound according to any one of [1] to [17].

[22] An organic light emitting device including: the compound according to any one of [1] to [17].

[23] The organic light emitting device according to [22], in which the organic light emitting device includes a layer containing the compound, and the layer also contains a host material.

[24] The organic light emitting device according to [23], in which the layer containing the compound further contains a delayed fluorescent material in addition to the compound and the host material, and the delayed fluorescent material has a lowest excited singlet energy lower than a lowest excited singlet energy of the host material and higher than a lowest excited singlet energy of the compound.

[25] The organic light emitting device according to [23] or [24], in which the device includes a layer containing the compound, and the layer also contains a light emitting material having a structure different from a structure of the compound.

[26] The organic light emitting device according to any one of [23] to [25], in which an amount of light emitted from the compound is largest among the materials contained in the organic light emitting device.

[27] The organic light emitting device according to [25], in which an amount of light emitted from the light emitting material is larger than an amount of light emitted from the compound.

[28] The organic light emitting device according to any one of [22] to [27], which is an organic electroluminescent device.

[29] The organic light emitting device according to any one of [22] to [28], which emits delayed fluorescence.

Advantageous Effects of Invention

The compound of the present invention shows excellent light emission characteristics. The compound of the present invention is useful as a material for an organic light emitting device.

Description of Embodiments

The contents of the present invention will be described in detail below. The constitutional elements may be described below with reference to representative embodiments and specific examples of the present invention, but the present invention is not limited to the embodiments and the specific examples. In the description herein, a numerical range expressed as "to" means a range that includes the numerical values described before and after "to" as the lower limit and the upper limit. A part or all of hydrogen atoms existing in the molecule of the compound for use in the present invention can be substituted with deuterium atoms ($^2H$, deuterium D). In the chemical structural formulae in the description herein, the hydrogen atom is expressed as H, or the expression thereof is omitted. For example, when expression of the atoms bonding to the ring skeleton-constituting carbon atoms of a benzene ring is omitted, H is considered to bond to the ring skeleton-constituting carbon atom at the site having the omitted expression. In the present description, the term "substituent" means an atom or an atomic group except a hydrogen atom and a deuterium atom. Also, the term "substituted or unsubstituted" means that a hydrogen atom can be substituted with a deuterium atom or a substituent.

[Compound Represented by General Formula (1)]

The compound represented by the following general formula (1) is described.

General Formula (1)

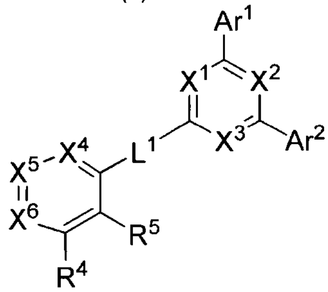

In the general formula (1), $X^1$ to $X^3$ each independently represent N or C(R). At least one of $X^1$ to $X^3$ represents N. R represents a hydrogen atom, a deuterium atom or a substituent. As referred to herein, the substituent can be selected from Substituent Group A, can be selected from Substituent Group B, can be selected from Substituent Group C, can be selected from Substituent Group D, or can be selected from Substituent Group E. In one preferred aspect of the present invention, $X^1$ to $X^3$ represent N. In one aspect of the present invention, $X^1$ and $X^3$ represent N, and $X^2$ represents C(R). In one aspect of the present invention, $X^1$ and $X^2$ represent N, and $X^3$ represents C(R). In one aspect of the present invention, $X^1$ represents N, and $X^2$ and $X^3$ represent C(R). In one aspect of the present invention, $X^2$ represents N, and $X^1$ and $X^3$ represent C(R). In one aspect of the present invention, R represents a hydrogen atom or a deuterium atom. In one aspect of the present invention, R represents an alkyl group optionally substituted with a deuterium atom. In one aspect of the present invention, R represents an aryl group optionally substituted with a deuterium atom, an alkyl group or an aryl group.

In the general formula (1), $X^4$ represents N or $C(R^1)$, $X^5$ represents N or $C(R^2)$, and $X^6$ represents N or $C(R^3)$, and only one of $X^4$ to $X^6$ represents N. In one preferred aspect of the present invention, $X^5$ represents N, $X^4$ represents $C(R^1)$, and $X^6$ represents $C(R^3)$. In another preferred aspect of the present invention, $X^6$ represents N, $X^4$ represents $C(R^1)$, and $X^5$ represents $C(R^2)$. In one aspect of the present invention, $X^4$ represents N, $X^5$ represents $C(R^2)$, and $X^6$ represents $C(R^3)$.

In the general formula (1), $R^1$ to $R^5$ each independently represent a hydrogen atom, a deuterium atom, a cyano group, a substituted or unsubstituted alkyl group, a substituted or unsubstituted aryl group, or a donor group. $Ar^1$ and $Ar^2$ each independently represent a substituted or unsubstituted aryl group, or a substituted or unsubstituted heteroaryl group containing a nitrogen atom as a ring skeleton-constituting atom.

The alkyl group that $R^1$ to $R^5$ can take can be linear, branched or cyclic. Two or more of a linear moiety, a cyclic moiety and a branched moiety can be in the group as mixed. The carbon number of the alkyl group can be, for example, 1 or more, 2 or more, or 4 or more. The carbon number can also be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, and a cycloheptyl group. The alkyl group which is the substituent can be further substituted with, for example, a deuterium atom, an aryl group, an alkoxy group, an aryloxy group, and a halogen atom. In one aspect of the present invention, the substituent for the alkyl group is one or more selected from the group consisting of an aryl group and a deuterium atom. In one preferred aspect of the present invention, the alkyl group is unsubstituted, and, for example, can be selected from the group consisting of a methyl group, an ethyl group, an isopropyl group and a tert-butyl group.

The aryl group that $R^1$ to $R^5$ and $Ar^1$ and $Ar^2$ can take each can be a monocyclic ring or can be a fused ring of two or more kinds of rings. In the case of a fused ring, the number of fused rings is preferably 2 to 6, and can be selected from, for example, 2 to 4. Specific examples of the ring include a benzene ring, a naphthalene ring, an anthracene ring, a phenanthrene ring, and a triphenylene ring. In one aspect of the present invention, the aryl group is a substituted or unsubstituted phenyl group, a substituted or unsubstituted naphthalen-1-yl group, or a substituted or unsubstituted naphthalen-2-yl group, and is preferably a substituted or unsubstituted phenyl group. For example, the substituent for the aryl group can be selected from Substituent Group A, can be selected from Substituent Group B, can be selected from Substituent Group C, can be selected from Substituent Group D, or can be selected from Substituent Group E. In one aspect of the present invention, the substituent for the aryl group is one or more selected from the group consisting of an alkyl group, an aryl group and a deuterium atom. In one preferred aspect of the present invention, the aryl group is substituted with at least one deuterium atom. In one aspect of the present invention, the aryl group is unsubstituted.

Specific examples of the substituted or unsubstituted aryl group that $R^1$ to $R^5$ and $Ar^1$ and $Ar^2$ can take are shown below. The aryl group which can be employed in the present invention should not be limitatively interpreted by the following specific examples. In the following specific examples, * indicates a bonding site. A methyl group is not shown. Consequently, Ar2 to Ar7 represent structures substituted with a methyl group.

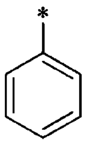

Ar1

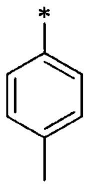

Ar2

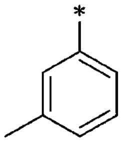

Ar3

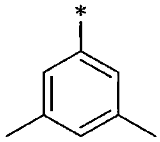

Ar4

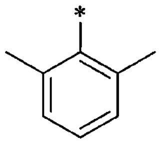

Ar5

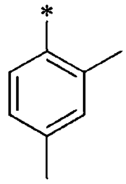

Ar6

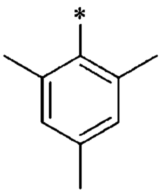

Ar7

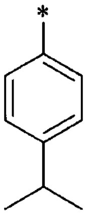

Ar8

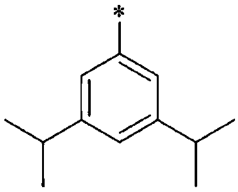

Ar9

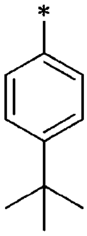

Ar10

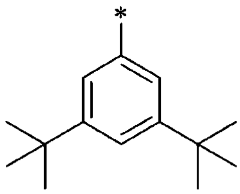

Ar11

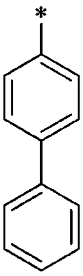

Ar12

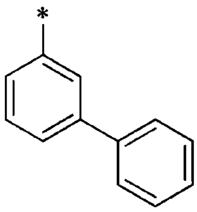

Ar13

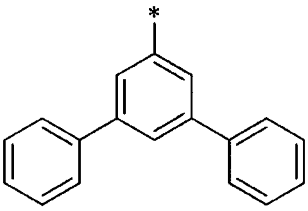

Ar14

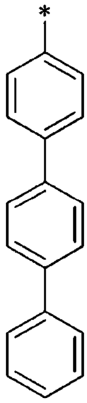

Ar15

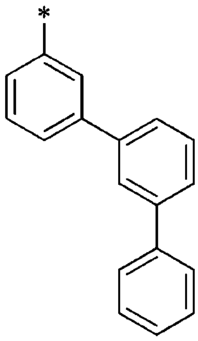

Ar16

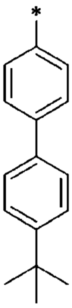

Ar17

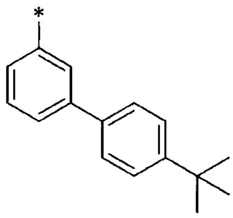

Ar18

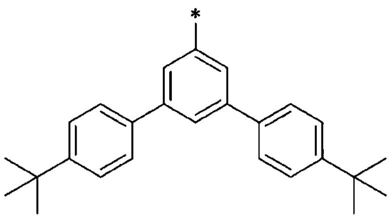

Ar19

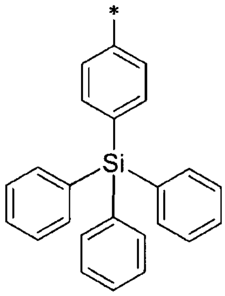

Ar20

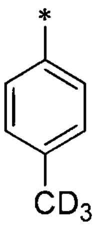

Ar21

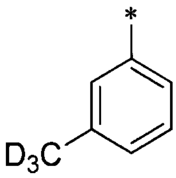

Ar22

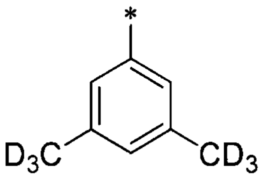

Ar23

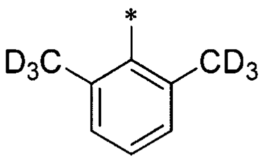

Ar24

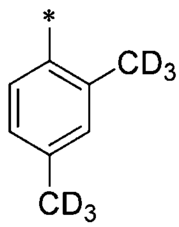

Ar25

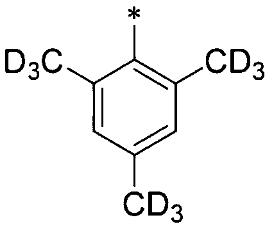

Ar26

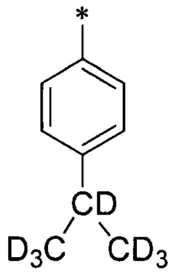

Ar27

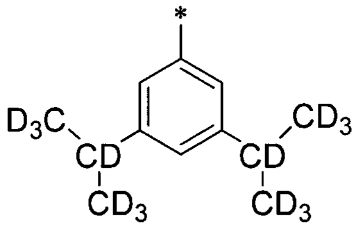

Ar28

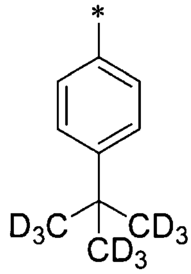

Ar29

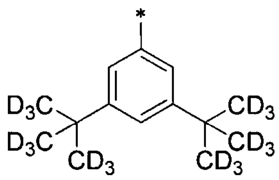

Ar30

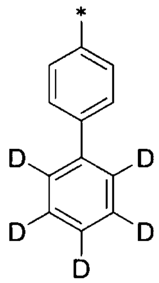

Ar31

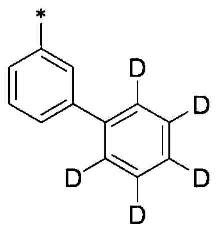

Ar32

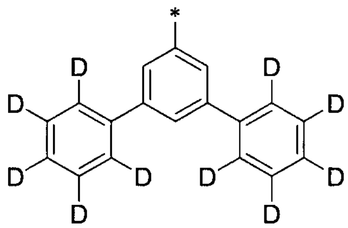

Ar33

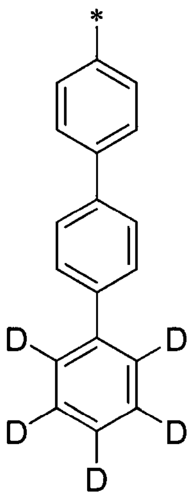

Ar34

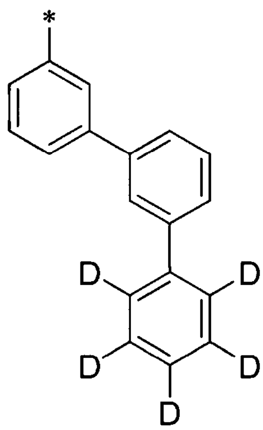

Ar35

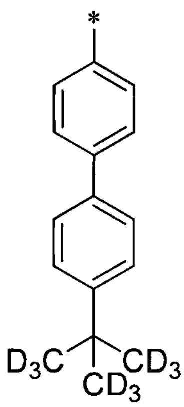

Ar36

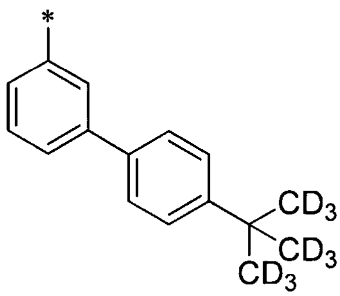

Ar37

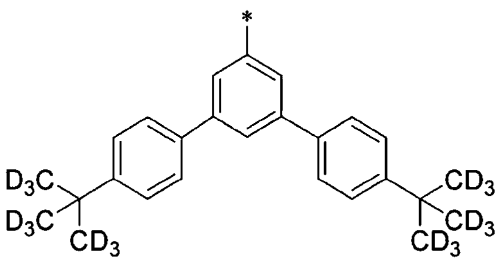

Ar38

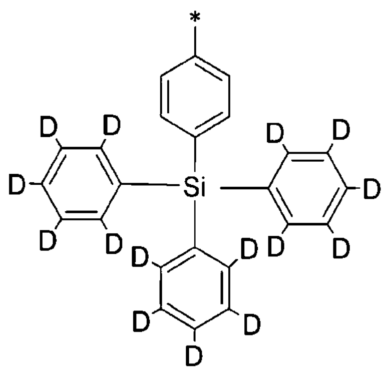

Ar39

In addition to the above-mentioned specific examples, groups obtained by substituting all hydrogen atoms present in Ar1 to Ar20 with deuterium atoms are disclosed as Ar40 to Ar59, respectively.

In one aspect of the present invention, the aryl group that $R^1$ to $R^5$ can take is Ar1 or Ar40. In one aspect of the

present invention, the aryl group that $R^1$ to $R^5$ can take is selected from the group consisting of Ar2 to Ar11, Ar21 to Ar30 and Ar41 to Ar50. In one aspect of the present invention, the aryl group that $R^1$ to $R^5$ can take is selected from the group consisting of Ar12 to Ar16, Ar31 to Ar35 and Ar51 to Ar55. In one aspect of the present invention, the aryl group that $R^1$ to $R^5$ can take is selected from the group consisting of Ar1, Ar12 to Ar16, Ar40, Ar31 to Ar35 and Ar51 to Ar55. In one aspect of the present invention, the aryl group that $R^1$ to $R^5$ can take is selected from the group consisting of Ar21 to Ar59.

In one aspect of the present invention, the aryl group that $Ar^1$ and $Ar^2$ can take is Ar1 or Ar40. In one aspect of the present invention, the aryl group that $Ar^1$ and $Ar^2$ can take is selected from the group consisting of Ar2 to Ar11, Ar21 to Ar30 and Ar41 to Ar50. In one aspect of the present invention, the aryl group that $Ar^1$ and $Ar^2$ can take is selected from the group consisting of Ar12 to Arl6, Ar31 to Ar35 and Ar51 to Ar55. In one aspect of the present invention, the aryl group that $Ar^1$ and $Ar^2$ can take is selected from the group consisting of Ar1, Ar12 to Ar16, Ar40, Ar31 to Ar35 and Ar51 to Ar55. In one aspect of the present invention, the aryl group that $Ar^1$ and $Ar^2$ can take is selected from the group consisting of Ar21 to Ar59.

At least one of $R^1$ to $R^5$ in the general formula (1) represents a donor group. The donor group that $R^1$ to $R^5$ can take does not include a substituted or unsubstituted aryl group.

The "donor group" can be selected from groups having a negative Hammett's σp value. The Hammett's σp value is proposed by L. P. Hammett and quantifies the influence of a substituent on the reaction rate or equilibrium of a para-substituted benzene derivative. Specifically, the value is a constant (σp) specific to the substituent in the following formula that is established between substituents and reaction rate constants or equilibrium constants in para-substituted benzene derivatives:

$$\log(k/k_0) = \rho\sigma p$$

or

$$\log(K/K_0) = \rho\sigma p.$$

. In the above equations, $k_0$ represents a rate constant of a benzene derivative not having a substituent; k represents a rate constant of a benzene derivative substituted with a substituent; $K_0$ represents an equilibrium constant of a benzene derivative not having a substituent; K represents an equilibrium constant of a benzene derivative substituted with a substituent; and ρ represents a reaction constant to be determined by the kind and the condition of reaction. Regarding the description relating to the "Hammett's σp value" and the numerical value of each substituent in the present invention, reference can be made to the description relating to σp value in Hansch, C. et. al., Chem. Rev., 91, 165-195 (1991).

The donor group that $R^1$ to $R^5$ can take preferably has σp of -0.3 or less, more preferably -0.5 or less, and still more preferably -0.7 or less. For example, the value can be selected from a range of -0.9 or less, or from a range of -1.1 or less.

The donor group in the present invention is preferably a group containing a substituted amino group. The donor group can be a substituted amino group, or can be a substituted amino group-bonded aryl group, especially a substituted amino group-bonded phenyl group. In one preferred aspect of the present invention, the donor group is a substituted amino group.

The substituent bonding to the nitrogen atom of a substituted amino group is preferably a substituted or unsubstituted alkyl group, a substituted or unsubstituted alkenyl group, a substituted or unsubstituted aryl group, or a substituted or unsubstituted heteroaryl group, and more preferably a substituted or unsubstituted aryl group or a substituted or unsubstituted heteroaryl group. Particularly, the substituted amino group is preferably a substituted or unsubstituted diarylamino group, or a substituted or unsubstituted diheteroarylamino group. As referred to herein, the two aryl groups constituting the diarylamino group can bond to each other, and the two heteroaryl groups constituting the diheteroarylamino group can bond to each other.

The donor group that $R^1$ to $R^5$ can take is preferably a group represented by the following general formula (a).

General Formula (a)

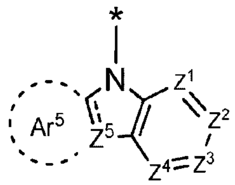

In the general formula (a), $Z^1$ represents C-$R^{14}$ or N, $Z^2$ represents C-$R^{15}$ or N, $Z^3$ represents C-$R^{16}$ or N, and $Z^4$ represents C-$R^{17}$ or N. $Z^5$ represents C or N, $Ar^5$ represents a substituted or unsubstituted aromatic ring or a substituted or

unsubstituted heteroaromatic ring. $R^{14}$ and $R^{15}$, $R^{15}$ and $R^{16}$, and $R^{16}$ and $R^{17}$ each can bond to each other to form a cyclic structure.

Among $Z^1$ to $Z^4$, the number of groups represented by N is preferably 0 to 3, and preferably 0 to 2. In one aspect of the present invention, among $Z^1$ to $Z^4$, the number of groups represented by N is 1. In one aspect of the present invention, among $Z^1$ to $Z^4$, the number of groups represented by N is 0.

$R^{14}$ to $R^{17}$ each independently represent a hydrogen atom, a deuterium atom, or a substituent.

For example, the substituent can be selected from Substituent Group A, can be selected from Substituent Group B, can be selected from Substituent Group C, can be selected from Substituent Group D, or can be selected from Substituent Group E. When two or more of $R^{14}$ to $R^{17}$ represent substituents, the two or more substituents can be the same or different. Zero to two of $R^{14}$ to $R^{17}$ preferably represent a substituent, and for example, one can be a substituent, or zero can be a substituent ($R^{14}$ to $R^{17}$ represents a hydrogen atom or a deuterium atom).

$R^{14}$ and $R^{15}$, $R^{15}$ and $R^{16}$, and $R^{16}$ and $R^{17}$ each can bond to each other to form a cyclic structure. The cyclic structure can be any of an aromatic ring, an heteroaromatic ring, an aliphatic hydrocarbon ring, and an aliphatic heterocyclic ring, and can be a ring obtained by fusing these rings. The structure is preferably an aromatic ring or a heteroaromatic ring. Examples of the aromatic ring include a substituted or unsubstituted benzene ring. Another benzene ring can be further fused to the benzene ring, and a heterocyclic ring such as a pyridine ring can be fused to the benzene ring. The heteroaromatic ring means a ring exhibiting aromaticity including a heteroatom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole ring can be employed as the heteroaromatic ring. In one preferred aspect of the present invention, the cyclic structure is a furan ring of a substituted or unsubstituted benzofuran, a thiophene ring of a substituted or unsubstituted benzothiophene, or a pyrrole ring of a substituted or unsubstituted indole. The benzofuran, benzothiophene, and indole referred to herein can be unsubstituted, can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. It is preferable that a substituted or unsubstituted aryl group bonds to the nitrogen atom constituting the pyrrole ring of indole, and examples of the substituent include a substituent selected from any of Substituent Group A to Substituent Group E. The cyclic structure can be a substituted or unsubstituted cyclopentadiene ring. In one aspect of the present invention, a pair of $R^{14}$ and $R^{15}$, $R^{15}$ and $R^{16}$, and $R^{16}$ and $R^{17}$ bonds to each other to form a cyclic structure. In one aspect of the present invention, none of $R^{14}$ and $R^{15}$, $R^{15}$ and $R^{16}$, and $R^{16}$ and $R^{17}$ bonds to each other to form a cyclic structure.

In the general formula (a), $Z^5$ represents C or N, $Ar^5$ represents a substituted or unsubstituted aromatic ring or a substituted or unsubstituted heteroaromatic ring. In one aspect of the present invention, $Z^5$ represents C, and $Ar^5$ represents a substituted or unsubstituted aromatic ring or a substituted or unsubstituted heteroaromatic ring. In one aspect of the present invention, $Z^5$ represents N, and $Ar^5$ represents a substituted or unsubstituted heteroaromatic ring.

Examples of the aromatic ring that $Ar^5$ can take include a benzene ring. Another benzene ring can be further fused to the benzene ring, and a heterocyclic ring such as a pyridine ring can be fused to the benzene ring. The heteroaromatic ring that $Ar^5$ can take is preferably a 5- to 7-membered ring, and for example, a 5-membered ring or a 6-membered ring can be employed. In one aspect of the present invention, as the heteroaromatic ring, a furan ring, a thiophene ring, a pyrrole ring, an imidazole ring, a pyridine ring, a pyridazine ring, a pyrimidine ring, or a pyrazine ring can be employed. In one aspect of the present invention, $Z^5$ represents C, and the heteroaromatic ring is a furan ring of a substituted or unsubstituted benzofuran, a thiophene ring of a substituted or unsubstituted benzothiophene, a pyridine ring of a substituted or unsubstituted quinoline, or a pyridine ring of a substituted or unsubstituted isoquinoline. In one aspect of the present invention, $Z^5$ represents N, and the heteroaromatic ring is a pyrrole ring of a substituted or unsubstituted indole, or an imidazole ring of a substituted or unsubstituted benzimidazole. The benzofuran, benzothiophene, quinoline, isoquinoline, indole and benzimidazole referred to herein can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E.

When $Z^5$ in the general formula (a) represents C, a group represented by the following general formula (b) is preferred.

General Formula (b)

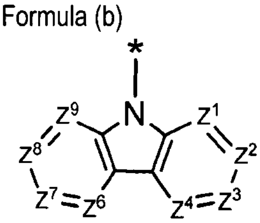

In the general formula (b), $Z^1$ represents C-$R^{14}$ or N, $Z^2$ represents C-$R^{15}$ or N, $Z^3$ represents C-$R^{16}$ or N, $Z^4$ represents C-$R^{17}$ or N, $Z^6$ represents C-$R^{18}$ or N, $Z^7$ represents C-$R^{19}$ or N, $Z^8$ represents C-$R^{20}$ or N, and $Z^9$ represents C-$R^{21}$ or N. $R^{14}$ and $R^{15}$, $R^{15}$ and $R^{16}$, $R^{16}$ and $R^{17}$, $R^{13}$ and $R^{19}$, $R^{19}$ and $R^{20}$, and $R^{20}$ and $R^{21}$ each can bond to each other to form a cyclic structure.

For $Z^1$ to $Z^4$ and $R^{14}$ to $R^{17}$ in the general formula (b), the corresponding description of the general formula (a) can be referred to. $Z^6$ to $Z^9$ and $R^{18}$ to $R^{21}$ in the general formula (b) correspond to $Z^1$ to $Z^4$ and $R^{14}$ to $R^{17}$ in the general formula (a), respectively, and for the contents thereof, reference can be made to the descriptions of $Z^1$ to $Z^4$ and $R^{14}$ to $R^{17}$ in the general formula (a).

In one aspect of the present invention, among $Z^1$ to $Z^4$ and $Z^6$ to $Z^9$, the number of groups represented by N is preferably 0 to 2, and more preferably 0 or 1. In one aspect of the present invention, among $Z^1$ to $Z^4$ and $Z^6$ to $Z^9$, the number of groups represented by N is 1. In one preferred aspect of the present invention, among $Z^1$ to $Z^4$ and $Z^6$ to $Z^9$, the number of groups represented by N is 0. When the number is 0, the formula represents a substituted or unsubstituted carbazol-9-yl group.

The donor group that $R^1$ to $R^5$ can take is preferably a substituted or unsubstituted carbazol-9-yl group. The carbazol-9-yl group referred to herein can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. Further one or more rings can be fused to the two benzene rings constituting the carbazol-9-yl group. In one preferred aspect of the present invention, the donor group that $R^1$ to $R^5$ can take is a carbazol-9-yl group optionally substituted with a group selected from Substituent Group E, and optionally fused with one or more rings. In the case where the carbazol-9-yl group not fused with a ring is substituted, the substitution site is not specifically limited, but is preferably at least one of 2 to 7-positions, more preferably at least one of 3 to 6-positions, and still more preferably a 3-position and a 6-position.

In one aspect of the present invention, the donor group that $R^1$ to $R^5$ can take is a carbazol-9-yl group fused with one or more rings, and hereinafter, this will be referred to as a "ring-fused carbazol-9-yl group". The ring-fused carbazol-9-yl group that $R^1$ to $R^5$ can take can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. Preferably, the group is unsubstituted, or substituted with a substituent selected from Substituent Group E. In one aspect of the present invention, the ring-fused carbazol-9-yl group is unsubstituted. In one preferred aspect of the present invention, the ring-fused carbazol-9-yl group is substituted with an aryl group optionally substituted with one atom or group selected from the group consisting of a deuterium atom, an alkyl group and an aryl group or with a group formed by combining two or more thereof.

The number of rings constituting the fused ring in the ring-fused carbazol-9-yl group is 4 or more, preferably 5 or more, more preferably 5 to 9, and still more preferably 5 to 7. In one preferred aspect of the present invention, the number of rings constituting the fused ring is 5. Here, the number of rings includes the number of rings of carbazole to be fused (i.e. 3).

The ring-fused carbazol-9-yl group is a group that bonds via the nitrogen atom constituting the ring skeleton of carbazole, and has a structure in which a ring is fused to at least one of the two benzene rings constituting carbazole. The fused ring can be any of an aromatic hydrocarbon ring, an aromatic heterocyclic ring, an aliphatic hydrocarbon ring, and an aliphatic heterocyclic ring, and can be a ring obtained by further fusing these rings. An aromatic hydrocarbon ring and an aromatic heterocyclic ring are preferable. Examples of the aromatic hydrocarbon ring include a substituted or unsubstituted benzene ring. Another benzene ring can be further fused to the benzene ring, and a heterocyclic ring such as a pyridine ring can be fused to the benzene ring. The aromatic heterocyclic ring means a ring exhibiting aromaticity including a heteroatom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole ring can be employed as the aromatic heterocyclic ring. In one aspect of the present invention, the fused ring is a furan ring of a substituted or unsubstituted benzofuran, a thiophene ring of a substituted or unsubstituted benzothiophene, or a pyrrole ring of a substituted or unsubstituted indole. It is preferable that a substituent selected from Substituent Group E (but except for a deuterium atom alone) bonds to the nitrogen atom of the pyrrole ring, and it is more preferable that an aryl group which can be substituted with an alkyl group or an aryl group is bonded. In the present

invention, it is preferable to employ a carbazol-9-yl group in which a ring having one or more atoms selected from the group consisting of an oxygen atom, a sulfur atom, and a nitrogen atom as a ring skeleton-constituting atom is fused. Above all, preferably employed are a benzofuro structure-fused carbazol-9-yl group, a benzothieno structure-fused carbazol-9-yl group, and an indole structure-fused carbazol-9-yl group. In one aspect of the present invention, the compound has at least one benzofuro structure-fused carbazol-9-yl group, and for example, has two or more such groups. In one aspect of the present invention, the compound has at least one benzothieno structure-fused carbazol-9-yl group, and for example, has two or more such groups.

The ring-fused carbazol-9-yl group employable herein includes a benzofuro[2,3-a]carbazol-9-yl group, a benzofuro[3,2-a]carbazol-9-yl group, a benzofuro[2,3-b]carbazol-9-yl group, a benzofuro[3,2-b]carbazol-9-yl group, a benzofuro[2,3-c]carbazol-9-yl group, and a benzofuro[3,2-c]carbazol-9-yl group. In addition, the ring-fused carbazol-9-yl group also employable herein includes a benzothieno[2,3-a]carbazol-9-yl group, a benzothieno[3,2-a]carbazol-9-yl group, a benzothieno[2,3-b]carbazol-9-yl group, a benzothieno[3,2-b]carbazol-9-yl group, a benzothieno[2,3-c]carbazol-9-yl group, and a benzothieno[3,2-c]carbazol-9-yl group. The ring-fused carbazol-9-yl group also employable herein includes an indolo[2,3-a]carbazol-9-yl group, an indolo[3,2-a]carbazol-9-yl group, an indolo[2,3-b]carbazol-9-yl group, an indolo[3,2-b]carbazol-9-yl group, an indolo[2,3-c]carbazol-9-yl group, and an indolo[3,2-c]carbazol-9-yl group.

The number of the substituents substituted on the ring-fused carbazol-9-yl group is preferably 1 to 10, more preferably 1 to 6, still more preferably 1 to 4, and can be, for example 1, or can be, for example 2. In one preferred aspect of the present invention, any of the 3-position or the 6-position of the ring-fused carbazol-9-yl group is substituted. In one preferred aspect of the present invention, the compound has at least one substituent on the para-position of the benzene ring viewed from the heteroatom present in the ring-fused carbazol-9-yl group. In one preferred aspect of the present invention, the compound has at least one substituent only on the para-position of the benzene ring viewed from the heteroatom present in the ring-fused carbazol-9-yl group. In one preferred aspect of the present invention, the compound has substituents on all the substitutable para-positions of the benzene ring viewed from the heteroatom present in the ring-fused carbazol-9-yl group.

Specific examples of the donor group that $R^1$ to $R^5$ in the general formula (1) can take are shown below. The donor group which can be employed in the present invention shall not be construed as being limited by the following specific examples. In the following specific examples, Ph indicates a phenyl group ($C_6H_5$), and * indicates a bonding site. A methyl group is not shown, and for example, D2 has one methyl group. A deuterated methyl group is expressed as $CD_3$. $C_6D_5$ represents a phenyl group in which all hydrogen atoms are deuterated. D represents a deuterium atom.

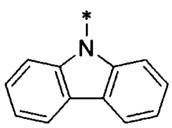 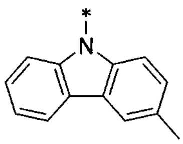 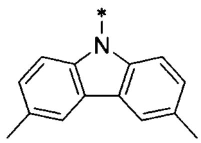 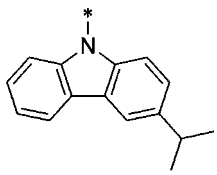 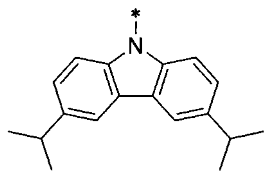

D1 D2 D3 D4 D5

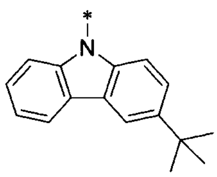 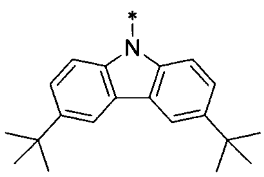 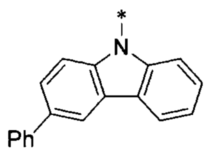 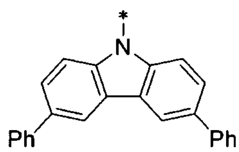

D6 D7 D8 D9

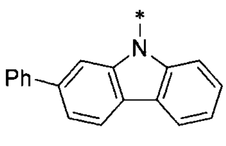 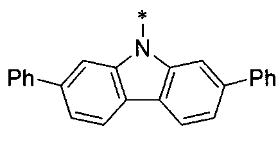 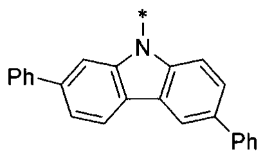 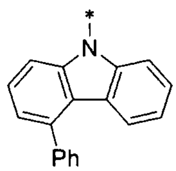

D10 D11 D12 D13

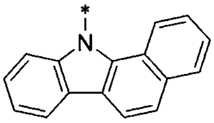
D14

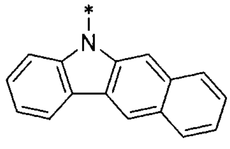
D15

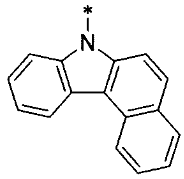
D16

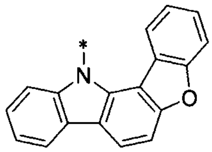
D17

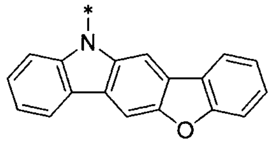
D18

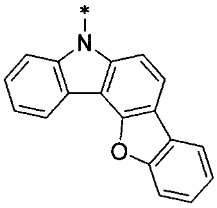
D19

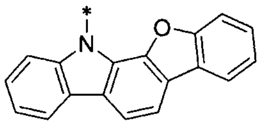
D20

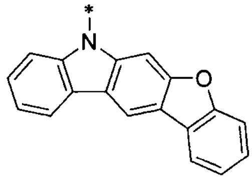
D21

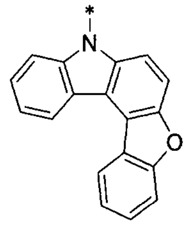
D22

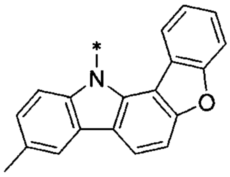
D23

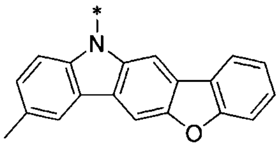
D24

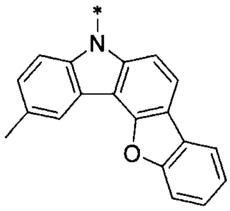
D25

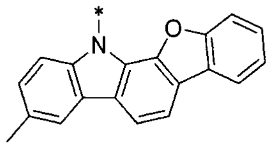
D26

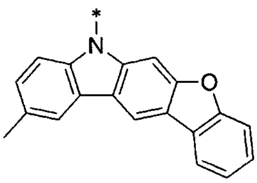
D27

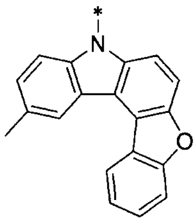
D28

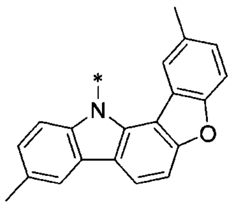
D29

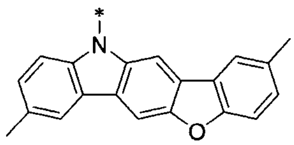
D30

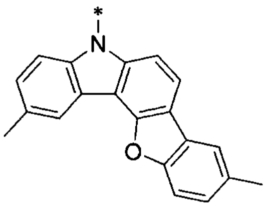
D31

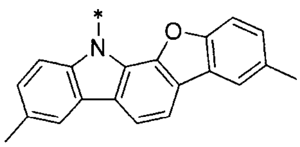
D32

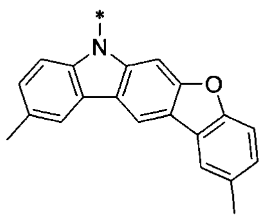
D33

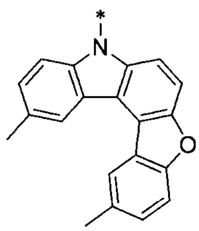
D34

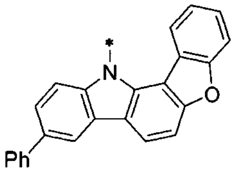

D35

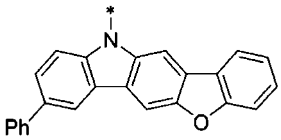

D36

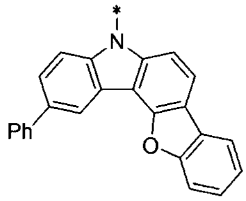

D37

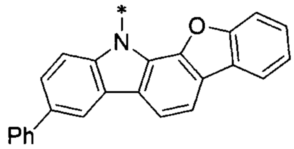

D38

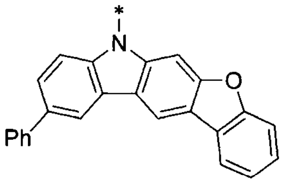

D39

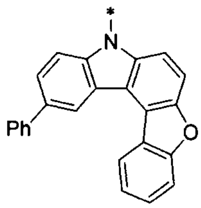

D40

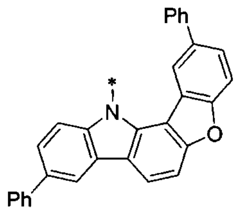

D41

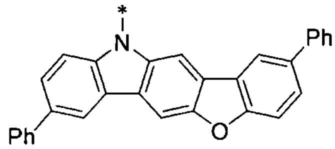

D42

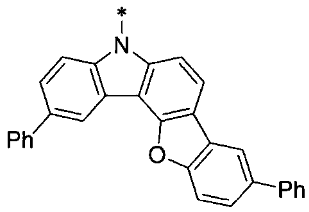

D43

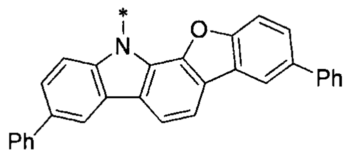

D44

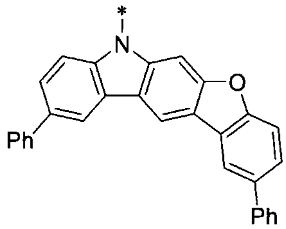

D45

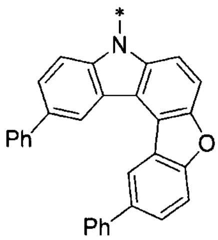

D46

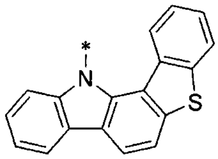

D47

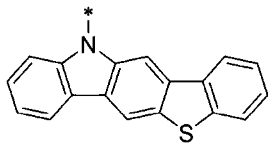

D48

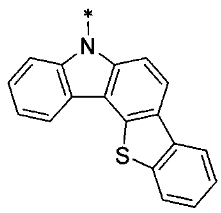

D49

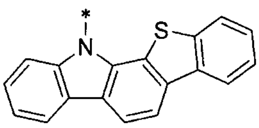

D50

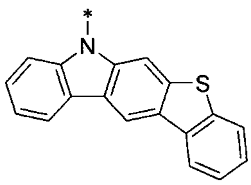

D51

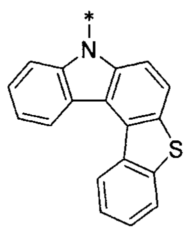

D52

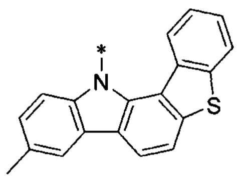

D53

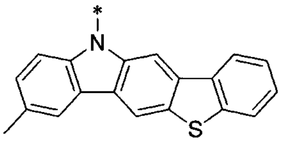
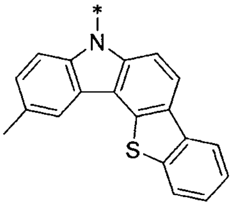
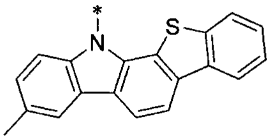
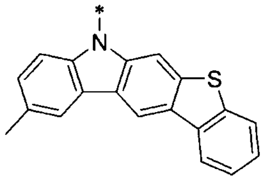

D54 D55 D56 D57

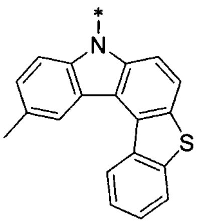
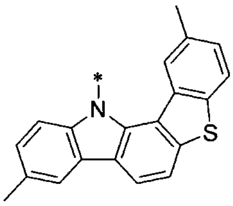
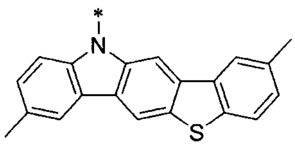
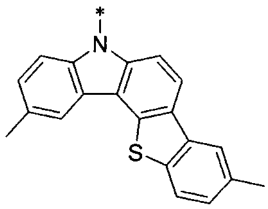

D58 D59 D60 D61

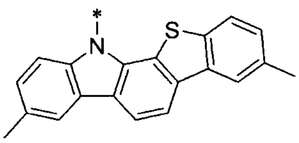
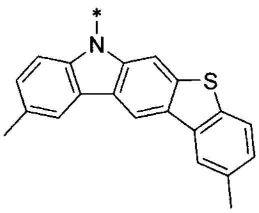
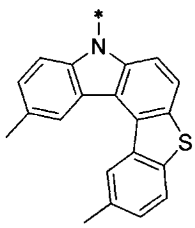
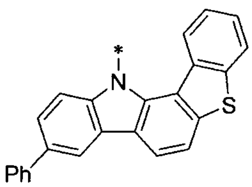

D62 D63 D64 D65

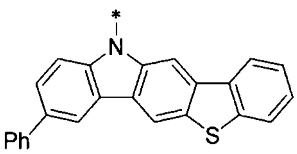
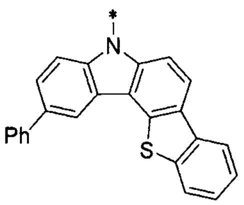
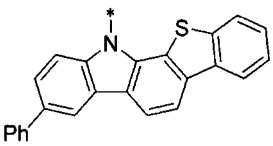
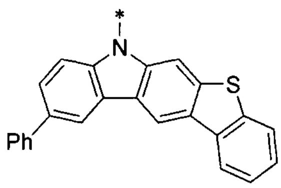

D66 D67 D68 D69

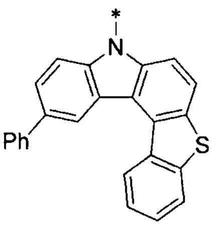
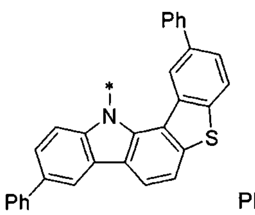
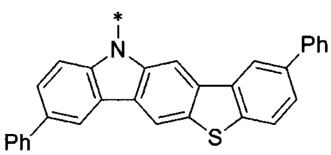
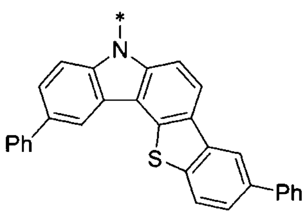

D70 D71 D72 D73

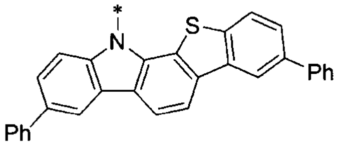

D74

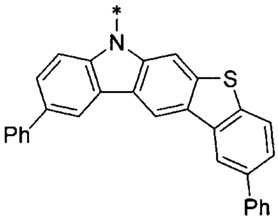

D75

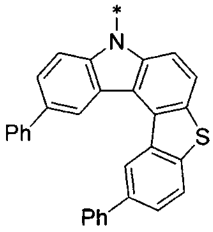

D76

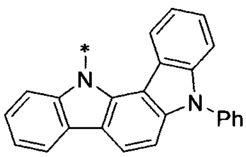

D77

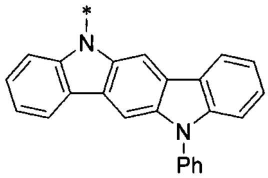

D78

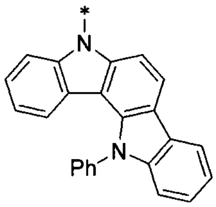

D79

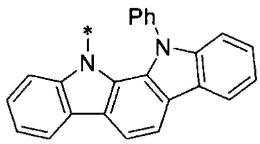

D80

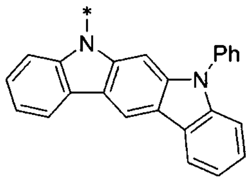

D81

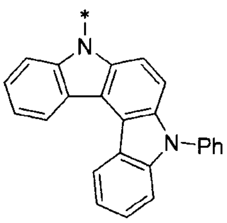

D82

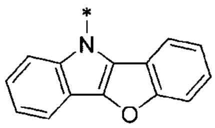

D83

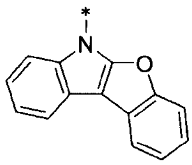

D84

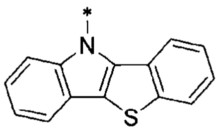

D85

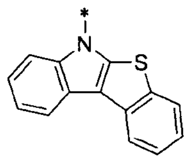

D86

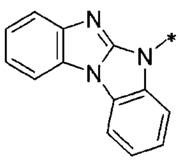

D87

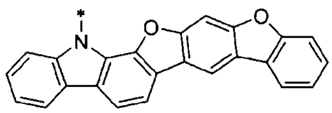

D88

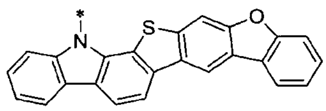

D89

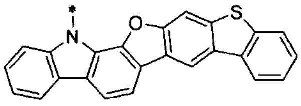

D90

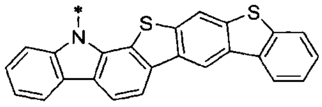

D91

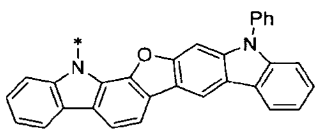

D92

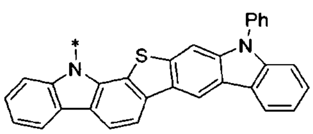

D93

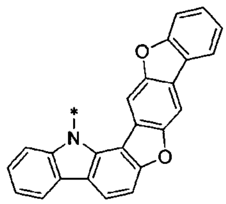

D94

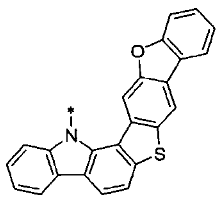

D95

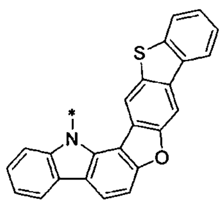

D96

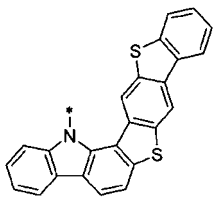

D97

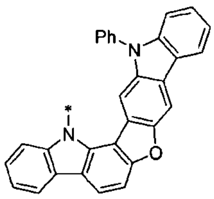

D98

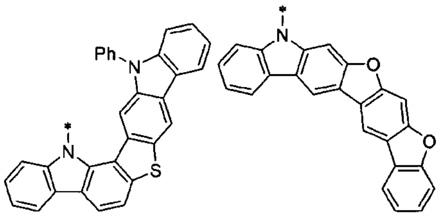

D99

D100

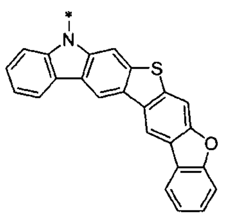

D101

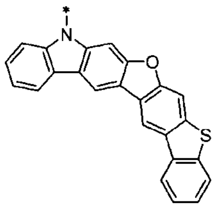

D102

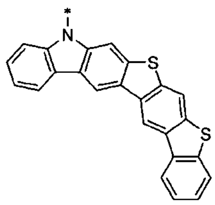

D103

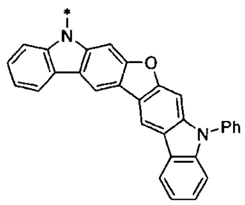

D104

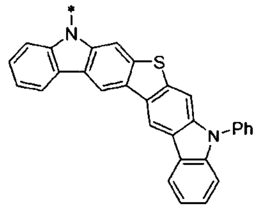

D105

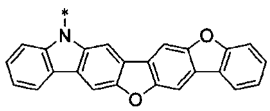

D106

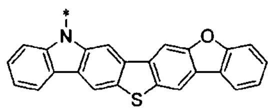

D107

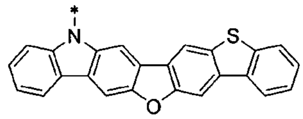

D108

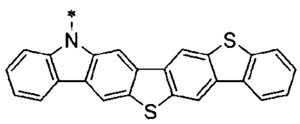

D109

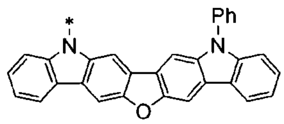

D110

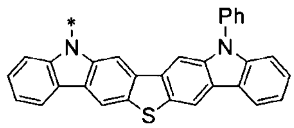

D111

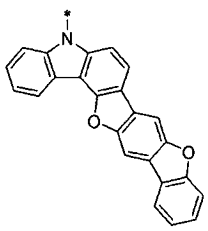

D112

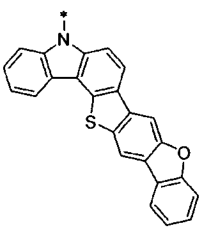

D113

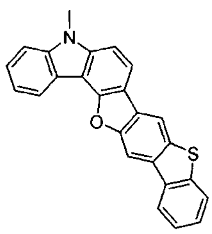

D114

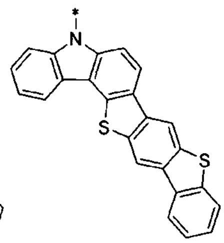

D115

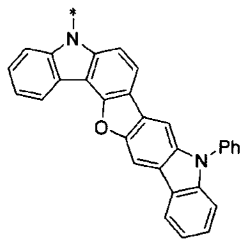

D116

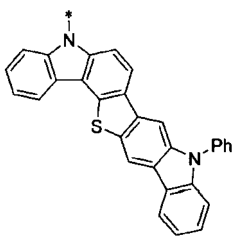

D117

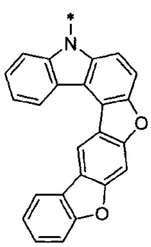

D118

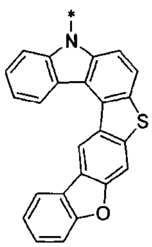

D119

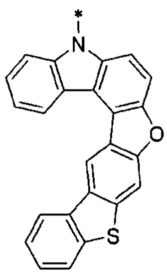

D120

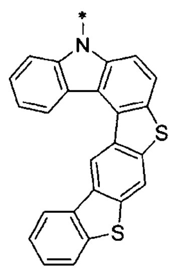

D121

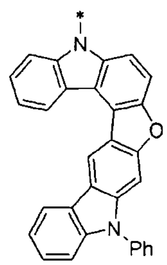

D122

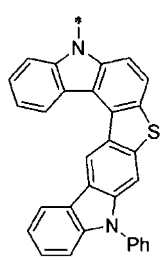
D123

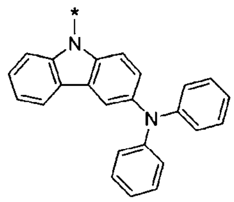
D124

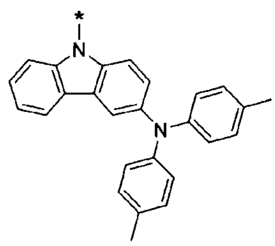
D125

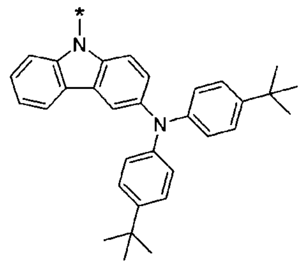
D126

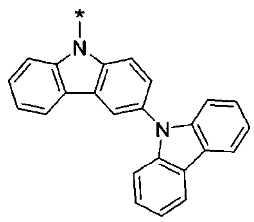
D127

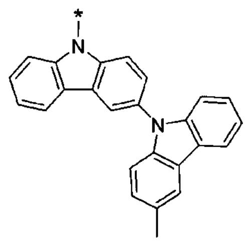
D128

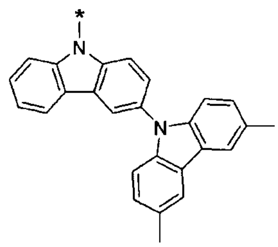
D129

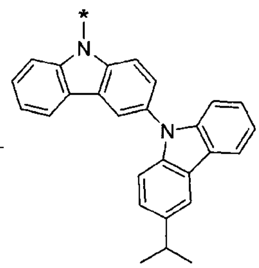
D130

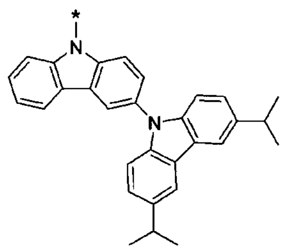
D131

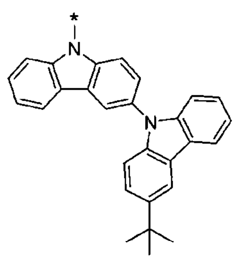
D132

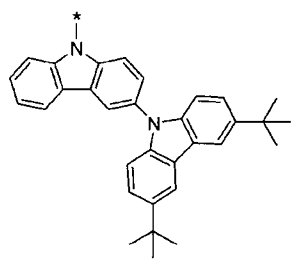
D133

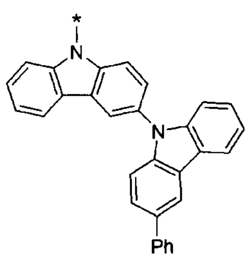
D134

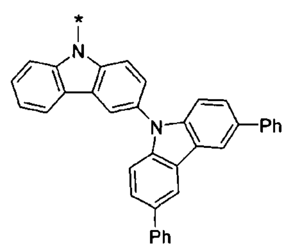
D135

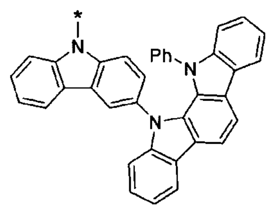
D136

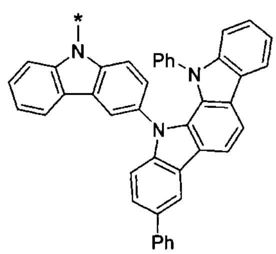
D137

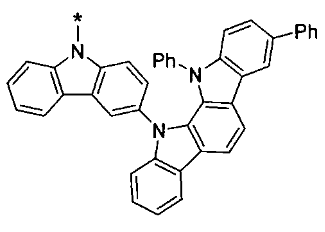
D138

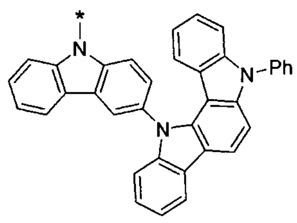
D139

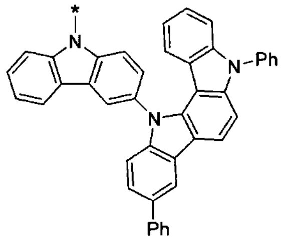

D140

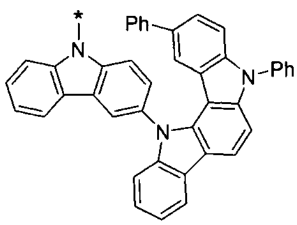

D141

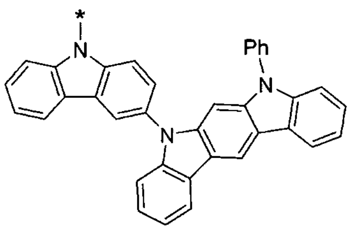

D142

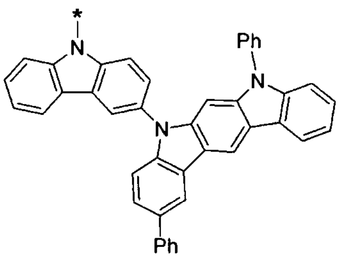

D143

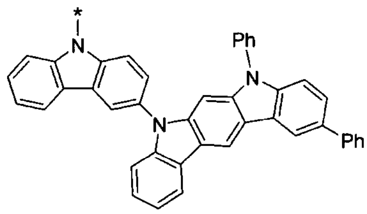

D144

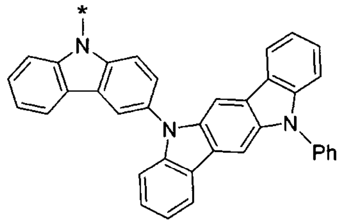

D145

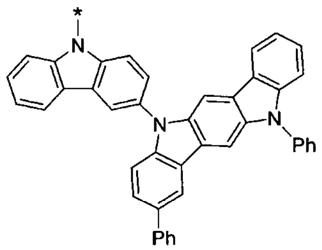

D146

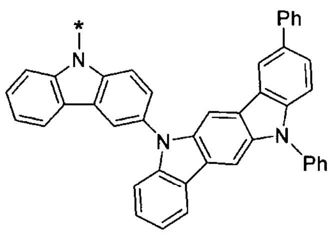

D147

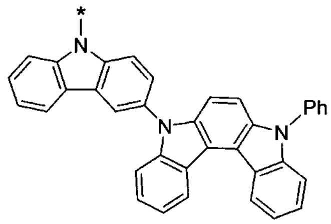

D148

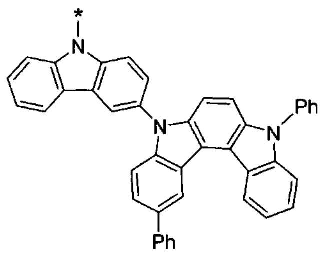

D149

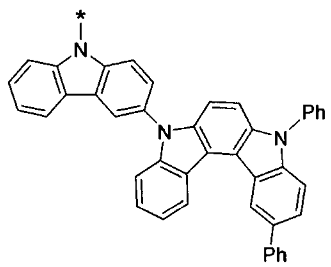

D150

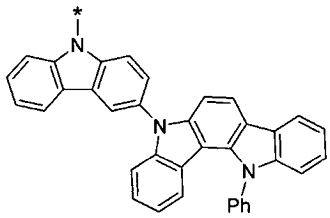

D151

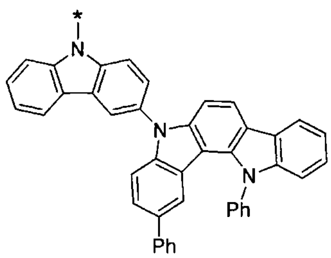

D152

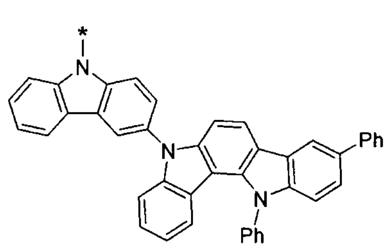

D153

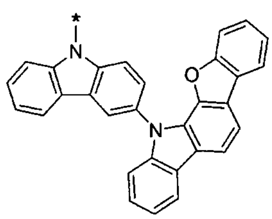

D154

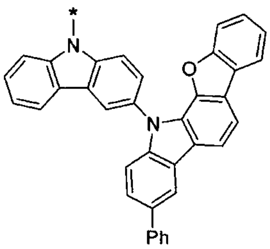

D155

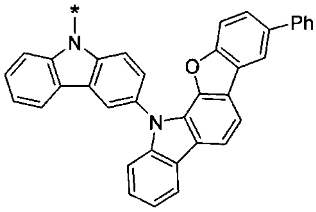

D156

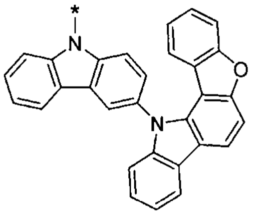

D157

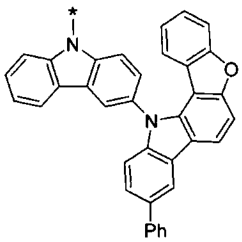

D158

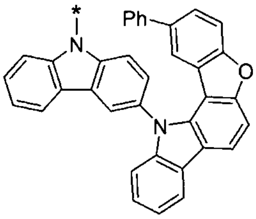

D159

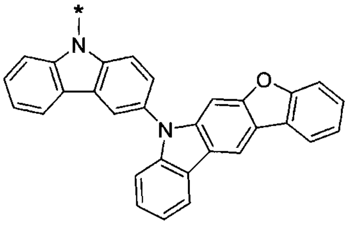

D160

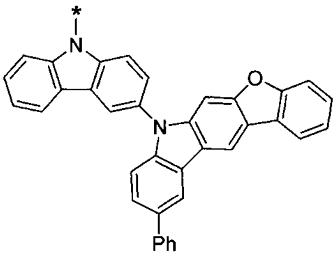

D161

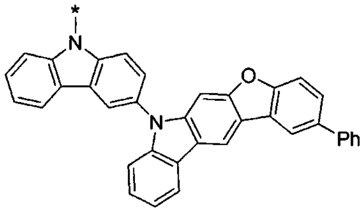

D162

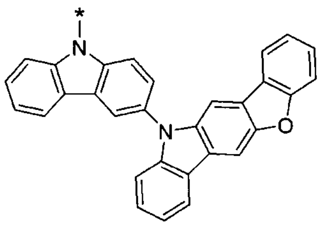

D163

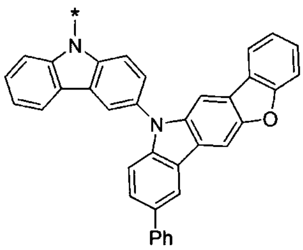

D164

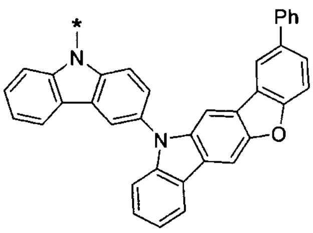

D165

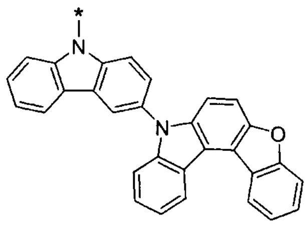

D166

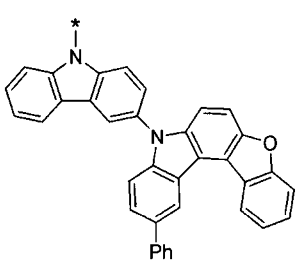

D167

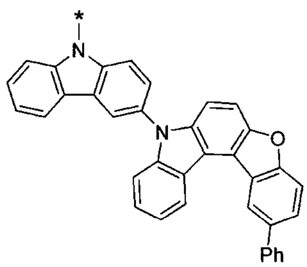

D168

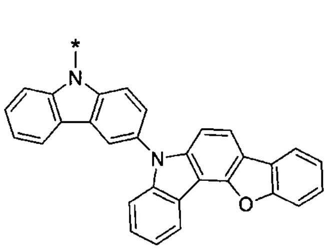

D169

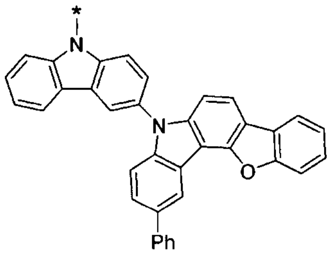

D170

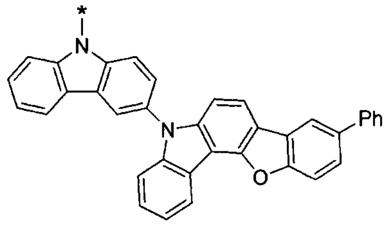

D171

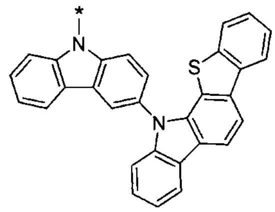

D172

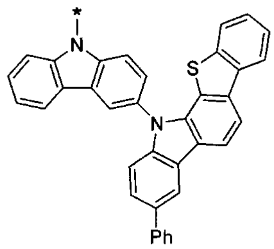

D173

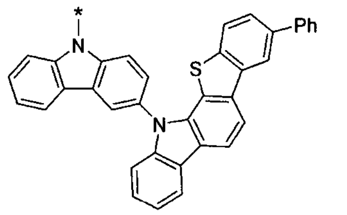

D174

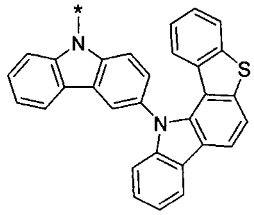

D175

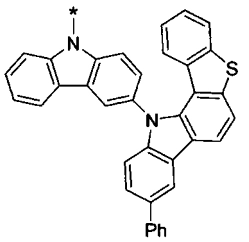

D176

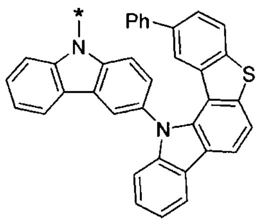

D177

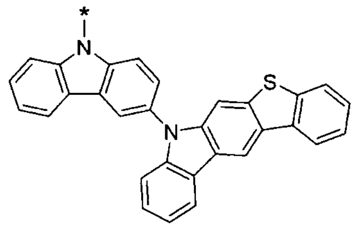

D178

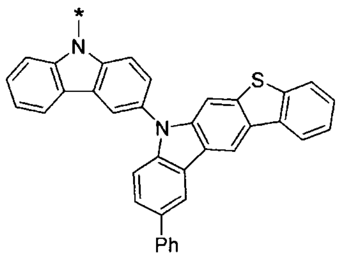

D179

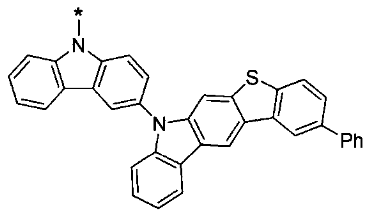

D180

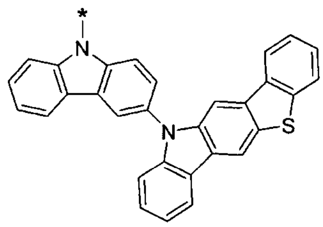

D181

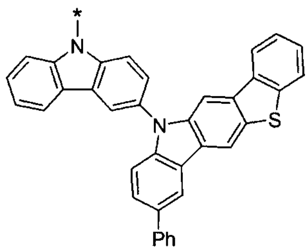

D182

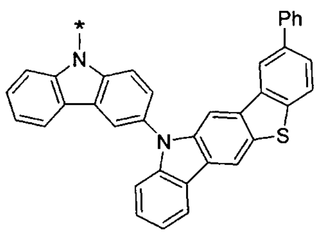

D183

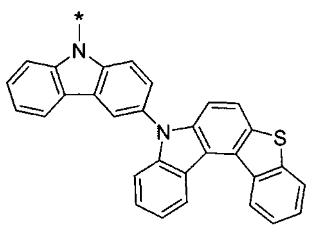

D184

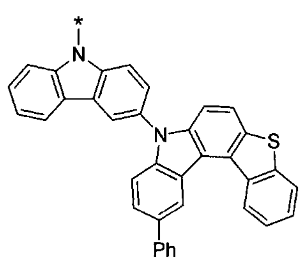

D185

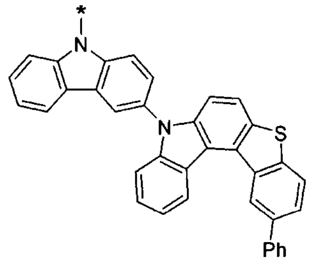

D186

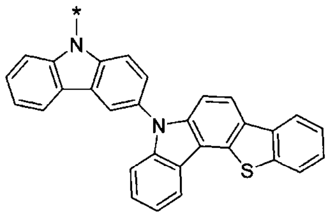

D187

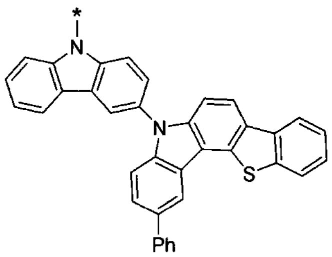

D188

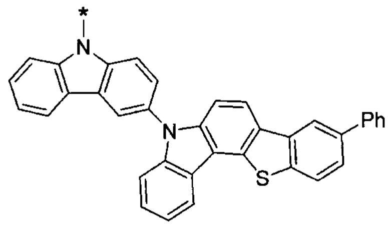

D189

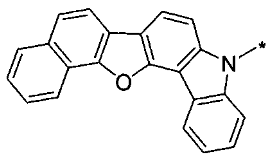

D190

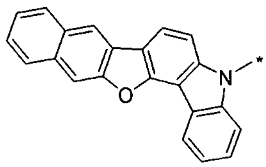

D191

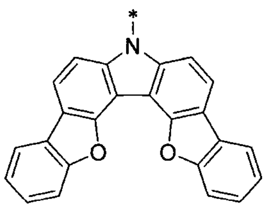

D192

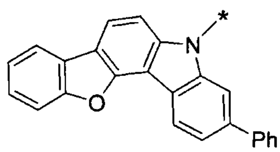

D193

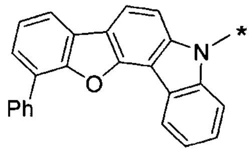

D194

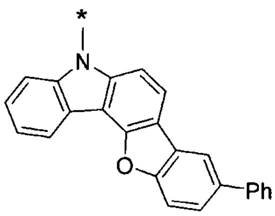

D195

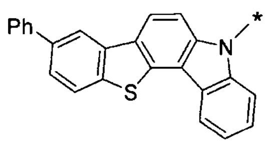

D196

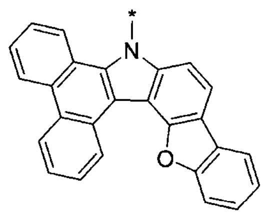

D197

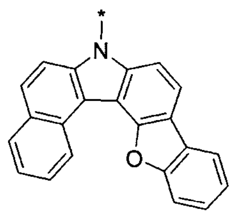

D198

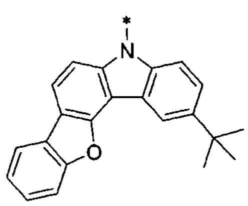

D199

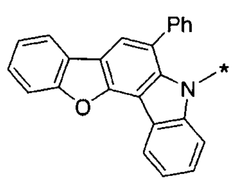

D200

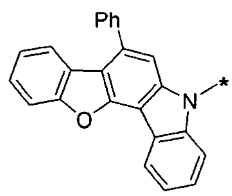

D201

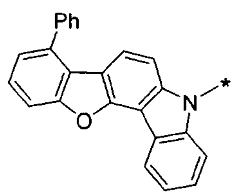

D202

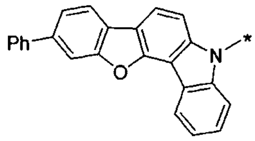 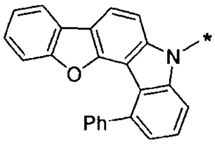 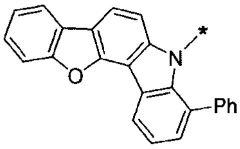 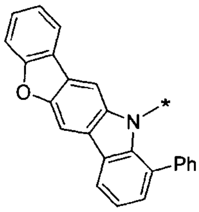 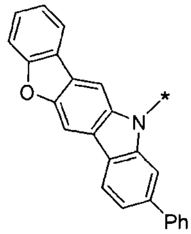

D203 D204 D205 D206 D207

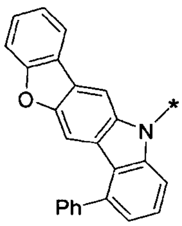 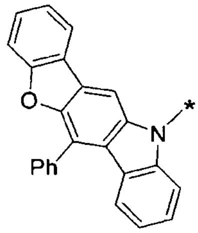 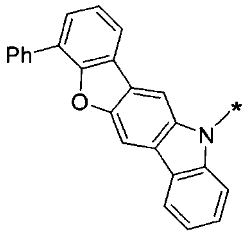 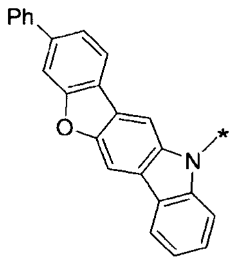 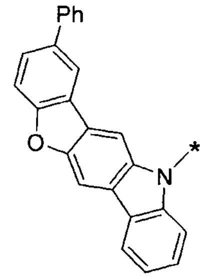

D208 D209 D210 D211 D212

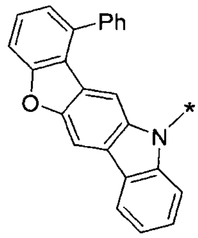 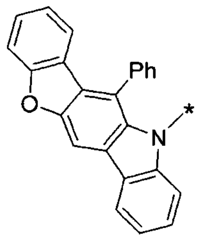 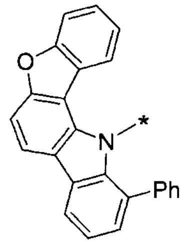 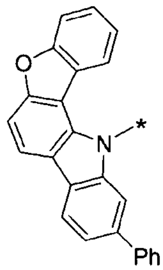

D213 D214 D215 D216

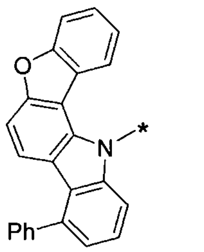 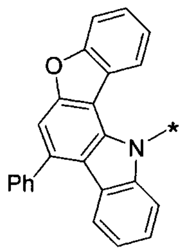 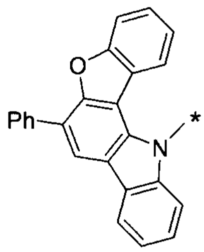 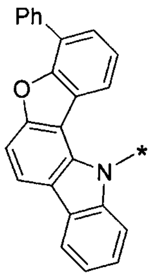

D217 D218 D219 D220

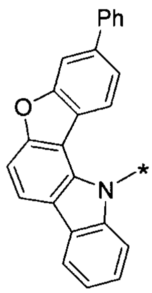

D221

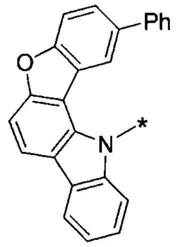

D222

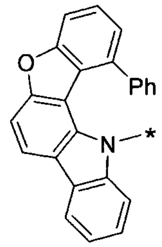

D223

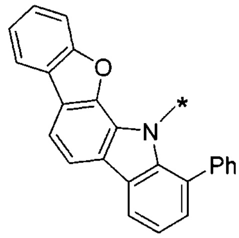

D224

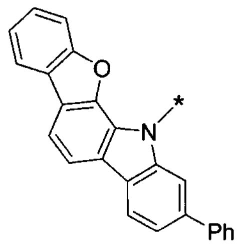

D225

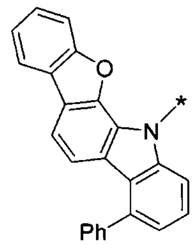

D226

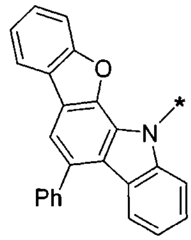

D227

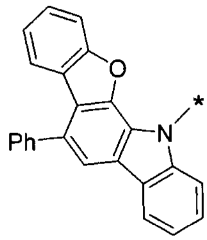

D228

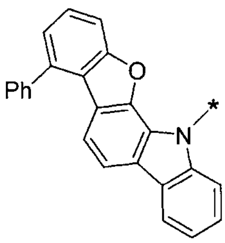

D229

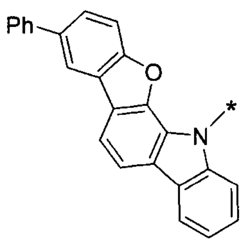

D230

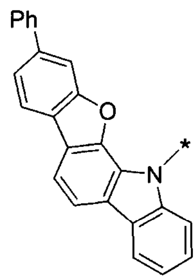

D231

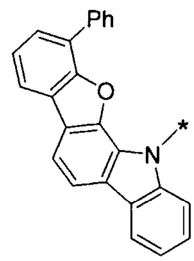

D232

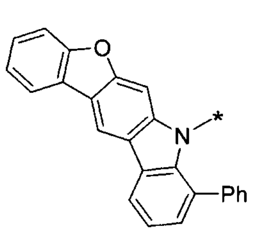

D233

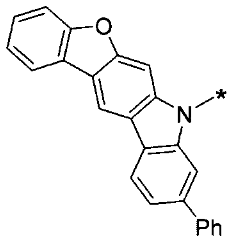

D234

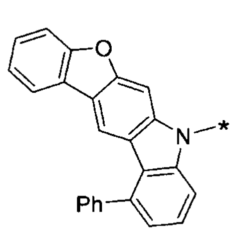

D235

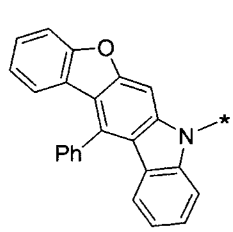

D236

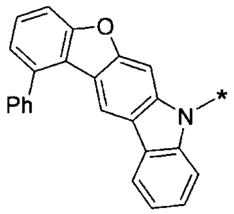
D237

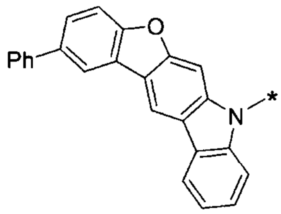
D238

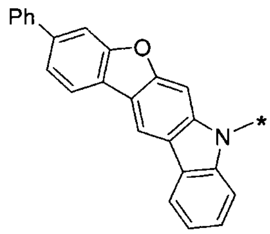
D239

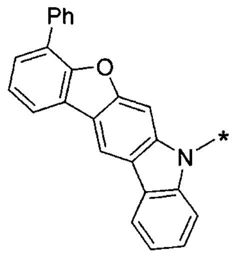
D240

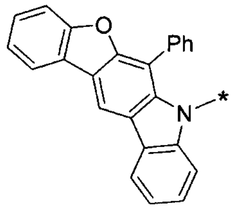
D241

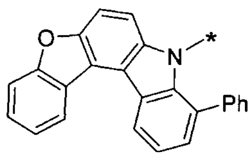
D242

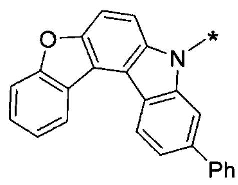
D243

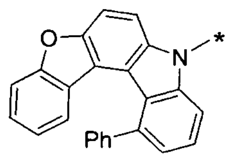
D244

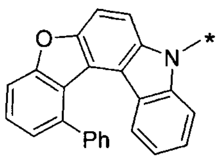
D245

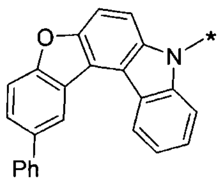
D246

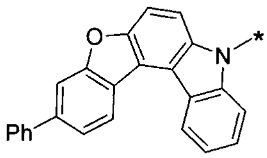
D247

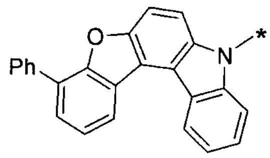
D248

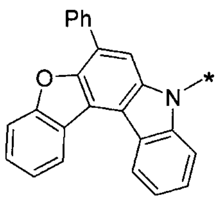
D249

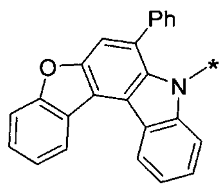
D250

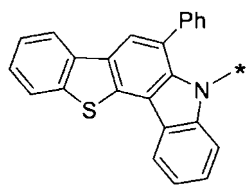
D251

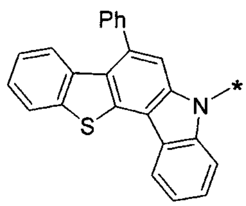
D252

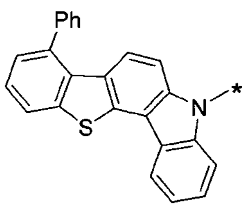
D253

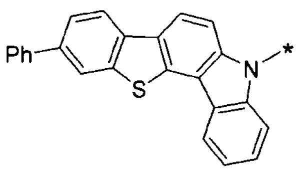
D254

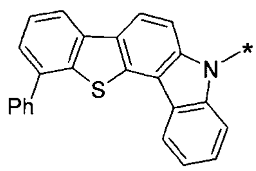
D255

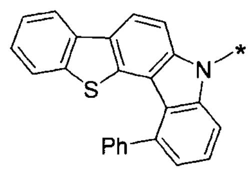
D256

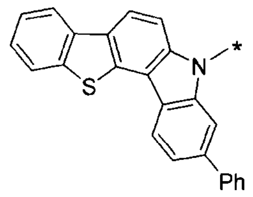

D257

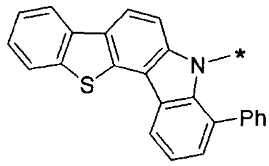

D258

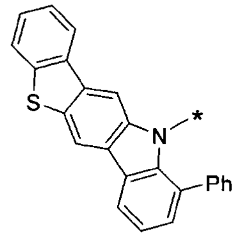

D259

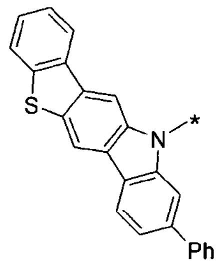

D260

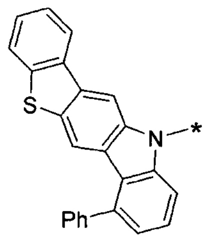

D261

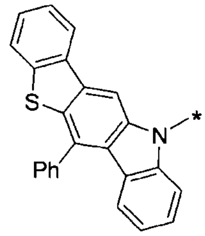

D262

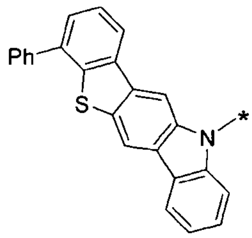

D263

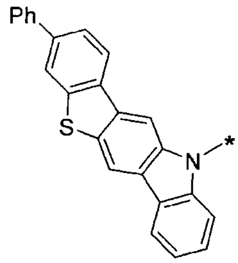

D264

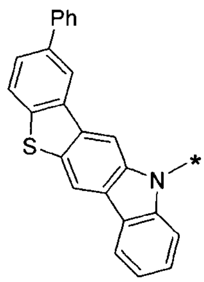

D265

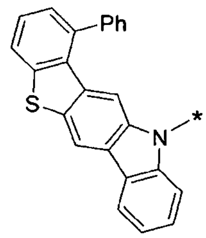

D266

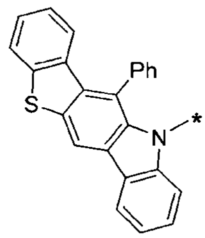

D267

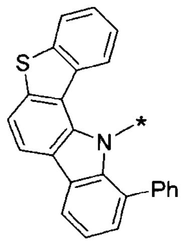

D268

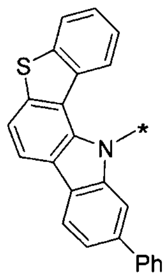

D269

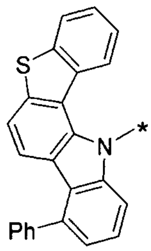

D270

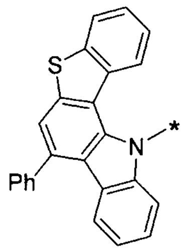

D271

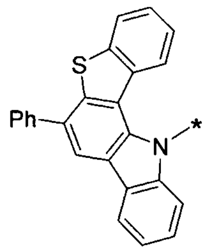

D272

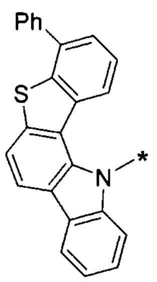
D273

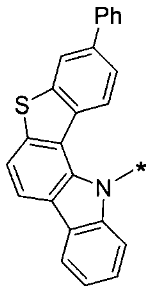
D274

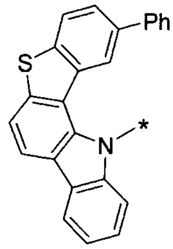
D275

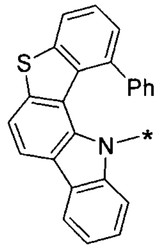
D276

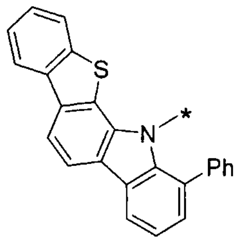
D277

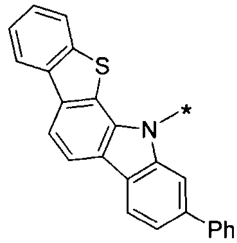
D278

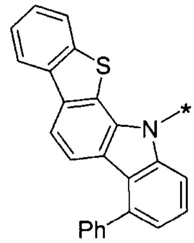
D279

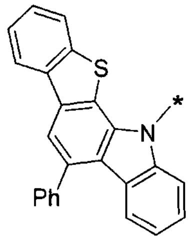
D280

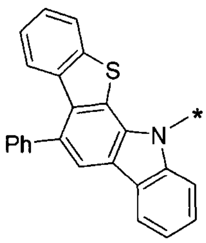
D281

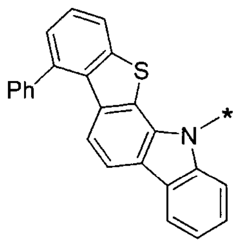
D282

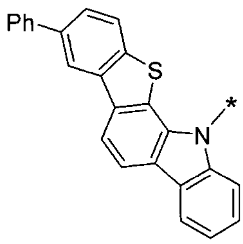
D283

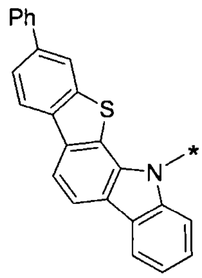
D284

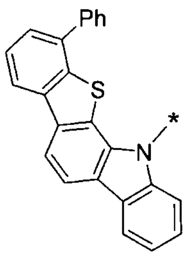
D285

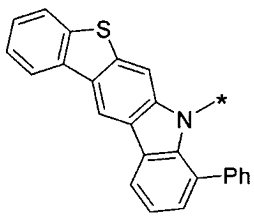
D286

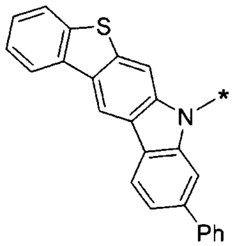
D287

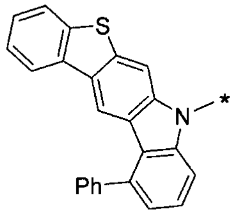
D288

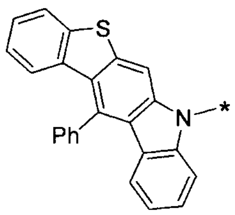
D289

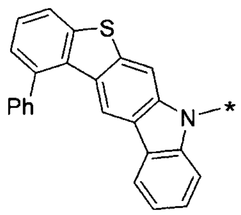
D290

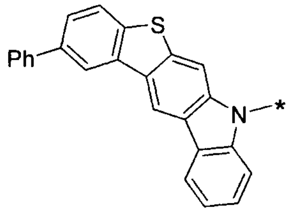
D291

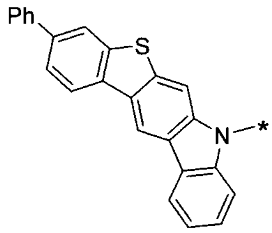
D292

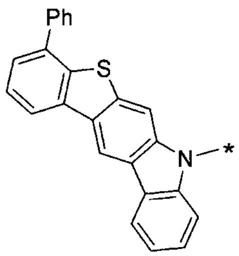
D293

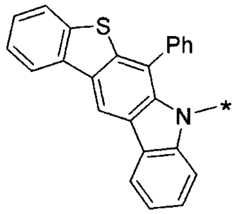
D294

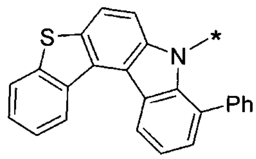
D295

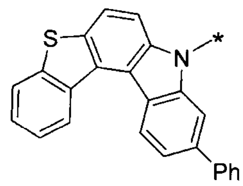
D296

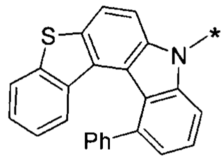
D297

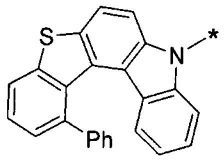
D298

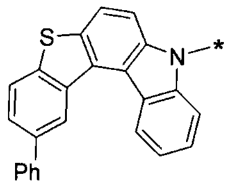
D299

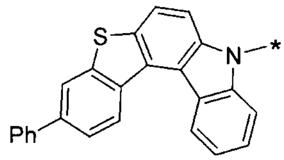
D300

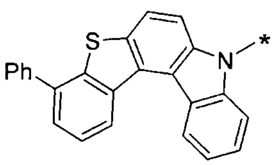
D301

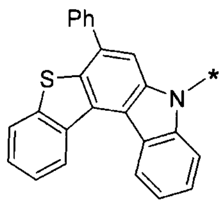
D302

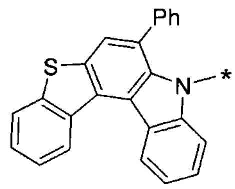
D303

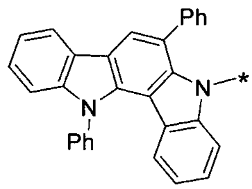
D304

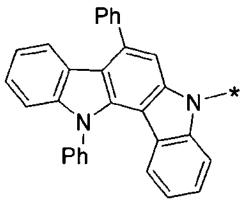
D305

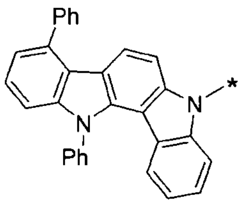
D306

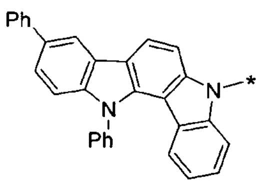
D307

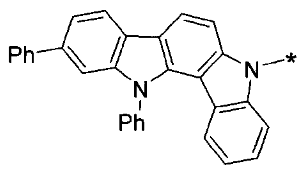
D308

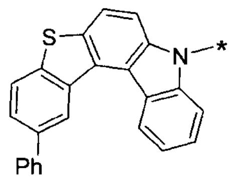
D309

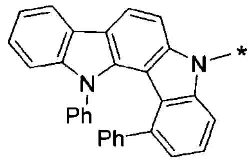
D310

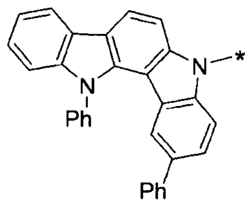
D311

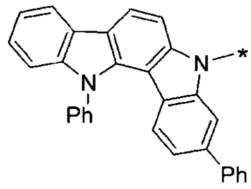
D312

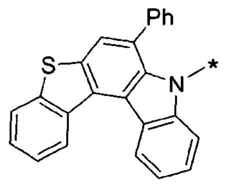
D313

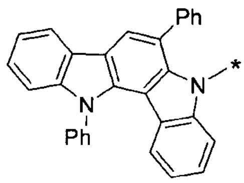
D314

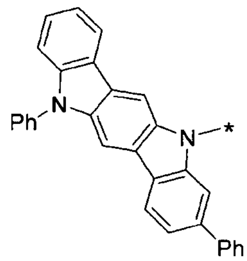
D315

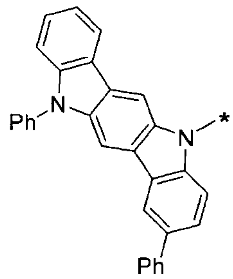
D316

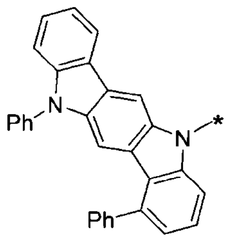
D317

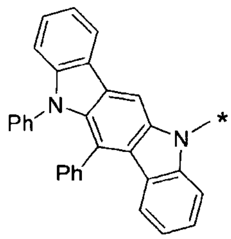
D318

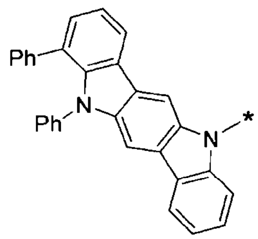
D319

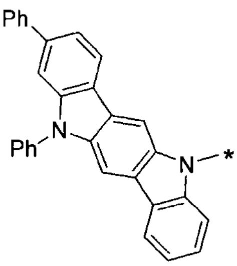
D320

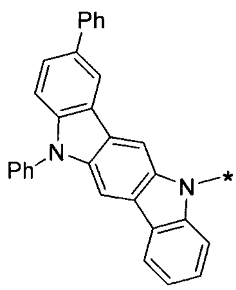
D321

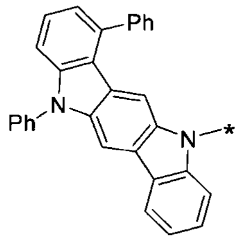
D322

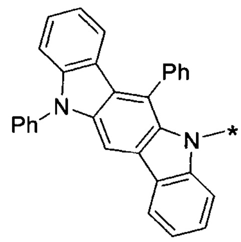
D323

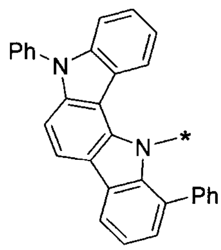
D324

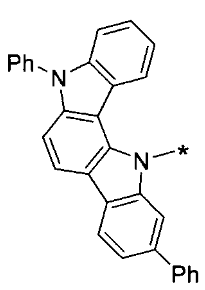

D325

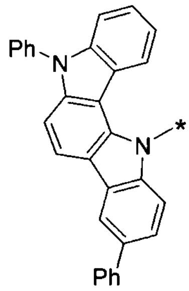

D326

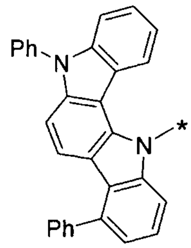

D327

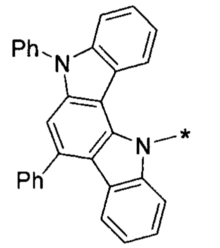

D328

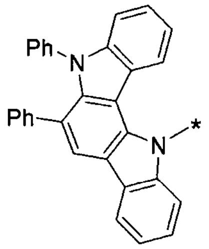

D329

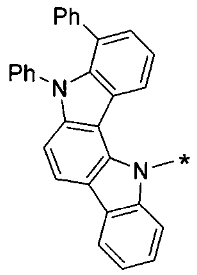

D330

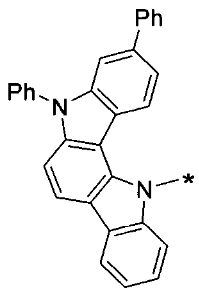

D331

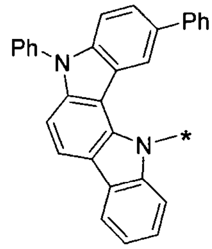

D332

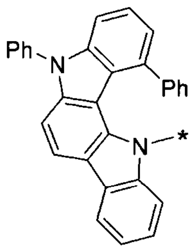

D333

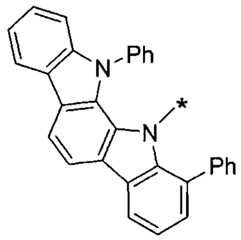

D334

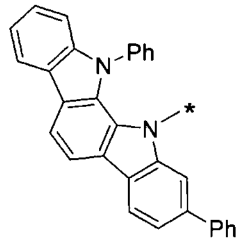

D335

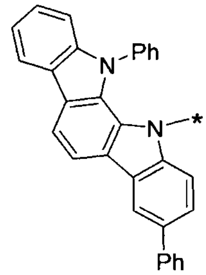

D336

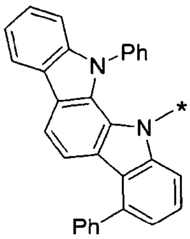

D337

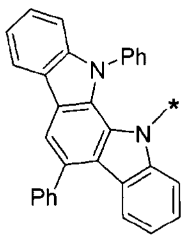

D338

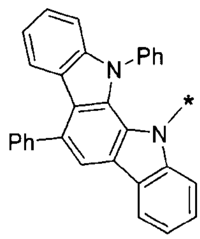

D339

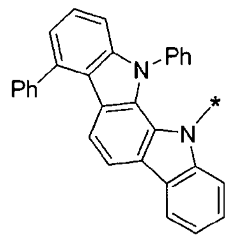

D340

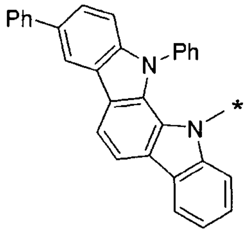 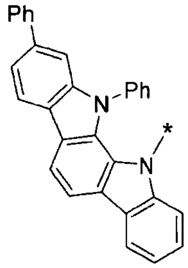 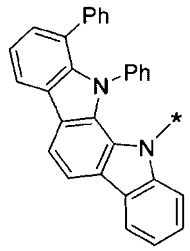 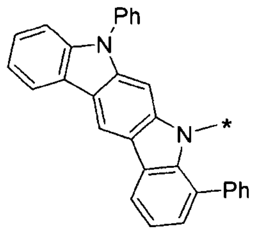

D341 D342 D343 D344

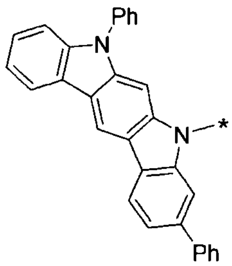 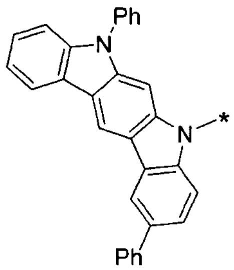 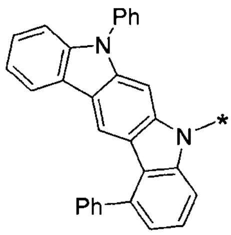 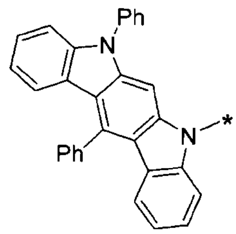

D345 D346 D347 D348

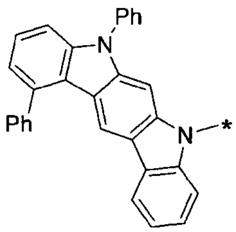 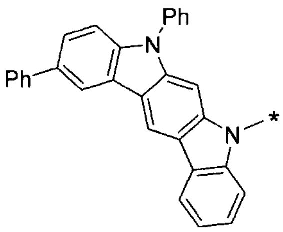 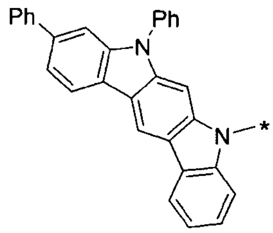 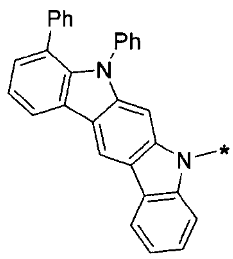

D349 D350 D351 D352

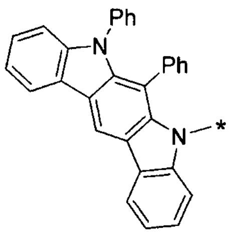 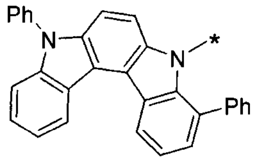 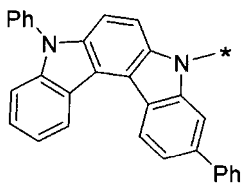 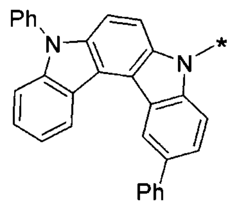

D353 D354 D355 D356

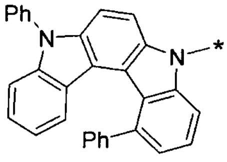 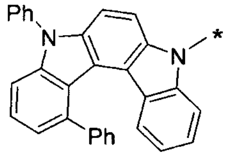 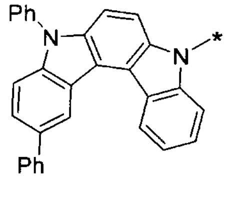 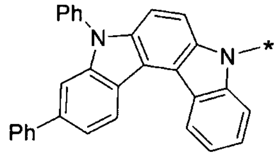

D357 D358 D359 D360

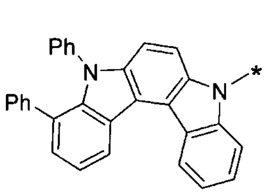 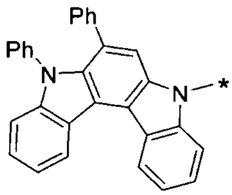 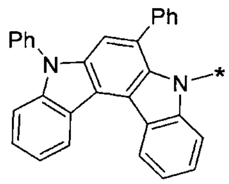 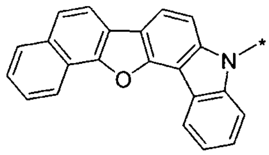

D361 D362 D363 D364

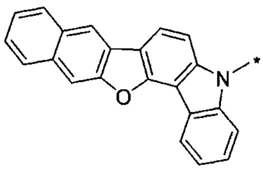 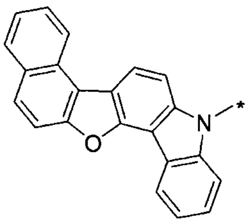 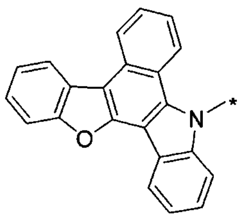 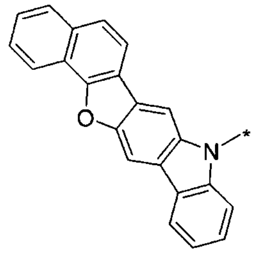

D365 D366 D367 D368

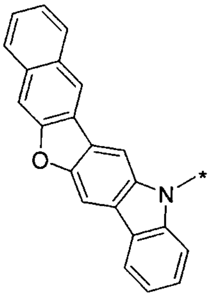 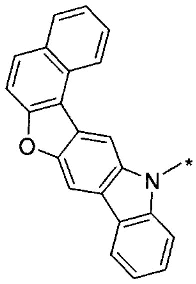 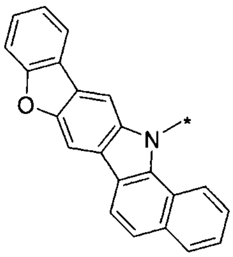 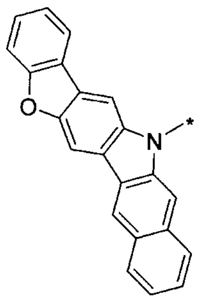 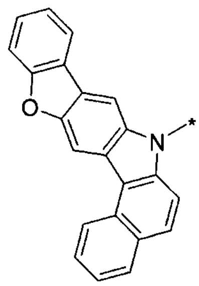

D369 D370 D371 D372 D373

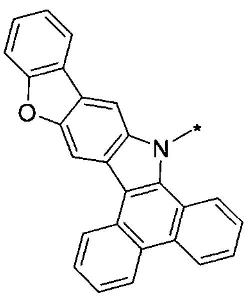 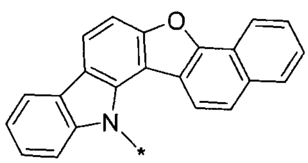 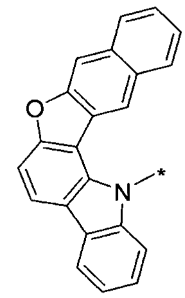 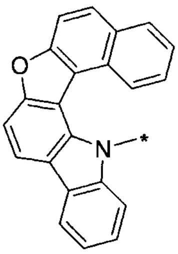

D374 D375 D376 D377

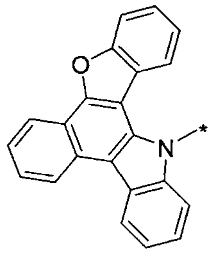
D378

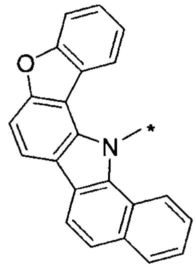
D379

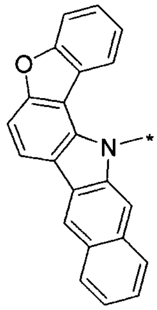
D380

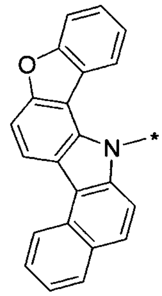
D381

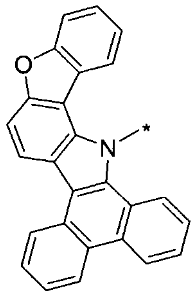
D382

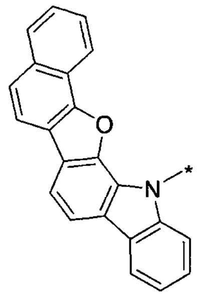
D383

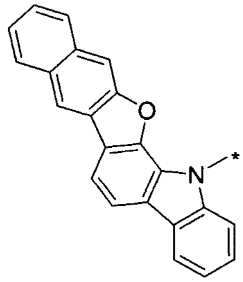
D384

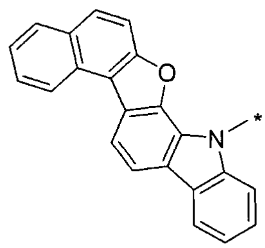
D385

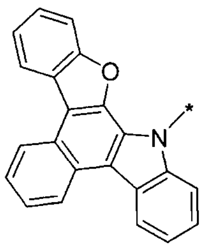
D386

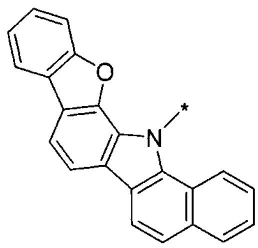
D387

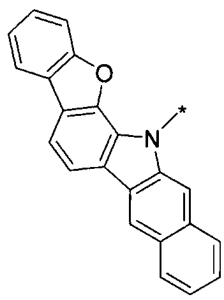
D388

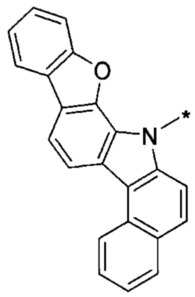
D389

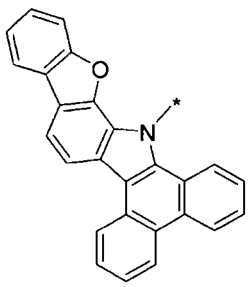
D390

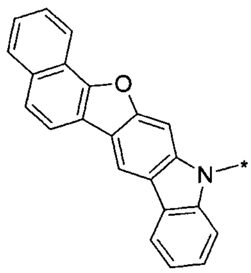
D391

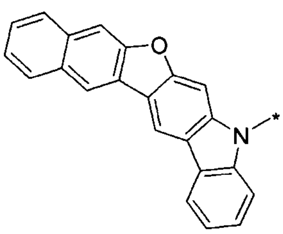
D392

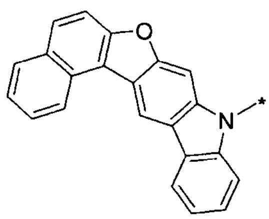
D393

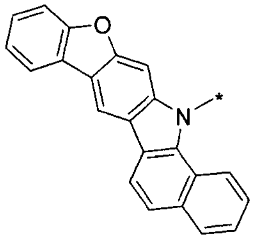
D394

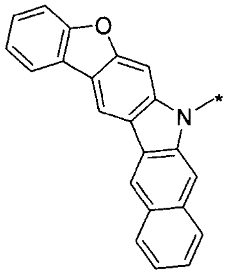
D395

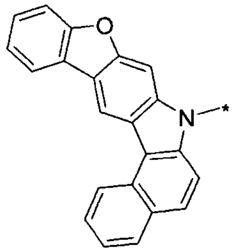
D396

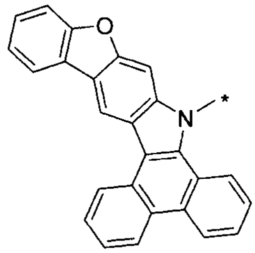
D397

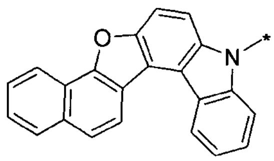
D398

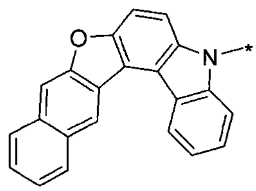
D399

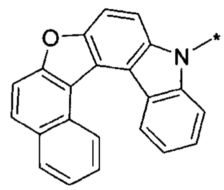
D400

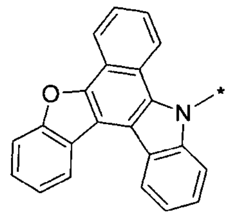
D401

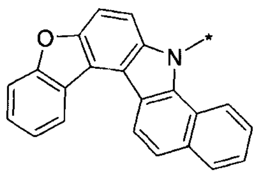
D402

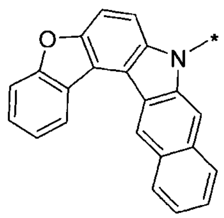
D403

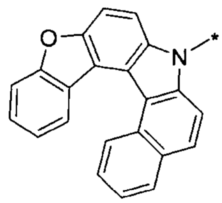
D404

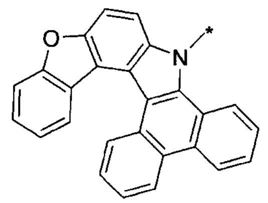
D405

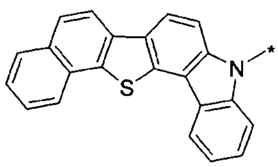
D406

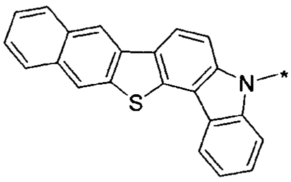
D407

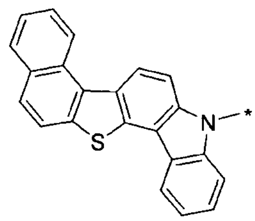
D408

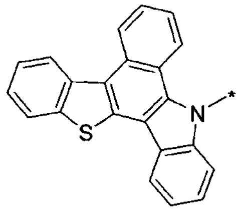
D409

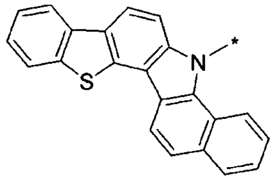
D410

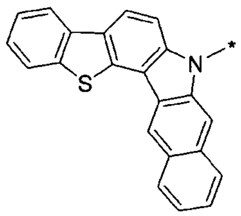
D411

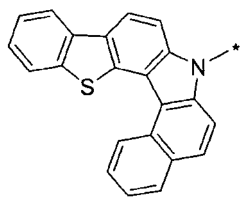
D412

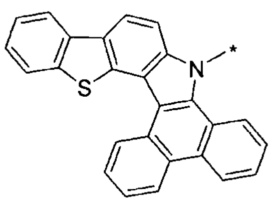
D413

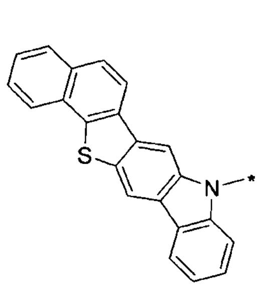
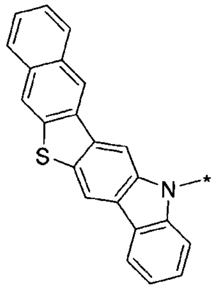
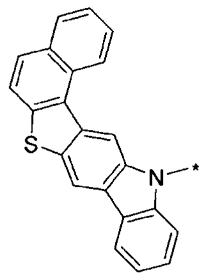
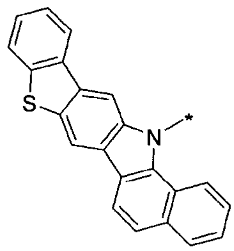

D414 D415 D416 D417

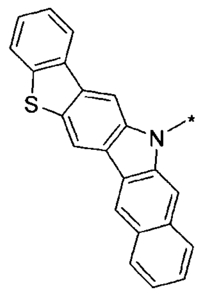
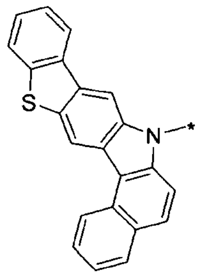
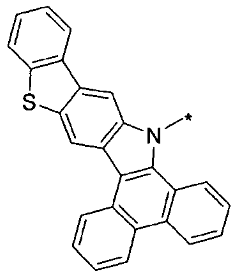
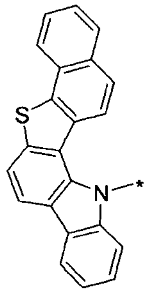

D418 D419 D420 D421

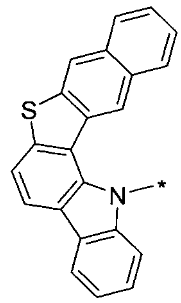
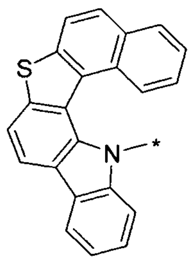
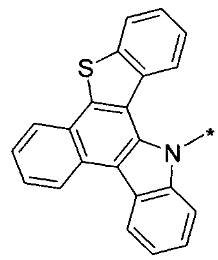
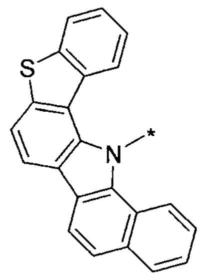

D422 D423 D424 D425

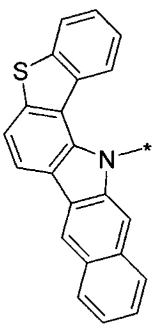
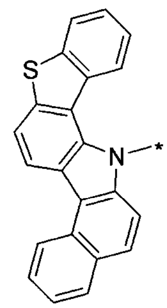
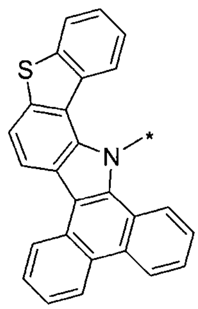
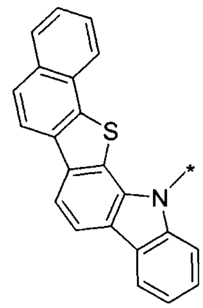

D426 D427 D428 D429

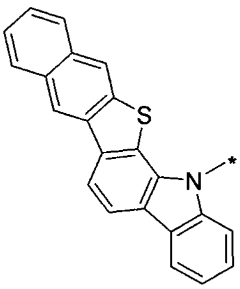
D430

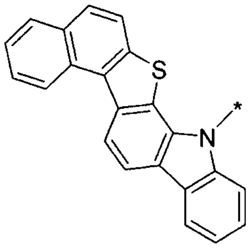
D431

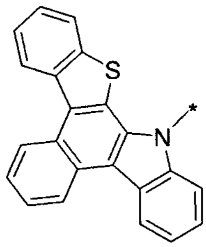
D432

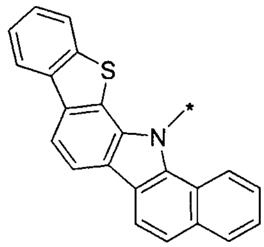
D433

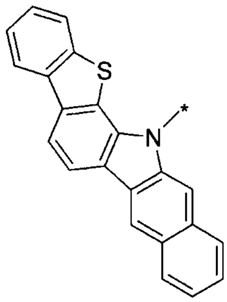
D434

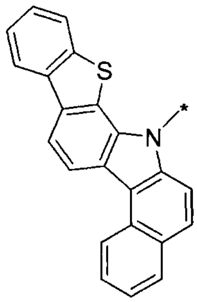
D435

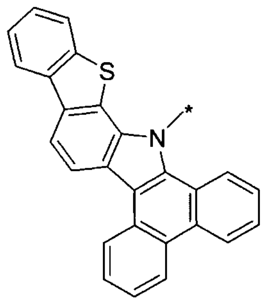
D436

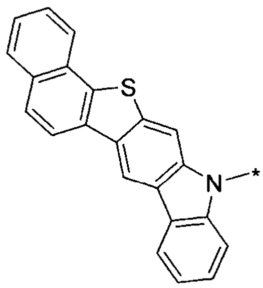
D437

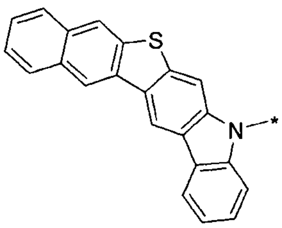
D438

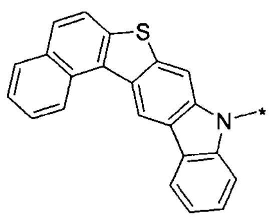
D439

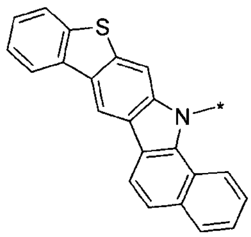
D440

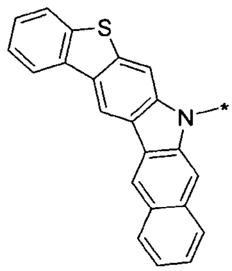
D441

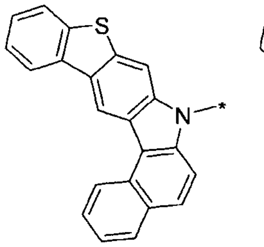
D442

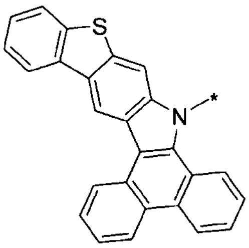
D443

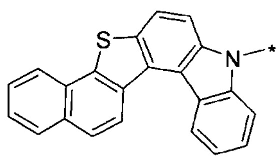
D444

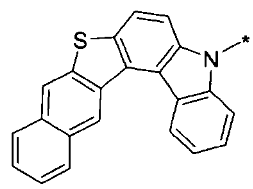
D445

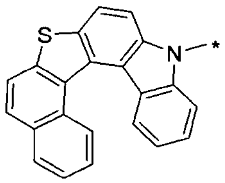
D446

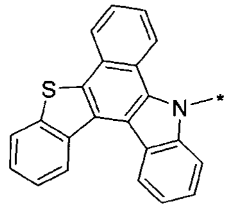
D447

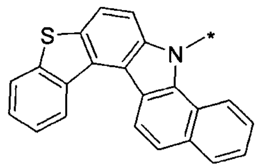
D448

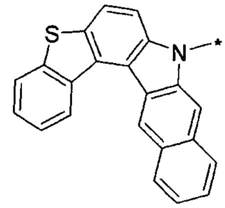
D449

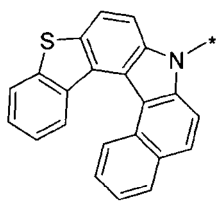
D450

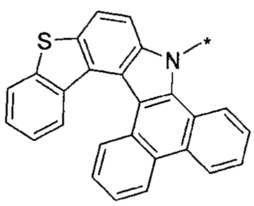
D451

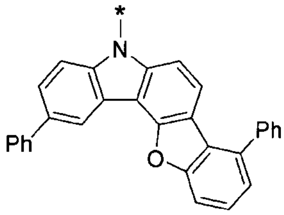
D452

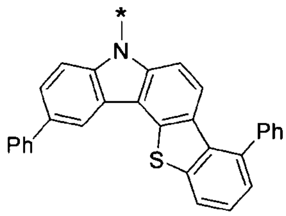
D453

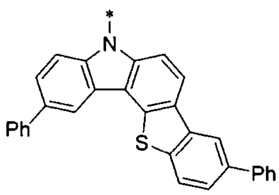
D454

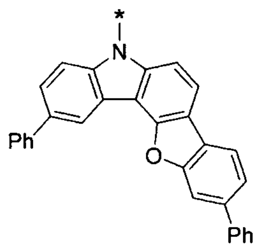
D455

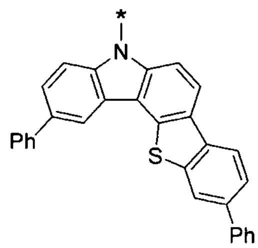
D456

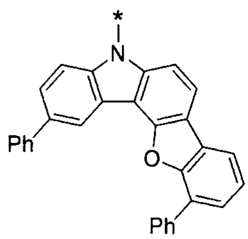
D457

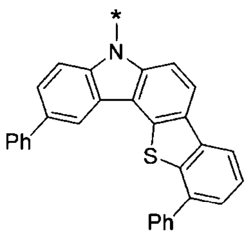
D458

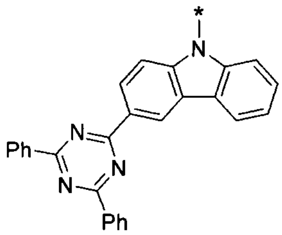
D459

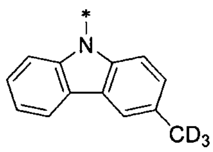
D460

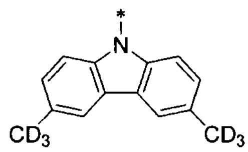
D461

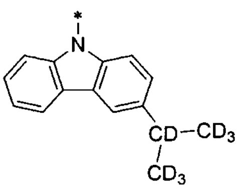
D462

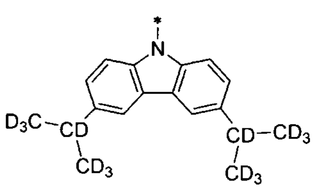
D463

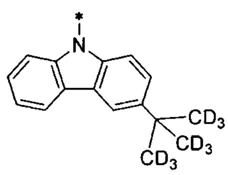
D464

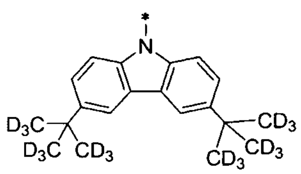
D465

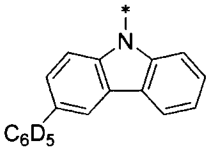 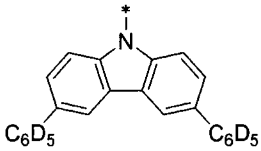 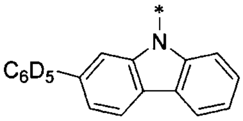 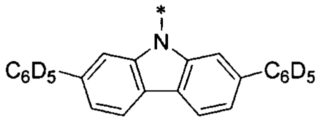

D466 D467 D468 D469

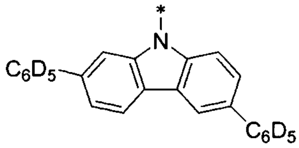 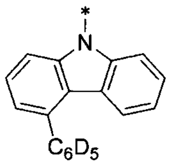 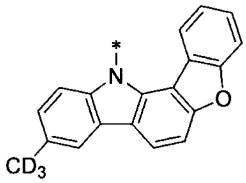 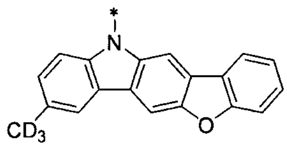

D470 D471 D472 D473

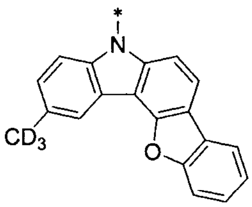 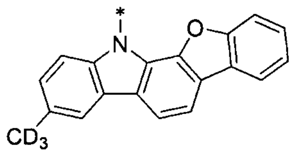 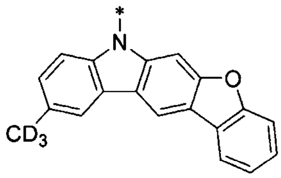 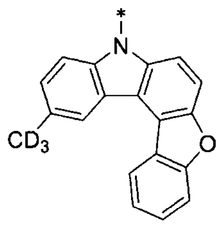

D474 D475 D476 D477

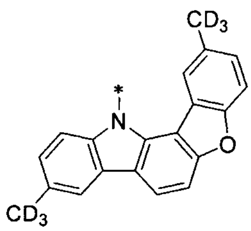 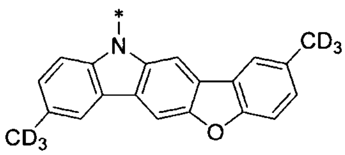 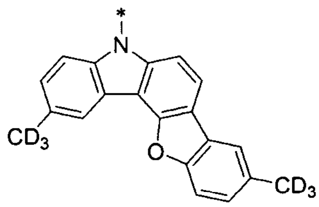

D478 D479 D480

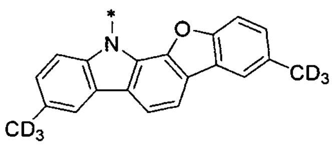 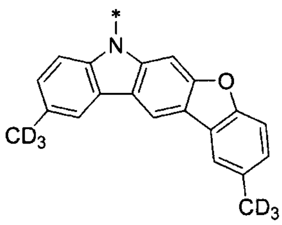 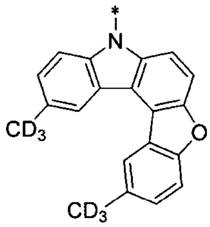 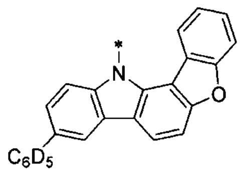

D481 D482 D483 D484

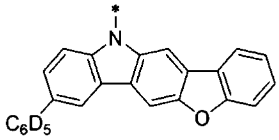
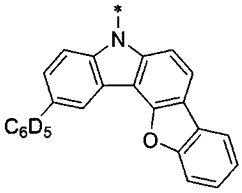
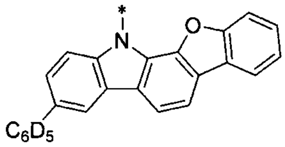
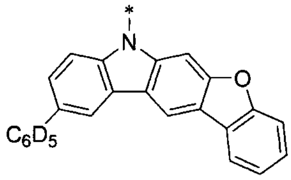

D485 D486 D487 D488

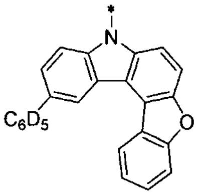
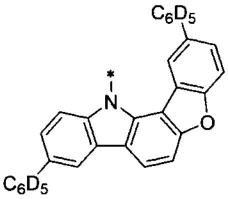
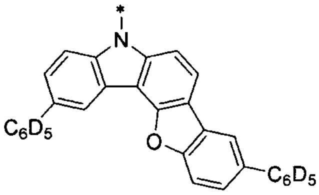
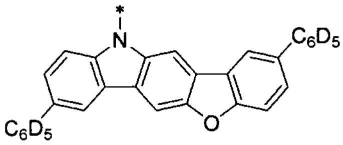

D489 D490 D491 D492

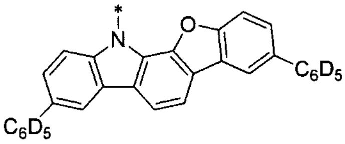
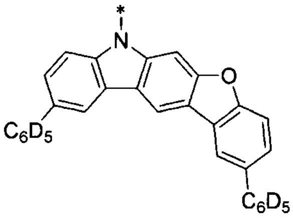
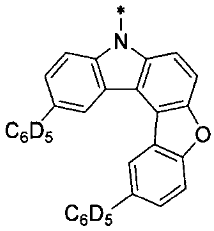
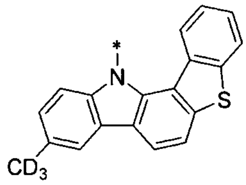

D493 D494 D495 D496

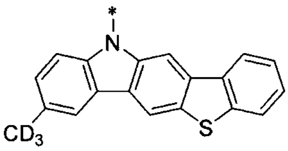
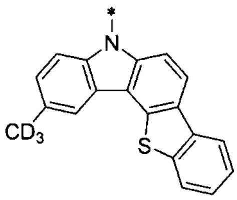
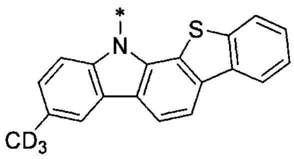
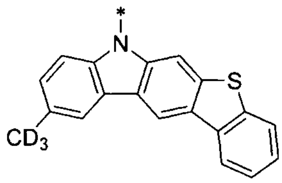

D497 D498 D499 D500

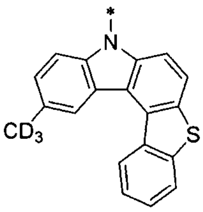
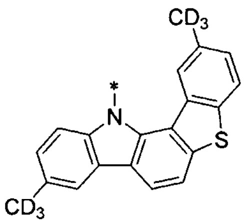
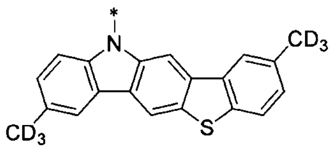
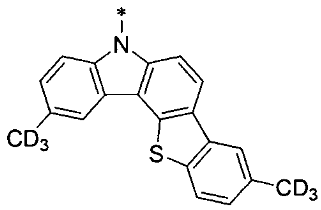

D501 D502 D503 D504

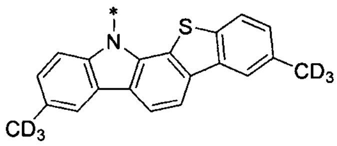

D505

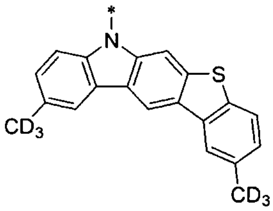

D506

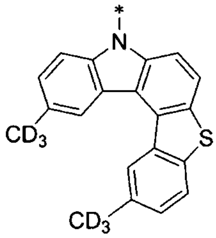

D507

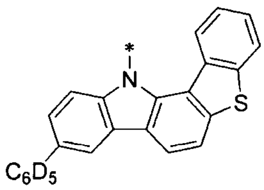

D508

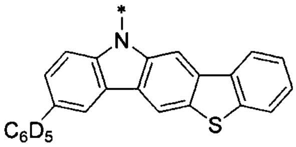

D509

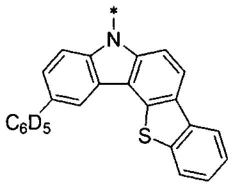

D510

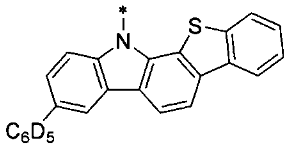

D511

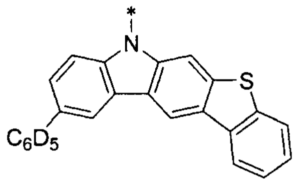

D512

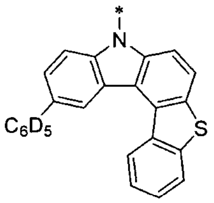

D513

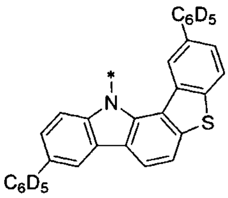

D514

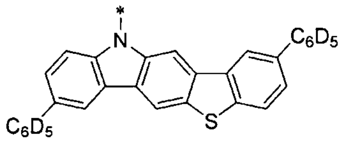

D515

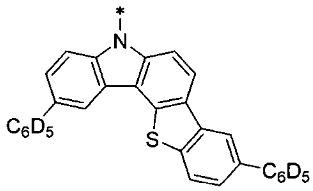

D516

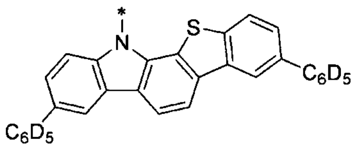

D517

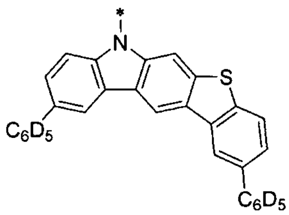

D518

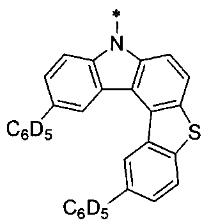

D519

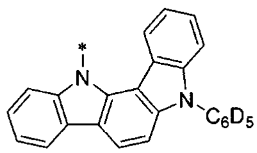

D520

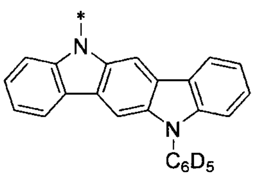

D521

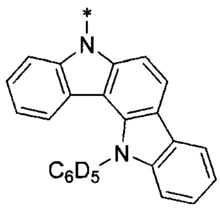

D522

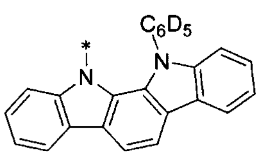

D523

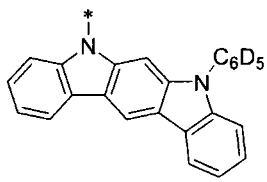

D524

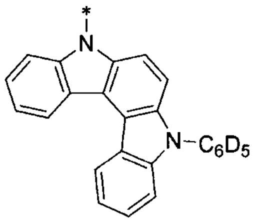

D525

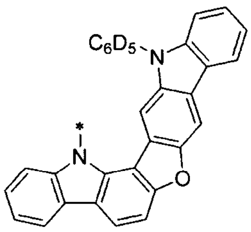

D526

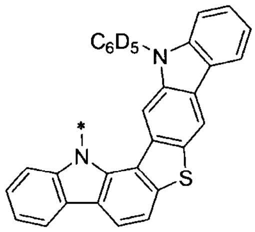

D527

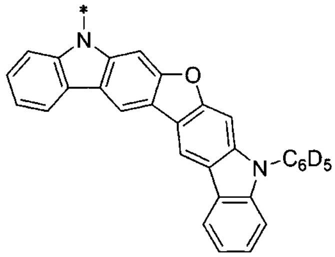

D528

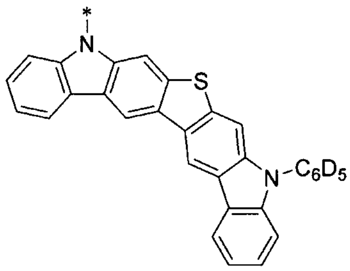

D529

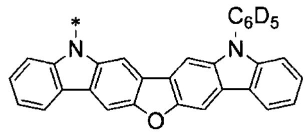

D530

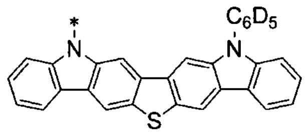

D531

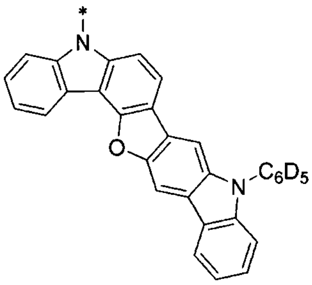

D532

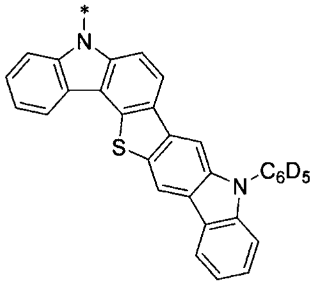

D533

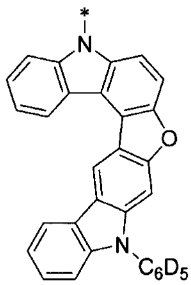

D534

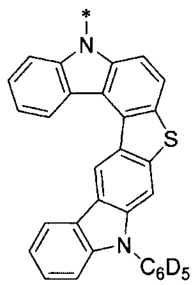

D535

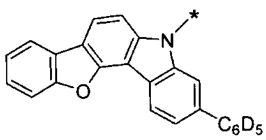

D536

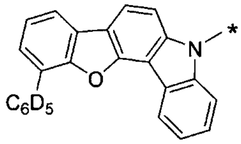

D537

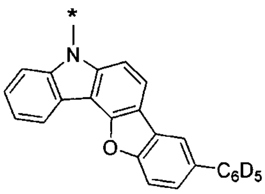

D538

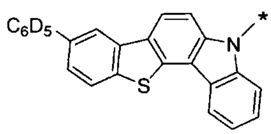

D539

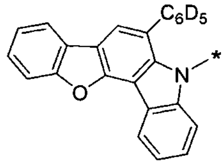

D540

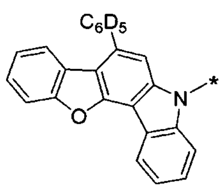

D541

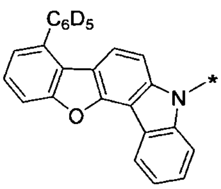

D542

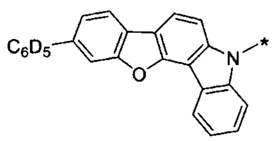

D543

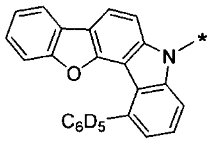
D544

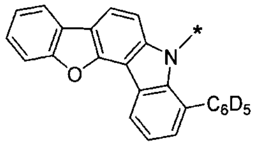
D545

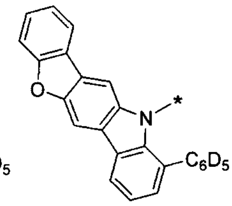
D546

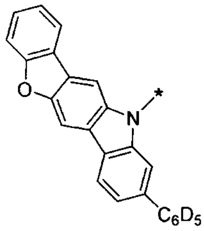
D547

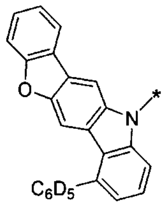
D548

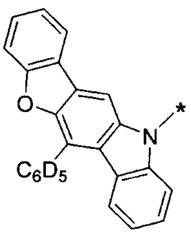
D549

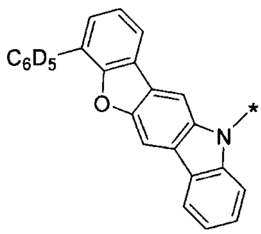
D550

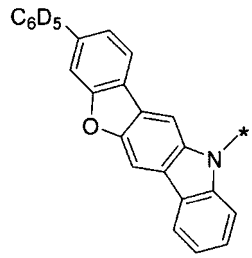
D551

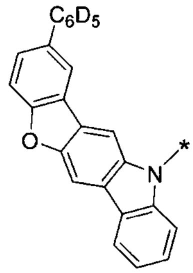
D552

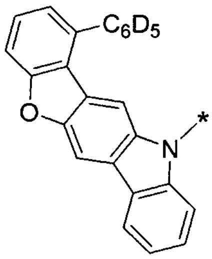
D553

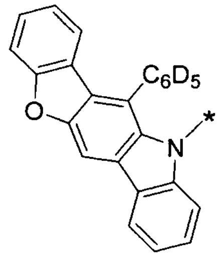
D554

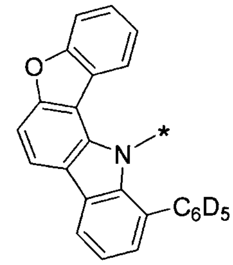
D555

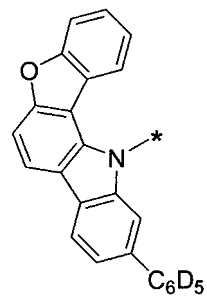
D556

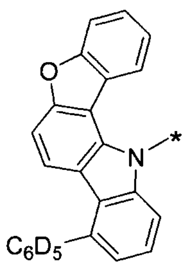
D557

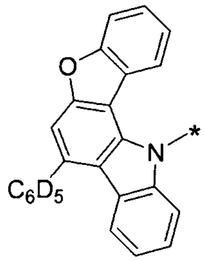
D558

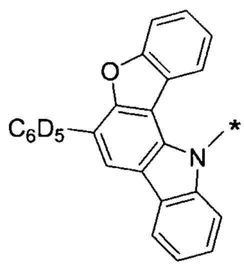
D559

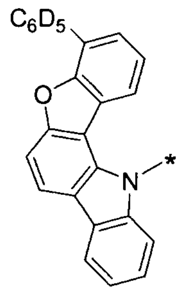
D560

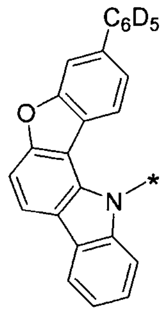
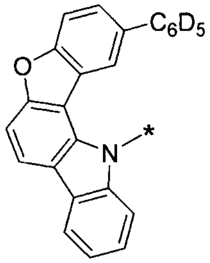
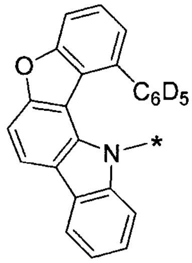
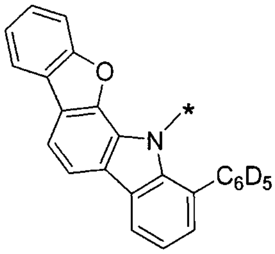

D561 D562 D563 D564

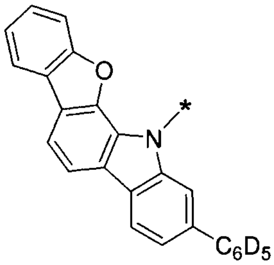
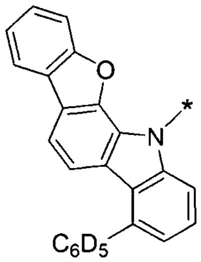
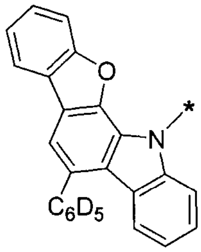
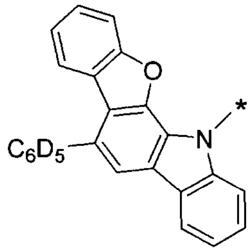

D565 D566 D567 D568

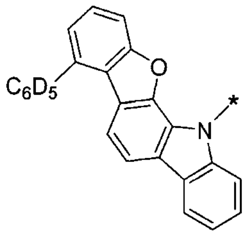
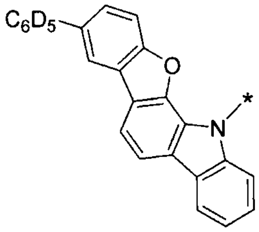
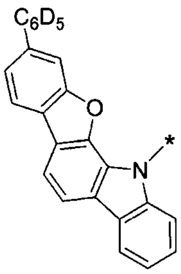
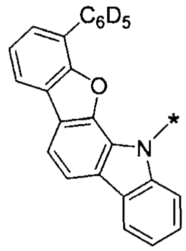

D569 D570 D571 D572

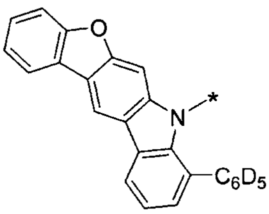
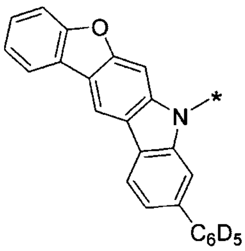
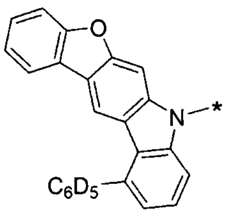
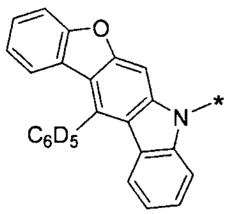

D573 D574 D575 D576

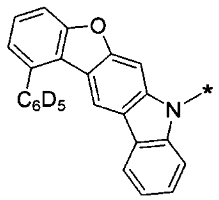

D577

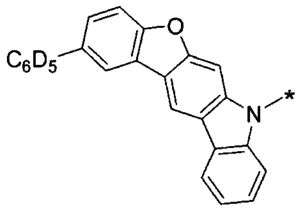

D578

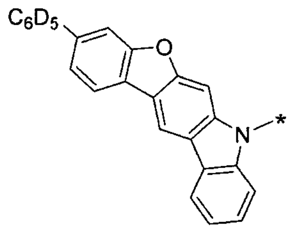

D579

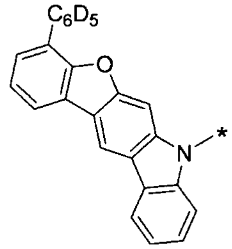

D580

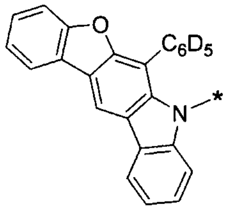

D581

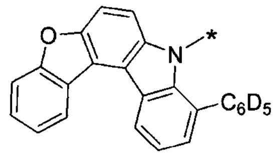

D582

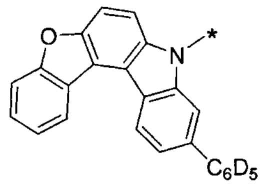

D583

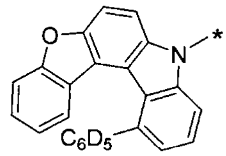

D584

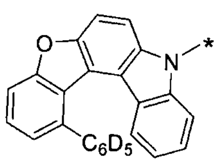

D585

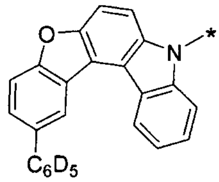

D586

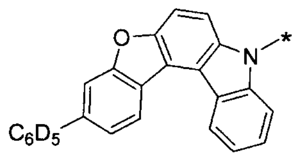

D587

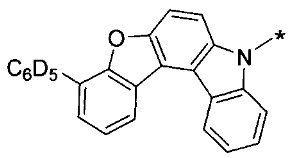

D588

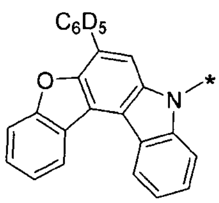

D589

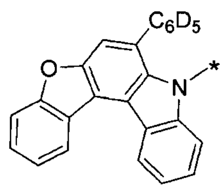

D590

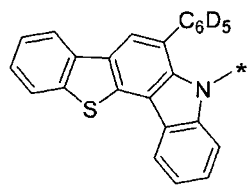

D591

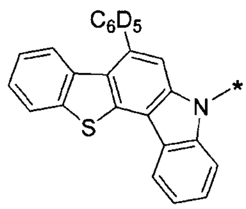

D592

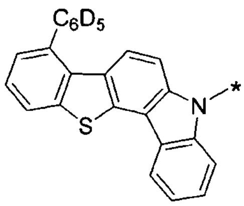

D593

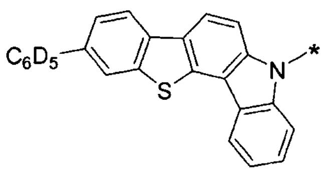

D594

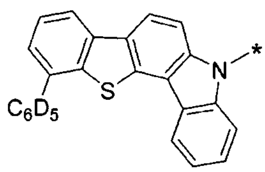

D595

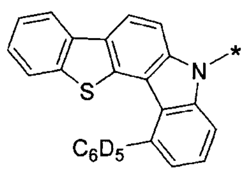

D596

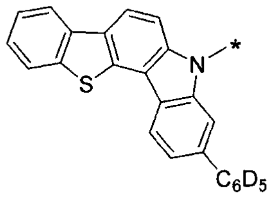

D597

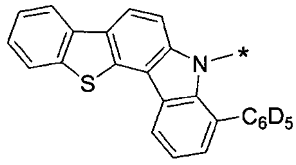

D598

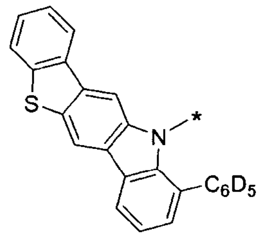

D599

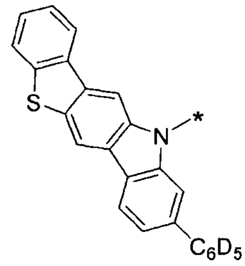

D600

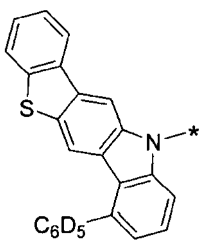

D601

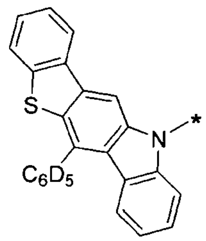

D602

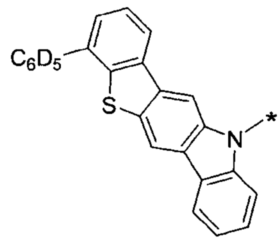

D603

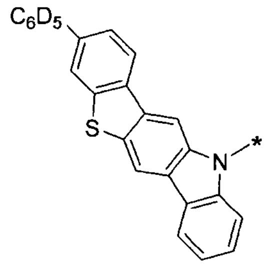

D604

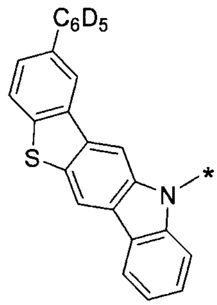

D605

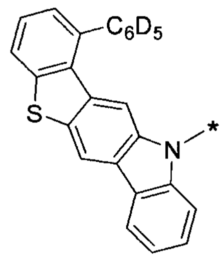

D606

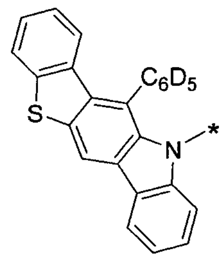

D607

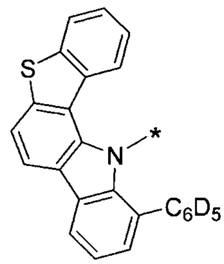

D608

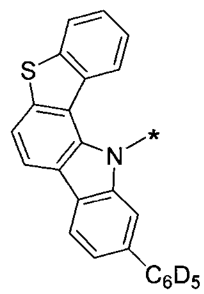

D609

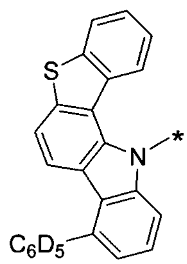

D610

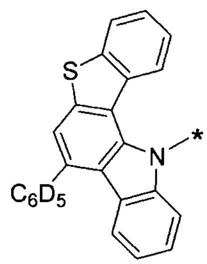

D611

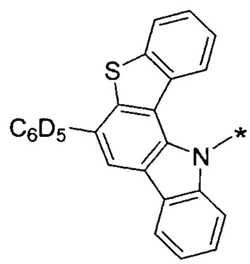

D612

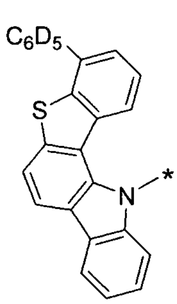 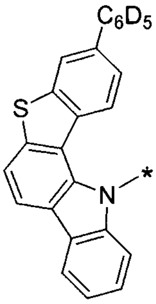 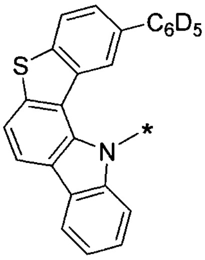 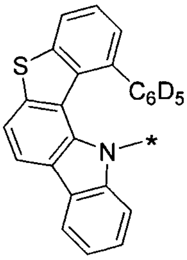

D613 D614 D615 D616

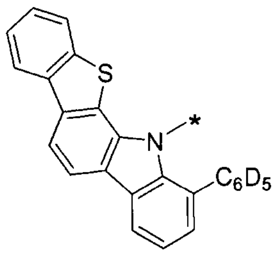 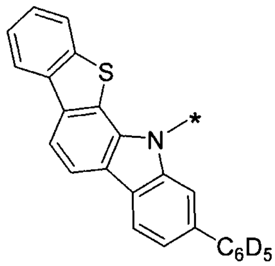 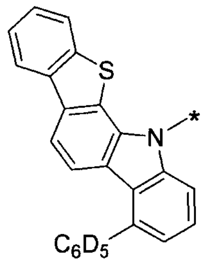 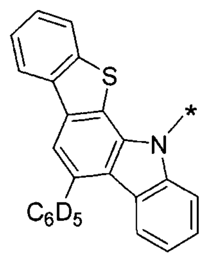

D617 D618 D619 D620

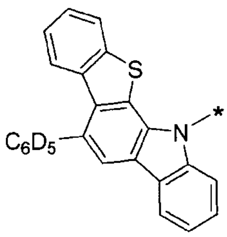 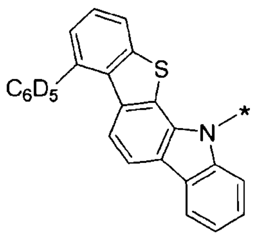 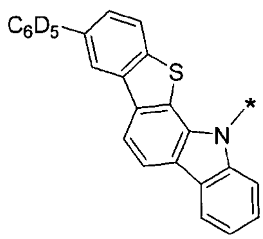 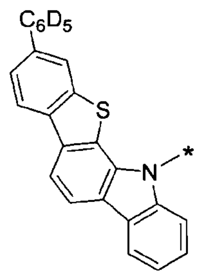

D621 D622 D623 D624

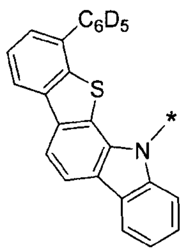 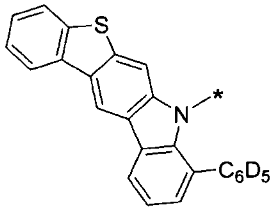 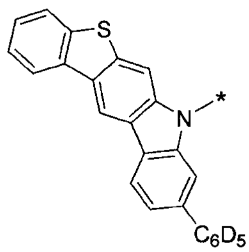 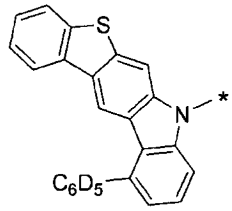

D625 D626 D627 D628

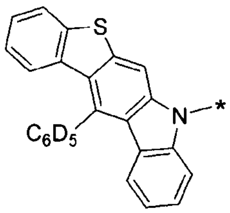
D629

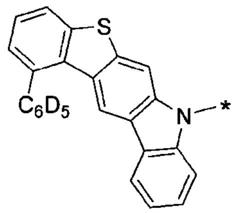
D630

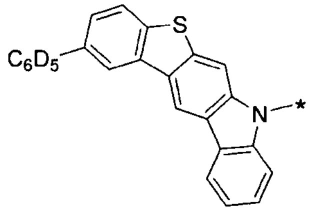
D631

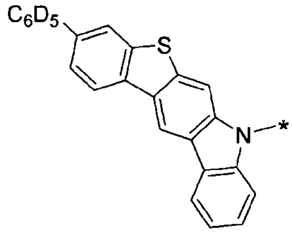
D632

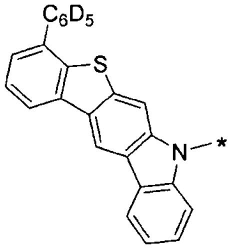
D633

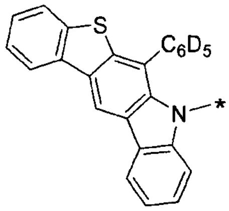
D634

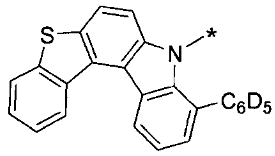
D635

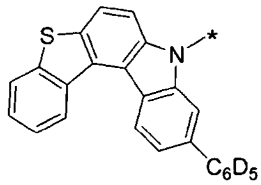
D636

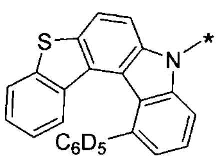
D637

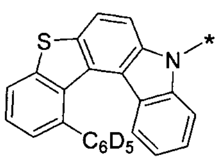
D638

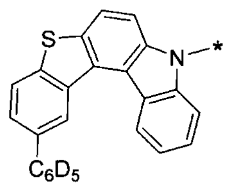
D639

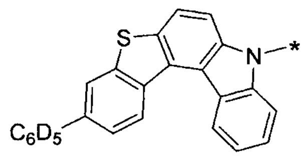
D640

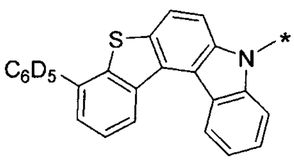
D641

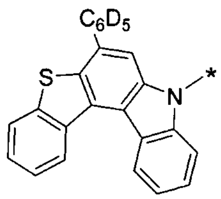
D642

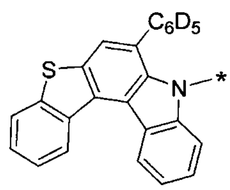
D643

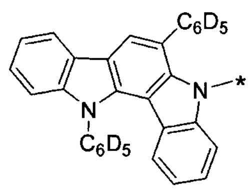
D644

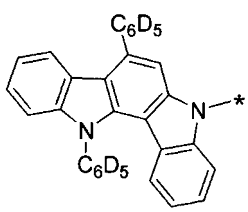
D645

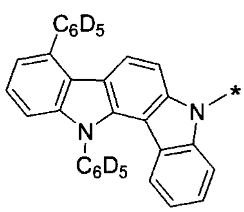
D646

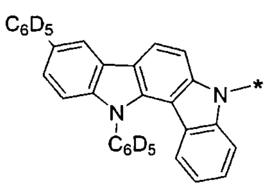
D647

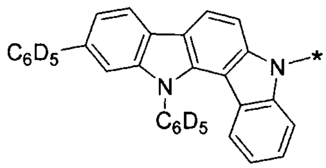
D648

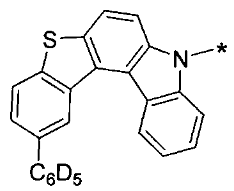

D649

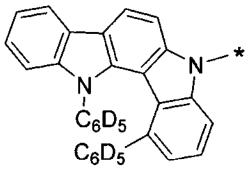

D650

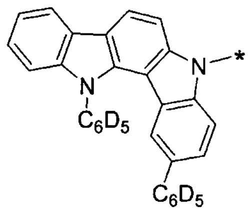

D651

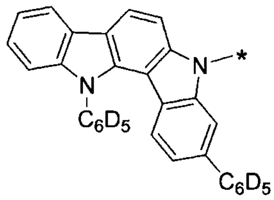

D652

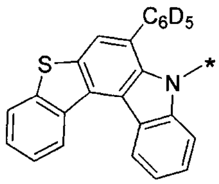

D653

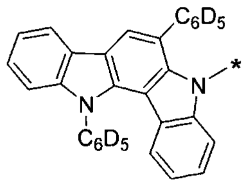

D654

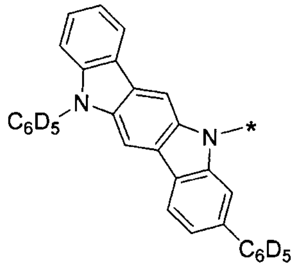

D655

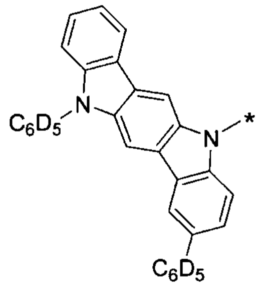

D656

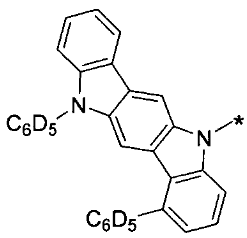

D657

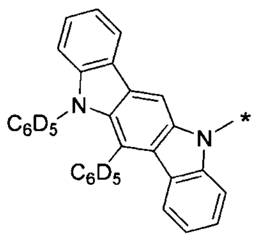

D658

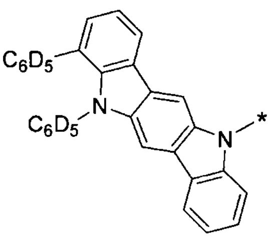

D659

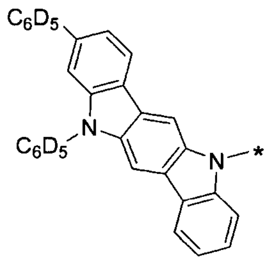

D660

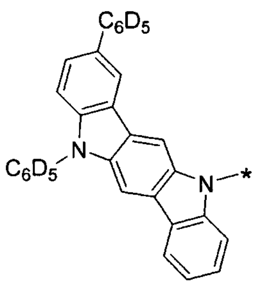

D661

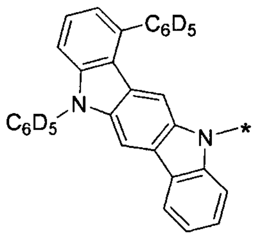

D662

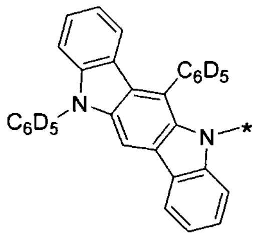

D663

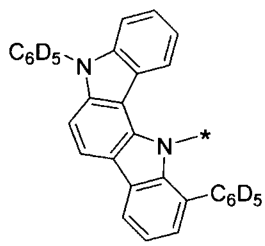

D664

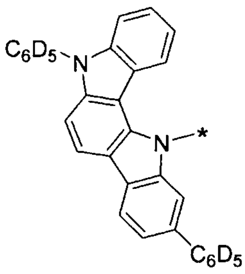
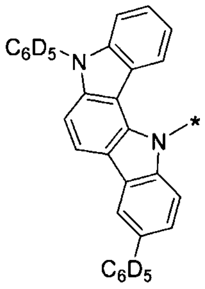
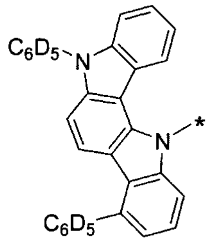
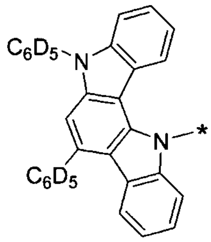

D665 D666 D667 D668

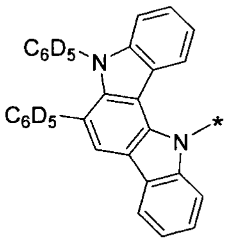
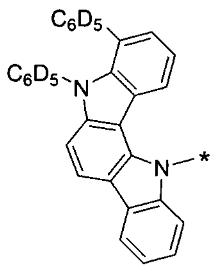
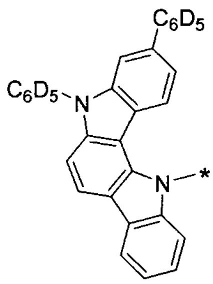
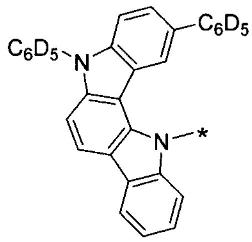

D669 D670 D671 D672

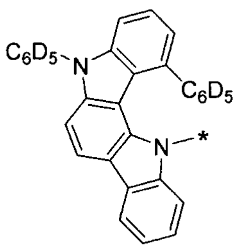
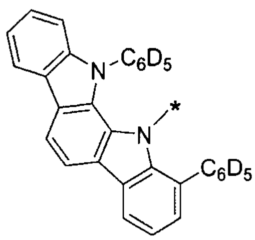
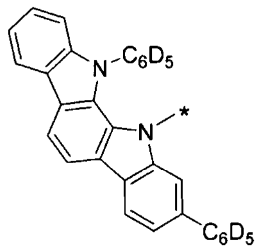
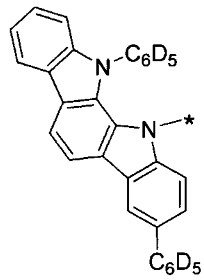

D673 D674 D675 D676

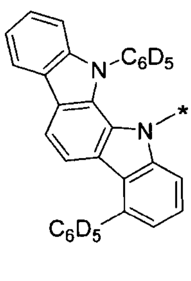
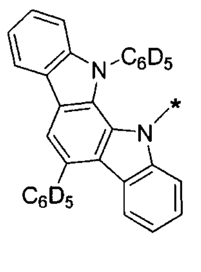
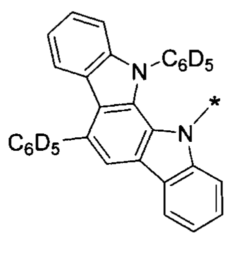
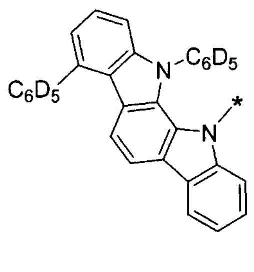

D677 D678 D679 D680

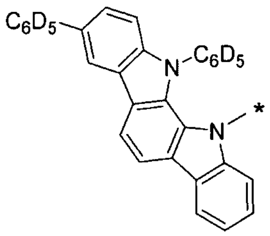

D681

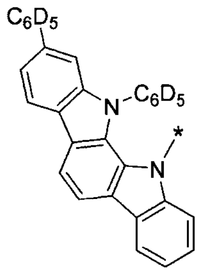

D682

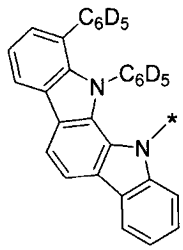

D683

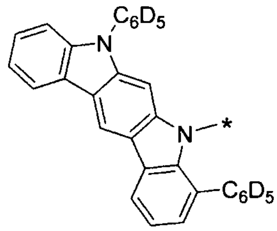

D684

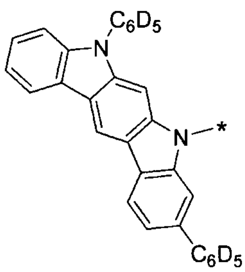

D685

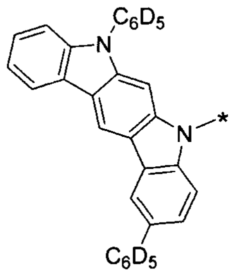

D686

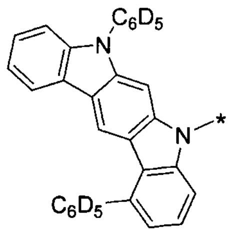

D687

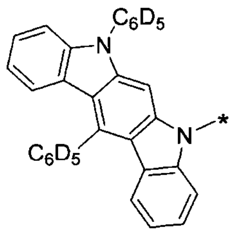

D688

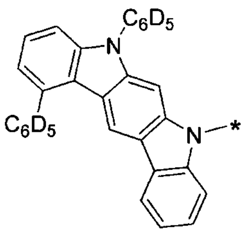

D689

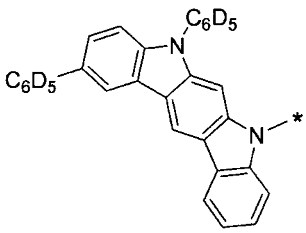

D690

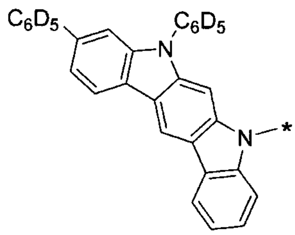

D691

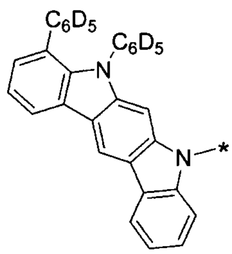

D692

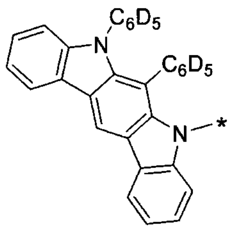

D693

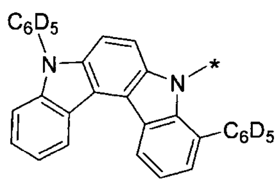

D694

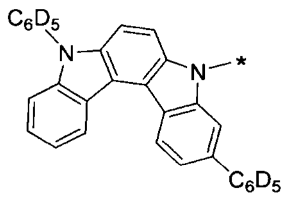

D695

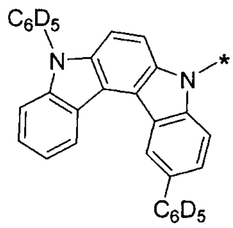

D696

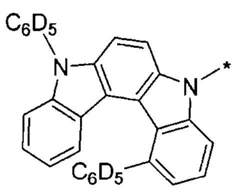

D697

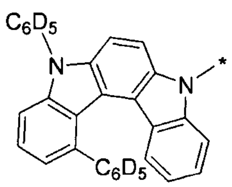

D698

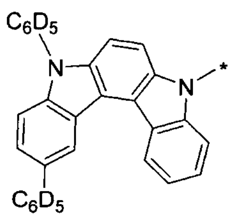

D699

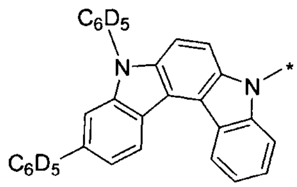

D700

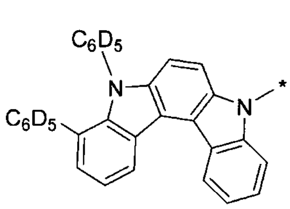

D701

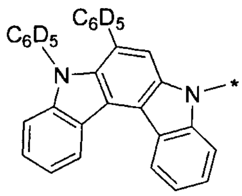

D702

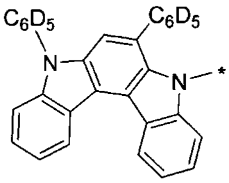

D703

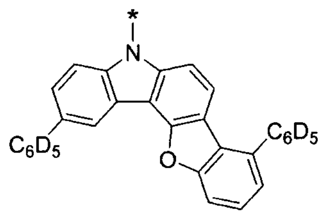

D704

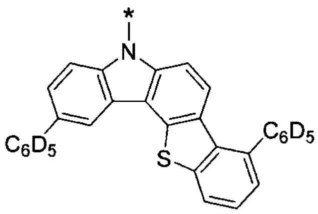

D705

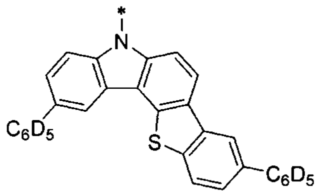

D706

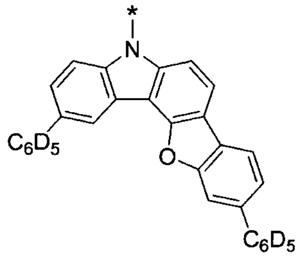

D707

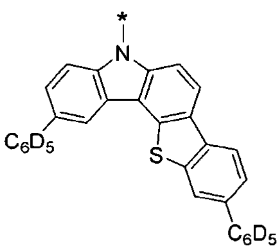

D708

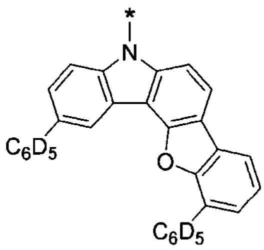

D709

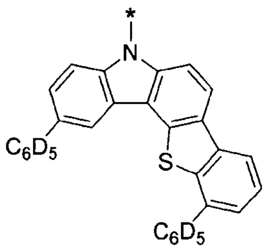

D710

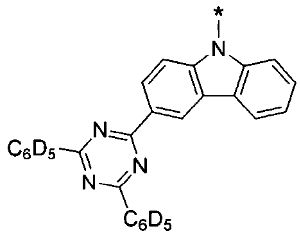

D711

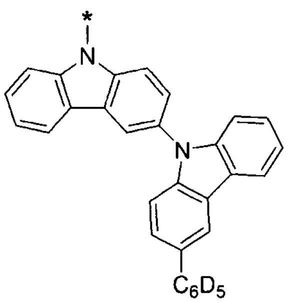

D712

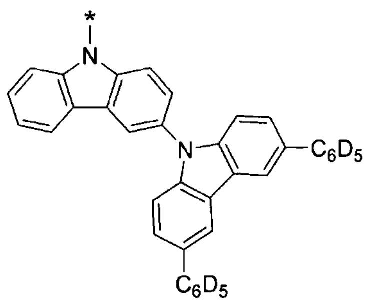

D713

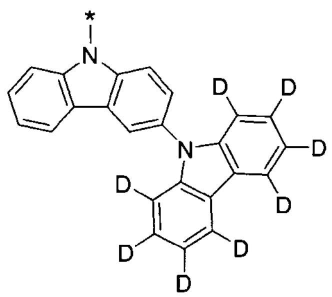

D714

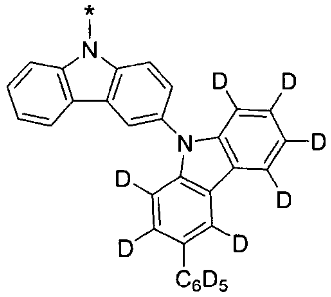

D715

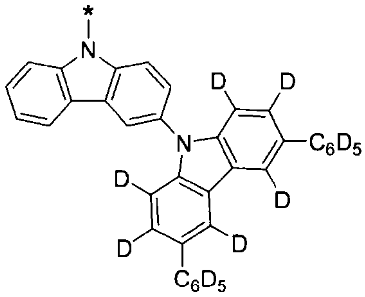

D716

Groups obtained by substituting all hydrogen atoms present in the above D1 to D459 with deuterium atoms are

disclosed as D717 to D1175. Phenyl groups with any of the above D1 to D1175 bonding to the 3-position (that is, groups with a metaphenylene group further bonding to * of D1 to D1175) are disclosed as D1(m) to D1175(m). Phenyl groups with any of the above D1 to D1175 bonding to the 4-position (that is, groups with a para-phenylene group further bonding to * of D1 to D1175) are disclosed as D1(p) to D1175(p).

In one preferred aspect of the present invention, the donor group that $R^1$ to $R^5$ in the general formula (1) can take is selected from the group consisting of D1 to D1175. In one aspect of the present invention, the donor group that $R^1$ to $R^5$ can take is selected from the group consisting of D460 to D1175. In one aspect of the present invention, the donor group that $R^1$ to $R^5$ can take is selected from the group consisting of D1(m) to D1175(m). In one aspect of the present invention, the donor group that $R^1$ to $R^5$ can take is selected from the group consisting of D1(p) to D1175(p).

In one aspect of the present invention, the donor group that $R^1$ to $R^5$ can take is selected from the group consisting of D1 to D13, and D717 to D729. In one aspect of the present invention, the donor group that $R^1$ to $R^5$ can take is selected from the group consisting of D14 to D16, and D730 to D732. In one aspect of the present invention, the donor group that $R^1$ to $R^5$ can take is selected from the group consisting of D17 to D87, and D733 to D803. In one aspect of the present invention, the donor group that $R^1$ to $R^5$ can take is selected from the group consisting of D88 to D123, and D804 to D839. In one aspect of the present invention, the donor group that $R^1$ to $R^5$ can take is selected from the group consisting of D124 to D189, and D840 to D905. In one aspect of the present invention, the donor group that $R^1$ to $R^5$ can take is selected from the group consisting of D190 to D363, D452 to D459, D906 to D1079, and D1168 to D1175. In one aspect of the present invention, the donor group that $R^1$ to $R^5$ can take is selected from the group consisting of D364 to D451, and D1080 to D1167. In one aspect of the present invention, the donor group that $R^1$ to $R^5$ can take is selected from the group consisting of D460 to D716.

One or more of $R^1$ to $R^5$ in the general formula (1) represent a donor group. In one aspect of the present invention, one to three of $R^1$ to $R^5$ represent a donor group. In one aspect of the present invention, one, two or three of $R^1$ to $R^5$ represent a donor group. In one aspect of the present invention, one of $R^1$ to $R^5$ represents a donor group. In one preferred aspect of the present invention, two of $R^1$ to $R^5$ represent a donor group. In one aspect of the present invention, three of $R^1$ to $R^5$ represent a donor group. In one aspect of the present invention, at least $R^1$ represents a donor group. In one aspect of the present invention, at least $R^3$ represents a donor group. In one aspect of the present invention, at least $R^4$ represents a donor group. In one aspect of the present invention, at least $R^5$ represents a donor group. In one aspect of the present invention, only $R^1$ represents a donor group. In one aspect of the present invention, only $R^3$ represents a donor group. In one aspect of the present invention, only $R^4$ represents a donor group. In one aspect of the present invention, only $R^5$ represents a donor group. In one preferred aspect of the present invention, only $R^3$ and $R^5$ represent a donor group. In one preferred aspect of the present invention, only $R^2$ and $R^5$ represent a donor group. In one aspect of the present invention, only $R^2$ and $R^4$ represent a donor group. In one aspect of the present invention, only $R^3$, $R^4$ and $R^5$ represent a donor group. In one aspect of the present invention, only $R^2$, $R^4$ and $R^5$ represent a donor group. When two or more of $R^1$ to $R^5$ represent a donor group, these can be the same or different.

The number of $R^1$ to $R^5$ that are a hydrogen atom or a deuterium atom is 0 to 2, preferably 0 or 1, and is, for example, 1, or for example, 0. These show more excellent light emission characteristics than the compounds where the number of $R^1$ to $R^5$ that are a hydrogen atom or a deuterium atom is 3. The number of $R^1$ to $R^5$ that are a substituted or unsubstituted aryl group is 0 or 1, and is preferably 1. The number can be 0. The number of $R^1$ to $R^5$ that are a substituted or unsubstituted alkyl group is 0 to 3, preferably 0 to 2, and can be 1 or can be 0.

In one preferred aspect of the present invention, two of $R^1$ to $R^5$ represent a donor group, one represents a hydrogen atom or a deuterium atom, and one represents a substituted or unsubstituted aryl group. In one aspect of the present invention, two donor groups are the same. In one aspect of the present invention, two donor groups are different from each other. In one aspect of the present invention, $X^5$ or $X^6$ represents N, and $R^1$ represents a hydrogen atom or a deuterium atom. In one preferred aspect of the present invention, $X^5$ represents N, $R^1$ represents a hydrogen atom or a deuterium atom, $R^3$ and $R^5$ represent a donor group, and $R^4$ represents a substituted or unsubstituted aryl group. In one aspect of the present invention, $X^5$ represents N, $R^1$ represent a hydrogen atom or a deuterium atom, $R^4$ and $R^5$ represent a donor group, and $R^3$ represents a substituted or unsubstituted aryl group. In one aspect of the present invention, $X^5$ represents N, $R^1$ represents a hydrogen atom or a deuterium atom, $R^3$ and $R^4$ represent a donor group, and $R^5$ represents a substituted or unsubstituted aryl group. In one preferred aspect of the present invention, $X^6$ represents N, $R^1$ represents a hydrogen atom or a deuterium atom, $R^2$ and $R^5$ represent a donor group, and $R^4$ represents a substituted or unsubstituted aryl group. In one aspect of the present invention, $X^6$ represents N, $R^1$ represents a hydrogen atom or a deuterium atom, $R^4$ and $R^5$ represent a donor group, and $R^2$ represents a substituted or unsubstituted aryl group.

In one aspect of the present invention, three of $R^1$ to $R^5$ represent a donor group, and one represents a hydrogen atom or a deuterium atom. In one aspect of the present invention, three donor groups are the same. In one aspect of the present invention, two of the three donor groups are the same, and one differs. In one aspect of the present invention, $X^5$ represents N, $R^1$ represents a hydrogen atom or a deuterium atom, and $R^3$ to $R^5$ represent a donor group. In one aspect of the present invention, $X^6$ represents N, $R^1$ represents a hydrogen atom or a deuterium atom, and $R^2$, $R^4$ and $R^5$ represent a donor group.

In one aspect of the present invention, one of $R^1$ to $R^5$ represents a donor group, one represents a substituted or unsubstituted aryl group, and two represent a hydrogen atom or a deuterium atom. In one aspect of the present invention, $X^5$ represents N, $R^1$ and $R^3$ represent a hydrogen atom or a deuterium atom, $R^4$ represents a substituted or unsubstituted aryl group, and $R^5$ represents a donor group. In one aspect of the present invention, $X^5$ represents N, $R^1$ and $R^3$ represent a hydrogen atom or a deuterium atom, $R^4$ represents a donor group, and $R^5$ represents a substituted or unsubstituted aryl group. In one aspect of the present invention, $X^5$ represents N, $R^1$ and $R^5$ represent a hydrogen atom or a deuterium atom, $R^3$ represents a donor group, and $R^4$ represents a substituted or unsubstituted aryl group. In one aspect of the present invention, $X^5$ represents N, $R^1$ and $R^4$ represent a hydrogen atom or a deuterium atom, $R^3$ represents a donor group, and $R^5$ represents a substituted or unsubstituted aryl group. In one aspect of the present invention, $X^5$ represents N, $R^1$ represents a donor group, $R^3$ and $R^5$ represent a hydrogen atom or a deuterium atom, and $R^4$ represents a substituted or unsubstituted aryl group. In one aspect of the present invention, $X^5$ represents N, $R^1$ represents a donor group, $R^3$ and $R^4$ represent a hydrogen atom or a deuterium atom, and $R^5$ represents a substituted or unsubstituted aryl group.

In one preferred aspect of the present invention, two of $R^1$ to $R^5$ represent a donor group, and two represent a hydrogen atom or a deuterium atom. In one aspect of the present invention, $X^5$ represents N, $R^1$ and $R^4$ represent a hydrogen atom or a deuterium atom, and $R^3$ and $R^5$ represent a donor group. In one aspect of the present invention, $X^5$ represents N, $R^1$ and $R^5$ represent a hydrogen atom or a deuterium atom, and $R^3$ and $R^4$ represent a donor group. In one aspect of the present invention, $X^5$ represents N, $R^1$ and $R^3$ represent a hydrogen atom or a deuterium atom, and $R^4$ and $R^5$ represent a donor group. In one preferred aspect of the present invention, $X^6$ represents N, $R^1$ and $R^4$ represent a hydrogen atom or a deuterium atom, and $R^2$ and $R^5$ represent a donor group. In one aspect of the present invention, $X^6$ represents N, $R^1$ and $R^2$ represent a hydrogen atom or a deuterium atom, and $R^4$ and $R^5$ represent a donor group.

In one aspect of the present invention, one of $R^1$ to $R^5$ represents a donor group, and three represent a hydrogen atom or a deuterium atom. In one aspect of the present invention, $X^5$ represents N, $R^1$, $R^3$ and $R^4$ represent a hydrogen atom or a deuterium atom, and $R^5$ represents a donor group. In one aspect of the present invention, $X^5$ represents N, $R^1$, $R^3$ and $R^5$ represent a hydrogen atom or a deuterium atom, and $R^4$ represents a donor group. In one aspect of the present invention, $X^5$ represents N, $R^1$, $R^4$ and $R^5$ represent a hydrogen atom or a deuterium atom, and $R^3$ represents a donor group. In one aspect of the present invention, $X^5$ represents N, $R^1$ represents a donor group, and $R^3$, $R^4$ and $R^5$ represent a hydrogen atom or a deuterium atom.

In one aspect of the present invention, $R^3$ to $R^5$ each independently represent a substituted or unsubstituted aryl group, or a donor group. In one aspect of the present invention, two of $R^3$ to $R^5$ represent a donor group, and one represents a substituted or unsubstituted aryl group. In one preferred aspect of the present invention, $R^3$ and $R^5$ represent a donor group, and $R^4$ represents a substituted or unsubstituted aryl group. In one aspect of the present invention, $R^3$, $R^4$ and $R^5$ represent a donor group.

In one aspect of the present invention, $R^2$, $R^4$ and $R^5$ each independently represent a substituted or unsubstituted aryl group, or a donor group. In one aspect of the present invention, two of $R^2$, $R^4$ and $R^5$ represent a donor group, and one represents a substituted or unsubstituted aryl group. In one preferred aspect of the present invention, $R^2$ and $R^5$ represent a donor group, and $R^4$ represents a substituted or unsubstituted aryl group. In one aspect of the present invention, $R^2$, $R^4$ and $R^5$ represent a donor group.

The heteroaryl group that $Ar^1$ and $Ar^2$ in the general formula (1) can take each can be a monocyclic ring or can be a fused ring of two or more kinds of rings. In the case of a fused ring, the number of fused rings is preferably 2 to 6, and can be selected from, for example, 2 to 4. Specific examples of the ring include a pyridine ring, a pyrimidine ring and a pyrrole ring, and these rings can be fused with any other ring. Specific examples of the heteroaryl group include a 2-pyridyl group, a 3-pyridyl group, a 4-pyridyl group, a carbazol-9-yl group, a carbazol-1-yl group, a carbazol-2-yl group, a carbazol-3-yl group, and a carbazol-4-yl group. The number of the ring skeleton-constituting atoms of the heteroaryl group is preferably 4 to 40, more preferably 5 to 20, and can be selected from a range of 5 to 16, or can be selected from a range of 5 to 12.

At least one of $Ar^1$ an $Ar^2$ in the general formula (1) represents a substituted or unsubstituted heteroaryl group bonding via a nitrogen atom. Specifically, the group is a substituted or unsubstituted heteroaryl group containing a nitrogen atom as a ring skeleton-constituting atom, which bonds via the nitrogen atom which is one of the ring skeleton-constituting atoms. A typical example of the group is a substituted or unsubstituted pyrrol-1-yl group, preferably a substituted or unsubstituted ring-fused pyrrol-1-yl group, more preferably a substituted or unsubstituted carbazol-9-yl group, of which the carbazole skeleton can be fused with any other ring. Specific examples of the substituted or unsubstituted heteroaryl group bonding via a nitrogen atom, which at least one of $Ar^1$ and $Ar^2$ can take, include the above-mentioned D1 to D1175.

In one aspect of the present invention, the group that at least one of $Ar^1$ and $Ar^2$ can take is selected from the group consisting of D1 to D13, and D717 to D729. In one aspect of the present invention, the group that at least one of $Ar^1$ and $Ar^2$ can take is selected from the group consisting of D14 to D16, and D730 to D732. In one aspect of the present invention, the group that at least one of $Ar^1$ and $Ar^2$ can take is selected from the group consisting of D17 to D87, and D733 to D803. In one aspect of the present invention, the group that at least one of $Ar^1$ and $Ar^2$ can take is selected from the group consisting of D88 to D123, and D804 to D839. In one aspect of the present invention, the group that at least one of $Ar^1$ and $Ar^2$ can take is selected from the group consisting of D124 to D189, and D840 to D905. In one aspect of the present

invention, the group that at least one of $Ar^1$ and $Ar^2$ can take is selected from the group consisting of D190 to D363, D452 to D459, D906 to D1079, and D1168 to D1175. In one aspect of the present invention, the group that at least one of $Ar^1$ and $Ar^2$ can take is selected from the group consisting of D364 to D451, and D1080 to D1167. In one aspect of the present invention, the group that at least one of $Ar^1$ and $Ar^2$ can take is selected from the group consisting of D460 to D716.

In one aspect of the present invention, $Ar^1$ and $Ar^2$ each independently represent a substituted or unsubstituted heteroaryl group bonding via a nitrogen atom. In one aspect of the present invention, $Ar^1$ and $Ar^2$ represent a substituted or unsubstituted heteroaryl group bonding via a nitrogen atom, which have the same structure. In one aspect of the present invention, only $Ar^1$ represents a substituted or unsubstituted heteroaryl group bonding via a nitrogen atom, and $Ar^2$ represents a substituted or unsubstituted aryl group.

In the general formula (1), $L^1$ represents a single bond or a divalent linking group. The divalent linking group includes a substituted or unsubstituted arylene group, and a substituted or unsubstituted heteroarylene group. In one preferred aspect of the present invention, $L^1$ represents a single bond. In one aspect of the present invention, $L^1$ represents a substituted or unsubstituted arylene group. In one aspect of the present invention, $L^1$ represents a substituted or unsubstituted heteroarylene group. Regarding the aryl moiety constituting the arylene group, reference can be made to the description and the preferred range of the aryl group in the description section of $R^1$ to $R^5$ mentioned above. The heteroarylene group includes a linking group formed by substituting at least one ring skeleton carbon atom constituting the arylene group with a nitrogen atom.

Specific examples of $L^1$ are shown below. $L^1$ which can be employed in the present invention shall not be construed as being limited by the following specific examples. In the following specific examples, expression of a methyl group is omitted. Consequently, for example, L3 to L5 are substituted with a methyl group. * indicates a bonding site. L1 represents a single bond.

none (direct bond)

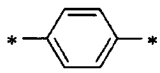 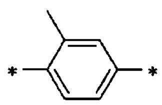 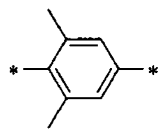 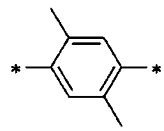

L1 L2 L3 L4 L5

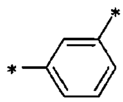 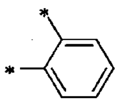 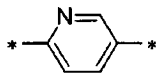 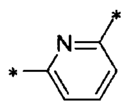 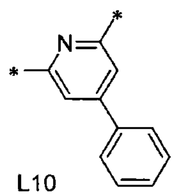

L6 L7 L8 L9 L10

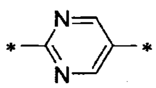 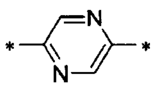 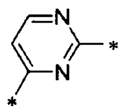 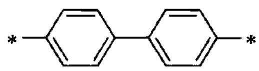 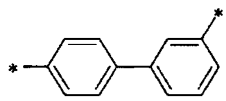

L11 L12 L13 L14 L15

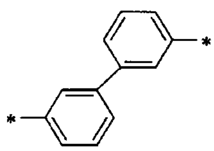 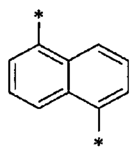 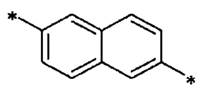 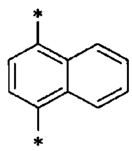 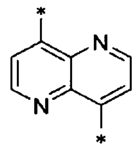

L16 L17 L18 L19 L20

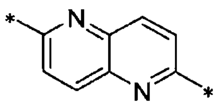

L21

Groups obtained by substituting all hydrogen atoms present in the above L2 to L21 with deuterium atoms are disclosed as L22 to L41. In one aspect of the present invention, $L^1$ is selected from the group consisting of L1 to L7, and L22 to L27. In one aspect of the present invention, $L^1$ is selected from the group consisting of L2 to L7, and L22 to L27. In one aspect of the present invention, $L^1$ is selected from the group consisting of L1, L8 to L13, L20, L21, L28 to L33, L40 and L41. In one aspect of the present invention, $L^1$ is selected from the group consisting of L8 to L13, L20, L21, L28 to L33, L40 and L41. In one aspect of the present invention, $L^1$ is selected from the group consisting of L1, L14 to L19, and L34 to L39. In one aspect of the present invention, $L^1$ is selected from the group consisting of L14 to L19, and L34 to L39.

In one preferred aspect of the present invention, $X^1$ to $X^3$ and $X^5$ represent N, $L^1$ represents a single bond, $Ar^1$ and $Ar^2$ each independently represent a substituted or unsubstituted heteroaryl group bonding via a nitrogen atom (preferably, a substituted or unsubstituted carbazol-9-yl group), two of $R^1$ and $R^3$ to $R^5$ represent a donor group (preferably, a substituted or unsubstituted carbazol-9-yl group), one represents a substituted or unsubstituted aryl group, and one (preferably $R^1$) represents a hydrogen atom or a deuterium atom.

In one preferred aspect of the present invention, $X^1$ to $X^3$ and $X^5$ represent N, $L^1$ represents a single bond, $Ar^1$ represents a substituted or unsubstituted heteroaryl group bonding via a nitrogen atom (preferably, a substituted or unsubstituted carbazol-9-yl group), $Ar^2$ represents a substituted or unsubstituted aryl group, two of $R^1$ and $R^3$ to $R^5$ represent a donor group (preferably, a substituted or unsubstituted carbazol-9-yl group), one represents a substituted or unsubstituted aryl group, and one (preferably $R^1$) represents a hydrogen atom or a deuterium atom.

In one aspect of the present invention, $X^1$ to $X^3$ and $X^5$ represent N, $L^1$ represents a single bond, $Ar^1$ and $Ar^2$ each independently represent a substituted or unsubstituted heteroaryl group bonding via a nitrogen atom (preferably, a substituted or unsubstituted carbazol-9-yl group), three of $R^1$ and $R^3$ to $R^5$ represent a donor group (preferably, a substituted or unsubstituted carbazol-9-yl group), and one (preferably $R^1$) represents a hydrogen atom or a deuterium atom.

In one aspect of the present invention, $X^1$ to $X^3$ and $X^5$ represent N, $L^1$ represents a single bond, $Ar^1$ represents a substituted or unsubstituted heteroaryl group bonding via a nitrogen atom (preferably, a substituted or unsubstituted carbazol-9-yl group), $Ar^2$ represents a substituted or unsubstituted aryl group, three of $R^1$ and $R^3$ to $R^3$ represent a donor group (preferably, a substituted or unsubstituted carbazol-9-yl group), and one (preferably $R^1$) represents a hydrogen atom or a deuterium atom.

In one aspect of the present invention, $X^1$ to $X^3$ and $X^6$ represent N, $L^1$ represents a single bond, $Ar^1$ and $Ar^2$ each independently represent a substituted or unsubstituted heteroaryl group bonding via a nitrogen atom (preferably, a substituted or unsubstituted carbazol-9-yl group), two or three of $R^1$, $R^2$, $R^4$ and $R^5$ represent a donor group (preferably, a substituted or unsubstituted carbazol-9-yl group), one or two (preferably at least $R^1$) represent a hydrogen atom or a deuterium atom, and zero or one represents a substituted or unsubstituted aryl group.

In one aspect of the present invention, $X^1$ to $X^3$ and $X^6$ represent N, $L^1$ represents a single bond, $Ar^1$ represents a substituted or unsubstituted heteroaryl group bonding via a nitrogen atom (preferably, a substituted or unsubstituted carbazol-9-yl group), $Ar^2$ represents a substituted or unsubstituted aryl group, two or three of $R^1$, $R^2$, $R^4$ and $R^5$ represent a donor group (preferably, a substituted or unsubstituted carbazol-9-yl group), one or two (preferably at least $R^1$) represent a hydrogen atom or a deuterium atom, and zero or one represents a substituted or unsubstituted aryl group.

In one preferred aspect of the present invention, $X^1$ to $X^3$ and $X^6$ represent N, $L^1$ represents a single bond, $Ar^1$ and $Ar^2$ each independently represent a substituted or unsubstituted heteroaryl group bonding via a nitrogen atom (preferably, a substituted or unsubstituted carbazol-9-yl group), two of $R^1$, $R^2$, $R^4$ and $R^5$ represent a donor group (preferably, a substituted or unsubstituted carbazol-9-yl group), one represents a substituted or unsubstituted aryl group, and one (preferably at least $R^1$) represents a hydrogen atom or a deuterium atom.

In one preferred aspect of the present invention, $X^1$ to $X^3$ and $X^6$ represent N, $L^1$ represents a single bond, $Ar^1$ represents a substituted or unsubstituted heteroaryl group bonding via a nitrogen atom (preferably, a substituted or unsubstituted carbazol-9-yl group), $Ar^2$ represents a substituted or unsubstituted aryl group, two of $R^1$, $R^2$, $R^4$ and $R^5$ represent a donor group (preferably, a substituted or unsubstituted carbazol-9-yl group), one represents a substituted or unsubstituted aryl group, and one (preferably at least $R^1$) represents a hydrogen atom or a deuterium atom.

In one preferred aspect of the present invention, $X^1$ to $X^3$ and $X^6$ represents N, $L^1$ represents a single bond, $Ar^1$ and $Ar^2$ each independently represent a substituted or unsubstituted heteroaryl group bonding via a nitrogen atom (preferably, a substituted or unsubstituted carbazol-9-yl group), two of $R^1$, $R^2$, $R^4$ and $R^5$ represent a donor group (preferably, a substituted or unsubstituted carbazol-9-yl group), and two (preferably at least $R^1$) represents a hydrogen

atom or a deuterium atom.

In one preferred aspect of the present invention, $X^1$ to $X^3$ and $X^6$ represent N, $L^1$ represents a single bond, $Ar^1$ represents a substituted or unsubstituted heteroaryl group bonding via a nitrogen atom (preferably, a substituted or unsubstituted carbazol-9-yl group), $Ar^2$ represents a substituted or unsubstituted aryl group, two of $R^1$, $R^2$, $R^4$ and $R^5$ represent a donor group (preferably, a substituted or unsubstituted carbazol-9-yl group), and two (preferably at least $R^1$) represents a hydrogen atom or a deuterium atom.

The compound represented by the general formula (1) preferably does not contain a metal atom, and can be a compound composed only of atoms selected from the group consisting of a carbon atom, a hydrogen atom, a deuterium atom, a nitrogen atom, an oxygen atom, and a sulfur atom. In one preferred aspect of the present invention, the compound represented by the general formula (1) is composed only of atoms selected from the group consisting of a carbon atom, a hydrogen atom, a deuterium atom, a nitrogen atom, and an oxygen atom. In addition, the compound represented by the general formula (1) can be a compound composed only of atoms selected from the group consisting of a carbon atom, a hydrogen atom, a deuterium atom, a nitrogen atom, and a sulfur atom. The compound represented by the general formula (1) can be a compound composed only of atoms selected from the group consisting of a carbon atom, a hydrogen atom, a deuterium atom, and a nitrogen atom. The compound represented by the general formula (1) can be a compound composed only of atoms selected from the group consisting of a carbon atom, a hydrogen atom, and a nitrogen atom. Further, the compound represented by the general formula (1) can be a compound which does not contain a hydrogen atom but contains a deuterium atom.

In the description herein, the term "Substituent Group A" means one atom or group or a combination of two or more thereof selected from the group consisting of a deuterium atom, a hydroxyl group, a halogen atom (for example, a fluorine atom, a chlorine atom, a bromine atom, and an iodine atom), an alkyl group (for example, having 1 to 40 carbon atoms), an alkoxy group (for example, having 1 to 40 carbon atoms), an alkylthio group (for example, having 1 to 40 carbon atoms), an aryl group (for example, having 6 to 30 carbon atoms), an aryloxy group (for example, having 6 to 30 carbon atoms), an arylthio group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), a heteroarylthio group (for example, having 5 to 30 ring skeleton-constituting atoms), an acyl group (for example, having 1 to 40 carbon atoms), an alkenyl group (for example, having 1 to 40 carbon atoms), an alkynyl group (for example, having 1 to 40 carbon atoms), an alkoxycarbonyl group (for example, having 1 to 40 carbon atoms), an aryloxycarbonyl group (for example, having 7 to 40 carbon atoms), a heteroaryloxycarbonyl group (for example, having 5 to 40 carbon atoms), a silyl group (for example, a trialkylsilyl group having 1 to 40 carbon atoms), and a nitro group.

In the description herein, the term "Substituent Group B" means one atom or group or a combination of two or more thereof selected from the group consisting of a deuterium atom, an alkyl group (for example, having 1 to 40 carbon atoms), an alkoxy group (for example, having 1 to 40 carbon atoms), an aryl group (for example, having 6 to 30 carbon atoms), an aryloxy group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), and a diarylamino group (for example, having 12 to 20 carbon atoms).

In the description herein, the term "Substituent Group C" means one atom or group or a combination of two or more thereof selected from the group consisting of a deuterium atom, an alkyl group (for example, having 1 to 20 carbon atoms), an aryl group (for example, having 6 to 22 carbon atoms), a heteroaryl group (for example, having 5 to 20 ring skeleton-constituting atoms), and a diarylamino group (for example, having 12 to 20 carbon atoms).

In the description herein, the term "Substituent Group D" means one atom or group or a combination of two or more thereof selected from the group consisting of a deuterium atom, an alkyl group (for example, having 1 to 20 carbon atoms), an aryl group (for example, having 6 to 22 carbon atoms), and a heteroaryl group (for example, having 5 to 20 ring skeleton-constituting atoms).

In the description herein, the term "Substituent Group E" means one atom or group or a combination of two or more groups selected from the group consisting of a deuterium atom, an alkyl group (for example, having 1 to 20 carbon atoms), and an aryl group (for example, having 6 to 22 carbon atoms).

In the description herein, the substituent meant by an expression of "substituted or unsubstituted" or "optionally substituted" can be selected, for example, from Substituent Group A, can be selected from Substituent Group B, can be selected from Substituent Group C, can be selected from Substituent Group D, or can be selected from Substituent Group E.

Specific examples of the compound represented by the general formula (1) are shown in the following Tables 1 to 5. The compound represented by the general formula (1) that can be used in the present invention should not be construed as being limited by these specific examples.

In Table 1, structures of the compounds are individually shown by specifying $R^3$ to $R^5$ of the following general formula (1a) for each compound. Specifically, the structures where $Ar^1$ and $Ar^2$ represent a deuterated carbazolyl group (D717), $X^1$ to $X^3$ represent a nitrogen atom (N), $L^1$ represents a single bond, $X^5$ represents N, $R^1$ represents a hydrogen atom, and $R^3$ to $R^5$ represent a group specified in Table 1 are individually shown as structures of Compounds 1 to 1175.

General Formula (1a)

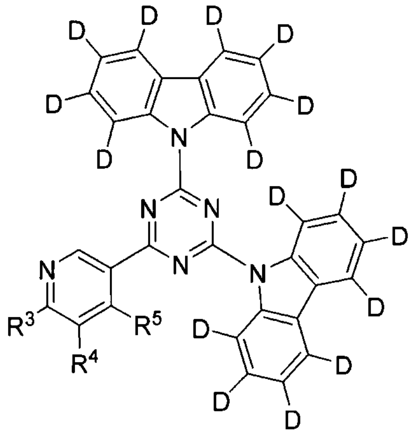

In Table 2, structures of Compounds 1 to 1015655 are shown by collectively displaying $R^3$ to $R^5$ of a plurality of compounds in each row. For example, in the row of Compounds 1 to 1175 in Table 2, compounds in which $R^4$ is fixed to Ar1, and $R^3$ and $R^5$ both represent D1 to D1175 are referred to as Compounds 1 to 1175 in that order. $R^3$ and $R^5$ are the same. That is, the row of Compounds 1 to 1175 in Table 2 collectively represents Compounds 1 to 1175 specified in Table 1. Similarly, in the row of Compounds 1176 to 2350 in Table 2, those in which $R^4$ is fixed to Ar2, and $R^3$ and $R^5$ both represent D1 to D1175 are referred to as Compounds 1176 to 2350 in that order. In the same manner, Compounds 2351 to 1015655 in Table 2 are also specified.

[Table 1]

| No. | $R^3$ | $R^4$ | $R^5$ |
|---|---|---|---|
| 1 | D1 | Ar1 | D1 |
| 2 | D2 | Ar1 | D2 |
| 3 | D3 | Ar1 | D3 |
| 4 | D4 | Ar1 | D4 |
| 5 | D5 | Ar1 | D5 |
| 6 | D6 | Ar1 | D6 |
| 7 | D7 | Ar1 | D7 |
| 8 | D8 | Ar1 | D8 |
| 9 | D9 | Ar1 | D9 |
| 10 | D10 | Ar1 | D10 |
| 11 | D11 | Ar1 | D11 |
| 12 | D12 | Ar1 | D12 |
| 13 | D13 | Ar1 | D13 |
| 14 | D14 | Ar1 | D14 |
| 15 | D15 | Ar1 | D15 |
| 16 | D16 | Ar1 | D16 |
| 17 | D17 | Ar1 | D17 |
| 18 | D18 | Ar1 | D18 |
| 19 | D19 | Ar1 | D19 |
| 20 | D20 | Ar1 | D20 |
| 21 | D21 | Ar1 | D21 |
| 22 | D22 | Ar1 | D22 |
| 23 | D23 | Ar1 | D23 |
| 24 | D24 | Ar1 | D24 |
| 25 | D25 | Ar1 | D25 |
| 26 | D26 | Ar1 | D26 |
| 27 | D27 | Ar1 | D27 |
| 28 | D28 | Ar1 | D28 |
| 29 | D29 | Ar1 | D29 |
| 30 | D30 | Ar1 | D30 |
| 31 | D31 | Ar1 | D31 |
| 32 | D32 | Ar1 | D32 |
| 33 | D33 | Ar1 | D33 |
| 34 | D34 | Ar1 | D34 |
| 35 | D35 | Ar1 | D35 |
| 36 | D36 | Ar1 | D36 |
| 37 | D37 | Ar1 | D37 |
| 38 | D38 | Ar1 | D38 |
| 39 | D39 | Ar1 | D39 |
| 40 | D40 | Ar1 | D40 |
| 41 | D41 | Ar1 | D41 |
| 42 | D42 | Ar1 | D42 |
| 43 | D43 | Ar1 | D43 |
| 44 | D44 | Ar1 | D44 |
| 45 | D45 | Ar1 | D45 |
| 46 | D46 | Ar1 | D46 |
| 47 | D47 | Ar1 | D47 |
| 48 | D48 | Ar1 | D48 |
| 49 | D49 | Ar1 | D49 |
| 50 | D50 | Ar1 | D50 |
| 51 | D51 | Ar1 | D51 |
| 52 | D52 | Ar1 | D52 |
| 53 | D53 | Ar1 | D53 |
| 54 | D54 | Ar1 | D54 |
| 55 | D55 | Ar1 | D55 |
| 56 | D56 | Ar1 | D56 |
| 57 | D57 | Ar1 | D57 |
| 58 | D58 | Ar1 | D58 |
| 59 | D59 | Ar1 | D59 |
| 60 | D60 | Ar1 | D60 |

| No. | $R^3$ | $R^4$ | $R^5$ |
|---|---|---|---|
| 61 | D61 | Ar1 | D61 |
| 62 | D62 | Ar1 | D62 |
| 63 | D63 | Ar1 | D63 |
| 64 | D64 | Ar1 | D64 |
| 65 | D65 | Ar1 | D65 |
| 66 | D66 | Ar1 | D66 |
| 67 | D67 | Ar1 | D67 |
| 68 | D68 | Ar1 | D68 |
| 69 | D69 | Ar1 | D69 |
| 70 | D70 | Ar1 | D70 |
| 71 | D71 | Ar1 | D71 |
| 72 | D72 | Ar1 | D72 |
| 73 | D73 | Ar1 | D73 |
| 74 | D74 | Ar1 | D74 |
| 75 | D75 | Ar1 | D75 |
| 76 | D76 | Ar1 | D76 |
| 77 | D77 | Ar1 | D77 |
| 78 | D78 | Ar1 | D78 |
| 79 | D79 | Ar1 | D79 |
| 80 | D80 | Ar1 | D80 |
| 81 | D81 | Ar1 | D81 |
| 82 | D82 | Ar1 | D82 |
| 83 | D83 | Ar1 | D83 |
| 84 | D84 | Ar1 | D84 |
| 85 | D85 | Ar1 | D85 |
| 86 | D86 | Ar1 | D86 |
| 87 | D87 | Ar1 | D87 |
| 88 | D88 | Ar1 | D88 |
| 89 | D89 | Ar1 | D89 |
| 90 | D90 | Ar1 | D90 |
| 91 | D91 | Ar1 | D91 |
| 92 | D92 | Ar1 | D92 |
| 93 | D93 | Ar1 | D93 |
| 94 | D94 | Ar1 | D94 |
| 95 | D95 | Ar1 | D95 |
| 96 | D96 | Ar1 | D96 |
| 97 | D97 | Ar1 | D97 |
| 98 | D98 | Ar1 | D98 |
| 99 | D99 | Ar1 | D99 |
| 100 | D100 | Ar1 | D100 |
| 101 | D101 | Ar1 | D101 |
| 102 | D102 | Ar1 | D102 |
| 103 | D103 | Ar1 | D103 |
| 104 | D104 | Ar1 | D104 |
| 105 | D105 | Ar1 | D105 |
| 106 | D106 | Ar1 | D106 |
| 107 | D107 | Ar1 | D107 |
| 108 | D108 | Ar1 | D108 |
| 109 | D109 | Ar1 | D109 |
| 110 | D110 | Ar1 | D110 |
| 111 | D111 | Ar1 | D111 |
| 112 | D112 | Ar1 | D112 |
| 113 | D113 | Ar1 | D113 |
| 114 | D114 | Ar1 | D114 |
| 115 | D115 | Ar1 | D115 |
| 116 | D116 | Ar1 | D116 |
| 117 | D117 | Ar1 | D117 |
| 118 | D118 | Ar1 | D118 |
| 119 | D119 | Ar1 | D119 |
| 120 | D120 | Ar1 | D120 |

| No. | $R^3$ | $R^4$ | $R^5$ |
|---|---|---|---|
| 121 | D121 | Ar1 | D121 |
| 122 | D122 | Ar1 | D122 |
| 123 | D123 | Ar1 | D123 |
| 124 | D124 | Ar1 | D124 |
| 125 | D125 | Ar1 | D125 |
| 126 | D126 | Ar1 | D126 |
| 127 | D127 | Ar1 | D127 |
| 128 | D128 | Ar1 | D128 |
| 129 | D129 | Ar1 | D129 |
| 130 | D130 | Ar1 | D130 |
| 131 | D131 | Ar1 | D131 |
| 132 | D132 | Ar1 | D132 |
| 133 | D133 | Ar1 | D133 |
| 134 | D134 | Ar1 | D134 |
| 135 | D135 | Ar1 | D135 |
| 136 | D136 | Ar1 | D136 |
| 137 | D137 | Ar1 | D137 |
| 138 | D138 | Ar1 | D138 |
| 139 | D139 | Ar1 | D139 |
| 140 | D140 | Ar1 | D140 |
| 141 | D141 | Ar1 | D141 |
| 142 | D142 | Ar1 | D142 |
| 143 | D143 | Ar1 | D143 |
| 144 | D144 | Ar1 | D144 |
| 145 | D145 | Ar1 | D145 |
| 146 | D146 | Ar1 | D146 |
| 147 | D147 | Ar1 | D147 |
| 148 | D148 | Ar1 | D148 |
| 149 | D149 | Ar1 | D149 |
| 150 | D150 | Ar1 | D150 |
| 151 | D151 | Ar1 | D151 |
| 152 | D152 | Ar1 | D152 |
| 153 | D153 | Ar1 | D153 |
| 154 | D154 | Ar1 | D154 |
| 155 | D155 | Ar1 | D155 |
| 156 | D156 | Ar1 | D156 |
| 157 | D157 | Ar1 | D157 |
| 158 | D158 | Ar1 | D158 |
| 159 | D159 | Ar1 | D159 |
| 160 | D160 | Ar1 | D160 |
| 161 | D161 | Ar1 | D161 |
| 162 | D162 | Ar1 | D162 |
| 163 | D163 | Ar1 | D163 |
| 164 | D164 | Ar1 | D164 |
| 165 | D165 | Ar1 | D165 |
| 166 | D166 | Ar1 | D166 |
| 167 | D167 | Ar1 | D167 |
| 168 | D168 | Ar1 | D168 |
| 169 | D169 | Ar1 | D169 |
| 170 | D170 | Ar1 | D170 |
| 171 | D171 | Ar1 | D171 |
| 172 | D172 | Ar1 | D172 |
| 173 | D173 | Ar1 | D173 |
| 174 | D174 | Ar1 | D174 |
| 175 | D175 | Ar1 | D175 |
| 176 | D176 | Ar1 | D176 |
| 177 | D177 | Ar1 | D177 |
| 178 | D178 | Ar1 | D178 |
| 179 | D179 | Ar1 | D179 |
| 180 | D180 | Ar1 | D180 |

| No. | $R^3$ | $R^4$ | $R^5$ |
|---|---|---|---|
| 181 | D181 | Ar1 | D181 |
| 182 | D182 | Ar1 | D182 |
| 183 | D183 | Ar1 | D183 |
| 184 | D184 | Ar1 | D184 |
| 185 | D185 | Ar1 | D185 |
| 186 | D186 | Ar1 | D186 |
| 187 | D187 | Ar1 | D187 |
| 188 | D188 | Ar1 | D188 |
| 189 | D189 | Ar1 | D189 |
| 190 | D190 | Ar1 | D190 |
| 191 | D191 | Ar1 | D191 |
| 192 | D192 | Ar1 | D192 |
| 193 | D193 | Ar1 | D193 |
| 194 | D194 | Ar1 | D194 |
| 195 | D195 | Ar1 | D195 |
| 196 | D196 | Ar1 | D196 |
| 197 | D197 | Ar1 | D197 |
| 198 | D198 | Ar1 | D198 |
| 199 | D199 | Ar1 | D199 |
| 200 | D200 | Ar1 | D200 |
| 201 | D201 | Ar1 | D201 |
| 202 | D202 | Ar1 | D202 |
| 203 | D203 | Ar1 | D203 |
| 204 | D204 | Ar1 | D204 |
| 205 | D205 | Ar1 | D205 |
| 206 | D206 | Ar1 | D206 |
| 207 | D207 | Ar1 | D207 |
| 208 | D208 | Ar1 | D208 |
| 209 | D209 | Ar1 | D209 |
| 210 | D210 | Ar1 | D210 |
| 211 | D211 | Ar1 | D211 |
| 212 | D212 | Ar1 | D212 |
| 213 | D213 | Ar1 | D213 |
| 214 | D214 | Ar1 | D214 |
| 215 | D215 | Ar1 | D215 |
| 216 | D216 | Ar1 | D216 |
| 217 | D217 | Ar1 | D217 |
| 218 | D218 | Ar1 | D218 |
| 219 | D219 | Ar1 | D219 |
| 220 | D220 | Ar1 | D220 |
| 221 | D221 | Ar1 | D221 |
| 222 | D222 | Ar1 | D222 |
| 223 | D223 | Ar1 | D223 |
| 224 | D224 | Ar1 | D224 |
| 225 | D225 | Ar1 | D225 |
| 226 | D226 | Ar1 | D226 |
| 227 | D227 | Ar1 | D227 |
| 228 | D228 | Ar1 | D228 |
| 229 | D229 | Ar1 | D229 |
| 230 | D230 | Ar1 | D230 |
| 231 | D231 | Ar1 | D231 |
| 232 | D232 | Ar1 | D232 |
| 233 | D233 | Ar1 | D233 |
| 234 | D234 | Ar1 | D234 |
| 235 | D235 | Ar1 | D235 |
| 236 | D236 | Ar1 | D236 |
| 237 | D237 | Ar1 | D237 |
| 238 | D238 | Ar1 | D238 |
| 239 | D239 | Ar1 | D239 |
| 240 | D240 | Ar1 | D240 |

| No. | $R^3$ | $R^4$ | $R^5$ |
|---|---|---|---|
| 241 | D241 | Ar1 | D241 |
| 242 | D242 | Ar1 | D242 |
| 243 | D243 | Ar1 | D243 |
| 244 | D244 | Ar1 | D244 |
| 245 | D245 | Ar1 | D245 |
| 246 | D246 | Ar1 | D246 |
| 247 | D247 | Ar1 | D247 |
| 248 | D248 | Ar1 | D248 |
| 249 | D249 | Ar1 | D249 |
| 250 | D250 | Ar1 | D250 |
| 251 | D251 | Ar1 | D251 |
| 252 | D252 | Ar1 | D252 |
| 253 | D253 | Ar1 | D253 |
| 254 | D254 | Ar1 | D254 |
| 255 | D255 | Ar1 | D255 |
| 256 | D256 | Ar1 | D256 |
| 257 | D257 | Ar1 | D257 |
| 258 | D258 | Ar1 | D258 |
| 259 | D259 | Ar1 | D259 |
| 260 | D260 | Ar1 | D260 |
| 261 | D261 | Ar1 | D261 |
| 262 | D262 | Ar1 | D262 |
| 263 | D263 | Ar1 | D263 |
| 264 | D264 | Ar1 | D264 |
| 265 | D265 | Ar1 | D265 |
| 266 | D266 | Ar1 | D266 |
| 267 | D267 | Ar1 | D267 |
| 268 | D268 | Ar1 | D268 |
| 269 | D269 | Ar1 | D269 |
| 270 | D270 | Ar1 | D270 |
| 271 | D271 | Ar1 | D271 |
| 272 | D272 | Ar1 | D272 |
| 273 | D273 | Ar1 | D273 |
| 274 | D274 | Ar1 | D274 |
| 275 | D275 | Ar1 | D275 |
| 276 | D276 | Ar1 | D276 |
| 277 | D277 | Ar1 | D277 |
| 278 | D278 | Ar1 | D278 |
| 279 | D279 | Ar1 | D279 |
| 280 | D280 | Ar1 | D280 |
| 281 | D281 | Ar1 | D281 |
| 282 | D282 | Ar1 | D282 |
| 283 | D283 | Ar1 | D283 |
| 284 | D284 | Ar1 | D284 |
| 285 | D285 | Ar1 | D285 |
| 286 | D286 | Ar1 | D286 |
| 287 | D287 | Ar1 | D287 |
| 288 | D288 | Ar1 | D288 |
| 289 | D289 | Ar1 | D289 |
| 290 | D290 | Ar1 | D290 |
| 291 | D291 | Ar1 | D291 |
| 292 | D292 | Ar1 | D292 |
| 293 | D293 | Ar1 | D293 |
| 294 | D294 | Ar1 | D294 |
| 295 | D295 | Ar1 | D295 |
| 296 | D296 | Ar1 | D296 |
| 297 | D297 | Ar1 | D297 |
| 298 | D298 | Ar1 | D298 |
| 299 | D299 | Ar1 | D299 |
| 300 | D300 | Ar1 | D300 |
| 301 | D301 | Ar1 | D301 |
| 302 | D302 | Ar1 | D302 |
| 303 | D303 | Ar1 | D303 |
| 304 | D304 | Ar1 | D304 |
| 305 | D305 | Ar1 | D305 |
| 306 | D306 | Ar1 | D306 |
| 307 | D307 | Ar1 | D307 |
| 308 | D308 | Ar1 | D308 |
| 309 | D309 | Ar1 | D309 |
| 310 | D310 | Ar1 | D310 |
| 311 | D311 | Ar1 | D311 |
| 312 | D312 | Ar1 | D312 |
| 313 | D313 | Ar1 | D313 |
| 314 | D314 | Ar1 | D314 |
| 315 | D315 | Ar1 | D315 |
| 316 | D316 | Ar1 | D316 |
| 317 | D317 | Ar1 | D317 |
| 318 | D318 | Ar1 | D318 |
| 319 | D319 | Ar1 | D319 |
| 320 | D320 | Ar1 | D320 |
| 321 | D321 | Ar1 | D321 |
| 322 | D322 | Ar1 | D322 |
| 323 | D323 | Ar1 | D323 |
| 324 | D324 | Ar1 | D324 |
| 325 | D325 | Ar1 | D325 |
| 326 | D326 | Ar1 | D326 |
| 327 | D327 | Ar1 | D327 |
| 328 | D328 | Ar1 | D328 |
| 329 | D329 | Ar1 | D329 |
| 330 | D330 | Ar1 | D330 |
| 331 | D331 | Ar1 | D331 |
| 332 | D332 | Ar1 | D332 |
| 333 | D333 | Ar1 | D333 |
| 334 | D334 | Ar1 | D334 |
| 335 | D335 | Ar1 | D335 |
| 336 | D336 | Ar1 | D336 |
| 337 | D337 | Ar1 | D337 |
| 338 | D338 | Ar1 | D338 |
| 339 | D339 | Ar1 | D339 |
| 340 | D340 | Ar1 | D340 |
| 341 | D341 | Ar1 | D341 |
| 342 | D342 | Ar1 | D342 |
| 343 | D343 | Ar1 | D343 |
| 344 | D344 | Ar1 | D344 |
| 345 | D345 | Ar1 | D345 |
| 346 | D346 | Ar1 | D346 |
| 347 | D347 | Ar1 | D347 |
| 348 | D348 | Ar1 | D348 |
| 349 | D349 | Ar1 | D349 |
| 350 | D350 | Ar1 | D350 |
| 351 | D351 | Ar1 | D351 |
| 352 | D352 | Ar1 | D352 |
| 353 | D353 | Ar1 | D353 |
| 354 | D354 | Ar1 | D354 |
| 355 | D355 | Ar1 | D355 |
| 356 | D356 | Ar1 | D356 |
| 357 | D357 | Ar1 | D357 |
| 358 | D358 | Ar1 | D358 |
| 359 | D359 | Ar1 | D359 |
| 360 | D360 | Ar1 | D360 |
| 361 | D361 | Ar1 | D361 |
| 362 | D362 | Ar1 | D362 |
| 363 | D363 | Ar1 | D363 |
| 364 | D364 | Ar1 | D364 |
| 365 | D365 | Ar1 | D365 |
| 366 | D366 | Ar1 | D366 |
| 367 | D367 | Ar1 | D367 |
| 368 | D368 | Ar1 | D368 |
| 369 | D369 | Ar1 | D369 |
| 370 | D370 | Ar1 | D370 |
| 371 | D371 | Ar1 | D371 |
| 372 | D372 | Ar1 | D372 |
| 373 | D373 | Ar1 | D373 |
| 374 | D374 | Ar1 | D374 |
| 375 | D375 | Ar1 | D375 |
| 376 | D376 | Ar1 | D376 |
| 377 | D377 | Ar1 | D377 |
| 378 | D378 | Ar1 | D378 |
| 379 | D379 | Ar1 | D379 |
| 380 | D380 | Ar1 | D380 |
| 381 | D381 | Ar1 | D381 |
| 382 | D382 | Ar1 | D382 |
| 383 | D383 | Ar1 | D383 |
| 384 | D384 | Ar1 | D384 |
| 385 | D385 | Ar1 | D385 |
| 386 | D386 | Ar1 | D386 |
| 387 | D387 | Ar1 | D387 |
| 388 | D388 | Ar1 | D388 |
| 389 | D389 | Ar1 | D389 |
| 390 | D390 | Ar1 | D390 |
| 391 | D391 | Ar1 | D391 |
| 392 | D392 | Ar1 | D392 |
| 393 | D393 | Ar1 | D393 |
| 394 | D394 | Ar1 | D394 |
| 395 | D395 | Ar1 | D395 |
| 396 | D396 | Ar1 | D396 |
| 397 | D397 | Ar1 | D397 |
| 398 | D398 | Ar1 | D398 |
| 399 | D399 | Ar1 | D399 |
| 400 | D400 | Ar1 | D400 |
| 401 | D401 | Ar1 | D401 |
| 402 | D402 | Ar1 | D402 |
| 403 | D403 | Ar1 | D403 |
| 404 | D404 | Ar1 | D404 |
| 405 | D405 | Ar1 | D405 |
| 406 | D406 | Ar1 | D406 |
| 407 | D407 | Ar1 | D407 |
| 408 | D408 | Ar1 | D408 |
| 409 | D409 | Ar1 | D409 |
| 410 | D410 | Ar1 | D410 |
| 411 | D411 | Ar1 | D411 |
| 412 | D412 | Ar1 | D412 |
| 413 | D413 | Ar1 | D413 |
| 414 | D414 | Ar1 | D414 |
| 415 | D415 | Ar1 | D415 |
| 416 | D416 | Ar1 | D416 |
| 417 | D417 | Ar1 | D417 |
| 418 | D418 | Ar1 | D418 |
| 419 | D419 | Ar1 | D419 |
| 420 | D420 | Ar1 | D420 |
| 421 | D421 | Ar1 | D421 |
| 422 | D422 | Ar1 | D422 |
| 423 | D423 | Ar1 | D423 |
| 424 | D424 | Ar1 | D424 |
| 425 | D425 | Ar1 | D425 |
| 426 | D426 | Ar1 | D426 |
| 427 | D427 | Ar1 | D427 |
| 428 | D428 | Ar1 | D428 |
| 429 | D429 | Ar1 | D429 |
| 430 | D430 | Ar1 | D430 |
| 431 | D431 | Ar1 | D431 |
| 432 | D432 | Ar1 | D432 |
| 433 | D433 | Ar1 | D433 |
| 434 | D434 | Ar1 | D434 |
| 435 | D435 | Ar1 | D435 |
| 436 | D436 | Ar1 | D436 |
| 437 | D437 | Ar1 | D437 |
| 438 | D438 | Ar1 | D438 |
| 439 | D439 | Ar1 | D439 |
| 440 | D440 | Ar1 | D440 |
| 441 | D441 | Ar1 | D441 |
| 442 | D442 | Ar1 | D442 |
| 443 | D443 | Ar1 | D443 |
| 444 | D444 | Ar1 | D444 |
| 445 | D445 | Ar1 | D445 |
| 446 | D446 | Ar1 | D446 |
| 447 | D447 | Ar1 | D447 |
| 448 | D448 | Ar1 | D448 |
| 449 | D449 | Ar1 | D449 |
| 450 | D450 | Ar1 | D450 |
| 451 | D451 | Ar1 | D451 |
| 452 | D452 | Ar1 | D452 |
| 453 | D453 | Ar1 | D453 |
| 454 | D454 | Ar1 | D454 |
| 455 | D455 | Ar1 | D455 |
| 456 | D456 | Ar1 | D456 |
| 457 | D457 | Ar1 | D457 |
| 458 | D458 | Ar1 | D458 |
| 459 | D459 | Ar1 | D459 |
| 460 | D460 | Ar1 | D460 |
| 461 | D461 | Ar1 | D461 |
| 462 | D462 | Ar1 | D462 |
| 463 | D463 | Ar1 | D463 |
| 464 | D464 | Ar1 | D464 |
| 465 | D465 | Ar1 | D465 |
| 466 | D466 | Ar1 | D466 |
| 467 | D467 | Ar1 | D467 |
| 468 | D468 | Ar1 | D468 |
| 469 | D469 | Ar1 | D469 |
| 470 | D470 | Ar1 | D470 |
| 471 | D471 | Ar1 | D471 |
| 472 | D472 | Ar1 | D472 |
| 473 | D473 | Ar1 | D473 |
| 474 | D474 | Ar1 | D474 |
| 475 | D475 | Ar1 | D475 |
| 476 | D476 | Ar1 | D476 |
| 477 | D477 | Ar1 | D477 |
| 478 | D478 | Ar1 | D478 |
| 479 | D479 | Ar1 | D479 |
| 480 | D480 | Ar1 | D480 |

| No. | $R^3$ | $R^4$ | $R^5$ |
|---|---|---|---|
| 481 | D481 | Ar1 | D481 |
| 482 | D482 | Ar1 | D482 |
| 483 | D483 | Ar1 | D483 |
| 484 | D484 | Ar1 | D484 |
| 485 | D485 | Ar1 | D485 |
| 486 | D486 | Ar1 | D486 |
| 487 | D487 | Ar1 | D487 |
| 488 | D488 | Ar1 | D488 |
| 489 | D489 | Ar1 | D489 |
| 490 | D490 | Ar1 | D490 |
| 491 | D491 | Ar1 | D491 |
| 492 | D492 | Ar1 | D492 |
| 493 | D493 | Ar1 | D493 |
| 494 | D494 | Ar1 | D494 |
| 495 | D495 | Ar1 | D495 |
| 496 | D496 | Ar1 | D496 |
| 497 | D497 | Ar1 | D497 |
| 498 | D498 | Ar1 | D498 |
| 499 | D499 | Ar1 | D499 |
| 500 | D500 | Ar1 | D500 |
| 501 | D501 | Ar1 | D501 |
| 502 | D502 | Ar1 | D502 |
| 503 | D503 | Ar1 | D503 |
| 504 | D504 | Ar1 | D504 |
| 505 | D505 | Ar1 | D505 |
| 506 | D506 | Ar1 | D506 |
| 507 | D507 | Ar1 | D507 |
| 508 | D508 | Ar1 | D508 |
| 509 | D509 | Ar1 | D509 |
| 510 | D510 | Ar1 | D510 |
| 511 | D511 | Ar1 | D511 |
| 512 | D512 | Ar1 | D512 |
| 513 | D513 | Ar1 | D513 |
| 514 | D514 | Ar1 | D514 |
| 515 | D515 | Ar1 | D515 |
| 516 | D516 | Ar1 | D516 |
| 517 | D517 | Ar1 | D517 |
| 518 | D518 | Ar1 | D518 |
| 519 | D519 | Ar1 | D519 |
| 520 | D520 | Ar1 | D520 |
| 521 | D521 | Ar1 | D521 |
| 522 | D522 | Ar1 | D522 |
| 523 | D523 | Ar1 | D523 |
| 524 | D524 | Ar1 | D524 |
| 525 | D525 | Ar1 | D525 |
| 526 | D526 | Ar1 | D526 |
| 527 | D527 | Ar1 | D527 |
| 528 | D528 | Ar1 | D528 |
| 529 | D529 | Ar1 | D529 |
| 530 | D530 | Ar1 | D530 |
| 531 | D531 | Ar1 | D531 |
| 532 | D532 | Ar1 | D532 |
| 533 | D533 | Ar1 | D533 |
| 534 | D534 | Ar1 | D534 |
| 535 | D535 | Ar1 | D535 |
| 536 | D536 | Ar1 | D536 |
| 537 | D537 | Ar1 | D537 |
| 538 | D538 | Ar1 | D538 |
| 539 | D539 | Ar1 | D539 |
| 540 | D540 | Ar1 | D540 |

| No. | $R^3$ | $R^4$ | $R^5$ |
|---|---|---|---|
| 541 | D541 | Ar1 | D541 |
| 542 | D542 | Ar1 | D542 |
| 543 | D543 | Ar1 | D543 |
| 544 | D544 | Ar1 | D544 |
| 545 | D545 | Ar1 | D545 |
| 546 | D546 | Ar1 | D546 |
| 547 | D547 | Ar1 | D547 |
| 548 | D548 | Ar1 | D548 |
| 549 | D549 | Ar1 | D549 |
| 550 | D550 | Ar1 | D550 |
| 551 | D551 | Ar1 | D551 |
| 552 | D552 | Ar1 | D552 |
| 553 | D553 | Ar1 | D553 |
| 554 | D554 | Ar1 | D554 |
| 555 | D555 | Ar1 | D555 |
| 556 | D556 | Ar1 | D556 |
| 557 | D557 | Ar1 | D557 |
| 558 | D558 | Ar1 | D558 |
| 559 | D559 | Ar1 | D559 |
| 560 | D560 | Ar1 | D560 |
| 561 | D561 | Ar1 | D561 |
| 562 | D562 | Ar1 | D562 |
| 563 | D563 | Ar1 | D563 |
| 564 | D564 | Ar1 | D564 |
| 565 | D565 | Ar1 | D565 |
| 566 | D566 | Ar1 | D566 |
| 567 | D567 | Ar1 | D567 |
| 568 | D568 | Ar1 | D568 |
| 569 | D569 | Ar1 | D569 |
| 570 | D570 | Ar1 | D570 |
| 571 | D571 | Ar1 | D571 |
| 572 | D572 | Ar1 | D572 |
| 573 | D573 | Ar1 | D573 |
| 574 | D574 | Ar1 | D574 |
| 575 | D575 | Ar1 | D575 |
| 576 | D576 | Ar1 | D576 |
| 577 | D577 | Ar1 | D577 |
| 578 | D578 | Ar1 | D578 |
| 579 | D579 | Ar1 | D579 |
| 580 | D580 | Ar1 | D580 |
| 581 | D581 | Ar1 | D581 |
| 582 | D582 | Ar1 | D582 |
| 583 | D583 | Ar1 | D583 |
| 584 | D584 | Ar1 | D584 |
| 585 | D585 | Ar1 | D585 |
| 586 | D586 | Ar1 | D586 |
| 587 | D587 | Ar1 | D587 |
| 588 | D588 | Ar1 | D588 |
| 589 | D589 | Ar1 | D589 |
| 590 | D590 | Ar1 | D590 |
| 591 | D591 | Ar1 | D591 |
| 592 | D592 | Ar1 | D592 |
| 593 | D593 | Ar1 | D593 |
| 594 | D594 | Ar1 | D594 |
| 595 | D595 | Ar1 | D595 |
| 596 | D596 | Ar1 | D596 |
| 597 | D597 | Ar1 | D597 |
| 598 | D598 | Ar1 | D598 |
| 599 | D599 | Ar1 | D599 |
| 600 | D600 | Ar1 | D600 |

| No. | $R^3$ | $R^4$ | $R^5$ |
|---|---|---|---|
| 601 | D601 | Ar1 | D601 |
| 602 | D602 | Ar1 | D602 |
| 603 | D603 | Ar1 | D603 |
| 604 | D604 | Ar1 | D604 |
| 605 | D605 | Ar1 | D605 |
| 606 | D606 | Ar1 | D606 |
| 607 | D607 | Ar1 | D607 |
| 608 | D608 | Ar1 | D608 |
| 609 | D609 | Ar1 | D609 |
| 610 | D610 | Ar1 | D610 |
| 611 | D611 | Ar1 | D611 |
| 612 | D612 | Ar1 | D612 |
| 613 | D613 | Ar1 | D613 |
| 614 | D614 | Ar1 | D614 |
| 615 | D615 | Ar1 | D615 |
| 616 | D616 | Ar1 | D616 |
| 617 | D617 | Ar1 | D617 |
| 618 | D618 | Ar1 | D618 |
| 619 | D619 | Ar1 | D619 |
| 620 | D620 | Ar1 | D620 |
| 621 | D621 | Ar1 | D621 |
| 622 | D622 | Ar1 | D622 |
| 623 | D623 | Ar1 | D623 |
| 624 | D624 | Ar1 | D624 |
| 625 | D625 | Ar1 | D625 |
| 626 | D626 | Ar1 | D626 |
| 627 | D627 | Ar1 | D627 |
| 628 | D628 | Ar1 | D628 |
| 629 | D629 | Ar1 | D629 |
| 630 | D630 | Ar1 | D630 |
| 631 | D631 | Ar1 | D631 |
| 632 | D632 | Ar1 | D632 |
| 633 | D633 | Ar1 | D633 |
| 634 | D634 | Ar1 | D634 |
| 635 | D635 | Ar1 | D635 |
| 636 | D636 | Ar1 | D636 |
| 637 | D637 | Ar1 | D637 |
| 638 | D638 | Ar1 | D638 |
| 639 | D639 | Ar1 | D639 |
| 640 | D640 | Ar1 | D640 |
| 641 | D641 | Ar1 | D641 |
| 642 | D642 | Ar1 | D642 |
| 643 | D643 | Ar1 | D643 |
| 644 | D644 | Ar1 | D644 |
| 645 | D645 | Ar1 | D645 |
| 646 | D646 | Ar1 | D646 |
| 647 | D647 | Ar1 | D647 |
| 648 | D648 | Ar1 | D648 |
| 649 | D649 | Ar1 | D649 |
| 650 | D650 | Ar1 | D650 |
| 651 | D651 | Ar1 | D651 |
| 652 | D652 | Ar1 | D652 |
| 653 | D653 | Ar1 | D653 |
| 654 | D654 | Ar1 | D654 |
| 655 | D655 | Ar1 | D655 |
| 656 | D656 | Ar1 | D656 |
| 657 | D657 | Ar1 | D657 |
| 658 | D658 | Ar1 | D658 |
| 659 | D659 | Ar1 | D659 |
| 660 | D660 | Ar1 | D660 |

| No. | $R^3$ | $R^4$ | $R^5$ |
|---|---|---|---|
| 661 | D661 | Ar1 | D661 |
| 662 | D662 | Ar1 | D662 |
| 663 | D663 | Ar1 | D663 |
| 664 | D664 | Ar1 | D664 |
| 665 | D665 | Ar1 | D665 |
| 666 | D666 | Ar1 | D666 |
| 667 | D667 | Ar1 | D667 |
| 668 | D668 | Ar1 | D668 |
| 669 | D669 | Ar1 | D669 |
| 670 | D670 | Ar1 | D670 |
| 671 | D671 | Ar1 | D671 |
| 672 | D672 | Ar1 | D672 |
| 673 | D673 | Ar1 | D673 |
| 674 | D674 | Ar1 | D674 |
| 675 | D675 | Ar1 | D675 |
| 676 | D676 | Ar1 | D676 |
| 677 | D677 | Ar1 | D677 |
| 678 | D678 | Ar1 | D678 |
| 679 | D679 | Ar1 | D679 |
| 680 | D680 | Ar1 | D680 |
| 681 | D681 | Ar1 | D681 |
| 682 | D682 | Ar1 | D682 |
| 683 | D683 | Ar1 | D683 |
| 684 | D684 | Ar1 | D684 |
| 685 | D685 | Ar1 | D685 |
| 686 | D686 | Ar1 | D686 |
| 687 | D687 | Ar1 | D687 |
| 688 | D688 | Ar1 | D688 |
| 689 | D689 | Ar1 | D689 |
| 690 | D690 | Ar1 | D690 |
| 691 | D691 | Ar1 | D691 |
| 692 | D692 | Ar1 | D692 |
| 693 | D693 | Ar1 | D693 |
| 694 | D694 | Ar1 | D694 |
| 695 | D695 | Ar1 | D695 |
| 696 | D696 | Ar1 | D696 |
| 697 | D697 | Ar1 | D697 |
| 698 | D698 | Ar1 | D698 |
| 699 | D699 | Ar1 | D699 |
| 700 | D700 | Ar1 | D700 |
| 701 | D701 | Ar1 | D701 |
| 702 | D702 | Ar1 | D702 |
| 703 | D703 | Ar1 | D703 |
| 704 | D704 | Ar1 | D704 |
| 705 | D705 | Ar1 | D705 |
| 706 | D706 | Ar1 | D706 |
| 707 | D707 | Ar1 | D707 |
| 708 | D708 | Ar1 | D708 |
| 709 | D709 | Ar1 | D709 |
| 710 | D710 | Ar1 | D710 |
| 711 | D711 | Ar1 | D711 |
| 712 | D712 | Ar1 | D712 |
| 713 | D713 | Ar1 | D713 |
| 714 | D714 | Ar1 | D714 |
| 715 | D715 | Ar1 | D715 |
| 716 | D716 | Ar1 | D716 |
| 717 | D717 | Ar1 | D717 |
| 718 | D718 | Ar1 | D718 |
| 719 | D719 | Ar1 | D719 |
| 720 | D720 | Ar1 | D720 |

| No. | $R^3$ | $R^4$ | $R^5$ |
|---|---|---|---|
| 721 | D721 | Ar1 | D721 |
| 722 | D722 | Ar1 | D722 |
| 723 | D723 | Ar1 | D723 |
| 724 | D724 | Ar1 | D724 |
| 725 | D725 | Ar1 | D725 |
| 726 | D726 | Ar1 | D726 |
| 727 | D727 | Ar1 | D727 |
| 728 | D728 | Ar1 | D728 |
| 729 | D729 | Ar1 | D729 |
| 730 | D730 | Ar1 | D730 |
| 731 | D731 | Ar1 | D731 |
| 732 | D732 | Ar1 | D732 |
| 733 | D733 | Ar1 | D733 |
| 734 | D734 | Ar1 | D734 |
| 735 | D735 | Ar1 | D735 |
| 736 | D736 | Ar1 | D736 |
| 737 | D737 | Ar1 | D737 |
| 738 | D738 | Ar1 | D738 |
| 739 | D739 | Ar1 | D739 |
| 740 | D740 | Ar1 | D740 |
| 741 | D741 | Ar1 | D741 |
| 742 | D742 | Ar1 | D742 |
| 743 | D743 | Ar1 | D743 |
| 744 | D744 | Ar1 | D744 |
| 745 | D745 | Ar1 | D745 |
| 746 | D746 | Ar1 | D746 |
| 747 | D747 | Ar1 | D747 |
| 748 | D748 | Ar1 | D748 |
| 749 | D749 | Ar1 | D749 |
| 750 | D750 | Ar1 | D750 |
| 751 | D751 | Ar1 | D751 |
| 752 | D752 | Ar1 | D752 |
| 753 | D753 | Ar1 | D753 |
| 754 | D754 | Ar1 | D754 |
| 755 | D755 | Ar1 | D755 |
| 756 | D756 | Ar1 | D756 |
| 757 | D757 | Ar1 | D757 |
| 758 | D758 | Ar1 | D758 |
| 759 | D759 | Ar1 | D759 |
| 760 | D760 | Ar1 | D760 |
| 761 | D761 | Ar1 | D761 |
| 762 | D762 | Ar1 | D762 |
| 763 | D763 | Ar1 | D763 |
| 764 | D764 | Ar1 | D764 |
| 765 | D765 | Ar1 | D765 |
| 766 | D766 | Ar1 | D766 |
| 767 | D767 | Ar1 | D767 |
| 768 | D768 | Ar1 | D768 |
| 769 | D769 | Ar1 | D769 |
| 770 | D770 | Ar1 | D770 |
| 771 | D771 | Ar1 | D771 |
| 772 | D772 | Ar1 | D772 |
| 773 | D773 | Ar1 | D773 |
| 774 | D774 | Ar1 | D774 |
| 775 | D775 | Ar1 | D775 |
| 776 | D776 | Ar1 | D776 |
| 777 | D777 | Ar1 | D777 |
| 778 | D778 | Ar1 | D778 |
| 779 | D779 | Ar1 | D779 |
| 780 | D780 | Ar1 | D780 |

| No. | $R^3$ | $R^4$ | $R^5$ |
|---|---|---|---|
| 781 | D781 | Ar1 | D781 |
| 782 | D782 | Ar1 | D782 |
| 783 | D783 | Ar1 | D783 |
| 784 | D784 | Ar1 | D784 |
| 785 | D785 | Ar1 | D785 |
| 786 | D786 | Ar1 | D786 |
| 787 | D787 | Ar1 | D787 |
| 788 | D788 | Ar1 | D788 |
| 789 | D789 | Ar1 | D789 |
| 790 | D790 | Ar1 | D790 |
| 791 | D791 | Ar1 | D791 |
| 792 | D792 | Ar1 | D792 |
| 793 | D793 | Ar1 | D793 |
| 794 | D794 | Ar1 | D794 |
| 795 | D795 | Ar1 | D795 |
| 796 | D796 | Ar1 | D796 |
| 797 | D797 | Ar1 | D797 |
| 798 | D798 | Ar1 | D798 |
| 799 | D799 | Ar1 | D799 |
| 800 | D800 | Ar1 | D800 |
| 801 | D801 | Ar1 | D801 |
| 802 | D802 | Ar1 | D802 |
| 803 | D803 | Ar1 | D803 |
| 804 | D804 | Ar1 | D804 |
| 805 | D805 | Ar1 | D805 |
| 806 | D806 | Ar1 | D806 |
| 807 | D807 | Ar1 | D807 |
| 808 | D808 | Ar1 | D808 |
| 809 | D809 | Ar1 | D809 |
| 810 | D810 | Ar1 | D810 |
| 811 | D811 | Ar1 | D811 |
| 812 | D812 | Ar1 | D812 |
| 813 | D813 | Ar1 | D813 |
| 814 | D814 | Ar1 | D814 |
| 815 | D815 | Ar1 | D815 |
| 816 | D816 | Ar1 | D816 |
| 817 | D817 | Ar1 | D817 |
| 818 | D818 | Ar1 | D818 |
| 819 | D819 | Ar1 | D819 |
| 820 | D820 | Ar1 | D820 |
| 821 | D821 | Ar1 | D821 |
| 822 | D822 | Ar1 | D822 |
| 823 | D823 | Ar1 | D823 |
| 824 | D824 | Ar1 | D824 |
| 825 | D825 | Ar1 | D825 |
| 826 | D826 | Ar1 | D826 |
| 827 | D827 | Ar1 | D827 |
| 828 | D828 | Ar1 | D828 |
| 829 | D829 | Ar1 | D829 |
| 830 | D830 | Ar1 | D830 |
| 831 | D831 | Ar1 | D831 |
| 832 | D832 | Ar1 | D832 |
| 833 | D833 | Ar1 | D833 |
| 834 | D834 | Ar1 | D834 |
| 835 | D835 | Ar1 | D835 |
| 836 | D836 | Ar1 | D836 |
| 837 | D837 | Ar1 | D837 |
| 838 | D838 | Ar1 | D838 |
| 839 | D839 | Ar1 | D839 |
| 840 | D840 | Ar1 | D840 |

| No. | $R^3$ | $R^4$ | $R^5$ |
|---|---|---|---|
| 841 | D841 | Ar1 | D841 |
| 842 | D842 | Ar1 | D842 |
| 843 | D843 | Ar1 | D843 |
| 844 | D844 | Ar1 | D844 |
| 845 | D845 | Ar1 | D845 |
| 846 | D846 | Ar1 | D846 |
| 847 | D847 | Ar1 | D847 |
| 848 | D848 | Ar1 | D848 |
| 849 | D849 | Ar1 | D849 |
| 850 | D850 | Ar1 | D850 |
| 851 | D851 | Ar1 | D851 |
| 852 | D852 | Ar1 | D852 |
| 853 | D853 | Ar1 | D853 |
| 854 | D854 | Ar1 | D854 |
| 855 | D855 | Ar1 | D855 |
| 856 | D856 | Ar1 | D856 |
| 857 | D857 | Ar1 | D857 |
| 858 | D858 | Ar1 | D858 |
| 859 | D859 | Ar1 | D859 |
| 860 | D860 | Ar1 | D860 |
| 861 | D861 | Ar1 | D861 |
| 862 | D862 | Ar1 | D862 |
| 863 | D863 | Ar1 | D863 |
| 864 | D864 | Ar1 | D864 |
| 865 | D865 | Ar1 | D865 |
| 866 | D866 | Ar1 | D866 |
| 867 | D867 | Ar1 | D867 |
| 868 | D868 | Ar1 | D868 |
| 869 | D869 | Ar1 | D869 |
| 870 | D870 | Ar1 | D870 |
| 871 | D871 | Ar1 | D871 |
| 872 | D872 | Ar1 | D872 |
| 873 | D873 | Ar1 | D873 |
| 874 | D874 | Ar1 | D874 |
| 875 | D875 | Ar1 | D875 |
| 876 | D876 | Ar1 | D876 |
| 877 | D877 | Ar1 | D877 |
| 878 | D878 | Ar1 | D878 |
| 879 | D879 | Ar1 | D879 |
| 880 | D880 | Ar1 | D880 |
| 881 | D881 | Ar1 | D881 |
| 882 | D882 | Ar1 | D882 |
| 883 | D883 | Ar1 | D883 |
| 884 | D884 | Ar1 | D884 |
| 885 | D885 | Ar1 | D885 |
| 886 | D886 | Ar1 | D886 |
| 887 | D887 | Ar1 | D887 |
| 888 | D888 | Ar1 | D888 |
| 889 | D889 | Ar1 | D889 |
| 890 | D890 | Ar1 | D890 |
| 891 | D891 | Ar1 | D891 |
| 892 | D892 | Ar1 | D892 |
| 893 | D893 | Ar1 | D893 |
| 894 | D894 | Ar1 | D894 |
| 895 | D895 | Ar1 | D895 |
| 896 | D896 | Ar1 | D896 |
| 897 | D897 | Ar1 | D897 |
| 898 | D898 | Ar1 | D898 |
| 899 | D899 | Ar1 | D899 |
| 900 | D900 | Ar1 | D900 |

| No. | $R^3$ | $R^4$ | $R^5$ |
|---|---|---|---|
| 901 | D901 | Ar1 | D901 |
| 902 | D902 | Ar1 | D902 |
| 903 | D903 | Ar1 | D903 |
| 904 | D904 | Ar1 | D904 |
| 905 | D905 | Ar1 | D905 |
| 906 | D906 | Ar1 | D906 |
| 907 | D907 | Ar1 | D907 |
| 908 | D908 | Ar1 | D908 |
| 909 | D909 | Ar1 | D909 |
| 910 | D910 | Ar1 | D910 |
| 911 | D911 | Ar1 | D911 |
| 912 | D912 | Ar1 | D912 |
| 913 | D913 | Ar1 | D913 |
| 914 | D914 | Ar1 | D914 |
| 915 | D915 | Ar1 | D915 |
| 916 | D916 | Ar1 | D916 |
| 917 | D917 | Ar1 | D917 |
| 918 | D918 | Ar1 | D918 |
| 919 | D919 | Ar1 | D919 |
| 920 | D920 | Ar1 | D920 |
| 921 | D921 | Ar1 | D921 |
| 922 | D922 | Ar1 | D922 |
| 923 | D923 | Ar1 | D923 |
| 924 | D924 | Ar1 | D924 |
| 925 | D925 | Ar1 | D925 |
| 926 | D926 | Ar1 | D926 |
| 927 | D927 | Ar1 | D927 |
| 928 | D928 | Ar1 | D928 |
| 929 | D929 | Ar1 | D929 |
| 930 | D930 | Ar1 | D930 |
| 931 | D931 | Ar1 | D931 |
| 932 | D932 | Ar1 | D932 |
| 933 | D933 | Ar1 | D933 |
| 934 | D934 | Ar1 | D934 |
| 935 | D935 | Ar1 | D935 |
| 936 | D936 | Ar1 | D936 |
| 937 | D937 | Ar1 | D937 |
| 938 | D938 | Ar1 | D938 |
| 939 | D939 | Ar1 | D939 |
| 940 | D940 | Ar1 | D940 |
| 941 | D941 | Ar1 | D941 |
| 942 | D942 | Ar1 | D942 |
| 943 | D943 | Ar1 | D943 |
| 944 | D944 | Ar1 | D944 |
| 945 | D945 | Ar1 | D945 |
| 946 | D946 | Ar1 | D946 |
| 947 | D947 | Ar1 | D947 |
| 948 | D948 | Ar1 | D948 |
| 949 | D949 | Ar1 | D949 |
| 950 | D950 | Ar1 | D950 |
| 951 | D951 | Ar1 | D951 |
| 952 | D952 | Ar1 | D952 |
| 953 | D953 | Ar1 | D953 |
| 954 | D954 | Ar1 | D954 |
| 955 | D955 | Ar1 | D955 |
| 956 | D956 | Ar1 | D956 |
| 957 | D957 | Ar1 | D957 |
| 958 | D958 | Ar1 | D958 |
| 959 | D959 | Ar1 | D959 |
| 960 | D960 | Ar1 | D960 |

| No. | $R^3$ | $R^4$ | $R^5$ |
|---|---|---|---|
| 961 | D961 | Ar1 | D961 |
| 962 | D962 | Ar1 | D962 |
| 963 | D963 | Ar1 | D963 |
| 964 | D964 | Ar1 | D964 |
| 965 | D965 | Ar1 | D965 |
| 966 | D966 | Ar1 | D966 |
| 967 | D967 | Ar1 | D967 |
| 968 | D968 | Ar1 | D968 |
| 969 | D969 | Ar1 | D969 |
| 970 | D970 | Ar1 | D970 |
| 971 | D971 | Ar1 | D971 |
| 972 | D972 | Ar1 | D972 |
| 973 | D973 | Ar1 | D973 |
| 974 | D974 | Ar1 | D974 |
| 975 | D975 | Ar1 | D975 |
| 976 | D976 | Ar1 | D976 |
| 977 | D977 | Ar1 | D977 |
| 978 | D978 | Ar1 | D978 |
| 979 | D979 | Ar1 | D979 |
| 980 | D980 | Ar1 | D980 |
| 981 | D981 | Ar1 | D981 |
| 982 | D982 | Ar1 | D982 |
| 983 | D983 | Ar1 | D983 |
| 984 | D984 | Ar1 | D984 |
| 985 | D985 | Ar1 | D985 |
| 986 | D986 | Ar1 | D986 |
| 987 | D987 | Ar1 | D987 |
| 988 | D988 | Ar1 | D988 |
| 989 | D989 | Ar1 | D989 |
| 990 | D990 | Ar1 | D990 |
| 991 | D991 | Ar1 | D991 |
| 992 | D992 | Ar1 | D992 |
| 993 | D993 | Ar1 | D993 |
| 994 | D994 | Ar1 | D994 |
| 995 | D995 | Ar1 | D995 |
| 996 | D996 | Ar1 | D996 |
| 997 | D997 | Ar1 | D997 |
| 998 | D998 | Ar1 | D998 |
| 999 | D999 | Ar1 | D999 |
| 1000 | D1000 | Ar1 | D1000 |
| 1001 | D1001 | Ar1 | D1001 |
| 1002 | D1002 | Ar1 | D1002 |
| 1003 | D1003 | Ar1 | D1003 |
| 1004 | D1004 | Ar1 | D1004 |
| 1005 | D1005 | Ar1 | D1005 |
| 1006 | D1006 | Ar1 | D1006 |
| 1007 | D1007 | Ar1 | D1007 |
| 1008 | D1008 | Ar1 | D1008 |
| 1009 | D1009 | Ar1 | D1009 |
| 1010 | D1010 | Ar1 | D1010 |
| 1011 | D1011 | Ar1 | D1011 |
| 1012 | D1012 | Ar1 | D1012 |
| 1013 | D1013 | Ar1 | D1013 |
| 1014 | D1014 | Ar1 | D1014 |
| 1015 | D1015 | Ar1 | D1015 |
| 1016 | D1016 | Ar1 | D1016 |
| 1017 | D1017 | Ar1 | D1017 |
| 1018 | D1018 | Ar1 | D1018 |
| 1019 | D1019 | Ar1 | D1019 |
| 1020 | D1020 | Ar1 | D1020 |
| 1021 | D1021 | Ar1 | D1021 |
| 1022 | D1022 | Ar1 | D1022 |
| 1023 | D1023 | Ar1 | D1023 |
| 1024 | D1024 | Ar1 | D1024 |
| 1025 | D1025 | Ar1 | D1025 |
| 1026 | D1026 | Ar1 | D1026 |
| 1027 | D1027 | Ar1 | D1027 |
| 1028 | D1028 | Ar1 | D1028 |
| 1029 | D1029 | Ar1 | D1029 |
| 1030 | D1030 | Ar1 | D1030 |
| 1031 | D1031 | Ar1 | D1031 |
| 1032 | D1032 | Ar1 | D1032 |
| 1033 | D1033 | Ar1 | D1033 |
| 1034 | D1034 | Ar1 | D1034 |
| 1035 | D1035 | Ar1 | D1035 |
| 1036 | D1036 | Ar1 | D1036 |
| 1037 | D1037 | Ar1 | D1037 |
| 1038 | D1038 | Ar1 | D1038 |
| 1039 | D1039 | Ar1 | D1039 |
| 1040 | D1040 | Ar1 | D1040 |
| 1041 | D1041 | Ar1 | D1041 |
| 1042 | D1042 | Ar1 | D1042 |
| 1043 | D1043 | Ar1 | D1043 |
| 1044 | D1044 | Ar1 | D1044 |
| 1045 | D1045 | Ar1 | D1045 |
| 1046 | D1046 | Ar1 | D1046 |
| 1047 | D1047 | Ar1 | D1047 |
| 1048 | D1048 | Ar1 | D1048 |
| 1049 | D1049 | Ar1 | D1049 |
| 1050 | D1050 | Ar1 | D1050 |
| 1051 | D1051 | Ar1 | D1051 |
| 1052 | D1052 | Ar1 | D1052 |
| 1053 | D1053 | Ar1 | D1053 |
| 1054 | D1054 | Ar1 | D1054 |
| 1055 | D1055 | Ar1 | D1055 |
| 1056 | D1056 | Ar1 | D1056 |
| 1057 | D1057 | Ar1 | D1057 |
| 1058 | D1058 | Ar1 | D1058 |
| 1059 | D1059 | Ar1 | D1059 |
| 1060 | D1060 | Ar1 | D1060 |
| 1061 | D1061 | Ar1 | D1061 |
| 1062 | D1062 | Ar1 | D1062 |
| 1063 | D1063 | Ar1 | D1063 |
| 1064 | D1064 | Ar1 | D1064 |
| 1065 | D1065 | Ar1 | D1065 |
| 1066 | D1066 | Ar1 | D1066 |
| 1067 | D1067 | Ar1 | D1067 |
| 1068 | D1068 | Ar1 | D1068 |
| 1069 | D1069 | Ar1 | D1069 |
| 1070 | D1070 | Ar1 | D1070 |
| 1071 | D1071 | Ar1 | D1071 |
| 1072 | D1072 | Ar1 | D1072 |
| 1073 | D1073 | Ar1 | D1073 |
| 1074 | D1074 | Ar1 | D1074 |
| 1075 | D1075 | Ar1 | D1075 |
| 1076 | D1076 | Ar1 | D1076 |
| 1077 | D1077 | Ar1 | D1077 |
| 1078 | D1078 | Ar1 | D1078 |
| 1079 | D1079 | Ar1 | D1079 |
| 1080 | D1080 | Ar1 | D1080 |
| 1081 | D1081 | Ar1 | D1081 |
| 1082 | D1082 | Ar1 | D1082 |
| 1083 | D1083 | Ar1 | D1083 |
| 1084 | D1084 | Ar1 | D1084 |
| 1085 | D1085 | Ar1 | D1085 |
| 1086 | D1086 | Ar1 | D1086 |
| 1087 | D1087 | Ar1 | D1087 |
| 1088 | D1088 | Ar1 | D1088 |
| 1089 | D1089 | Ar1 | D1089 |
| 1090 | D1090 | Ar1 | D1090 |
| 1091 | D1091 | Ar1 | D1091 |
| 1092 | D1092 | Ar1 | D1092 |
| 1093 | D1093 | Ar1 | D1093 |
| 1094 | D1094 | Ar1 | D1094 |
| 1095 | D1095 | Ar1 | D1095 |
| 1096 | D1096 | Ar1 | D1096 |
| 1097 | D1097 | Ar1 | D1097 |
| 1098 | D1098 | Ar1 | D1098 |
| 1099 | D1099 | Ar1 | D1099 |
| 1100 | D1100 | Ar1 | D1100 |
| 1101 | D1101 | Ar1 | D1101 |
| 1102 | D1102 | Ar1 | D1102 |
| 1103 | D1103 | Ar1 | D1103 |
| 1104 | D1104 | Ar1 | D1104 |
| 1105 | D1105 | Ar1 | D1105 |
| 1106 | D1106 | Ar1 | D1106 |
| 1107 | D1107 | Ar1 | D1107 |
| 1108 | D1108 | Ar1 | D1108 |
| 1109 | D1109 | Ar1 | D1109 |
| 1110 | D1110 | Ar1 | D1110 |
| 1111 | D1111 | Ar1 | D1111 |
| 1112 | D1112 | Ar1 | D1112 |
| 1113 | D1113 | Ar1 | D1113 |
| 1114 | D1114 | Ar1 | D1114 |
| 1115 | D1115 | Ar1 | D1115 |
| 1116 | D1116 | Ar1 | D1116 |
| 1117 | D1117 | Ar1 | D1117 |
| 1118 | D1118 | Ar1 | D1118 |
| 1119 | D1119 | Ar1 | D1119 |
| 1120 | D1120 | Ar1 | D1120 |
| 1121 | D1121 | Ar1 | D1121 |
| 1122 | D1122 | Ar1 | D1122 |
| 1123 | D1123 | Ar1 | D1123 |
| 1124 | D1124 | Ar1 | D1124 |
| 1125 | D1125 | Ar1 | D1125 |
| 1126 | D1126 | Ar1 | D1126 |
| 1127 | D1127 | Ar1 | D1127 |
| 1128 | D1128 | Ar1 | D1128 |
| 1129 | D1129 | Ar1 | D1129 |
| 1130 | D1130 | Ar1 | D1130 |
| 1131 | D1131 | Ar1 | D1131 |
| 1132 | D1132 | Ar1 | D1132 |
| 1133 | D1133 | Ar1 | D1133 |
| 1134 | D1134 | Ar1 | D1134 |
| 1135 | D1135 | Ar1 | D1135 |
| 1136 | D1136 | Ar1 | D1136 |
| 1137 | D1137 | Ar1 | D1137 |
| 1138 | D1138 | Ar1 | D1138 |
| 1139 | D1139 | Ar1 | D1139 |
| 1140 | D1140 | Ar1 | D1140 |
| 1141 | D1141 | Ar1 | D1141 |
| 1142 | D1142 | Ar1 | D1142 |
| 1143 | D1143 | Ar1 | D1143 |
| 1144 | D1144 | Ar1 | D1144 |
| 1145 | D1145 | Ar1 | D1145 |
| 1146 | D1146 | Ar1 | D1146 |
| 1147 | D1147 | Ar1 | D1147 |
| 1148 | D1148 | Ar1 | D1148 |
| 1149 | D1149 | Ar1 | D1149 |
| 1150 | D1150 | Ar1 | D1150 |
| 1151 | D1151 | Ar1 | D1151 |
| 1152 | D1152 | Ar1 | D1152 |
| 1153 | D1153 | Ar1 | D1153 |
| 1154 | D1154 | Ar1 | D1154 |
| 1155 | D1155 | Ar1 | D1155 |
| 1156 | D1156 | Ar1 | D1156 |
| 1157 | D1157 | Ar1 | D1157 |
| 1158 | D1158 | Ar1 | D1158 |
| 1159 | D1159 | Ar1 | D1159 |
| 1160 | D1160 | Ar1 | D1160 |
| 1161 | D1161 | Ar1 | D1161 |
| 1162 | D1162 | Ar1 | D1162 |
| 1163 | D1163 | Ar1 | D1163 |
| 1164 | D1164 | Ar1 | D1164 |
| 1165 | D1165 | Ar1 | D1165 |
| 1166 | D1166 | Ar1 | D1166 |
| 1167 | D1167 | Ar1 | D1167 |
| 1168 | D1168 | Ar1 | D1168 |
| 1169 | D1169 | Ar1 | D1169 |
| 1170 | D1170 | Ar1 | D1170 |
| 1171 | D1171 | Ar1 | D1171 |
| 1172 | D1172 | Ar1 | D1172 |
| 1173 | D1173 | Ar1 | D1173 |
| 1174 | D1174 | Ar1 | D1174 |
| 1175 | D1175 | Ar1 | D1175 |

[Table 2]

| No. | $R^3$ | $R^4$ | $R^5$ | = |
|---|---|---|---|---|
| 1 ~ 1175 | D1~D1175 | Ar1 | D1~D1175 | |
| 1176 ~ 2350 | D1~D1175 | Ar2 | D1~D1175 | |
| 2351 ~ 3525 | D1~D1175 | Ar3 | D1~D1175 | |
| 3526 ~ 4700 | D1~D1175 | Ar4 | D1~D1175 | |
| 4701 ~ 5875 | D1~D1175 | Ar5 | D1~D1175 | |
| 5876 ~ 7050 | D1~D1175 | Ar6 | D1~D1175 | |
| 7051 ~ 8225 | D1~D1175 | Ar7 | D1~D1175 | |
| 8226 ~ 9400 | D1~D1175 | Ar8 | D1~D1175 | |
| 9401 ~ 10575 | D1~D1175 | Ar9 | D1~D1175 | |
| 10576 ~ 11750 | D1~D1175 | Ar10 | D1~D1175 | |
| 11751 ~ 12925 | D1~D1175 | Ar11 | D1~D1175 | |
| 12926 ~ 14100 | D1~D1175 | Ar12 | D1~D1175 | |
| 14101 ~ 15275 | D1~D1175 | Ar13 | D1~D1175 | |
| 15276 ~ 16450 | D1~D1175 | Ar14 | D1~D1175 | |
| 16451 ~ 17625 | D1~D1175 | Ar15 | D1~D1175 | |
| 17626 ~ 18800 | D1~D1175 | Ar16 | D1~D1175 | |
| 18801 ~ 19975 | D1~D1175 | Ar17 | D1~D1175 | |
| 19976 ~ 21150 | D1~D1175 | Ar18 | D1~D1175 | |
| 21151 ~ 22325 | D1~D1175 | Ar19 | D1~D1175 | |
| 22326 ~ 23500 | D1~D1175 | Ar20 | D1~D1175 | |
| 23501 ~ 24675 | D1~D1175 | Ar21 | D1~D1175 | |
| 24676 ~ 25850 | D1~D1175 | Ar22 | D1~D1175 | |
| 25851 ~ 27025 | D1~D1175 | Ar23 | D1~D1175 | |
| 27026 ~ 28200 | D1~D1175 | Ar24 | D1~D1175 | |
| 28201 ~ 29375 | D1~D1175 | Ar25 | D1~D1175 | |
| 29376 ~ 30550 | D1~D1175 | Ar26 | D1~D1175 | |
| 30551 ~ 31725 | D1~D1175 | Ar27 | D1~D1175 | |
| 31726 ~ 32900 | D1~D1175 | Ar28 | D1~D1175 | |
| 32901 ~ 34075 | D1~D1175 | Ar29 | D1~D1175 | |
| 34076 ~ 35250 | D1~D1175 | Ar30 | D1~D1175 | |
| 35251 ~ 36425 | D1~D1175 | Ar31 | D1~D1175 | $R^3 = R^5$ |
| 36426 ~ 37600 | D1~D1175 | Ar32 | D1~D1175 | |
| 37601 ~ 38775 | D1~D1175 | Ar33 | D1~D1175 | |
| 38776 ~ 39950 | D1~D1175 | Ar34 | D1~D1175 | |
| 39951 ~ 41125 | D1~D1175 | Ar35 | D1~D1175 | |
| 41126 ~ 42300 | D1~D1175 | Ar36 | D1~D1175 | |
| 42301 ~ 43475 | D1~D1175 | Ar37 | D1~D1175 | |
| 43476 ~ 44650 | D1~D1175 | Ar38 | D1~D1175 | |
| 44651 ~ 45825 | D1~D1175 | Ar39 | D1~D1175 | |
| 45826 ~ 47000 | D1~D1175 | Ar40 | D1~D1175 | |
| 47001 ~ 48175 | D1~D1175 | Ar41 | D1~D1175 | |
| 48176 ~ 49350 | D1~D1175 | Ar42 | D1~D1175 | |
| 49351 ~ 50525 | D1~D1175 | Ar43 | D1~D1175 | |
| 50526 ~ 51700 | D1~D1175 | Ar44 | D1~D1175 | |
| 51701 ~ 52875 | D1~D1175 | Ar45 | D1~D1175 | |
| 52876 ~ 54050 | D1~D1175 | Ar46 | D1~D1175 | |
| 54051 ~ 55225 | D1~D1175 | Ar47 | D1~D1175 | |
| 55226 ~ 56400 | D1~D1175 | Ar48 | D1~D1175 | |
| 56401 ~ 57575 | D1~D1175 | Ar49 | D1~D1175 | |
| 57576 ~ 58750 | D1~D1175 | Ar50 | D1~D1175 | |
| 58751 ~ 59925 | D1~D1175 | Ar51 | D1~D1175 | |
| 59926 ~ 61100 | D1~D1175 | Ar52 | D1~D1175 | |
| 61101 ~ 62275 | D1~D1175 | Ar53 | D1~D1175 | |
| 62276 ~ 63450 | D1~D1175 | Ar54 | D1~D1175 | |
| 63451 ~ 64625 | D1~D1175 | Ar55 | D1~D1175 | |
| 64626 ~ 65800 | D1~D1175 | Ar56 | D1~D1175 | |
| 65801 ~ 66975 | D1~D1175 | Ar57 | D1~D1175 | |
| 66976 ~ 68150 | D1~D1175 | Ar58 | D1~D1175 | |
| 68151 ~ 69325 | D1~D1175 | Ar59 | D1~D1175 | |
| 69326 ~ 70500 | D1~D1175 | H | D1~D1175 | |

| No. | $R^3$ | $R^4$ | $R^5$ |
|---|---|---|---|
| 70501 ～ 71674 | D1 | Ar1 | D2～D1175 |
| 71675 ～ 72848 | D1 | Ar2 | D2～D1175 |
| 72849 ～ 74022 | D1 | Ar3 | D2～D1175 |
| 74023 ～ 75196 | D1 | Ar4 | D2～D1175 |
| 75197 ～ 76370 | D1 | Ar5 | D2～D1175 |
| 76371 ～ 77544 | D1 | Ar6 | D2～D1175 |
| 77545 ～ 78718 | D1 | Ar7 | D2～D1175 |
| 78719 ～ 79892 | D1 | Ar8 | D2～D1175 |
| 79893 ～ 81066 | D1 | Ar9 | D2～D1175 |
| 81067 ～ 82240 | D1 | Ar10 | D2～D1175 |
| 82241 ～ 83414 | D1 | Ar11 | D2～D1175 |
| 83415 ～ 84588 | D1 | Ar12 | D2～D1175 |
| 84589 ～ 85762 | D1 | Ar13 | D2～D1175 |
| 85763 ～ 86936 | D1 | Ar14 | D2～D1175 |
| 86937 ～ 88110 | D1 | Ar15 | D2～D1175 |
| 88111 ～ 89284 | D1 | Ar16 | D2～D1175 |
| 89285 ～ 90458 | D1 | Ar17 | D2～D1175 |
| 90459 ～ 91632 | D1 | Ar18 | D2～D1175 |
| 91633 ～ 92806 | D1 | Ar19 | D2～D1175 |
| 92807 ～ 93980 | D1 | Ar20 | D2～D1175 |
| 93981 ～ 95154 | D1 | Ar21 | D2～D1175 |
| 95155 ～ 96328 | D1 | Ar22 | D2～D1175 |
| 96329 ～ 97502 | D1 | Ar23 | D2～D1175 |
| 97503 ～ 98676 | D1 | Ar24 | D2～D1175 |
| 98677 ～ 99850 | D1 | Ar25 | D2～D1175 |
| 99851 ～ 101024 | D1 | Ar26 | D2～D1175 |
| 101025 ～ 102198 | D1 | Ar27 | D2～D1175 |
| 102199 ～ 103372 | D1 | Ar28 | D2～D1175 |
| 103373 ～ 104546 | D1 | Ar29 | D2～D1175 |
| 104547 ～ 105720 | D1 | Ar30 | D2～D1175 |
| 105721 ～ 106894 | D1 | Ar31 | D2～D1175 |
| 106895 ～ 108068 | D1 | Ar32 | D2～D1175 |
| 108069 ～ 109242 | D1 | Ar33 | D2～D1175 |
| 109243 ～ 110416 | D1 | Ar34 | D2～D1175 |
| 110417 ～ 111590 | D1 | Ar35 | D2～D1175 |
| 111591 ～ 112764 | D1 | Ar36 | D2～D1175 |
| 112765 ～ 113938 | D1 | Ar37 | D2～D1175 |
| 113939 ～ 115112 | D1 | Ar38 | D2～D1175 |
| 115113 ～ 116286 | D1 | Ar39 | D2～D1175 |
| 116287 ～ 117460 | D1 | Ar40 | D2～D1175 |
| 117461 ～ 118634 | D1 | Ar41 | D2～D1175 |
| 118635 ～ 119808 | D1 | Ar42 | D2～D1175 |
| 119809 ～ 120982 | D1 | Ar43 | D2～D1175 |
| 120983 ～ 122156 | D1 | Ar44 | D2～D1175 |
| 122157 ～ 123330 | D1 | Ar45 | D2～D1175 |
| 123331 ～ 124504 | D1 | Ar46 | D2～D1175 |
| 124505 ～ 125678 | D1 | Ar47 | D2～D1175 |
| 125679 ～ 126852 | D1 | Ar48 | D2～D1175 |
| 126853 ～ 128026 | D1 | Ar49 | D2～D1175 |
| 128027 ～ 129200 | D1 | Ar50 | D2～D1175 |
| 129201 ～ 130374 | D1 | Ar51 | D2～D1175 |
| 130375 ～ 131548 | D1 | Ar52 | D2～D1175 |
| 131549 ～ 132722 | D1 | Ar53 | D2～D1175 |
| 132723 ～ 133896 | D1 | Ar54 | D2～D1175 |
| 133897 ～ 135070 | D1 | Ar55 | D2～D1175 |
| 135071 ～ 136244 | D1 | Ar56 | D2～D1175 |
| 136245 ～ 137418 | D1 | Ar57 | D2～D1175 |
| 137419 ～ 138592 | D1 | Ar58 | D2～D1175 |
| 138593 ～ 139766 | D1 | Ar59 | D2～D1175 |
| 139767 ～ 140940 | D1 | H | D2～D1175 |

| No. | $R^3$ | $R^4$ | $R^5$ |
|---|---|---|---|
| 140941 ～ 142114 | D7 | Ar1 | D1～6,D8～1175 |
| 142115 ～ 143288 | D7 | Ar2 | D1～6,D8～1175 |
| 143289 ～ 144462 | D7 | Ar3 | D1～6,D8～1175 |
| 144463 ～ 145636 | D7 | Ar4 | D1～6,D8～1175 |
| 145637 ～ 146810 | D7 | Ar5 | D1～6,D8～1175 |
| 146811 ～ 147984 | D7 | Ar6 | D1～6,D8～1175 |
| 147985 ～ 149158 | D7 | Ar7 | D1～6,D8～1175 |
| 149159 ～ 150332 | D7 | Ar8 | D1～6,D8～1175 |
| 150333 ～ 151506 | D7 | Ar9 | D1～6,D8～1175 |
| 151507 ～ 152680 | D7 | Ar10 | D1～6,D8～1175 |
| 152681 ～ 153854 | D7 | Ar11 | D1～6,D8～1175 |
| 153855 ～ 155028 | D7 | Ar12 | D1～6,D8～1175 |
| 155029 ～ 156202 | D7 | Ar13 | D1～6,D8～1175 |
| 156203 ～ 157376 | D7 | Ar14 | D1～6,D8～1175 |
| 157377 ～ 158550 | D7 | Ar15 | D1～6,D8～1175 |
| 158551 ～ 159724 | D7 | Ar16 | D1～6,D8～1175 |
| 159725 ～ 160898 | D7 | Ar17 | D1～6,D8～1175 |
| 160899 ～ 162072 | D7 | Ar18 | D1～6,D8～1175 |
| 162073 ～ 163246 | D7 | Ar19 | D1～6,D8～1175 |
| 163247 ～ 164420 | D7 | Ar20 | D1～6,D8～1175 |
| 164421 ～ 165594 | D7 | Ar21 | D1～6,D8～1175 |
| 165595 ～ 166768 | D7 | Ar22 | D1～6,D8～1175 |
| 166769 ～ 167942 | D7 | Ar23 | D1～6,D8～1175 |
| 167943 ～ 169116 | D7 | Ar24 | D1～6,D8～1175 |
| 169117 ～ 170290 | D7 | Ar25 | D1～6,D8～1175 |
| 170291 ～ 171464 | D7 | Ar26 | D1～6,D8～1175 |
| 171465 ～ 172638 | D7 | Ar27 | D1～6,D8～1175 |
| 172639 ～ 173812 | D7 | Ar28 | D1～6,D8～1175 |
| 173813 ～ 174986 | D7 | Ar29 | D1～6,D8～1175 |
| 174987 ～ 176160 | D7 | Ar30 | D1～6,D8～1175 |
| 176161 ～ 177334 | D7 | Ar31 | D1～6,D8～1175 |
| 177335 ～ 178508 | D7 | Ar32 | D1～6,D8～1175 |
| 178509 ～ 179682 | D7 | Ar33 | D1～6,D8～1175 |
| 179683 ～ 180856 | D7 | Ar34 | D1～6,D8～1175 |
| 180857 ～ 182030 | D7 | Ar35 | D1～6,D8～1175 |
| 182031 ～ 183204 | D7 | Ar36 | D1～6,D8～1175 |
| 183205 ～ 184378 | D7 | Ar37 | D1～6,D8～1175 |
| 184379 ～ 185552 | D7 | Ar38 | D1～6,D8～1175 |
| 185553 ～ 186726 | D7 | Ar39 | D1～6,D8～1175 |
| 186727 ～ 187900 | D7 | Ar40 | D1～6,D8～1175 |
| 187901 ～ 189074 | D7 | Ar41 | D1～6,D8～1175 |
| 189075 ～ 190248 | D7 | Ar42 | D1～6,D8～1175 |
| 190249 ～ 191422 | D7 | Ar43 | D1～6,D8～1175 |
| 191423 ～ 192596 | D7 | Ar44 | D1～6,D8～1175 |
| 192597 ～ 193770 | D7 | Ar45 | D1～6,D8～1175 |
| 193771 ～ 194944 | D7 | Ar46 | D1～6,D8～1175 |
| 194945 ～ 196118 | D7 | Ar47 | D1～6,D8～1175 |
| 196119 ～ 197292 | D7 | Ar48 | D1～6,D8～1175 |
| 197293 ～ 198466 | D7 | Ar49 | D1～6,D8～1175 |
| 198467 ～ 199640 | D7 | Ar50 | D1～6,D8～1175 |
| 199641 ～ 200814 | D7 | Ar51 | D1～6,D8～1175 |
| 200815 ～ 201988 | D7 | Ar52 | D1～6,D8～1175 |
| 201989 ～ 203162 | D7 | Ar53 | D1～6,D8～1175 |
| 203163 ～ 204336 | D7 | Ar54 | D1～6,D8～1175 |
| 204337 ～ 205510 | D7 | Ar55 | D1～6,D8～1175 |
| 205511 ～ 206684 | D7 | Ar56 | D1～6,D8～1175 |
| 206685 ～ 207858 | D7 | Ar57 | D1～6,D8～1175 |
| 207859 ～ 209032 | D7 | Ar58 | D1～6,D8～1175 |
| 209033 ～ 210206 | D7 | Ar59 | D1～6,D8～1175 |
| 210207 ～ 211380 | D7 | H | D1～6,D8～1175 |

| No. | $R^3$ | $R^4$ | $R^5$ |
|---|---|---|---|
| 211381 ～212554 | D8 | Ar1 | D1～7,D9～1175 |
| 212555 ～213728 | D8 | Ar2 | D1～7,D9～1175 |
| 213729 ～214902 | D8 | Ar3 | D1～7,D9～1175 |
| 214903 ～216076 | D8 | Ar4 | D1～7,D9～1175 |
| 216077 ～217250 | D8 | Ar5 | D1～7,D9～1175 |
| 217251 ～218424 | D8 | Ar6 | D1～7,D9～1175 |
| 218425 ～219598 | D8 | Ar7 | D1～7,D9～1175 |
| 219599 ～220772 | D8 | Ar8 | D1～7,D9～1175 |
| 220773 ～221946 | D8 | Ar9 | D1～7,D9～1175 |
| 221947 ～223120 | D8 | Ar10 | D1～7,D9～1175 |
| 223121 ～224294 | D8 | Ar11 | D1～7,D9～1175 |
| 224295 ～225468 | D8 | Ar12 | D1～7,D9～1175 |
| 225469 ～226642 | D8 | Ar13 | D1～7,D9～1175 |
| 226643 ～227816 | D8 | Ar14 | D1～7,D9～1175 |
| 227817 ～228990 | D8 | Ar15 | D1～7,D9～1175 |
| 228991 ～230164 | D8 | Ar16 | D1～7,D9～1175 |
| 230165 ～231338 | D8 | Ar17 | D1～7,D9～1175 |
| 231339 ～232512 | D8 | Ar18 | D1～7,D9～1175 |
| 232513 ～233686 | D8 | Ar19 | D1～7,D9～1175 |
| 233687 ～234860 | D8 | Ar20 | D1～7,D9～1175 |
| 234861 ～236034 | D8 | Ar21 | D1～7,D9～1175 |
| 236035 ～237208 | D8 | Ar22 | D1～7,D9～1175 |
| 237209 ～238382 | D8 | Ar23 | D1～7,D9～1175 |
| 238383 ～239556 | D8 | Ar24 | D1～7,D9～1175 |
| 239557 ～240730 | D8 | Ar25 | D1～7,D9～1175 |
| 240731 ～241904 | D8 | Ar26 | D1～7,D9～1175 |
| 241905 ～243078 | D8 | Ar27 | D1～7,D9～1175 |
| 243079 ～244252 | D8 | Ar28 | D1～7,D9～1175 |
| 244253 ～245426 | D8 | Ar29 | D1～7,D9～1175 |
| 245427 ～246600 | D8 | Ar30 | D1～7,D9～1175 |
| 246601 ～247774 | D8 | Ar31 | D1～7,D9～1175 |
| 247775 ～248948 | D8 | Ar32 | D1～7,D9～1175 |
| 248949 ～250122 | D8 | Ar33 | D1～7,D9～1175 |
| 250123 ～251296 | D8 | Ar34 | D1～7,D9～1175 |
| 251297 ～252470 | D8 | Ar35 | D1～7,D9～1175 |
| 252471 ～253644 | D8 | Ar36 | D1～7,D9～1175 |
| 253645 ～254818 | D8 | Ar37 | D1～7,D9～1175 |
| 254819 ～255992 | D8 | Ar38 | D1～7,D9～1175 |
| 255993 ～257166 | D8 | Ar39 | D1～7,D9～1175 |
| 257167 ～258340 | D8 | Ar40 | D1～7,D9～1175 |
| 258341 ～259514 | D8 | Ar41 | D1～7,D9～1175 |
| 259515 ～260688 | D8 | Ar42 | D1～7,D9～1175 |
| 260689 ～261862 | D8 | Ar43 | D1～7,D9～1175 |
| 261863 ～263036 | D8 | Ar44 | D1～7,D9～1175 |
| 263037 ～264210 | D8 | Ar45 | D1～7,D9～1175 |
| 264211 ～265384 | D8 | Ar46 | D1～7,D9～1175 |
| 265385 ～266558 | D8 | Ar47 | D1～7,D9～1175 |
| 266559 ～267732 | D8 | Ar48 | D1～7,D9～1175 |
| 267733 ～268906 | D8 | Ar49 | D1～7,D9～1175 |
| 268907 ～270080 | D8 | Ar50 | D1～7,D9～1175 |
| 270081 ～271254 | D8 | Ar51 | D1～7,D9～1175 |
| 271255 ～272428 | D8 | Ar52 | D1～7,D9～1175 |
| 272429 ～273602 | D8 | Ar53 | D1～7,D9～1175 |
| 273603 ～274776 | D8 | Ar54 | D1～7,D9～1175 |
| 274777 ～275950 | D8 | Ar55 | D1～7,D9～1175 |
| 275951 ～277124 | D8 | Ar56 | D1～7,D9～1175 |
| 277125 ～278298 | D8 | Ar57 | D1～7,D9～1175 |
| 278299 ～279472 | D8 | Ar58 | D1～7,D9～1175 |
| 279473 ～280646 | D8 | Ar59 | D1～7,D9～1175 |
| 280647 ～281820 | D8 | H | D1～7,D9～1175 |

| No. | $R^3$ | $R^4$ | $R^5$ |
|---|---|---|---|
| 281821 ～282994 | D9 | Ar1 | D1～8,D10～1175 |
| 282995 ～284168 | D9 | Ar2 | D1～8,D10～1175 |
| 284169 ～285342 | D9 | Ar3 | D1～8,D10～1175 |
| 285343 ～286516 | D9 | Ar4 | D1～8,D10～1175 |
| 286517 ～287690 | D9 | Ar5 | D1～8,D10～1175 |
| 287691 ～288864 | D9 | Ar6 | D1～8,D10～1175 |
| 288865 ～290038 | D9 | Ar7 | D1～8,D10～1175 |
| 290039 ～291212 | D9 | Ar8 | D1～8,D10～1175 |
| 291213 ～292386 | D9 | Ar9 | D1～8,D10～1175 |
| 292387 ～293560 | D9 | Ar10 | D1～8,D10～1175 |
| 293561 ～294734 | D9 | Ar11 | D1～8,D10～1175 |
| 294735 ～295908 | D9 | Ar12 | D1～8,D10～1175 |
| 295909 ～297082 | D9 | Ar13 | D1～8,D10～1175 |
| 297083 ～298256 | D9 | Ar14 | D1～8,D10～1175 |
| 298257 ～299430 | D9 | Ar15 | D1～8,D10～1175 |
| 299431 ～300604 | D9 | Ar16 | D1～8,D10～1175 |
| 300605 ～301778 | D9 | Ar17 | D1～8,D10～1175 |
| 301779 ～302952 | D9 | Ar18 | D1～8,D10～1175 |
| 302953 ～304126 | D9 | Ar19 | D1～8,D10～1175 |
| 304127 ～305300 | D9 | Ar20 | D1～8,D10～1175 |
| 305301 ～306474 | D9 | Ar21 | D1～8,D10～1175 |
| 306475 ～307648 | D9 | Ar22 | D1～8,D10～1175 |
| 307649 ～308822 | D9 | Ar23 | D1～8,D10～1175 |
| 308823 ～309996 | D9 | Ar24 | D1～8,D10～1175 |
| 309997 ～311170 | D9 | Ar25 | D1～8,D10～1175 |
| 311171 ～312344 | D9 | Ar26 | D1～8,D10～1175 |
| 312345 ～313518 | D9 | Ar27 | D1～8,D10～1175 |
| 313519 ～314692 | D9 | Ar28 | D1～8,D10～1175 |
| 314693 ～315866 | D9 | Ar29 | D1～8,D10～1175 |
| 315867 ～317040 | D9 | Ar30 | D1～8,D10～1175 |
| 317041 ～318214 | D9 | Ar31 | D1～8,D10～1175 |
| 318215 ～319388 | D9 | Ar32 | D1～8,D10～1175 |
| 319389 ～320562 | D9 | Ar33 | D1～8,D10～1175 |
| 320563 ～321736 | D9 | Ar34 | D1～8,D10～1175 |
| 321737 ～322910 | D9 | Ar35 | D1～8,D10～1175 |
| 322911 ～324084 | D9 | Ar36 | D1～8,D10～1175 |
| 324085 ～325258 | D9 | Ar37 | D1～8,D10～1175 |
| 325259 ～326432 | D9 | Ar38 | D1～8,D10～1175 |
| 326433 ～327606 | D9 | Ar39 | D1～8,D10～1175 |
| 327607 ～328780 | D9 | Ar40 | D1～8,D10～1175 |
| 328781 ～329954 | D9 | Ar41 | D1～8,D10～1175 |
| 329955 ～331128 | D9 | Ar42 | D1～8,D10～1175 |
| 331129 ～332302 | D9 | Ar43 | D1～8,D10～1175 |
| 332303 ～333476 | D9 | Ar44 | D1～8,D10～1175 |
| 333477 ～334650 | D9 | Ar45 | D1～8,D10～1175 |
| 334651 ～335824 | D9 | Ar46 | D1～8,D10～1175 |
| 335825 ～336998 | D9 | Ar47 | D1～8,D10～1175 |
| 336999 ～338172 | D9 | Ar48 | D1～8,D10～1175 |
| 338173 ～339346 | D9 | Ar49 | D1～8,D10～1175 |
| 339347 ～340520 | D9 | Ar50 | D1～8,D10～1175 |
| 340521 ～341694 | D9 | Ar51 | D1～8,D10～1175 |
| 341695 ～342868 | D9 | Ar52 | D1～8,D10～1175 |
| 342869 ～344042 | D9 | Ar53 | D1～8,D10～1175 |
| 344043 ～345216 | D9 | Ar54 | D1～8,D10～1175 |
| 345217 ～346390 | D9 | Ar55 | D1～8,D10～1175 |
| 346391 ～347564 | D9 | Ar56 | D1～8,D10～1175 |
| 347565 ～348738 | D9 | Ar57 | D1～8,D10～1175 |
| 348739 ～349912 | D9 | Ar58 | D1～8,D10～1175 |
| 349913 ～351086 | D9 | Ar59 | D1～8,D10～1175 |
| 351087 ～352260 | D9 | H | D1～8,D10～1175 |

| No. | $R^3$ | $R^4$ | $R^5$ |
|---|---|---|---|
| 352261 ～353434 | D19 | Ar1 | D1～18,D20～1175 |
| 353435 ～354608 | D19 | Ar2 | D1～18,D20～1175 |
| 354609 ～355782 | D19 | Ar3 | D1～18,D20～1175 |
| 355783 ～356956 | D19 | Ar4 | D1～18,D20～1175 |
| 356957 ～358130 | D19 | Ar5 | D1～18,D20～1175 |
| 358131 ～359304 | D19 | Ar6 | D1～18,D20～1175 |
| 359305 ～360478 | D19 | Ar7 | D1～18,D20～1175 |
| 360479 ～361652 | D19 | Ar8 | D1～18,D20～1175 |
| 361653 ～362826 | D19 | Ar9 | D1～18,D20～1175 |
| 362827 ～364000 | D19 | Ar10 | D1～18,D20～1175 |
| 364001 ～365174 | D19 | Ar11 | D1～18,D20～1175 |
| 365175 ～366348 | D19 | Ar12 | D1～18,D20～1175 |
| 366349 ～367522 | D19 | Ar13 | D1～18,D20～1175 |
| 367523 ～368696 | D19 | Ar14 | D1～18,D20～1175 |
| 368697 ～369870 | D19 | Ar15 | D1～18,D20～1175 |
| 369871 ～371044 | D19 | Ar16 | D1～18,D20～1175 |
| 371045 ～372218 | D19 | Ar17 | D1～18,D20～1175 |
| 372219 ～373392 | D19 | Ar18 | D1～18,D20～1175 |
| 373393 ～374566 | D19 | Ar19 | D1～18,D20～1175 |
| 374567 ～375740 | D19 | Ar20 | D1～18,D20～1175 |
| 375741 ～376914 | D19 | Ar21 | D1～18,D20～1175 |
| 376915 ～378088 | D19 | Ar22 | D1～18,D20～1175 |
| 378089 ～379262 | D19 | Ar23 | D1～18,D20～1175 |
| 379263 ～380436 | D19 | Ar24 | D1～18,D20～1175 |
| 380437 ～381610 | D19 | Ar25 | D1～18,D20～1175 |
| 381611 ～382784 | D19 | Ar26 | D1～18,D20～1175 |
| 382785 ～383958 | D19 | Ar27 | D1～18,D20～1175 |
| 383959 ～385132 | D19 | Ar28 | D1～18,D20～1175 |
| 385133 ～386306 | D19 | Ar29 | D1～18,D20～1175 |
| 386307 ～387480 | D19 | Ar30 | D1～18,D20～1175 |
| 387481 ～388654 | D19 | Ar31 | D1～18,D20～1175 |
| 388655 ～389828 | D19 | Ar32 | D1～18,D20～1175 |
| 389829 ～391002 | D19 | Ar33 | D1～18,D20～1175 |
| 391003 ～392176 | D19 | Ar34 | D1～18,D20～1175 |
| 392177 ～393350 | D19 | Ar35 | D1～18,D20～1175 |
| 393351 ～394524 | D19 | Ar36 | D1～18,D20～1175 |
| 394525 ～395698 | D19 | Ar37 | D1～18,D20～1175 |
| 395699 ～396872 | D19 | Ar38 | D1～18,D20～1175 |
| 396873 ～398046 | D19 | Ar39 | D1～18,D20～1175 |
| 398047 ～399220 | D19 | Ar40 | D1～18,D20～1175 |
| 399221 ～400394 | D19 | Ar41 | D1～18,D20～1175 |
| 400395 ～401568 | D19 | Ar42 | D1～18,D20～1175 |
| 401569 ～402742 | D19 | Ar43 | D1～18,D20～1175 |
| 402743 ～403916 | D19 | Ar44 | D1～18,D20～1175 |
| 403917 ～405090 | D19 | Ar45 | D1～18,D20～1175 |
| 405091 ～406264 | D19 | Ar46 | D1～18,D20～1175 |
| 406265 ～407438 | D19 | Ar47 | D1～18,D20～1175 |
| 407439 ～408612 | D19 | Ar48 | D1～18,D20～1175 |
| 408613 ～409786 | D19 | Ar49 | D1～18,D20～1175 |
| 409787 ～410960 | D19 | Ar50 | D1～18,D20～1175 |
| 410961 ～412134 | D19 | Ar51 | D1～18,D20～1175 |
| 412135 ～413308 | D19 | Ar52 | D1～18,D20～1175 |
| 413309 ～414482 | D19 | Ar53 | D1～18,D20～1175 |
| 414483 ～415656 | D19 | Ar54 | D1～18,D20～1175 |
| 415657 ～416830 | D19 | Ar55 | D1～18,D20～1175 |
| 416831 ～418004 | D19 | Ar56 | D1～18,D20～1175 |
| 418005 ～419178 | D19 | Ar57 | D1～18,D20～1175 |
| 419179 ～420352 | D19 | Ar58 | D1～18,D20～1175 |
| 420353 ～421526 | D19 | Ar59 | D1～18,D20～1175 |
| 421527 ～422700 | D19 | H | D1～18,D20～1175 |

| No. | $R^3$ | $R^4$ | $R^5$ |
|---|---|---|---|
| 422701 ～423874 | D37 | Ar1 | D1～36,D38～1175 |
| 423875 ～425048 | D37 | Ar2 | D1～36,D38～1175 |
| 425049 ～426222 | D37 | Ar3 | D1～36,D38～1175 |
| 426223 ～427396 | D37 | Ar4 | D1～36,D38～1175 |
| 427397 ～428570 | D37 | Ar5 | D1～36,D38～1175 |
| 428571 ～429744 | D37 | Ar6 | D1～36,D38～1175 |
| 429745 ～430918 | D37 | Ar7 | D1～36,D38～1175 |
| 430919 ～432092 | D37 | Ar8 | D1～36,D38～1175 |
| 432093 ～433266 | D37 | Ar9 | D1～36,D38～1175 |
| 433267 ～434440 | D37 | Ar10 | D1～36,D38～1175 |
| 434441 ～435614 | D37 | Ar11 | D1～36,D38～1175 |
| 435615 ～436788 | D37 | Ar12 | D1～36,D38～1175 |
| 436789 ～437962 | D37 | Ar13 | D1～36,D38～1175 |
| 437963 ～439136 | D37 | Ar14 | D1～36,D38～1175 |
| 439137 ～440310 | D37 | Ar15 | D1～36,D38～1175 |
| 440311 ～441484 | D37 | Ar16 | D1～36,D38～1175 |
| 441485 ～442658 | D37 | Ar17 | D1～36,D38～1175 |
| 442659 ～443832 | D37 | Ar18 | D1～36,D38～1175 |
| 443833 ～445006 | D37 | Ar19 | D1～36,D38～1175 |
| 445007 ～446180 | D37 | Ar20 | D1～36,D38～1175 |
| 446181 ～447354 | D37 | Ar21 | D1～36,D38～1175 |
| 447355 ～448528 | D37 | Ar22 | D1～36,D38～1175 |
| 448529 ～449702 | D37 | Ar23 | D1～36,D38～1175 |
| 449703 ～450876 | D37 | Ar24 | D1～36,D38～1175 |
| 450877 ～452050 | D37 | Ar25 | D1～36,D38～1175 |
| 452051 ～453224 | D37 | Ar26 | D1～36,D38～1175 |
| 453225 ～454398 | D37 | Ar27 | D1～36,D38～1175 |
| 454399 ～455572 | D37 | Ar28 | D1～36,D38～1175 |
| 455573 ～456746 | D37 | Ar29 | D1～36,D38～1175 |
| 456747 ～457920 | D37 | Ar30 | D1～36,D38～1175 |
| 457921 ～459094 | D37 | Ar31 | D1～36,D38～1175 |
| 459095 ～460268 | D37 | Ar32 | D1～36,D38～1175 |
| 460269 ～461442 | D37 | Ar33 | D1～36,D38～1175 |
| 461443 ～462616 | D37 | Ar34 | D1～36,D38～1175 |
| 462617 ～463790 | D37 | Ar35 | D1～36,D38～1175 |
| 463791 ～464964 | D37 | Ar36 | D1～36,D38～1175 |
| 464965 ～466138 | D37 | Ar37 | D1～36,D38～1175 |
| 466139 ～467312 | D37 | Ar38 | D1～36,D38～1175 |
| 467313 ～468486 | D37 | Ar39 | D1～36,D38～1175 |
| 468487 ～469660 | D37 | Ar40 | D1～36,D38～1175 |
| 469661 ～470834 | D37 | Ar41 | D1～36,D38～1175 |
| 470835 ～472008 | D37 | Ar42 | D1～36,D38～1175 |
| 472009 ～473182 | D37 | Ar43 | D1～36,D38～1175 |
| 473183 ～474356 | D37 | Ar44 | D1～36,D38～1175 |
| 474357 ～475530 | D37 | Ar45 | D1～36,D38～1175 |
| 475531 ～476704 | D37 | Ar46 | D1～36,D38～1175 |
| 476705 ～477878 | D37 | Ar47 | D1～36,D38～1175 |
| 477879 ～479052 | D37 | Ar48 | D1～36,D38～1175 |
| 479053 ～480226 | D37 | Ar49 | D1～36,D38～1175 |
| 480227 ～481400 | D37 | Ar50 | D1～36,D38～1175 |
| 481401 ～482574 | D37 | Ar51 | D1～36,D38～1175 |
| 482575 ～483748 | D37 | Ar52 | D1～36,D38～1175 |
| 483749 ～484922 | D37 | Ar53 | D1～36,D38～1175 |
| 484923 ～486096 | D37 | Ar54 | D1～36,D38～1175 |
| 486097 ～487270 | D37 | Ar55 | D1～36,D38～1175 |
| 487271 ～488444 | D37 | Ar56 | D1～36,D38～1175 |
| 488445 ～489618 | D37 | Ar57 | D1～36,D38～1175 |
| 489619 ～490792 | D37 | Ar58 | D1～36,D38～1175 |
| 490793 ～491966 | D37 | Ar59 | D1～36,D38～1175 |
| 491967 ～493140 | D37 | H | D1～36,D38～1175 |

| No. | $R^3$ | $R^4$ | $R^5$ |
|---|---|---|---|
| 493141 ～494314 | D50 | Ar1 | D1～49,D51～1175 |
| 494315 ～495488 | D50 | Ar2 | D1～49,D51～1175 |
| 495489 ～496662 | D50 | Ar3 | D1～49,D51～1175 |
| 496663 ～497836 | D50 | Ar4 | D1～49,D51～1175 |
| 497837 ～499010 | D50 | Ar5 | D1～49,D51～1175 |
| 499011 ～500184 | D50 | Ar6 | D1～49,D51～1175 |
| 500185 ～501358 | D50 | Ar7 | D1～49,D51～1175 |
| 501359 ～502532 | D50 | Ar8 | D1～49,D51～1175 |
| 502533 ～503706 | D50 | Ar9 | D1～49,D51～1175 |
| 503707 ～504880 | D50 | Ar10 | D1～49,D51～1175 |
| 504881 ～506054 | D50 | Ar11 | D1～49,D51～1175 |
| 506055 ～507228 | D50 | Ar12 | D1～49,D51～1175 |
| 507229 ～508402 | D50 | Ar13 | D1～49,D51～1175 |
| 508403 ～509576 | D50 | Ar14 | D1～49,D51～1175 |
| 509577 ～510750 | D50 | Ar15 | D1～49,D51～1175 |
| 510751 ～511924 | D50 | Ar16 | D1～49,D51～1175 |
| 511925 ～513098 | D50 | Ar17 | D1～49,D51～1175 |
| 513099 ～514272 | D50 | Ar18 | D1～49,D51～1175 |
| 514273 ～515446 | D50 | Ar19 | D1～49,D51～1175 |
| 515447 ～516620 | D50 | Ar20 | D1～49,D51～1175 |
| 516621 ～517794 | D50 | Ar21 | D1～49,D51～1175 |
| 517795 ～518968 | D50 | Ar22 | D1～49,D51～1175 |
| 518969 ～520142 | D50 | Ar23 | D1～49,D51～1175 |
| 520143 ～521316 | D50 | Ar24 | D1～49,D51～1175 |
| 521317 ～522490 | D50 | Ar25 | D1～49,D51～1175 |
| 522491 ～523664 | D50 | Ar26 | D1～49,D51～1175 |
| 523665 ～524838 | D50 | Ar27 | D1～49,D51～1175 |
| 524839 ～526012 | D50 | Ar28 | D1～49,D51～1175 |
| 526013 ～527186 | D50 | Ar29 | D1～49,D51～1175 |
| 527187 ～528360 | D50 | Ar30 | D1～49,D51～1175 |
| 528361 ～529534 | D50 | Ar31 | D1～49,D51～1175 |
| 529535 ～530708 | D50 | Ar32 | D1～49,D51～1175 |
| 530709 ～531882 | D50 | Ar33 | D1～49,D51～1175 |
| 531883 ～533056 | D50 | Ar34 | D1～49,D51～1175 |
| 533057 ～534230 | D50 | Ar35 | D1～49,D51～1175 |
| 534231 ～535404 | D50 | Ar36 | D1～49,D51～1175 |
| 535405 ～536578 | D50 | Ar37 | D1～49,D51～1175 |
| 536579 ～537752 | D50 | Ar38 | D1～49,D51～1175 |
| 537753 ～538926 | D50 | Ar39 | D1～49,D51～1175 |
| 538927 ～540100 | D50 | Ar40 | D1～49,D51～1175 |
| 540101 ～541274 | D50 | Ar41 | D1～49,D51～1175 |
| 541275 ～542448 | D50 | Ar42 | D1～49,D51～1175 |
| 542449 ～543622 | D50 | Ar43 | D1～49,D51～1175 |
| 543623 ～544796 | D50 | Ar44 | D1～49,D51～1175 |
| 544797 ～545970 | D50 | Ar45 | D1～49,D51～1175 |
| 545971 ～547144 | D50 | Ar46 | D1～49,D51～1175 |
| 547145 ～548318 | D50 | Ar47 | D1～49,D51～1175 |
| 548319 ～549492 | D50 | Ar48 | D1～49,D51～1175 |
| 549493 ～550666 | D50 | Ar49 | D1～49,D51～1175 |
| 550667 ～551840 | D50 | Ar50 | D1～49,D51～1175 |
| 551841 ～553014 | D50 | Ar51 | D1～49,D51～1175 |
| 553015 ～554188 | D50 | Ar52 | D1～49,D51～1175 |
| 554189 ～555362 | D50 | Ar53 | D1～49,D51～1175 |
| 555363 ～556536 | D50 | Ar54 | D1～49,D51～1175 |
| 556537 ～557710 | D50 | Ar55 | D1～49,D51～1175 |
| 557711 ～558884 | D50 | Ar56 | D1～49,D51～1175 |
| 558885 ～560058 | D50 | Ar57 | D1～49,D51～1175 |
| 560059 ～561232 | D50 | Ar58 | D1～49,D51～1175 |
| 561233 ～562406 | D50 | Ar59 | D1～49,D51～1175 |
| 562407 ～563580 | D50 | H | D1～49,D51～1175 |

| No. | $R^3$ | $R^4$ | $R^5$ |
|---|---|---|---|
| 563581 ～564754 | D465 | Ar1 | D1～464,D466～1175 |
| 564755 ～565928 | D465 | Ar2 | D1～464,D466～1175 |
| 565929 ～567102 | D465 | Ar3 | D1～464,D466～1175 |
| 567103 ～568276 | D465 | Ar4 | D1～464,D466～1175 |
| 568277 ～569450 | D465 | Ar5 | D1～464,D466～1175 |
| 569451 ～570624 | D465 | Ar6 | D1～464,D466～1175 |
| 570625 ～571798 | D465 | Ar7 | D1～464,D466～1175 |
| 571799 ～572972 | D465 | Ar8 | D1～464,D466～1175 |
| 572973 ～574146 | D465 | Ar9 | D1～464,D466～1175 |
| 574147 ～575320 | D465 | Ar10 | D1～464,D466～1175 |
| 575321 ～576494 | D465 | Ar11 | D1～464,D466～1175 |
| 576495 ～577668 | D465 | Ar12 | D1～464,D466～1175 |
| 577669 ～578842 | D465 | Ar13 | D1～464,D466～1175 |
| 578843 ～580016 | D465 | Ar14 | D1～464,D466～1175 |
| 580017 ～581190 | D465 | Ar15 | D1～464,D466～1175 |
| 581191 ～582364 | D465 | Ar16 | D1～464,D466～1175 |
| 582365 ～583538 | D465 | Ar17 | D1～464,D466～1175 |
| 583539 ～584712 | D465 | Ar18 | D1～464,D466～1175 |
| 584713 ～585886 | D465 | Ar19 | D1～464,D466～1175 |
| 585887 ～587060 | D465 | Ar20 | D1～464,D466～1175 |
| 587061 ～588234 | D465 | Ar21 | D1～464,D466～1175 |
| 588235 ～589408 | D465 | Ar22 | D1～464,D466～1175 |
| 589409 ～590582 | D465 | Ar23 | D1～464,D466～1175 |
| 590583 ～591756 | D465 | Ar24 | D1～464,D466～1175 |
| 591757 ～592930 | D465 | Ar25 | D1～464,D466～1175 |
| 592931 ～594104 | D465 | Ar26 | D1～464,D466～1175 |
| 594105 ～595278 | D465 | Ar27 | D1～464,D466～1175 |
| 595279 ～596452 | D465 | Ar28 | D1～464,D466～1175 |
| 596453 ～597626 | D465 | Ar29 | D1～464,D466～1175 |
| 597627 ～598800 | D465 | Ar30 | D1～464,D466～1175 |
| 598801 ～599974 | D465 | Ar31 | D1～464,D466～1175 |
| 599975 ～601148 | D465 | Ar32 | D1～464,D466～1175 |
| 601149 ～602322 | D465 | Ar33 | D1～464,D466～1175 |
| 602323 ～603496 | D465 | Ar34 | D1～464,D466～1175 |
| 603497 ～604670 | D465 | Ar35 | D1～464,D466～1175 |
| 604671 ～605844 | D465 | Ar36 | D1～464,D466～1175 |
| 605845 ～607018 | D465 | Ar37 | D1～464,D466～1175 |
| 607019 ～608192 | D465 | Ar38 | D1～464,D466～1175 |
| 608193 ～609366 | D465 | Ar39 | D1～464,D466～1175 |
| 609367 ～610540 | D465 | Ar40 | D1～464,D466～1175 |
| 610541 ～611714 | D465 | Ar41 | D1～464,D466～1175 |
| 611715 ～612888 | D465 | Ar42 | D1～464,D466～1175 |
| 612889 ～614062 | D465 | Ar43 | D1～464,D466～1175 |
| 614063 ～615236 | D465 | Ar44 | D1～464,D466～1175 |
| 615237 ～616410 | D465 | Ar45 | D1～464,D466～1175 |
| 616411 ～617584 | D465 | Ar46 | D1～464,D466～1175 |
| 617585 ～618758 | D465 | Ar47 | D1～464,D466～1175 |
| 618759 ～619932 | D465 | Ar48 | D1～464,D466～1175 |
| 619933 ～621106 | D465 | Ar49 | D1～464,D466～1175 |
| 621107 ～622280 | D465 | Ar50 | D1～464,D466～1175 |
| 622281 ～623454 | D465 | Ar51 | D1～464,D466～1175 |
| 623455 ～624628 | D465 | Ar52 | D1～464,D466～1175 |
| 624629 ～625802 | D465 | Ar53 | D1～464,D466～1175 |
| 625803 ～626976 | D465 | Ar54 | D1～464,D466～1175 |
| 626977 ～628150 | D465 | Ar55 | D1～464,D466～1175 |
| 628151 ～629324 | D465 | Ar56 | D1～464,D466～1175 |
| 629325 ～630498 | D465 | Ar57 | D1～464,D466～1175 |
| 630499 ～631672 | D465 | Ar58 | D1～464,D466～1175 |
| 631673 ～632846 | D465 | Ar59 | D1～464,D466～1175 |
| 632847 ～634020 | D465 | H | D1～464,D466～1175 |

| No. | $R^3$ | $R^4$ | $R^5$ |
|---|---|---|---|
| 634021 ～635194 | D466 | Ar1 | D1～465,D467～1175 |
| 635195 ～636368 | D466 | Ar2 | D1～465,D467～1175 |
| 636369 ～637542 | D466 | Ar3 | D1～465,D467～1175 |
| 637543 ～638716 | D466 | Ar4 | D1～465,D467～1175 |
| 638717 ～639890 | D466 | Ar5 | D1～465,D467～1175 |
| 639891 ～641064 | D466 | Ar6 | D1～465,D467～1175 |
| 641065 ～642238 | D466 | Ar7 | D1～465,D467～1175 |
| 642239 ～643412 | D466 | Ar8 | D1～465,D467～1175 |
| 643413 ～644586 | D466 | Ar9 | D1～465,D467～1175 |
| 644587 ～645760 | D466 | Ar10 | D1～465,D467～1175 |
| 645761 ～646934 | D466 | Ar11 | D1～465,D467～1175 |
| 646935 ～648108 | D466 | Ar12 | D1～465,D467～1175 |
| 648109 ～649282 | D466 | Ar13 | D1～465,D467～1175 |
| 649283 ～650456 | D466 | Ar14 | D1～465,D467～1175 |
| 650457 ～651630 | D466 | Ar15 | D1～465,D467～1175 |
| 651631 ～652804 | D466 | Ar16 | D1～465,D467～1175 |
| 652805 ～653978 | D466 | Ar17 | D1～465,D467～1175 |
| 653979 ～655152 | D466 | Ar18 | D1～465,D467～1175 |
| 655153 ～656326 | D466 | Ar19 | D1～465,D467～1175 |
| 656327 ～657500 | D466 | Ar20 | D1～465,D467～1175 |
| 657501 ～658674 | D466 | Ar21 | D1～465,D467～1175 |
| 658675 ～659848 | D466 | Ar22 | D1～465,D467～1175 |
| 659849 ～661022 | D466 | Ar23 | D1～465,D467～1175 |
| 661023 ～662196 | D466 | Ar24 | D1～465,D467～1175 |
| 662197 ～663370 | D466 | Ar25 | D1～465,D467～1175 |
| 663371 ～664544 | D466 | Ar26 | D1～465,D467～1175 |
| 664545 ～665718 | D466 | Ar27 | D1～465,D467～1175 |
| 665719 ～666892 | D466 | Ar28 | D1～465,D467～1175 |
| 666893 ～668066 | D466 | Ar29 | D1～465,D467～1175 |
| 668067 ～669240 | D466 | Ar30 | D1～465,D467～1175 |
| 669241 ～670414 | D466 | Ar31 | D1～465,D467～1175 |
| 670415 ～671588 | D466 | Ar32 | D1～465,D467～1175 |
| 671589 ～672762 | D466 | Ar33 | D1～465,D467～1175 |
| 672763 ～673936 | D466 | Ar34 | D1～465,D467～1175 |
| 673937 ～675110 | D466 | Ar35 | D1～465,D467～1175 |
| 675111 ～676284 | D466 | Ar36 | D1～465,D467～1175 |
| 676285 ～677458 | D466 | Ar37 | D1～465,D467～1175 |
| 677459 ～678632 | D466 | Ar38 | D1～465,D467～1175 |
| 678633 ～679806 | D466 | Ar39 | D1～465,D467～1175 |
| 679807 ～680980 | D466 | Ar40 | D1～465,D467～1175 |
| 680981 ～682154 | D466 | Ar41 | D1～465,D467～1175 |
| 682155 ～683328 | D466 | Ar42 | D1～465,D467～1175 |
| 683329 ～684502 | D466 | Ar43 | D1～465,D467～1175 |
| 684503 ～685676 | D466 | Ar44 | D1～465,D467～1175 |
| 685677 ～686850 | D466 | Ar45 | D1～465,D467～1175 |
| 686851 ～688024 | D466 | Ar46 | D1～465,D467～1175 |
| 688025 ～689198 | D466 | Ar47 | D1～465,D467～1175 |
| 689199 ～690372 | D466 | Ar48 | D1～465,D467～1175 |
| 690373 ～691546 | D466 | Ar49 | D1～465,D467～1175 |
| 691547 ～692720 | D466 | Ar50 | D1～465,D467～1175 |
| 692721 ～693894 | D466 | Ar51 | D1～465,D467～1175 |
| 693895 ～695068 | D466 | Ar52 | D1～465,D467～1175 |
| 695069 ～696242 | D466 | Ar53 | D1～465,D467～1175 |
| 696243 ～697416 | D466 | Ar54 | D1～465,D467～1175 |
| 697417 ～698590 | D466 | Ar55 | D1～465,D467～1175 |
| 698591 ～699764 | D466 | Ar56 | D1～465,D467～1175 |
| 699765 ～700938 | D466 | Ar57 | D1～465,D467～1175 |
| 700939 ～702112 | D466 | Ar58 | D1～465,D467～1175 |
| 702113 ～703286 | D466 | Ar59 | D1～465,D467～1175 |
| 703287 ～704460 | D466 | H | D1～465,D467～1175 |

| No. | $R^3$ | $R^4$ | $R^5$ |
|---|---|---|---|
| 704461 ～705634 | D467 | Ar1 | D1～466,D468～1175 |
| 705635 ～706808 | D467 | Ar2 | D1～466,D468～1175 |
| 706809 ～707982 | D467 | Ar3 | D1～466,D468～1175 |
| 707983 ～709156 | D467 | Ar4 | D1～466,D468～1175 |
| 709157 ～710330 | D467 | Ar5 | D1～466,D468～1175 |
| 710331 ～711504 | D467 | Ar6 | D1～466,D468～1175 |
| 711505 ～712678 | D467 | Ar7 | D1～466,D468～1175 |
| 712679 ～713852 | D467 | Ar8 | D1～466,D468～1175 |
| 713853 ～715026 | D467 | Ar9 | D1～466,D468～1175 |
| 715027 ～716200 | D467 | Ar10 | D1～466,D468～1175 |
| 716201 ～717374 | D467 | Ar11 | D1～466,D468～1175 |
| 717375 ～718548 | D467 | Ar12 | D1～466,D468～1175 |
| 718549 ～719722 | D467 | Ar13 | D1～466,D468～1175 |
| 719723 ～720896 | D467 | Ar14 | D1～466,D468～1175 |
| 720897 ～722070 | D467 | Ar15 | D1～466,D468～1175 |
| 722071 ～723244 | D467 | Ar16 | D1～466,D468～1175 |
| 723245 ～724418 | D467 | Ar17 | D1～466,D468～1175 |
| 724419 ～725592 | D467 | Ar18 | D1～466,D468～1175 |
| 725593 ～726766 | D467 | Ar19 | D1～466,D468～1175 |
| 726767 ～727940 | D467 | Ar20 | D1～466,D468～1175 |
| 727941 ～729114 | D467 | Ar21 | D1～466,D468～1175 |
| 729115 ～730288 | D467 | Ar22 | D1～466,D468～1175 |
| 730289 ～731462 | D467 | Ar23 | D1～466,D468～1175 |
| 731463 ～732636 | D467 | Ar24 | D1～466,D468～1175 |
| 732637 ～733810 | D467 | Ar25 | D1～466,D468～1175 |
| 733811 ～734984 | D467 | Ar26 | D1～466,D468～1175 |
| 734985 ～736158 | D467 | Ar27 | D1～466,D468～1175 |
| 736159 ～737332 | D467 | Ar28 | D1～466,D468～1175 |
| 737333 ～738506 | D467 | Ar29 | D1～466,D468～1175 |
| 738507 ～739680 | D467 | Ar30 | D1～466,D468～1175 |
| 739681 ～740854 | D467 | Ar31 | D1～466,D468～1175 |
| 740855 ～742028 | D467 | Ar32 | D1～466,D468～1175 |
| 742029 ～743202 | D467 | Ar33 | D1～466,D468～1175 |
| 743203 ～744376 | D467 | Ar34 | D1～466,D468～1175 |
| 744377 ～745550 | D467 | Ar35 | D1～466,D468～1175 |
| 745551 ～746724 | D467 | Ar36 | D1～466,D468～1175 |
| 746725 ～747898 | D467 | Ar37 | D1～466,D468～1175 |
| 747899 ～749072 | D467 | Ar38 | D1～466,D468～1175 |
| 749073 ～750246 | D467 | Ar39 | D1～466,D468～1175 |
| 750247 ～751420 | D467 | Ar40 | D1～466,D468～1175 |
| 751421 ～752594 | D467 | Ar41 | D1～466,D468～1175 |
| 752595 ～753768 | D467 | Ar42 | D1～466,D468～1175 |
| 753769 ～754942 | D467 | Ar43 | D1～466,D468～1175 |
| 754943 ～756116 | D467 | Ar44 | D1～466,D468～1175 |
| 756117 ～757290 | D467 | Ar45 | D1～466,D468～1175 |
| 757291 ～758464 | D467 | Ar46 | D1～466,D468～1175 |
| 758465 ～759638 | D467 | Ar47 | D1～466,D468～1175 |
| 759639 ～760812 | D467 | Ar48 | D1～466,D468～1175 |
| 760813 ～761986 | D467 | Ar49 | D1～466,D468～1175 |
| 761987 ～763160 | D467 | Ar50 | D1～466,D468～1175 |
| 763161 ～764334 | D467 | Ar51 | D1～466,D468～1175 |
| 764335 ～765508 | D467 | Ar52 | D1～466,D468～1175 |
| 765509 ～766682 | D467 | Ar53 | D1～466,D468～1175 |
| 766683 ～767856 | D467 | Ar54 | D1～466,D468～1175 |
| 767857 ～769030 | D467 | Ar55 | D1～466,D468～1175 |
| 769031 ～770204 | D467 | Ar56 | D1～466,D468～1175 |
| 770205 ～771378 | D467 | Ar57 | D1～466,D468～1175 |
| 771379 ～772552 | D467 | Ar58 | D1～466,D468～1175 |
| 772553 ～773726 | D467 | Ar59 | D1～466,D468～1175 |
| 773727 ～774900 | D467 | H | D1～466,D468～1175 |

| No. | $R^3$ | $R^4$ | $R^5$ |
|---|---|---|---|
| 774901 ~ 776074 | D486 | Ar1 | D1~485,D487~1175 |
| 776075 ~ 777248 | D486 | Ar2 | D1~485,D487~1175 |
| 777249 ~ 778422 | D486 | Ar3 | D1~485,D487~1175 |
| 778423 ~ 779596 | D486 | Ar4 | D1~485,D487~1175 |
| 779597 ~ 780770 | D486 | Ar5 | D1~485,D487~1175 |
| 780771 ~ 781944 | D486 | Ar6 | D1~485,D487~1175 |
| 781945 ~ 783118 | D486 | Ar7 | D1~485,D487~1175 |
| 783119 ~ 784292 | D486 | Ar8 | D1~485,D487~1175 |
| 784293 ~ 785466 | D486 | Ar9 | D1~485,D487~1175 |
| 785467 ~ 786640 | D486 | Ar10 | D1~485,D487~1175 |
| 786641 ~ 787814 | D486 | Ar11 | D1~485,D487~1175 |
| 787815 ~ 788988 | D486 | Ar12 | D1~485,D487~1175 |
| 788989 ~ 790162 | D486 | Ar13 | D1~485,D487~1175 |
| 790163 ~ 791336 | D486 | Ar14 | D1~485,D487~1175 |
| 791337 ~ 792510 | D486 | Ar15 | D1~485,D487~1175 |
| 792511 ~ 793684 | D486 | Ar16 | D1~485,D487~1175 |
| 793685 ~ 794858 | D486 | Ar17 | D1~485,D487~1175 |
| 794859 ~ 796032 | D486 | Ar18 | D1~485,D487~1175 |
| 796033 ~ 797206 | D486 | Ar19 | D1~485,D487~1175 |
| 797207 ~ 798380 | D486 | Ar20 | D1~485,D487~1175 |
| 798381 ~ 799554 | D486 | Ar21 | D1~485,D487~1175 |
| 799555 ~ 800728 | D486 | Ar22 | D1~485,D487~1175 |
| 800729 ~ 801902 | D486 | Ar23 | D1~485,D487~1175 |
| 801903 ~ 803076 | D486 | Ar24 | D1~485,D487~1175 |
| 803077 ~ 804250 | D486 | Ar25 | D1~485,D487~1175 |
| 804251 ~ 805424 | D486 | Ar26 | D1~485,D487~1175 |
| 805425 ~ 806598 | D486 | Ar27 | D1~485,D487~1175 |
| 806599 ~ 807772 | D486 | Ar28 | D1~485,D487~1175 |
| 807773 ~ 808946 | D486 | Ar29 | D1~485,D487~1175 |
| 808947 ~ 810120 | D486 | Ar30 | D1~485,D487~1175 |
| 810121 ~ 811294 | D486 | Ar31 | D1~485,D487~1175 |
| 811295 ~ 812468 | D486 | Ar32 | D1~485,D487~1175 |
| 812469 ~ 813642 | D486 | Ar33 | D1~485,D487~1175 |
| 813643 ~ 814816 | D486 | Ar34 | D1~485,D487~1175 |
| 814817 ~ 815990 | D486 | Ar35 | D1~485,D487~1175 |
| 815991 ~ 817164 | D486 | Ar36 | D1~485,D487~1175 |
| 817165 ~ 818338 | D486 | Ar37 | D1~485,D487~1175 |
| 818339 ~ 819512 | D486 | Ar38 | D1~485,D487~1175 |
| 819513 ~ 820686 | D486 | Ar39 | D1~485,D487~1175 |
| 820687 ~ 821860 | D486 | Ar40 | D1~485,D487~1175 |
| 821861 ~ 823034 | D486 | Ar41 | D1~485,D487~1175 |
| 823035 ~ 824208 | D486 | Ar42 | D1~485,D487~1175 |
| 824209 ~ 825382 | D486 | Ar43 | D1~485,D487~1175 |
| 825383 ~ 826556 | D486 | Ar44 | D1~485,D487~1175 |
| 826557 ~ 827730 | D486 | Ar45 | D1~485,D487~1175 |
| 827731 ~ 828904 | D486 | Ar46 | D1~485,D487~1175 |
| 828905 ~ 830078 | D486 | Ar47 | D1~485,D487~1175 |
| 830079 ~ 831252 | D486 | Ar48 | D1~485,D487~1175 |
| 831253 ~ 832426 | D486 | Ar49 | D1~485,D487~1175 |
| 832427 ~ 833600 | D486 | Ar50 | D1~485,D487~1175 |
| 833601 ~ 834774 | D486 | Ar51 | D1~485,D487~1175 |
| 834775 ~ 835948 | D486 | Ar52 | D1~485,D487~1175 |
| 835949 ~ 837122 | D486 | Ar53 | D1~485,D487~1175 |
| 837123 ~ 838296 | D486 | Ar54 | D1~485,D487~1175 |
| 838297 ~ 839470 | D486 | Ar55 | D1~485,D487~1175 |
| 839471 ~ 840644 | D486 | Ar56 | D1~485,D487~1175 |
| 840645 ~ 841818 | D486 | Ar57 | D1~485,D487~1175 |
| 841819 ~ 842992 | D486 | Ar58 | D1~485,D487~1175 |
| 842993 ~ 844166 | D486 | Ar59 | D1~485,D487~1175 |
| 844167 ~ 845340 | D486 | H | D1~485,D487~1175 |

| No. | $R^3$ | $R^4$ | $R^5$ |
|---|---|---|---|
| 845341 ~ 846514 | D717 | Ar1 | D1~716,D718~1175 |
| 846515 ~ 847688 | D717 | Ar2 | D1~716,D718~1175 |
| 847689 ~ 848862 | D717 | Ar3 | D1~716,D718~1175 |
| 848863 ~ 850036 | D717 | Ar4 | D1~716,D718~1175 |
| 850037 ~ 851210 | D717 | Ar5 | D1~716,D718~1175 |
| 851211 ~ 852384 | D717 | Ar6 | D1~716,D718~1175 |
| 852385 ~ 853558 | D717 | Ar7 | D1~716,D718~1175 |
| 853559 ~ 854732 | D717 | Ar8 | D1~716,D718~1175 |
| 854733 ~ 855906 | D717 | Ar9 | D1~716,D718~1175 |
| 855907 ~ 857080 | D717 | Ar10 | D1~716,D718~1175 |
| 857081 ~ 858254 | D717 | Ar11 | D1~716,D718~1175 |
| 858255 ~ 859428 | D717 | Ar12 | D1~716,D718~1175 |
| 859429 ~ 860602 | D717 | Ar13 | D1~716,D718~1175 |
| 860603 ~ 861776 | D717 | Ar14 | D1~716,D718~1175 |
| 861777 ~ 862950 | D717 | Ar15 | D1~716,D718~1175 |
| 862951 ~ 864124 | D717 | Ar16 | D1~716,D718~1175 |
| 864125 ~ 865298 | D717 | Ar17 | D1~716,D718~1175 |
| 865299 ~ 866472 | D717 | Ar18 | D1~716,D718~1175 |
| 866473 ~ 867646 | D717 | Ar19 | D1~716,D718~1175 |
| 867647 ~ 868820 | D717 | Ar20 | D1~716,D718~1175 |
| 868821 ~ 869994 | D717 | Ar21 | D1~716,D718~1175 |
| 869995 ~ 871168 | D717 | Ar22 | D1~716,D718~1175 |
| 871169 ~ 872342 | D717 | Ar23 | D1~716,D718~1175 |
| 872343 ~ 873516 | D717 | Ar24 | D1~716,D718~1175 |
| 873517 ~ 874690 | D717 | Ar25 | D1~716,D718~1175 |
| 874691 ~ 875864 | D717 | Ar26 | D1~716,D718~1175 |
| 875865 ~ 877038 | D717 | Ar27 | D1~716,D718~1175 |
| 877039 ~ 878212 | D717 | Ar28 | D1~716,D718~1175 |
| 878213 ~ 879386 | D717 | Ar29 | D1~716,D718~1175 |
| 879387 ~ 880560 | D717 | Ar30 | D1~716,D718~1175 |
| 880561 ~ 881734 | D717 | Ar31 | D1~716,D718~1175 |
| 881735 ~ 882908 | D717 | Ar32 | D1~716,D718~1175 |
| 882909 ~ 884082 | D717 | Ar33 | D1~716,D718~1175 |
| 884083 ~ 885256 | D717 | Ar34 | D1~716,D718~1175 |
| 885257 ~ 886430 | D717 | Ar35 | D1~716,D718~1175 |
| 886431 ~ 887604 | D717 | Ar36 | D1~716,D718~1175 |
| 887605 ~ 888778 | D717 | Ar37 | D1~716,D718~1175 |
| 888779 ~ 889952 | D717 | Ar38 | D1~716,D718~1175 |
| 889953 ~ 891126 | D717 | Ar39 | D1~716,D718~1175 |
| 891127 ~ 892300 | D717 | Ar40 | D1~716,D718~1175 |
| 892301 ~ 893474 | D717 | Ar41 | D1~716,D718~1175 |
| 893475 ~ 894648 | D717 | Ar42 | D1~716,D718~1175 |
| 894649 ~ 895822 | D717 | Ar43 | D1~716,D718~1175 |
| 895823 ~ 896996 | D717 | Ar44 | D1~716,D718~1175 |
| 896997 ~ 898170 | D717 | Ar45 | D1~716,D718~1175 |
| 898171 ~ 899344 | D717 | Ar46 | D1~716,D718~1175 |
| 899345 ~ 900518 | D717 | Ar47 | D1~716,D718~1175 |
| 900519 ~ 901692 | D717 | Ar48 | D1~716,D718~1175 |
| 901693 ~ 902866 | D717 | Ar49 | D1~716,D718~1175 |
| 902867 ~ 904040 | D717 | Ar50 | D1~716,D718~1175 |
| 904041 ~ 905214 | D717 | Ar51 | D1~716,D718~1175 |
| 905215 ~ 906388 | D717 | Ar52 | D1~716,D718~1175 |
| 906389 ~ 907562 | D717 | Ar53 | D1~716,D718~1175 |
| 907563 ~ 908736 | D717 | Ar54 | D1~716,D718~1175 |
| 908737 ~ 909910 | D717 | Ar55 | D1~716,D718~1175 |
| 909911 ~ 911084 | D717 | Ar56 | D1~716,D718~1175 |
| 911085 ~ 912258 | D717 | Ar57 | D1~716,D718~1175 |
| 912259 ~ 913432 | D717 | Ar58 | D1~716,D718~1175 |
| 913433 ~ 914606 | D717 | Ar59 | D1~716,D718~1175 |
| 914607 ~ 915780 | D717 | H | D1~716,D718~1175 |

| No. | $R^3$ | $R^4$ | $R^5$ | = |
|---|---|---|---|---|
| 915781 ~ 916955 | D1~D1175 | D1 | D1~D1175 | $R^3 = R^5$ |
| 916956 ~ 918130 | D1~D1175 | D7 | D1~D1175 | |
| 918131 ~ 919305 | D1~D1175 | D8 | D1~D1175 | |
| 919306 ~ 920480 | D1~D1175 | D9 | D1~D1175 | |
| 920481 ~ 921655 | D1~D1175 | D19 | D1~D1175 | |
| 921656 ~ 922830 | D1~D1175 | D37 | D1~D1175 | |
| 922831 ~ 924005 | D1~D1175 | D50 | D1~D1175 | |
| 924006 ~ 925180 | D1~D1175 | D67 | D1~D1175 | |
| 925181 ~ 926355 | D1~D1175 | D77 | D1~D1175 | |
| 926356 ~ 927530 | D1~D1175 | D78 | D1~D1175 | |
| 927531 ~ 928705 | D1~D1175 | D79 | D1~D1175 | |
| 928706 ~ 929880 | D1~D1175 | D80 | D1~D1175 | |
| 929881 ~ 931055 | D1~D1175 | D81 | D1~D1175 | |
| 931056 ~ 932230 | D1~D1175 | D465 | D1~D1175 | |
| 932231 ~ 933405 | D1~D1175 | D466 | D1~D1175 | |
| 933406 ~ 934580 | D1~D1175 | D467 | D1~D1175 | |
| 934581 ~ 935755 | D1~D1175 | D471 | D1~D1175 | |
| 935756 ~ 936930 | D1~D1175 | D486 | D1~D1175 | |
| 936931 ~ 938105 | D1~D1175 | D717 | D1~D1175 | |
| 938106 ~ 939280 | D1~D1175 | D735 | D1~D1175 | |
| 939281 ~ 940455 | D1~D1175 | D783 | D1~D1175 | |
| 940456 ~ 941630 | D1~D1175 | D793 | D1~D1175 | |
| 941631 ~ 942805 | D1~D1175 | D794 | D1~D1175 | |
| 942806 ~ 943980 | D1~D1175 | D795 | D1~D1175 | |
| 943981 ~ 945155 | D1~D1175 | D796 | D1~D1175 | |
| 945156 ~ 946330 | D1~D1175 | D797 | D1~D1175 | |
| 946331 ~ 947505 | H | Ar1 | D1~D1175 | |
| 947506 ~ 948680 | H | Ar2 | D1~D1175 | |
| 948681 ~ 949855 | H | Ar3 | D1~D1175 | |
| 949856 ~ 951030 | H | Ar4 | D1~D1175 | |
| 951031 ~ 952205 | H | Ar5 | D1~D1175 | |
| 952206 ~ 953380 | H | Ar6 | D1~D1175 | |
| 953381 ~ 954555 | H | Ar7 | D1~D1175 | |
| 954556 ~ 955730 | H | Ar8 | D1~D1175 | |
| 955731 ~ 956905 | H | Ar9 | D1~D1175 | |
| 956906 ~ 958080 | H | Ar10 | D1~D1175 | |
| 958081 ~ 959255 | H | Ar11 | D1~D1175 | |
| 959256 ~ 960430 | H | Ar12 | D1~D1175 | |
| 960431 ~ 961605 | H | Ar13 | D1~D1175 | |
| 961606 ~ 962780 | H | Ar14 | D1~D1175 | |
| 962781 ~ 963955 | H | Ar15 | D1~D1175 | |
| 963956 ~ 965130 | H | Ar16 | D1~D1175 | |
| 965131 ~ 966305 | H | Ar17 | D1~D1175 | |
| 966306 ~ 967480 | H | Ar18 | D1~D1175 | |
| 967481 ~ 968655 | H | Ar19 | D1~D1175 | |
| 968656 ~ 969830 | H | Ar20 | D1~D1175 | |
| 969831 ~ 971005 | H | Ar21 | D1~D1175 | |
| 971006 ~ 972180 | H | Ar22 | D1~D1175 | |
| 972181 ~ 973355 | H | Ar23 | D1~D1175 | |
| 973356 ~ 974530 | H | Ar24 | D1~D1175 | |
| 974531 ~ 975705 | H | Ar25 | D1~D1175 | |
| 975706 ~ 976880 | H | Ar26 | D1~D1175 | |
| 976881 ~ 978055 | H | Ar27 | D1~D1175 | |
| 978056 ~ 979230 | H | Ar28 | D1~D1175 | |
| 979231 ~ 980405 | H | Ar29 | D1~D1175 | |
| 980406 ~ 981580 | H | Ar30 | D1~D1175 | |
| 981581 ~ 982755 | H | Ar31 | D1~D1175 | |
| 982756 ~ 983930 | H | Ar32 | D1~D1175 | |
| 983931 ~ 985105 | H | Ar33 | D1~D1175 | |
| 985106 ~ 986280 | H | Ar34 | D1~D1175 | |

| No. | $R^3$ | $R^4$ | $R^5$ |
|---|---|---|---|
| 986281 ~ 987455 | H | Ar35 | D1~D1175 |
| 987456 ~ 988630 | H | Ar36 | D1~D1175 |
| 988631 ~ 989805 | H | Ar37 | D1~D1175 |
| 989806 ~ 990980 | H | Ar38 | D1~D1175 |
| 990981 ~ 992155 | H | Ar39 | D1~D1175 |
| 992156 ~ 993330 | H | Ar40 | D1~D1175 |
| 993331 ~ 994505 | H | Ar41 | D1~D1175 |
| 994506 ~ 995680 | H | Ar42 | D1~D1175 |
| 995681 ~ 996855 | H | Ar43 | D1~D1175 |
| 996856 ~ 998030 | H | Ar44 | D1~D1175 |
| 998031 ~ 999205 | H | Ar45 | D1~D1175 |
| 999206 ~ 1000380 | H | Ar46 | D1~D1175 |
| 1000381 ~ 1001555 | H | Ar47 | D1~D1175 |
| 1001556 ~ 1002730 | H | Ar48 | D1~D1175 |
| 1002731 ~ 1003905 | H | Ar49 | D1~D1175 |
| 1003906 ~ 1005080 | H | Ar50 | D1~D1175 |
| 1005081 ~ 1006255 | H | Ar51 | D1~D1175 |
| 1006256 ~ 1007430 | H | Ar52 | D1~D1175 |
| 1007431 ~ 1008605 | H | Ar53 | D1~D1175 |
| 1008606 ~ 1009780 | H | Ar54 | D1~D1175 |
| 1009781 ~ 1010955 | H | Ar55 | D1~D1175 |
| 1010956 ~ 1012130 | H | Ar56 | D1~D1175 |
| 1012131 ~ 1013305 | H | Ar57 | D1~D1175 |
| 1013306 ~ 1014480 | H | Ar58 | D1~D1175 |
| 1014481 ~ 1015655 | H | Ar59 | D1~D1175 |

Next, specific examples of the compound having a structure represented by the following general formula (1b) are shown in Table 3. In Table 3, structures of the compounds are shown in the same manner as in Table 2.

General Formula (1b)

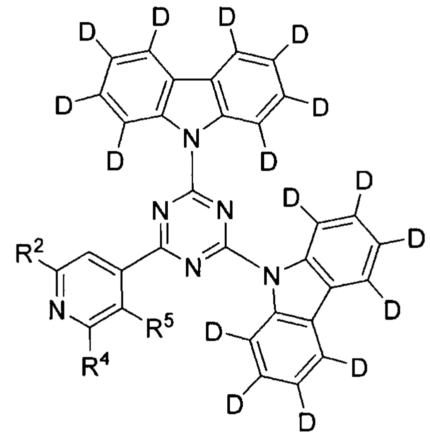

[Table 3]

| No. | $R^2$ | $R^4$ | $R^5$ | = |
|---|---|---|---|---|
| 1015656 ~ 1016830 | D1~D1175 | Ar1 | D1~D1175 | $R^2$ = $R^5$ |
| 1016831 ~ 1018005 | D1~D1175 | Ar2 | D1~D1175 | |
| 1018006 ~ 1019180 | D1~D1175 | Ar3 | D1~D1175 | |
| 1019181 ~ 1020355 | D1~D1175 | Ar4 | D1~D1175 | |
| 1020356 ~ 1021530 | D1~D1175 | Ar5 | D1~D1175 | |
| 1021531 ~ 1022705 | D1~D1175 | Ar6 | D1~D1175 | |
| 1022706 ~ 1023880 | D1~D1175 | Ar7 | D1~D1175 | |
| 1023881 ~ 1025055 | D1~D1175 | Ar8 | D1~D1175 | |
| 1025056 ~ 1026230 | D1~D1175 | Ar9 | D1~D1175 | |
| 1026231 ~ 1027405 | D1~D1175 | Ar10 | D1~D1175 | |
| 1027406 ~ 1028580 | D1~D1175 | Ar11 | D1~D1175 | |
| 1028581 ~ 1029755 | D1~D1175 | Ar12 | D1~D1175 | |
| 1029756 ~ 1030930 | D1~D1175 | Ar13 | D1~D1175 | |
| 1030931 ~ 1032105 | D1~D1175 | Ar14 | D1~D1175 | |
| 1032106 ~ 1033280 | D1~D1175 | Ar15 | D1~D1175 | |
| 1033281 ~ 1034455 | D1~D1175 | Ar16 | D1~D1175 | |
| 1034456 ~ 1035630 | D1~D1175 | Ar17 | D1~D1175 | |
| 1035631 ~ 1036805 | D1~D1175 | Ar18 | D1~D1175 | |
| 1036806 ~ 1037980 | D1~D1175 | Ar19 | D1~D1175 | |
| 1037981 ~ 1039155 | D1~D1175 | Ar20 | D1~D1175 | |
| 1039156 ~ 1040330 | D1~D1175 | Ar21 | D1~D1175 | |
| 1040331 ~ 1041505 | D1~D1175 | Ar22 | D1~D1175 | |
| 1041506 ~ 1042680 | D1~D1175 | Ar23 | D1~D1175 | |
| 1042681 ~ 1043855 | D1~D1175 | Ar24 | D1~D1175 | |
| 1043856 ~ 1045030 | D1~D1175 | Ar25 | D1~D1175 | |
| 1045031 ~ 1046205 | D1~D1175 | Ar26 | D1~D1175 | |
| 1046206 ~ 1047380 | D1~D1175 | Ar27 | D1~D1175 | |
| 1047381 ~ 1048555 | D1~D1175 | Ar28 | D1~D1175 | |
| 1048556 ~ 1049730 | D1~D1175 | Ar29 | D1~D1175 | |
| 1049731 ~ 1050905 | D1~D1175 | Ar30 | D1~D1175 | |
| 1050906 ~ 1052080 | D1~D1175 | Ar31 | D1~D1175 | |
| 1052081 ~ 1053255 | D1~D1175 | Ar32 | D1~D1175 | |
| 1053256 ~ 1054430 | D1~D1175 | Ar33 | D1~D1175 | |
| 1054431 ~ 1055605 | D1~D1175 | Ar34 | D1~D1175 | |
| 1055606 ~ 1056780 | D1~D1175 | Ar35 | D1~D1175 | |
| 1056781 ~ 1057955 | D1~D1175 | Ar36 | D1~D1175 | |
| 1057956 ~ 1059130 | D1~D1175 | Ar37 | D1~D1175 | |
| 1059131 ~ 1060305 | D1~D1175 | Ar38 | D1~D1175 | |
| 1060306 ~ 1061480 | D1~D1175 | Ar39 | D1~D1175 | |
| 1061481 ~ 1062655 | D1~D1175 | Ar40 | D1~D1175 | |
| 1062656 ~ 1063830 | D1~D1175 | Ar41 | D1~D1175 | |
| 1063831 ~ 1065005 | D1~D1175 | Ar42 | D1~D1175 | |
| 1065006 ~ 1066180 | D1~D1175 | Ar43 | D1~D1175 | |
| 1066181 ~ 1067355 | D1~D1175 | Ar44 | D1~D1175 | |
| 1067356 ~ 1068530 | D1~D1175 | Ar45 | D1~D1175 | |
| 1068531 ~ 1069705 | D1~D1175 | Ar46 | D1~D1175 | |
| 1069706 ~ 1070880 | D1~D1175 | Ar47 | D1~D1175 | |
| 1070881 ~ 1072055 | D1~D1175 | Ar48 | D1~D1175 | |
| 1072056 ~ 1073230 | D1~D1175 | Ar49 | D1~D1175 | |
| 1073231 ~ 1074405 | D1~D1175 | Ar50 | D1~D1175 | |
| 1074406 ~ 1075580 | D1~D1175 | Ar51 | D1~D1175 | |
| 1075581 ~ 1076755 | D1~D1175 | Ar52 | D1~D1175 | |
| 1076756 ~ 1077930 | D1~D1175 | Ar53 | D1~D1175 | |
| 1077931 ~ 1079105 | D1~D1175 | Ar54 | D1~D1175 | |
| 1079106 ~ 1080280 | D1~D1175 | Ar55 | D1~D1175 | |
| 1080281 ~ 1081455 | D1~D1175 | Ar56 | D1~D1175 | |
| 1081456 ~ 1082630 | D1~D1175 | Ar57 | D1~D1175 | |
| 1082631 ~ 1083805 | D1~D1175 | Ar58 | D1~D1175 | |
| 1083806 ~ 1084980 | D1~D1175 | Ar59 | D1~D1175 | |
| 1084981 ~ 1086155 | D1~D1175 | H | D1~D1175 | |

| No. | $R^2$ | $R^4$ | $R^5$ |
|---|---|---|---|
| 1086156 ~ 1087329 | D1 | Ar1 | D2~D1175 |
| 1087330 ~ 1088503 | D1 | Ar2 | D2~D1175 |
| 1088504 ~ 1089677 | D1 | Ar3 | D2~D1175 |
| 1089678 ~ 1090851 | D1 | Ar4 | D2~D1175 |
| 1090852 ~ 1092025 | D1 | Ar5 | D2~D1175 |
| 1092026 ~ 1093199 | D1 | Ar6 | D2~D1175 |
| 1093200 ~ 1094373 | D1 | Ar7 | D2~D1175 |
| 1094374 ~ 1095547 | D1 | Ar8 | D2~D1175 |
| 1095548 ~ 1096721 | D1 | Ar9 | D2~D1175 |
| 1096722 ~ 1097895 | D1 | Ar10 | D2~D1175 |
| 1097896 ~ 1099069 | D1 | Ar11 | D2~D1175 |
| 1099070 ~ 1100243 | D1 | Ar12 | D2~D1175 |
| 1100244 ~ 1101417 | D1 | Ar13 | D2~D1175 |
| 1101418 ~ 1102591 | D1 | Ar14 | D2~D1175 |
| 1102592 ~ 1103765 | D1 | Ar15 | D2~D1175 |
| 1103766 ~ 1104939 | D1 | Ar16 | D2~D1175 |
| 1104940 ~ 1106113 | D1 | Ar17 | D2~D1175 |
| 1106114 ~ 1107287 | D1 | Ar18 | D2~D1175 |
| 1107288 ~ 1108461 | D1 | Ar19 | D2~D1175 |
| 1108462 ~ 1109635 | D1 | Ar20 | D2~D1175 |
| 1109636 ~ 1110809 | D1 | Ar21 | D2~D1175 |
| 1110810 ~ 1111983 | D1 | Ar22 | D2~D1175 |
| 1111984 ~ 1113157 | D1 | Ar23 | D2~D1175 |
| 1113158 ~ 1114331 | D1 | Ar24 | D2~D1175 |
| 1114332 ~ 1115505 | D1 | Ar25 | D2~D1175 |
| 1115506 ~ 1116679 | D1 | Ar26 | D2~D1175 |
| 1116680 ~ 1117853 | D1 | Ar27 | D2~D1175 |
| 1117854 ~ 1119027 | D1 | Ar28 | D2~D1175 |
| 1119028 ~ 1120201 | D1 | Ar29 | D2~D1175 |
| 1120202 ~ 1121375 | D1 | Ar30 | D2~D1175 |
| 1121376 ~ 1122549 | D1 | Ar31 | D2~D1175 |
| 1122550 ~ 1123723 | D1 | Ar32 | D2~D1175 |
| 1123724 ~ 1124897 | D1 | Ar33 | D2~D1175 |
| 1124898 ~ 1126071 | D1 | Ar34 | D2~D1175 |
| 1126072 ~ 1127245 | D1 | Ar35 | D2~D1175 |
| 1127246 ~ 1128419 | D1 | Ar36 | D2~D1175 |
| 1128420 ~ 1129593 | D1 | Ar37 | D2~D1175 |
| 1129594 ~ 1130767 | D1 | Ar38 | D2~D1175 |
| 1130768 ~ 1131941 | D1 | Ar39 | D2~D1175 |
| 1131942 ~ 1133115 | D1 | Ar40 | D2~D1175 |
| 1133116 ~ 1134289 | D1 | Ar41 | D2~D1175 |
| 1134290 ~ 1135463 | D1 | Ar42 | D2~D1175 |
| 1135464 ~ 1136637 | D1 | Ar43 | D2~D1175 |
| 1136638 ~ 1137811 | D1 | Ar44 | D2~D1175 |
| 1137812 ~ 1138985 | D1 | Ar45 | D2~D1175 |
| 1138986 ~ 1140159 | D1 | Ar46 | D2~D1175 |
| 1140160 ~ 1141333 | D1 | Ar47 | D2~D1175 |
| 1141334 ~ 1142507 | D1 | Ar48 | D2~D1175 |
| 1142508 ~ 1143681 | D1 | Ar49 | D2~D1175 |
| 1143682 ~ 1144855 | D1 | Ar50 | D2~D1175 |
| 1144856 ~ 1146029 | D1 | Ar51 | D2~D1175 |
| 1146030 ~ 1147203 | D1 | Ar52 | D2~D1175 |
| 1147204 ~ 1148377 | D1 | Ar53 | D2~D1175 |
| 1148378 ~ 1149551 | D1 | Ar54 | D2~D1175 |
| 1149552 ~ 1150725 | D1 | Ar55 | D2~D1175 |
| 1150726 ~ 1151899 | D1 | Ar56 | D2~D1175 |
| 1151900 ~ 1153073 | D1 | Ar57 | D2~D1175 |
| 1153074 ~ 1154247 | D1 | Ar58 | D2~D1175 |
| 1154248 ~ 1155421 | D1 | Ar59 | D2~D1175 |
| 1155422 ~ 1156595 | D1 | H | D2~D1175 |

| No. | $R^2$ | $R^4$ | $R^5$ |
|---|---|---|---|
| 1156596 ~ 1157769 | D7 | Ar1 | D1~6,D8~1175 |
| 1157770 ~ 1158943 | D7 | Ar2 | D1~6,D8~1175 |
| 1158944 ~ 1160117 | D7 | Ar3 | D1~6,D8~1175 |
| 1160118 ~ 1161291 | D7 | Ar4 | D1~6,D8~1175 |
| 1161292 ~ 1162465 | D7 | Ar5 | D1~6,D8~1175 |
| 1162466 ~ 1163639 | D7 | Ar6 | D1~6,D8~1175 |
| 1163640 ~ 1164813 | D7 | Ar7 | D1~6,D8~1175 |
| 1164814 ~ 1165987 | D7 | Ar8 | D1~6,D8~1175 |
| 1165988 ~ 1167161 | D7 | Ar9 | D1~6,D8~1175 |
| 1167162 ~ 1168335 | D7 | Ar10 | D1~6,D8~1175 |
| 1168336 ~ 1169509 | D7 | Ar11 | D1~6,D8~1175 |
| 1169510 ~ 1170683 | D7 | Ar12 | D1~6,D8~1175 |
| 1170684 ~ 1171857 | D7 | Ar13 | D1~6,D8~1175 |
| 1171858 ~ 1173031 | D7 | Ar14 | D1~6,D8~1175 |
| 1173032 ~ 1174205 | D7 | Ar15 | D1~6,D8~1175 |
| 1174206 ~ 1175379 | D7 | Ar16 | D1~6,D8~1175 |
| 1175380 ~ 1176553 | D7 | Ar17 | D1~6,D8~1175 |
| 1176554 ~ 1177727 | D7 | Ar18 | D1~6,D8~1175 |
| 1177728 ~ 1178901 | D7 | Ar19 | D1~6,D8~1175 |
| 1178902 ~ 1180075 | D7 | Ar20 | D1~6,D8~1175 |
| 1180076 ~ 1181249 | D7 | Ar21 | D1~6,D8~1175 |
| 1181250 ~ 1182423 | D7 | Ar22 | D1~6,D8~1175 |
| 1182424 ~ 1183597 | D7 | Ar23 | D1~6,D8~1175 |
| 1183598 ~ 1184771 | D7 | Ar24 | D1~6,D8~1175 |
| 1184772 ~ 1185945 | D7 | Ar25 | D1~6,D8~1175 |
| 1185946 ~ 1187119 | D7 | Ar26 | D1~6,D8~1175 |
| 1187120 ~ 1188293 | D7 | Ar27 | D1~6,D8~1175 |
| 1188294 ~ 1189467 | D7 | Ar28 | D1~6,D8~1175 |
| 1189468 ~ 1190641 | D7 | Ar29 | D1~6,D8~1175 |
| 1190642 ~ 1191815 | D7 | Ar30 | D1~6,D8~1175 |
| 1191816 ~ 1192989 | D7 | Ar31 | D1~6,D8~1175 |
| 1192990 ~ 1194163 | D7 | Ar32 | D1~6,D8~1175 |
| 1194164 ~ 1195337 | D7 | Ar33 | D1~6,D8~1175 |
| 1195338 ~ 1196511 | D7 | Ar34 | D1~6,D8~1175 |
| 1196512 ~ 1197685 | D7 | Ar35 | D1~6,D8~1175 |
| 1197686 ~ 1198859 | D7 | Ar36 | D1~6,D8~1175 |
| 1198860 ~ 1200033 | D7 | Ar37 | D1~6,D8~1175 |
| 1200034 ~ 1201207 | D7 | Ar38 | D1~6,D8~1175 |
| 1201208 ~ 1202381 | D7 | Ar39 | D1~6,D8~1175 |
| 1202382 ~ 1203555 | D7 | Ar40 | D1~6,D8~1175 |
| 1203556 ~ 1204729 | D7 | Ar41 | D1~6,D8~1175 |
| 1204730 ~ 1205903 | D7 | Ar42 | D1~6,D8~1175 |
| 1205904 ~ 1207077 | D7 | Ar43 | D1~6,D8~1175 |
| 1207078 ~ 1208251 | D7 | Ar44 | D1~6,D8~1175 |
| 1208252 ~ 1209425 | D7 | Ar45 | D1~6,D8~1175 |
| 1209426 ~ 1210599 | D7 | Ar46 | D1~6,D8~1175 |
| 1210600 ~ 1211773 | D7 | Ar47 | D1~6,D8~1175 |
| 1211774 ~ 1212947 | D7 | Ar48 | D1~6,D8~1175 |
| 1212948 ~ 1214121 | D7 | Ar49 | D1~6,D8~1175 |
| 1214122 ~ 1215295 | D7 | Ar50 | D1~6,D8~1175 |
| 1215296 ~ 1216469 | D7 | Ar51 | D1~6,D8~1175 |
| 1216470 ~ 1217643 | D7 | Ar52 | D1~6,D8~1175 |
| 1217644 ~ 1218817 | D7 | Ar53 | D1~6,D8~1175 |
| 1218818 ~ 1219991 | D7 | Ar54 | D1~6,D8~1175 |
| 1219992 ~ 1221165 | D7 | Ar55 | D1~6,D8~1175 |
| 1221166 ~ 1222339 | D7 | Ar56 | D1~6,D8~1175 |
| 1222340 ~ 1223513 | D7 | Ar57 | D1~6,D8~1175 |
| 1223514 ~ 1224687 | D7 | Ar58 | D1~6,D8~1175 |
| 1224688 ~ 1225861 | D7 | Ar59 | D1~6,D8~1175 |
| 1225862 ~ 1227035 | D7 | H | D1~6,D8~1175 |

| No. | $R^2$ | $R^4$ | $R^5$ |
|---|---|---|---|
| 1227036 ~ 1228209 | D8 | Ar1 | D1~7,D9~1175 |
| 1228210 ~ 1229383 | D8 | Ar2 | D1~7,D9~1175 |
| 1229384 ~ 1230557 | D8 | Ar3 | D1~7,D9~1175 |
| 1230558 ~ 1231731 | D8 | Ar4 | D1~7,D9~1175 |
| 1231732 ~ 1232905 | D8 | Ar5 | D1~7,D9~1175 |
| 1232906 ~ 1234079 | D8 | Ar6 | D1~7,D9~1175 |
| 1234080 ~ 1235253 | D8 | Ar7 | D1~7,D9~1175 |
| 1235254 ~ 1236427 | D8 | Ar8 | D1~7,D9~1175 |
| 1236428 ~ 1237601 | D8 | Ar9 | D1~7,D9~1175 |
| 1237602 ~ 1238775 | D8 | Ar10 | D1~7,D9~1175 |
| 1238776 ~ 1239949 | D8 | Ar11 | D1~7,D9~1175 |
| 1239950 ~ 1241123 | D8 | Ar12 | D1~7,D9~1175 |
| 1241124 ~ 1242297 | D8 | Ar13 | D1~7,D9~1175 |
| 1242298 ~ 1243471 | D8 | Ar14 | D1~7,D9~1175 |
| 1243472 ~ 1244645 | D8 | Ar15 | D1~7,D9~1175 |
| 1244646 ~ 1245819 | D8 | Ar16 | D1~7,D9~1175 |
| 1245820 ~ 1246993 | D8 | Ar17 | D1~7,D9~1175 |
| 1246994 ~ 1248167 | D8 | Ar18 | D1~7,D9~1175 |
| 1248168 ~ 1249341 | D8 | Ar19 | D1~7,D9~1175 |
| 1249342 ~ 1250515 | D8 | Ar20 | D1~7,D9~1175 |
| 1250516 ~ 1251689 | D8 | Ar21 | D1~7,D9~1175 |
| 1251690 ~ 1252863 | D8 | Ar22 | D1~7,D9~1175 |
| 1252864 ~ 1254037 | D8 | Ar23 | D1~7,D9~1175 |
| 1254038 ~ 1255211 | D8 | Ar24 | D1~7,D9~1175 |
| 1255212 ~ 1256385 | D8 | Ar25 | D1~7,D9~1175 |
| 1256386 ~ 1257559 | D8 | Ar26 | D1~7,D9~1175 |
| 1257560 ~ 1258733 | D8 | Ar27 | D1~7,D9~1175 |
| 1258734 ~ 1259907 | D8 | Ar28 | D1~7,D9~1175 |
| 1259908 ~ 1261081 | D8 | Ar29 | D1~7,D9~1175 |
| 1261082 ~ 1262255 | D8 | Ar30 | D1~7,D9~1175 |
| 1262256 ~ 1263429 | D8 | Ar31 | D1~7,D9~1175 |
| 1263430 ~ 1264603 | D8 | Ar32 | D1~7,D9~1175 |
| 1264604 ~ 1265777 | D8 | Ar33 | D1~7,D9~1175 |
| 1265778 ~ 1266951 | D8 | Ar34 | D1~7,D9~1175 |
| 1266952 ~ 1268125 | D8 | Ar35 | D1~7,D9~1175 |
| 1268126 ~ 1269299 | D8 | Ar36 | D1~7,D9~1175 |
| 1269300 ~ 1270473 | D8 | Ar37 | D1~7,D9~1175 |
| 1270474 ~ 1271647 | D8 | Ar38 | D1~7,D9~1175 |
| 1271648 ~ 1272821 | D8 | Ar39 | D1~7,D9~1175 |
| 1272822 ~ 1273995 | D8 | Ar40 | D1~7,D9~1175 |
| 1273996 ~ 1275169 | D8 | Ar41 | D1~7,D9~1175 |
| 1275170 ~ 1276343 | D8 | Ar42 | D1~7,D9~1175 |
| 1276344 ~ 1277517 | D8 | Ar43 | D1~7,D9~1175 |
| 1277518 ~ 1278691 | D8 | Ar44 | D1~7,D9~1175 |
| 1278692 ~ 1279865 | D8 | Ar45 | D1~7,D9~1175 |
| 1279866 ~ 1281039 | D8 | Ar46 | D1~7,D9~1175 |
| 1281040 ~ 1282213 | D8 | Ar47 | D1~7,D9~1175 |
| 1282214 ~ 1283387 | D8 | Ar48 | D1~7,D9~1175 |
| 1283388 ~ 1284561 | D8 | Ar49 | D1~7,D9~1175 |
| 1284562 ~ 1285735 | D8 | Ar50 | D1~7,D9~1175 |
| 1285736 ~ 1286909 | D8 | Ar51 | D1~7,D9~1175 |
| 1286910 ~ 1288083 | D8 | Ar52 | D1~7,D9~1175 |
| 1288084 ~ 1289257 | D8 | Ar53 | D1~7,D9~1175 |
| 1289258 ~ 1290431 | D8 | Ar54 | D1~7,D9~1175 |
| 1290432 ~ 1291605 | D8 | Ar55 | D1~7,D9~1175 |
| 1291606 ~ 1292779 | D8 | Ar56 | D1~7,D9~1175 |
| 1292780 ~ 1293953 | D8 | Ar57 | D1~7,D9~1175 |
| 1293954 ~ 1295127 | D8 | Ar58 | D1~7,D9~1175 |
| 1295128 ~ 1296301 | D8 | Ar59 | D1~7,D9~1175 |
| 1296302 ~ 1297475 | D8 | H | D1~7,D9~1175 |

| No. | $R^2$ | $R^4$ | $R^5$ |
|---|---|---|---|
| 1297476 ~ 1298649 | D9 | Ar1 | D1~8,D10~1175 |
| 1298650 ~ 1299823 | D9 | Ar2 | D1~8,D10~1175 |
| 1299824 ~ 1300997 | D9 | Ar3 | D1~8,D10~1175 |
| 1300998 ~ 1302171 | D9 | Ar4 | D1~8,D10~1175 |
| 1302172 ~ 1303345 | D9 | Ar5 | D1~8,D10~1175 |
| 1303346 ~ 1304519 | D9 | Ar6 | D1~8,D10~1175 |
| 1304520 ~ 1305693 | D9 | Ar7 | D1~8,D10~1175 |
| 1305694 ~ 1306867 | D9 | Ar8 | D1~8,D10~1175 |
| 1306868 ~ 1308041 | D9 | Ar9 | D1~8,D10~1175 |
| 1308042 ~ 1309215 | D9 | Ar10 | D1~8,D10~1175 |
| 1309216 ~ 1310389 | D9 | Ar11 | D1~8,D10~1175 |
| 1310390 ~ 1311563 | D9 | Ar12 | D1~8,D10~1175 |
| 1311564 ~ 1312737 | D9 | Ar13 | D1~8,D10~1175 |
| 1312738 ~ 1313911 | D9 | Ar14 | D1~8,D10~1175 |
| 1313912 ~ 1315085 | D9 | Ar15 | D1~8,D10~1175 |
| 1315086 ~ 1316259 | D9 | Ar16 | D1~8,D10~1175 |
| 1316260 ~ 1317433 | D9 | Ar17 | D1~8,D10~1175 |
| 1317434 ~ 1318607 | D9 | Ar18 | D1~8,D10~1175 |
| 1318608 ~ 1319781 | D9 | Ar19 | D1~8,D10~1175 |
| 1319782 ~ 1320955 | D9 | Ar20 | D1~8,D10~1175 |
| 1320956 ~ 1322129 | D9 | Ar21 | D1~8,D10~1175 |
| 1322130 ~ 1323303 | D9 | Ar22 | D1~8,D10~1175 |
| 1323304 ~ 1324477 | D9 | Ar23 | D1~8,D10~1175 |
| 1324478 ~ 1325651 | D9 | Ar24 | D1~8,D10~1175 |
| 1325652 ~ 1326825 | D9 | Ar25 | D1~8,D10~1175 |
| 1326826 ~ 1327999 | D9 | Ar26 | D1~8,D10~1175 |
| 1328000 ~ 1329173 | D9 | Ar27 | D1~8,D10~1175 |
| 1329174 ~ 1330347 | D9 | Ar28 | D1~8,D10~1175 |
| 1330348 ~ 1331521 | D9 | Ar29 | D1~8,D10~1175 |
| 1331522 ~ 1332695 | D9 | Ar30 | D1~8,D10~1175 |
| 1332696 ~ 1333869 | D9 | Ar31 | D1~8,D10~1175 |
| 1333870 ~ 1335043 | D9 | Ar32 | D1~8,D10~1175 |
| 1335044 ~ 1336217 | D9 | Ar33 | D1~8,D10~1175 |
| 1336218 ~ 1337391 | D9 | Ar34 | D1~8,D10~1175 |
| 1337392 ~ 1338565 | D9 | Ar35 | D1~8,D10~1175 |
| 1338566 ~ 1339739 | D9 | Ar36 | D1~8,D10~1175 |
| 1339740 ~ 1340913 | D9 | Ar37 | D1~8,D10~1175 |
| 1340914 ~ 1342087 | D9 | Ar38 | D1~8,D10~1175 |
| 1342088 ~ 1343261 | D9 | Ar39 | D1~8,D10~1175 |
| 1343262 ~ 1344435 | D9 | Ar40 | D1~8,D10~1175 |
| 1344436 ~ 1345609 | D9 | Ar41 | D1~8,D10~1175 |
| 1345610 ~ 1346783 | D9 | Ar42 | D1~8,D10~1175 |
| 1346784 ~ 1347957 | D9 | Ar43 | D1~8,D10~1175 |
| 1347958 ~ 1349131 | D9 | Ar44 | D1~8,D10~1175 |
| 1349132 ~ 1350305 | D9 | Ar45 | D1~8,D10~1175 |
| 1350306 ~ 1351479 | D9 | Ar46 | D1~8,D10~1175 |
| 1351480 ~ 1352653 | D9 | Ar47 | D1~8,D10~1175 |
| 1352654 ~ 1353827 | D9 | Ar48 | D1~8,D10~1175 |
| 1353828 ~ 1355001 | D9 | Ar49 | D1~8,D10~1175 |
| 1355002 ~ 1356175 | D9 | Ar50 | D1~8,D10~1175 |
| 1356176 ~ 1357349 | D9 | Ar51 | D1~8,D10~1175 |
| 1357350 ~ 1358523 | D9 | Ar52 | D1~8,D10~1175 |
| 1358524 ~ 1359697 | D9 | Ar53 | D1~8,D10~1175 |
| 1359698 ~ 1360871 | D9 | Ar54 | D1~8,D10~1175 |
| 1360872 ~ 1362045 | D9 | Ar55 | D1~8,D10~1175 |
| 1362046 ~ 1363219 | D9 | Ar56 | D1~8,D10~1175 |
| 1363220 ~ 1364393 | D9 | Ar57 | D1~8,D10~1175 |
| 1364394 ~ 1365567 | D9 | Ar58 | D1~8,D10~1175 |
| 1365568 ~ 1366741 | D9 | Ar59 | D1~8,D10~1175 |
| 1366742 ~ 1367915 | D9 | H | D1~8,D10~1175 |

| No. | $R^2$ | $R^4$ | $R^5$ |
|---|---|---|---|
| 1367916 ~ 1369089 | D19 | Ar1 | D1~18,D20~1175 |
| 1369090 ~ 1370263 | D19 | Ar2 | D1~18,D20~1175 |
| 1370264 ~ 1371437 | D19 | Ar3 | D1~18,D20~1175 |
| 1371438 ~ 1372611 | D19 | Ar4 | D1~18,D20~1175 |
| 1372612 ~ 1373785 | D19 | Ar5 | D1~18,D20~1175 |
| 1373786 ~ 1374959 | D19 | Ar6 | D1~18,D20~1175 |
| 1374960 ~ 1376133 | D19 | Ar7 | D1~18,D20~1175 |
| 1376134 ~ 1377307 | D19 | Ar8 | D1~18,D20~1175 |
| 1377308 ~ 1378481 | D19 | Ar9 | D1~18,D20~1175 |
| 1378482 ~ 1379655 | D19 | Ar10 | D1~18,D20~1175 |
| 1379656 ~ 1380829 | D19 | Ar11 | D1~18,D20~1175 |
| 1380830 ~ 1382003 | D19 | Ar12 | D1~18,D20~1175 |
| 1382004 ~ 1383177 | D19 | Ar13 | D1~18,D20~1175 |
| 1383178 ~ 1384351 | D19 | Ar14 | D1~18,D20~1175 |
| 1384352 ~ 1385525 | D19 | Ar15 | D1~18,D20~1175 |
| 1385526 ~ 1386699 | D19 | Ar16 | D1~18,D20~1175 |
| 1386700 ~ 1387873 | D19 | Ar17 | D1~18,D20~1175 |
| 1387874 ~ 1389047 | D19 | Ar18 | D1~18,D20~1175 |
| 1389048 ~ 1390221 | D19 | Ar19 | D1~18,D20~1175 |
| 1390222 ~ 1391395 | D19 | Ar20 | D1~18,D20~1175 |
| 1391396 ~ 1392569 | D19 | Ar21 | D1~18,D20~1175 |
| 1392570 ~ 1393743 | D19 | Ar22 | D1~18,D20~1175 |
| 1393744 ~ 1394917 | D19 | Ar23 | D1~18,D20~1175 |
| 1394918 ~ 1396091 | D19 | Ar24 | D1~18,D20~1175 |
| 1396092 ~ 1397265 | D19 | Ar25 | D1~18,D20~1175 |
| 1397266 ~ 1398439 | D19 | Ar26 | D1~18,D20~1175 |
| 1398440 ~ 1399613 | D19 | Ar27 | D1~18,D20~1175 |
| 1399614 ~ 1400787 | D19 | Ar28 | D1~18,D20~1175 |
| 1400788 ~ 1401961 | D19 | Ar29 | D1~18,D20~1175 |
| 1401962 ~ 1403135 | D19 | Ar30 | D1~18,D20~1175 |
| 1403136 ~ 1404309 | D19 | Ar31 | D1~18,D20~1175 |
| 1404310 ~ 1405483 | D19 | Ar32 | D1~18,D20~1175 |
| 1405484 ~ 1406657 | D19 | Ar33 | D1~18,D20~1175 |
| 1406658 ~ 1407831 | D19 | Ar34 | D1~18,D20~1175 |
| 1407832 ~ 1409005 | D19 | Ar35 | D1~18,D20~1175 |
| 1409006 ~ 1410179 | D19 | Ar36 | D1~18,D20~1175 |
| 1410180 ~ 1411353 | D19 | Ar37 | D1~18,D20~1175 |
| 1411354 ~ 1412527 | D19 | Ar38 | D1~18,D20~1175 |
| 1412528 ~ 1413701 | D19 | Ar39 | D1~18,D20~1175 |
| 1413702 ~ 1414875 | D19 | Ar40 | D1~18,D20~1175 |
| 1414876 ~ 1416049 | D19 | Ar41 | D1~18,D20~1175 |
| 1416050 ~ 1417223 | D19 | Ar42 | D1~18,D20~1175 |
| 1417224 ~ 1418397 | D19 | Ar43 | D1~18,D20~1175 |
| 1418398 ~ 1419571 | D19 | Ar44 | D1~18,D20~1175 |
| 1419572 ~ 1420745 | D19 | Ar45 | D1~18,D20~1175 |
| 1420746 ~ 1421919 | D19 | Ar46 | D1~18,D20~1175 |
| 1421920 ~ 1423093 | D19 | Ar47 | D1~18,D20~1175 |
| 1423094 ~ 1424267 | D19 | Ar48 | D1~18,D20~1175 |
| 1424268 ~ 1425441 | D19 | Ar49 | D1~18,D20~1175 |
| 1425442 ~ 1426615 | D19 | Ar50 | D1~18,D20~1175 |
| 1426616 ~ 1427789 | D19 | Ar51 | D1~18,D20~1175 |
| 1427790 ~ 1428963 | D19 | Ar52 | D1~18,D20~1175 |
| 1428964 ~ 1430137 | D19 | Ar53 | D1~18,D20~1175 |
| 1430138 ~ 1431311 | D19 | Ar54 | D1~18,D20~1175 |
| 1431312 ~ 1432485 | D19 | Ar55 | D1~18,D20~1175 |
| 1432486 ~ 1433659 | D19 | Ar56 | D1~18,D20~1175 |
| 1433660 ~ 1434833 | D19 | Ar57 | D1~18,D20~1175 |
| 1434834 ~ 1436007 | D19 | Ar58 | D1~18,D20~1175 |
| 1436008 ~ 1437181 | D19 | Ar59 | D1~18,D20~1175 |
| 1437182 ~ 1438355 | D19 | H | D1~18,D20~1175 |
| 1438356 ~ 1439529 | D37 | Ar1 | D1~36,D38~1175 |
| 1439530 ~ 1440703 | D37 | Ar2 | D1~36,D38~1175 |
| 1440704 ~ 1441877 | D37 | Ar3 | D1~36,D38~1175 |
| 1441878 ~ 1443051 | D37 | Ar4 | D1~36,D38~1175 |
| 1443052 ~ 1444225 | D37 | Ar5 | D1~36,D38~1175 |
| 1444226 ~ 1445399 | D37 | Ar6 | D1~36,D38~1175 |
| 1445400 ~ 1446573 | D37 | Ar7 | D1~36,D38~1175 |
| 1446574 ~ 1447747 | D37 | Ar8 | D1~36,D38~1175 |
| 1447748 ~ 1448921 | D37 | Ar9 | D1~36,D38~1175 |
| 1448922 ~ 1450095 | D37 | Ar10 | D1~36,D38~1175 |
| 1450096 ~ 1451269 | D37 | Ar11 | D1~36,D38~1175 |
| 1451270 ~ 1452443 | D37 | Ar12 | D1~36,D38~1175 |
| 1452444 ~ 1453617 | D37 | Ar13 | D1~36,D38~1175 |
| 1453618 ~ 1454791 | D37 | Ar14 | D1~36,D38~1175 |
| 1454792 ~ 1455965 | D37 | Ar15 | D1~36,D38~1175 |
| 1455966 ~ 1457139 | D37 | Ar16 | D1~36,D38~1175 |
| 1457140 ~ 1458313 | D37 | Ar17 | D1~36,D38~1175 |
| 1458314 ~ 1459487 | D37 | Ar18 | D1~36,D38~1175 |
| 1459488 ~ 1460661 | D37 | Ar19 | D1~36,D38~1175 |
| 1460662 ~ 1461835 | D37 | Ar20 | D1~36,D38~1175 |
| 1461836 ~ 1463009 | D37 | Ar21 | D1~36,D38~1175 |
| 1463010 ~ 1464183 | D37 | Ar22 | D1~36,D38~1175 |
| 1464184 ~ 1465357 | D37 | Ar23 | D1~36,D38~1175 |
| 1465358 ~ 1466531 | D37 | Ar24 | D1~36,D38~1175 |
| 1466532 ~ 1467705 | D37 | Ar25 | D1~36,D38~1175 |
| 1467706 ~ 1468879 | D37 | Ar26 | D1~36,D38~1175 |
| 1468880 ~ 1470053 | D37 | Ar27 | D1~36,D38~1175 |
| 1470054 ~ 1471227 | D37 | Ar28 | D1~36,D38~1175 |
| 1471228 ~ 1472401 | D37 | Ar29 | D1~36,D38~1175 |
| 1472402 ~ 1473575 | D37 | Ar30 | D1~36,D38~1175 |
| 1473576 ~ 1474749 | D37 | Ar31 | D1~36,D38~1175 |
| 1474750 ~ 1475923 | D37 | Ar32 | D1~36,D38~1175 |
| 1475924 ~ 1477097 | D37 | Ar33 | D1~36,D38~1175 |
| 1477098 ~ 1478271 | D37 | Ar34 | D1~36,D38~1175 |
| 1478272 ~ 1479445 | D37 | Ar35 | D1~36,D38~1175 |
| 1479446 ~ 1480619 | D37 | Ar36 | D1~36,D38~1175 |
| 1480620 ~ 1481793 | D37 | Ar37 | D1~36,D38~1175 |
| 1481794 ~ 1482967 | D37 | Ar38 | D1~36,D38~1175 |
| 1482968 ~ 1484141 | D37 | Ar39 | D1~36,D38~1175 |
| 1484142 ~ 1485315 | D37 | Ar40 | D1~36,D38~1175 |
| 1485316 ~ 1486489 | D37 | Ar41 | D1~36,D38~1175 |
| 1486490 ~ 1487663 | D37 | Ar42 | D1~36,D38~1175 |
| 1487664 ~ 1488837 | D37 | Ar43 | D1~36,D38~1175 |
| 1488838 ~ 1490011 | D37 | Ar44 | D1~36,D38~1175 |
| 1490012 ~ 1491185 | D37 | Ar45 | D1~36,D38~1175 |
| 1491186 ~ 1492359 | D37 | Ar46 | D1~36,D38~1175 |
| 1492360 ~ 1493533 | D37 | Ar47 | D1~36,D38~1175 |
| 1493534 ~ 1494707 | D37 | Ar48 | D1~36,D38~1175 |
| 1494708 ~ 1495881 | D37 | Ar49 | D1~36,D38~1175 |
| 1495882 ~ 1497055 | D37 | Ar50 | D1~36,D38~1175 |
| 1497056 ~ 1498229 | D37 | Ar51 | D1~36,D38~1175 |
| 1498230 ~ 1499403 | D37 | Ar52 | D1~36,D38~1175 |
| 1499404 ~ 1500577 | D37 | Ar53 | D1~36,D38~1175 |
| 1500578 ~ 1501751 | D37 | Ar54 | D1~36,D38~1175 |
| 1501752 ~ 1502925 | D37 | Ar55 | D1~36,D38~1175 |
| 1502926 ~ 1504099 | D37 | Ar56 | D1~36,D38~1175 |
| 1504100 ~ 1505273 | D37 | Ar57 | D1~36,D38~1175 |
| 1505274 ~ 1506447 | D37 | Ar58 | D1~36,D38~1175 |
| 1506448 ~ 1507621 | D37 | Ar59 | D1~36,D38~1175 |
| 1507622 ~ 1508795 | D37 | H | D1~36,D38~1175 |

| No. | $R^2$ | $R^4$ | $R^5$ |
|---|---|---|---|
| 1508796 ～ 1509969 | D50 | Ar1 | D1～49,D51～1175 |
| 1509970 ～ 1511143 | D50 | Ar2 | D1～49,D51～1175 |
| 1511144 ～ 1512317 | D50 | Ar3 | D1～49,D51～1175 |
| 1512318 ～ 1513491 | D50 | Ar4 | D1～49,D51～1175 |
| 1513492 ～ 1514665 | D50 | Ar5 | D1～49,D51～1175 |
| 1514666 ～ 1515839 | D50 | Ar6 | D1～49,D51～1175 |
| 1515840 ～ 1517013 | D50 | Ar7 | D1～49,D51～1175 |
| 1517014 ～ 1518187 | D50 | Ar8 | D1～49,D51～1175 |
| 1518188 ～ 1519361 | D50 | Ar9 | D1～49,D51～1175 |
| 1519362 ～ 1520535 | D50 | Ar10 | D1～49,D51～1175 |
| 1520536 ～ 1521709 | D50 | Ar11 | D1～49,D51～1175 |
| 1521710 ～ 1522883 | D50 | Ar12 | D1～49,D51～1175 |
| 1522884 ～ 1524057 | D50 | Ar13 | D1～49,D51～1175 |
| 1524058 ～ 1525231 | D50 | Ar14 | D1～49,D51～1175 |
| 1525232 ～ 1526405 | D50 | Ar15 | D1～49,D51～1175 |
| 1526406 ～ 1527579 | D50 | Ar16 | D1～49,D51～1175 |
| 1527580 ～ 1528753 | D50 | Ar17 | D1～49,D51～1175 |
| 1528754 ～ 1529927 | D50 | Ar18 | D1～49,D51～1175 |
| 1529928 ～ 1531101 | D50 | Ar19 | D1～49,D51～1175 |
| 1531102 ～ 1532275 | D50 | Ar20 | D1～49,D51～1175 |
| 1532276 ～ 1533449 | D50 | Ar21 | D1～49,D51～1175 |
| 1533450 ～ 1534623 | D50 | Ar22 | D1～49,D51～1175 |
| 1534624 ～ 1535797 | D50 | Ar23 | D1～49,D51～1175 |
| 1535798 ～ 1536971 | D50 | Ar24 | D1～49,D51～1175 |
| 1536972 ～ 1538145 | D50 | Ar25 | D1～49,D51～1175 |
| 1538146 ～ 1539319 | D50 | Ar26 | D1～49,D51～1175 |
| 1539320 ～ 1540493 | D50 | Ar27 | D1～49,D51～1175 |
| 1540494 ～ 1541667 | D50 | Ar28 | D1～49,D51～1175 |
| 1541668 ～ 1542841 | D50 | Ar29 | D1～49,D51～1175 |
| 1542842 ～ 1544015 | D50 | Ar30 | D1～49,D51～1175 |
| 1544016 ～ 1545189 | D50 | Ar31 | D1～49,D51～1175 |
| 1545190 ～ 1546363 | D50 | Ar32 | D1～49,D51～1175 |
| 1546364 ～ 1547537 | D50 | Ar33 | D1～49,D51～1175 |
| 1547538 ～ 1548711 | D50 | Ar34 | D1～49,D51～1175 |
| 1548712 ～ 1549885 | D50 | Ar35 | D1～49,D51～1175 |
| 1549886 ～ 1551059 | D50 | Ar36 | D1～49,D51～1175 |
| 1551060 ～ 1552233 | D50 | Ar37 | D1～49,D51～1175 |
| 1552234 ～ 1553407 | D50 | Ar38 | D1～49,D51～1175 |
| 1553408 ～ 1554581 | D50 | Ar39 | D1～49,D51～1175 |
| 1554582 ～ 1555755 | D50 | Ar40 | D1～49,D51～1175 |
| 1555756 ～ 1556929 | D50 | Ar41 | D1～49,D51～1175 |
| 1556930 ～ 1558103 | D50 | Ar42 | D1～49,D51～1175 |
| 1558104 ～ 1559277 | D50 | Ar43 | D1～49,D51～1175 |
| 1559278 ～ 1560451 | D50 | Ar44 | D1～49,D51～1175 |
| 1560452 ～ 1561625 | D50 | Ar45 | D1～49,D51～1175 |
| 1561626 ～ 1562799 | D50 | Ar46 | D1～49,D51～1175 |
| 1562800 ～ 1563973 | D50 | Ar47 | D1～49,D51～1175 |
| 1563974 ～ 1565147 | D50 | Ar48 | D1～49,D51～1175 |
| 1565148 ～ 1566321 | D50 | Ar49 | D1～49,D51～1175 |
| 1566322 ～ 1567495 | D50 | Ar50 | D1～49,D51～1175 |
| 1567496 ～ 1568669 | D50 | Ar51 | D1～49,D51～1175 |
| 1568670 ～ 1569843 | D50 | Ar52 | D1～49,D51～1175 |
| 1569844 ～ 1571017 | D50 | Ar53 | D1～49,D51～1175 |
| 1571018 ～ 1572191 | D50 | Ar54 | D1～49,D51～1175 |
| 1572192 ～ 1573365 | D50 | Ar55 | D1～49,D51～1175 |
| 1573366 ～ 1574539 | D50 | Ar56 | D1～49,D51～1175 |
| 1574540 ～ 1575713 | D50 | Ar57 | D1～49,D51～1175 |
| 1575714 ～ 1576887 | D50 | Ar58 | D1～49,D51～1175 |
| 1576888 ～ 1578061 | D50 | Ar59 | D1～49,D51～1175 |
| 1578062 ～ 1579235 | D50 | H | D1～49,D51～1175 |

| No. | $R^2$ | $R^4$ | $R^5$ |
|---|---|---|---|
| 1579236 ～ 1580409 | D465 | Ar1 | D1～464,D466～1175 |
| 1580410 ～ 1581583 | D465 | Ar2 | D1～464,D466～1175 |
| 1581584 ～ 1582757 | D465 | Ar3 | D1～464,D466～1175 |
| 1582758 ～ 1583931 | D465 | Ar4 | D1～464,D466～1175 |
| 1583932 ～ 1585105 | D465 | Ar5 | D1～464,D466～1175 |
| 1585106 ～ 1586279 | D465 | Ar6 | D1～464,D466～1175 |
| 1586280 ～ 1587453 | D465 | Ar7 | D1～464,D466～1175 |
| 1587454 ～ 1588627 | D465 | Ar8 | D1～464,D466～1175 |
| 1588628 ～ 1589801 | D465 | Ar9 | D1～464,D466～1175 |
| 1589802 ～ 1590975 | D465 | Ar10 | D1～464,D466～1175 |
| 1590976 ～ 1592149 | D465 | Ar11 | D1～464,D466～1175 |
| 1592150 ～ 1593323 | D465 | Ar12 | D1～464,D466～1175 |
| 1593324 ～ 1594497 | D465 | Ar13 | D1～464,D466～1175 |
| 1594498 ～ 1595671 | D465 | Ar14 | D1～464,D466～1175 |
| 1595672 ～ 1596845 | D465 | Ar15 | D1～464,D466～1175 |
| 1596846 ～ 1598019 | D465 | Ar16 | D1～464,D466～1175 |
| 1598020 ～ 1599193 | D465 | Ar17 | D1～464,D466～1175 |
| 1599194 ～ 1600367 | D465 | Ar18 | D1～464,D466～1175 |
| 1600368 ～ 1601541 | D465 | Ar19 | D1～464,D466～1175 |
| 1601542 ～ 1602715 | D465 | Ar20 | D1～464,D466～1175 |
| 1602716 ～ 1603889 | D465 | Ar21 | D1～464,D466～1175 |
| 1603890 ～ 1605063 | D465 | Ar22 | D1～464,D466～1175 |
| 1605064 ～ 1606237 | D465 | Ar23 | D1～464,D466～1175 |
| 1606238 ～ 1607411 | D465 | Ar24 | D1～464,D466～1175 |
| 1607412 ～ 1608585 | D465 | Ar25 | D1～464,D466～1175 |
| 1608586 ～ 1609759 | D465 | Ar26 | D1～464,D466～1175 |
| 1609760 ～ 1610933 | D465 | Ar27 | D1～464,D466～1175 |
| 1610934 ～ 1612107 | D465 | Ar28 | D1～464,D466～1175 |
| 1612108 ～ 1613281 | D465 | Ar29 | D1～464,D466～1175 |
| 1613282 ～ 1614455 | D465 | Ar30 | D1～464,D466～1175 |
| 1614456 ～ 1615629 | D465 | Ar31 | D1～464,D466～1175 |
| 1615630 ～ 1616803 | D465 | Ar32 | D1～464,D466～1175 |
| 1616804 ～ 1617977 | D465 | Ar33 | D1～464,D466～1175 |
| 1617978 ～ 1619151 | D465 | Ar34 | D1～464,D466～1175 |
| 1619152 ～ 1620325 | D465 | Ar35 | D1～464,D466～1175 |
| 1620326 ～ 1621499 | D465 | Ar36 | D1～464,D466～1175 |
| 1621500 ～ 1622673 | D465 | Ar37 | D1～464,D466～1175 |
| 1622674 ～ 1623847 | D465 | Ar38 | D1～464,D466～1175 |
| 1623848 ～ 1625021 | D465 | Ar39 | D1～464,D466～1175 |
| 1625022 ～ 1626195 | D465 | Ar40 | D1～464,D466～1175 |
| 1626196 ～ 1627369 | D465 | Ar41 | D1～464,D466～1175 |
| 1627370 ～ 1628543 | D465 | Ar42 | D1～464,D466～1175 |
| 1628544 ～ 1629717 | D465 | Ar43 | D1～464,D466～1175 |
| 1629718 ～ 1630891 | D465 | Ar44 | D1～464,D466～1175 |
| 1630892 ～ 1632065 | D465 | Ar45 | D1～464,D466～1175 |
| 1632066 ～ 1633239 | D465 | Ar46 | D1～464,D466～1175 |
| 1633240 ～ 1634413 | D465 | Ar47 | D1～464,D466～1175 |
| 1634414 ～ 1635587 | D465 | Ar48 | D1～464,D466～1175 |
| 1635588 ～ 1636761 | D465 | Ar49 | D1～464,D466～1175 |
| 1636762 ～ 1637935 | D465 | Ar50 | D1～464,D466～1175 |
| 1637936 ～ 1639109 | D465 | Ar51 | D1～464,D466～1175 |
| 1639110 ～ 1640283 | D465 | Ar52 | D1～464,D466～1175 |
| 1640284 ～ 1641457 | D465 | Ar53 | D1～464,D466～1175 |
| 1641458 ～ 1642631 | D465 | Ar54 | D1～464,D466～1175 |
| 1642632 ～ 1643805 | D465 | Ar55 | D1～464,D466～1175 |
| 1643806 ～ 1644979 | D465 | Ar56 | D1～464,D466～1175 |
| 1644980 ～ 1646153 | D465 | Ar57 | D1～464,D466～1175 |
| 1646154 ～ 1647327 | D465 | Ar58 | D1～464,D466～1175 |
| 1647328 ～ 1648501 | D465 | Ar59 | D1～464,D466～1175 |
| 1648502 ～ 1649675 | D465 | H | D1～464,D466～1175 |

| No. | $R^2$ | $R^4$ | $R^5$ |
|---|---|---|---|
| 1649676 ~ 1650849 | D466 | Ar1 | D1~465,D467~1175 |
| 1650850 ~ 1652023 | D466 | Ar2 | D1~465,D467~1175 |
| 1652024 ~ 1653197 | D466 | Ar3 | D1~465,D467~1175 |
| 1653198 ~ 1654371 | D466 | Ar4 | D1~465,D467~1175 |
| 1654372 ~ 1655545 | D466 | Ar5 | D1~465,D467~1175 |
| 1655546 ~ 1656719 | D466 | Ar6 | D1~465,D467~1175 |
| 1656720 ~ 1657893 | D466 | Ar7 | D1~465,D467~1175 |
| 1657894 ~ 1659067 | D466 | Ar8 | D1~465,D467~1175 |
| 1659068 ~ 1660241 | D466 | Ar9 | D1~465,D467~1175 |
| 1660242 ~ 1661415 | D466 | Ar10 | D1~465,D467~1175 |
| 1661416 ~ 1662589 | D466 | Ar11 | D1~465,D467~1175 |
| 1662590 ~ 1663763 | D466 | Ar12 | D1~465,D467~1175 |
| 1663764 ~ 1664937 | D466 | Ar13 | D1~465,D467~1175 |
| 1664938 ~ 1666111 | D466 | Ar14 | D1~465,D467~1175 |
| 1666112 ~ 1667285 | D466 | Ar15 | D1~465,D467~1175 |
| 1667286 ~ 1668459 | D466 | Ar16 | D1~465,D467~1175 |
| 1668460 ~ 1669633 | D466 | Ar17 | D1~465,D467~1175 |
| 1669634 ~ 1670807 | D466 | Ar18 | D1~465,D467~1175 |
| 1670808 ~ 1671981 | D466 | Ar19 | D1~465,D467~1175 |
| 1671982 ~ 1673155 | D466 | Ar20 | D1~465,D467~1175 |
| 1673156 ~ 1674329 | D466 | Ar21 | D1~465,D467~1175 |
| 1674330 ~ 1675503 | D466 | Ar22 | D1~465,D467~1175 |
| 1675504 ~ 1676677 | D466 | Ar23 | D1~465,D467~1175 |
| 1676678 ~ 1677851 | D466 | Ar24 | D1~465,D467~1175 |
| 1677852 ~ 1679025 | D466 | Ar25 | D1~465,D467~1175 |
| 1679026 ~ 1680199 | D466 | Ar26 | D1~465,D467~1175 |
| 1680200 ~ 1681373 | D466 | Ar27 | D1~465,D467~1175 |
| 1681374 ~ 1682547 | D466 | Ar28 | D1~465,D467~1175 |
| 1682548 ~ 1683721 | D466 | Ar29 | D1~465,D467~1175 |
| 1683722 ~ 1684895 | D466 | Ar30 | D1~465,D467~1175 |
| 1684896 ~ 1686069 | D466 | Ar31 | D1~465,D467~1175 |
| 1686070 ~ 1687243 | D466 | Ar32 | D1~465,D467~1175 |
| 1687244 ~ 1688417 | D466 | Ar33 | D1~465,D467~1175 |
| 1688418 ~ 1689591 | D466 | Ar34 | D1~465,D467~1175 |
| 1689592 ~ 1690765 | D466 | Ar35 | D1~465,D467~1175 |
| 1690766 ~ 1691939 | D466 | Ar36 | D1~465,D467~1175 |
| 1691940 ~ 1693113 | D466 | Ar37 | D1~465,D467~1175 |
| 1693114 ~ 1694287 | D466 | Ar38 | D1~465,D467~1175 |
| 1694288 ~ 1695461 | D466 | Ar39 | D1~465,D467~1175 |
| 1695462 ~ 1696635 | D466 | Ar40 | D1~465,D467~1175 |
| 1696636 ~ 1697809 | D466 | Ar41 | D1~465,D467~1175 |
| 1697810 ~ 1698983 | D466 | Ar42 | D1~465,D467~1175 |
| 1698984 ~ 1700157 | D466 | Ar43 | D1~465,D467~1175 |
| 1700158 ~ 1701331 | D466 | Ar44 | D1~465,D467~1175 |
| 1701332 ~ 1702505 | D466 | Ar45 | D1~465,D467~1175 |
| 1702506 ~ 1703679 | D466 | Ar46 | D1~465,D467~1175 |
| 1703680 ~ 1704853 | D466 | Ar47 | D1~465,D467~1175 |
| 1704854 ~ 1706027 | D466 | Ar48 | D1~465,D467~1175 |
| 1706028 ~ 1707201 | D466 | Ar49 | D1~465,D467~1175 |
| 1707202 ~ 1708375 | D466 | Ar50 | D1~465,D467~1175 |
| 1708376 ~ 1709549 | D466 | Ar51 | D1~465,D467~1175 |
| 1709550 ~ 1710723 | D466 | Ar52 | D1~465,D467~1175 |
| 1710724 ~ 1711897 | D466 | Ar53 | D1~465,D467~1175 |
| 1711898 ~ 1713071 | D466 | Ar54 | D1~465,D467~1175 |
| 1713072 ~ 1714245 | D466 | Ar55 | D1~465,D467~1175 |
| 1714246 ~ 1715419 | D466 | Ar56 | D1~465,D467~1175 |
| 1715420 ~ 1716593 | D466 | Ar57 | D1~465,D467~1175 |
| 1716594 ~ 1717767 | D466 | Ar58 | D1~465,D467~1175 |
| 1717768 ~ 1718941 | D466 | Ar59 | D1~465,D467~1175 |
| 1718942 ~ 1720115 | D466 | H | D1~465,D467~1175 |

| No. | $R^2$ | $R^4$ | $R^5$ |
|---|---|---|---|
| 1720116 ~ 1721289 | D467 | Ar1 | D1~466,D468~1175 |
| 1721290 ~ 1722463 | D467 | Ar2 | D1~466,D468~1175 |
| 1722464 ~ 1723637 | D467 | Ar3 | D1~466,D468~1175 |
| 1723638 ~ 1724811 | D467 | Ar4 | D1~466,D468~1175 |
| 1724812 ~ 1725985 | D467 | Ar5 | D1~466,D468~1175 |
| 1725986 ~ 1727159 | D467 | Ar6 | D1~466,D468~1175 |
| 1727160 ~ 1728333 | D467 | Ar7 | D1~466,D468~1175 |
| 1728334 ~ 1729507 | D467 | Ar8 | D1~466,D468~1175 |
| 1729508 ~ 1730681 | D467 | Ar9 | D1~466,D468~1175 |
| 1730682 ~ 1731855 | D467 | Ar10 | D1~466,D468~1175 |
| 1731856 ~ 1733029 | D467 | Ar11 | D1~466,D468~1175 |
| 1733030 ~ 1734203 | D467 | Ar12 | D1~466,D468~1175 |
| 1734204 ~ 1735377 | D467 | Ar13 | D1~466,D468~1175 |
| 1735378 ~ 1736551 | D467 | Ar14 | D1~466,D468~1175 |
| 1736552 ~ 1737725 | D467 | Ar15 | D1~466,D468~1175 |
| 1737726 ~ 1738899 | D467 | Ar16 | D1~466,D468~1175 |
| 1738900 ~ 1740073 | D467 | Ar17 | D1~466,D468~1175 |
| 1740074 ~ 1741247 | D467 | Ar18 | D1~466,D468~1175 |
| 1741248 ~ 1742421 | D467 | Ar19 | D1~466,D468~1175 |
| 1742422 ~ 1743595 | D467 | Ar20 | D1~466,D468~1175 |
| 1743596 ~ 1744769 | D467 | Ar21 | D1~466,D468~1175 |
| 1744770 ~ 1745943 | D467 | Ar22 | D1~466,D468~1175 |
| 1745944 ~ 1747117 | D467 | Ar23 | D1~466,D468~1175 |
| 1747118 ~ 1748291 | D467 | Ar24 | D1~466,D468~1175 |
| 1748292 ~ 1749465 | D467 | Ar25 | D1~466,D468~1175 |
| 1749466 ~ 1750639 | D467 | Ar26 | D1~466,D468~1175 |
| 1750640 ~ 1751813 | D467 | Ar27 | D1~466,D468~1175 |
| 1751814 ~ 1752987 | D467 | Ar28 | D1~466,D468~1175 |
| 1752988 ~ 1754161 | D467 | Ar29 | D1~466,D468~1175 |
| 1754162 ~ 1755335 | D467 | Ar30 | D1~466,D468~1175 |
| 1755336 ~ 1756509 | D467 | Ar31 | D1~466,D468~1175 |
| 1756510 ~ 1757683 | D467 | Ar32 | D1~466,D468~1175 |
| 1757684 ~ 1758857 | D467 | Ar33 | D1~466,D468~1175 |
| 1758858 ~ 1760031 | D467 | Ar34 | D1~466,D468~1175 |
| 1760032 ~ 1761205 | D467 | Ar35 | D1~466,D468~1175 |
| 1761206 ~ 1762379 | D467 | Ar36 | D1~466,D468~1175 |
| 1762380 ~ 1763553 | D467 | Ar37 | D1~466,D468~1175 |
| 1763554 ~ 1764727 | D467 | Ar38 | D1~466,D468~1175 |
| 1764728 ~ 1765901 | D467 | Ar39 | D1~466,D468~1175 |
| 1765902 ~ 1767075 | D467 | Ar40 | D1~466,D468~1175 |
| 1767076 ~ 1768249 | D467 | Ar41 | D1~466,D468~1175 |
| 1768250 ~ 1769423 | D467 | Ar42 | D1~466,D468~1175 |
| 1769424 ~ 1770597 | D467 | Ar43 | D1~466,D468~1175 |
| 1770598 ~ 1771771 | D467 | Ar44 | D1~466,D468~1175 |
| 1771772 ~ 1772945 | D467 | Ar45 | D1~466,D468~1175 |
| 1772946 ~ 1774119 | D467 | Ar46 | D1~466,D468~1175 |
| 1774120 ~ 1775293 | D467 | Ar47 | D1~466,D468~1175 |
| 1775294 ~ 1776467 | D467 | Ar48 | D1~466,D468~1175 |
| 1776468 ~ 1777641 | D467 | Ar49 | D1~466,D468~1175 |
| 1777642 ~ 1778815 | D467 | Ar50 | D1~466,D468~1175 |
| 1778816 ~ 1779989 | D467 | Ar51 | D1~466,D468~1175 |
| 1779990 ~ 1781163 | D467 | Ar52 | D1~466,D468~1175 |
| 1781164 ~ 1782337 | D467 | Ar53 | D1~466,D468~1175 |
| 1782338 ~ 1783511 | D467 | Ar54 | D1~466,D468~1175 |
| 1783512 ~ 1784685 | D467 | Ar55 | D1~466,D468~1175 |
| 1784686 ~ 1785859 | D467 | Ar56 | D1~466,D468~1175 |
| 1785860 ~ 1787033 | D467 | Ar57 | D1~466,D468~1175 |
| 1787034 ~ 1788207 | D467 | Ar58 | D1~466,D468~1175 |
| 1788208 ~ 1789381 | D467 | Ar59 | D1~466,D468~1175 |
| 1789382 ~ 1790555 | D467 | H | D1~466,D468~1175 |

| No. | $R^2$ | $R^4$ | $R^5$ |
|---|---|---|---|
| 1790556 ~ 1791729 | D486 | Ar1 | D1~485,D487~1175 |
| 1791730 ~ 1792903 | D486 | Ar2 | D1~485,D487~1175 |
| 1792904 ~ 1794077 | D486 | Ar3 | D1~485,D487~1175 |
| 1794078 ~ 1795251 | D486 | Ar4 | D1~485,D487~1175 |
| 1795252 ~ 1796425 | D486 | Ar5 | D1~485,D487~1175 |
| 1796426 ~ 1797599 | D486 | Ar6 | D1~485,D487~1175 |
| 1797600 ~ 1798773 | D486 | Ar7 | D1~485,D487~1175 |
| 1798774 ~ 1799947 | D486 | Ar8 | D1~485,D487~1175 |
| 1799948 ~ 1801121 | D486 | Ar9 | D1~485,D487~1175 |
| 1801122 ~ 1802295 | D486 | Ar10 | D1~485,D487~1175 |
| 1802296 ~ 1803469 | D486 | Ar11 | D1~485,D487~1175 |
| 1803470 ~ 1804643 | D486 | Ar12 | D1~485,D487~1175 |
| 1804644 ~ 1805817 | D486 | Ar13 | D1~485,D487~1175 |
| 1805818 ~ 1806991 | D486 | Ar14 | D1~485,D487~1175 |
| 1806992 ~ 1808165 | D486 | Ar15 | D1~485,D487~1175 |
| 1808166 ~ 1809339 | D486 | Ar16 | D1~485,D487~1175 |
| 1809340 ~ 1810513 | D486 | Ar17 | D1~485,D487~1175 |
| 1810514 ~ 1811687 | D486 | Ar18 | D1~485,D487~1175 |
| 1811688 ~ 1812861 | D486 | Ar19 | D1~485,D487~1175 |
| 1812862 ~ 1814035 | D486 | Ar20 | D1~485,D487~1175 |
| 1814036 ~ 1815209 | D486 | Ar21 | D1~485,D487~1175 |
| 1815210 ~ 1816383 | D486 | Ar22 | D1~485,D487~1175 |
| 1816384 ~ 1817557 | D486 | Ar23 | D1~485,D487~1175 |
| 1817558 ~ 1818731 | D486 | Ar24 | D1~485,D487~1175 |
| 1818732 ~ 1819905 | D486 | Ar25 | D1~485,D487~1175 |
| 1819906 ~ 1821079 | D486 | Ar26 | D1~485,D487~1175 |
| 1821080 ~ 1822253 | D486 | Ar27 | D1~485,D487~1175 |
| 1822254 ~ 1823427 | D486 | Ar28 | D1~485,D487~1175 |
| 1823428 ~ 1824601 | D486 | Ar29 | D1~485,D487~1175 |
| 1824602 ~ 1825775 | D486 | Ar30 | D1~485,D487~1175 |
| 1825776 ~ 1826949 | D486 | Ar31 | D1~485,D487~1175 |
| 1826950 ~ 1828123 | D486 | Ar32 | D1~485,D487~1175 |
| 1828124 ~ 1829297 | D486 | Ar33 | D1~485,D487~1175 |
| 1829298 ~ 1830471 | D486 | Ar34 | D1~485,D487~1175 |
| 1830472 ~ 1831645 | D486 | Ar35 | D1~485,D487~1175 |
| 1831646 ~ 1832819 | D486 | Ar36 | D1~485,D487~1175 |
| 1832820 ~ 1833993 | D486 | Ar37 | D1~485,D487~1175 |
| 1833994 ~ 1835167 | D486 | Ar38 | D1~485,D487~1175 |
| 1835168 ~ 1836341 | D486 | Ar39 | D1~485,D487~1175 |
| 1836342 ~ 1837515 | D486 | Ar40 | D1~485,D487~1175 |
| 1837516 ~ 1838689 | D486 | Ar41 | D1~485,D487~1175 |
| 1838690 ~ 1839863 | D486 | Ar42 | D1~485,D487~1175 |
| 1839864 ~ 1841037 | D486 | Ar43 | D1~485,D487~1175 |
| 1841038 ~ 1842211 | D486 | Ar44 | D1~485,D487~1175 |
| 1842212 ~ 1843385 | D486 | Ar45 | D1~485,D487~1175 |
| 1843386 ~ 1844559 | D486 | Ar46 | D1~485,D487~1175 |
| 1844560 ~ 1845733 | D486 | Ar47 | D1~485,D487~1175 |
| 1845734 ~ 1846907 | D486 | Ar48 | D1~485,D487~1175 |
| 1846908 ~ 1848081 | D486 | Ar49 | D1~485,D487~1175 |
| 1848082 ~ 1849255 | D486 | Ar50 | D1~485,D487~1175 |
| 1849256 ~ 1850429 | D486 | Ar51 | D1~485,D487~1175 |
| 1850430 ~ 1851603 | D486 | Ar52 | D1~485,D487~1175 |
| 1851604 ~ 1852777 | D486 | Ar53 | D1~485,D487~1175 |
| 1852778 ~ 1853951 | D486 | Ar54 | D1~485,D487~1175 |
| 1853952 ~ 1855125 | D486 | Ar55 | D1~485,D487~1175 |
| 1855126 ~ 1856299 | D486 | Ar56 | D1~485,D487~1175 |
| 1856300 ~ 1857473 | D486 | Ar57 | D1~485,D487~1175 |
| 1857474 ~ 1858647 | D486 | Ar58 | D1~485,D487~1175 |
| 1858648 ~ 1859821 | D486 | Ar59 | D1~485,D487~1175 |
| 1859822 ~ 1860995 | D486 | H | D1~485,D487~1175 |

| No. | $R^2$ | $R^4$ | $R^5$ |
|---|---|---|---|
| 1860996 ~ 1862169 | D717 | Ar1 | D1~716,D718~1175 |
| 1862170 ~ 1863343 | D717 | Ar2 | D1~716,D718~1175 |
| 1863344 ~ 1864517 | D717 | Ar3 | D1~716,D718~1175 |
| 1864518 ~ 1865691 | D717 | Ar4 | D1~716,D718~1175 |
| 1865692 ~ 1866865 | D717 | Ar5 | D1~716,D718~1175 |
| 1866866 ~ 1868039 | D717 | Ar6 | D1~716,D718~1175 |
| 1868040 ~ 1869213 | D717 | Ar7 | D1~716,D718~1175 |
| 1869214 ~ 1870387 | D717 | Ar8 | D1~716,D718~1175 |
| 1870388 ~ 1871561 | D717 | Ar9 | D1~716,D718~1175 |
| 1871562 ~ 1872735 | D717 | Ar10 | D1~716,D718~1175 |
| 1872736 ~ 1873909 | D717 | Ar11 | D1~716,D718~1175 |
| 1873910 ~ 1875083 | D717 | Ar12 | D1~716,D718~1175 |
| 1875084 ~ 1876257 | D717 | Ar13 | D1~716,D718~1175 |
| 1876258 ~ 1877431 | D717 | Ar14 | D1~716,D718~1175 |
| 1877432 ~ 1878605 | D717 | Ar15 | D1~716,D718~1175 |
| 1878606 ~ 1879779 | D717 | Ar16 | D1~716,D718~1175 |
| 1879780 ~ 1880953 | D717 | Ar17 | D1~716,D718~1175 |
| 1880954 ~ 1882127 | D717 | Ar18 | D1~716,D718~1175 |
| 1882128 ~ 1883301 | D717 | Ar19 | D1~716,D718~1175 |
| 1883302 ~ 1884475 | D717 | Ar20 | D1~716,D718~1175 |
| 1884476 ~ 1885649 | D717 | Ar21 | D1~716,D718~1175 |
| 1885650 ~ 1886823 | D717 | Ar22 | D1~716,D718~1175 |
| 1886824 ~ 1887997 | D717 | Ar23 | D1~716,D718~1175 |
| 1887998 ~ 1889171 | D717 | Ar24 | D1~716,D718~1175 |
| 1889172 ~ 1890345 | D717 | Ar25 | D1~716,D718~1175 |
| 1890346 ~ 1891519 | D717 | Ar26 | D1~716,D718~1175 |
| 1891520 ~ 1892693 | D717 | Ar27 | D1~716,D718~1175 |
| 1892694 ~ 1893867 | D717 | Ar28 | D1~716,D718~1175 |
| 1893868 ~ 1895041 | D717 | Ar29 | D1~716,D718~1175 |
| 1895042 ~ 1896215 | D717 | Ar30 | D1~716,D718~1175 |
| 1896216 ~ 1897389 | D717 | Ar31 | D1~716,D718~1175 |
| 1897390 ~ 1898563 | D717 | Ar32 | D1~716,D718~1175 |
| 1898564 ~ 1899737 | D717 | Ar33 | D1~716,D718~1175 |
| 1899738 ~ 1900911 | D717 | Ar34 | D1~716,D718~1175 |
| 1900912 ~ 1902085 | D717 | Ar35 | D1~716,D718~1175 |
| 1902086 ~ 1903259 | D717 | Ar36 | D1~716,D718~1175 |
| 1903260 ~ 1904433 | D717 | Ar37 | D1~716,D718~1175 |
| 1904434 ~ 1905607 | D717 | Ar38 | D1~716,D718~1175 |
| 1905608 ~ 1906781 | D717 | Ar39 | D1~716,D718~1175 |
| 1906782 ~ 1907955 | D717 | Ar40 | D1~716,D718~1175 |
| 1907956 ~ 1909129 | D717 | Ar41 | D1~716,D718~1175 |
| 1909130 ~ 1910303 | D717 | Ar42 | D1~716,D718~1175 |
| 1910304 ~ 1911477 | D717 | Ar43 | D1~716,D718~1175 |
| 1911478 ~ 1912651 | D717 | Ar44 | D1~716,D718~1175 |
| 1912652 ~ 1913825 | D717 | Ar45 | D1~716,D718~1175 |
| 1913826 ~ 1914999 | D717 | Ar46 | D1~716,D718~1175 |
| 1915000 ~ 1916173 | D717 | Ar47 | D1~716,D718~1175 |
| 1916174 ~ 1917347 | D717 | Ar48 | D1~716,D718~1175 |
| 1917348 ~ 1918521 | D717 | Ar49 | D1~716,D718~1175 |
| 1918522 ~ 1919695 | D717 | Ar50 | D1~716,D718~1175 |
| 1919696 ~ 1920869 | D717 | Ar51 | D1~716,D718~1175 |
| 1920870 ~ 1922043 | D717 | Ar52 | D1~716,D718~1175 |
| 1922044 ~ 1923217 | D717 | Ar53 | D1~716,D718~1175 |
| 1923218 ~ 1924391 | D717 | Ar54 | D1~716,D718~1175 |
| 1924392 ~ 1925565 | D717 | Ar55 | D1~716,D718~1175 |
| 1925566 ~ 1926739 | D717 | Ar56 | D1~716,D718~1175 |
| 1926740 ~ 1927913 | D717 | Ar57 | D1~716,D718~1175 |
| 1927914 ~ 1929087 | D717 | Ar58 | D1~716,D718~1175 |
| 1929088 ~ 1930261 | D717 | Ar59 | D1~716,D718~1175 |
| 1930262 ~ 1931435 | D717 | H | D1~716,D718~1175 |

| No. | $R^2$ | $R^4$ | $R^5$ | = |
|---|---|---|---|---|
| 1931436 ~ 1932610 | D1~D1175 | D1 | D1~D1175 | |
| 1932611 ~ 1933785 | D1~D1175 | D7 | D1~D1175 | |
| 1933786 ~ 1934960 | D1~D1175 | D8 | D1~D1175 | |
| 1934961 ~ 1936135 | D1~D1175 | D9 | D1~D1175 | |
| 1936136 ~ 1937310 | D1~D1175 | D19 | D1~D1175 | |
| 1937311 ~ 1938485 | D1~D1175 | D37 | D1~D1175 | |
| 1938486 ~ 1939660 | D1~D1175 | D50 | D1~D1175 | |
| 1939661 ~ 1940835 | D1~D1175 | D67 | D1~D1175 | |
| 1940836 ~ 1942010 | D1~D1175 | D77 | D1~D1175 | |
| 1942011 ~ 1943185 | D1~D1175 | D78 | D1~D1175 | |
| 1943186 ~ 1944360 | D1~D1175 | D79 | D1~D1175 | |
| 1944361 ~ 1945535 | D1~D1175 | D80 | D1~D1175 | |
| 1945536 ~ 1946710 | D1~D1175 | D81 | D1~D1175 | $R^2 = R^5$ |
| 1946711 ~ 1947885 | D1~D1175 | D465 | D1~D1175 | |
| 1947886 ~ 1949060 | D1~D1175 | D466 | D1~D1175 | |
| 1949061 ~ 1950235 | D1~D1175 | D467 | D1~D1175 | |
| 1950236 ~ 1951410 | D1~D1175 | D471 | D1~D1175 | |
| 1951411 ~ 1952585 | D1~D1175 | D486 | D1~D1175 | |
| 1952586 ~ 1953760 | D1~D1175 | D717 | D1~D1175 | |
| 1953761 ~ 1954935 | D1~D1175 | D735 | D1~D1175 | |
| 1954936 ~ 1956110 | D1~D1175 | D783 | D1~D1175 | |
| 1956111 ~ 1957285 | D1~D1175 | D793 | D1~D1175 | |
| 1957286 ~ 1958460 | D1~D1175 | D794 | D1~D1175 | |
| 1958461 ~ 1959635 | D1~D1175 | D795 | D1~D1175 | |
| 1959636 ~ 1960810 | D1~D1175 | D796 | D1~D1175 | |
| 1960811 ~ 1961985 | D1~D1175 | D797 | D1~D1175 | |
| 1961986 ~ 1963160 | H | Ar1 | D1~D1175 | |
| 1963161 ~ 1964335 | H | Ar2 | D1~D1175 | |
| 1964336 ~ 1965510 | H | Ar3 | D1~D1175 | |
| 1965511 ~ 1966685 | H | Ar4 | D1~D1175 | |
| 1966686 ~ 1967860 | H | Ar5 | D1~D1175 | |
| 1967861 ~ 1969035 | H | Ar6 | D1~D1175 | |
| 1969036 ~ 1970210 | H | Ar7 | D1~D1175 | |
| 1970211 ~ 1971385 | H | Ar8 | D1~D1175 | |
| 1971386 ~ 1972560 | H | Ar9 | D1~D1175 | |
| 1972561 ~ 1973735 | H | Ar10 | D1~D1175 | |
| 1973736 ~ 1974910 | H | Ar11 | D1~D1175 | |
| 1974911 ~ 1976085 | H | Ar12 | D1~D1175 | |
| 1976086 ~ 1977260 | H | Ar13 | D1~D1175 | |
| 1977261 ~ 1978435 | H | Ar14 | D1~D1175 | |
| 1978436 ~ 1979610 | H | Ar15 | D1~D1175 | |
| 1979611 ~ 1980785 | H | Ar16 | D1~D1175 | |
| 1980786 ~ 1981960 | H | Ar17 | D1~D1175 | |
| 1981961 ~ 1983135 | H | Ar18 | D1~D1175 | |
| 1983136 ~ 1984310 | H | Ar19 | D1~D1175 | |
| 1984311 ~ 1985485 | H | Ar20 | D1~D1175 | |
| 1985486 ~ 1986660 | H | Ar21 | D1~D1175 | |
| 1986661 ~ 1987835 | H | Ar22 | D1~D1175 | |
| 1987836 ~ 1989010 | H | Ar23 | D1~D1175 | |
| 1989011 ~ 1990185 | H | Ar24 | D1~D1175 | |
| 1990186 ~ 1991360 | H | Ar25 | D1~D1175 | |
| 1991361 ~ 1992535 | H | Ar26 | D1~D1175 | |
| 1992536 ~ 1993710 | H | Ar27 | D1~D1175 | |
| 1993711 ~ 1994885 | H | Ar28 | D1~D1175 | |
| 1994886 ~ 1996060 | H | Ar29 | D1~D1175 | |
| 1996061 ~ 1997235 | H | Ar30 | D1~D1175 | |
| 1997236 ~ 1998410 | H | Ar31 | D1~D1175 | |
| 1998411 ~ 1999585 | H | Ar32 | D1~D1175 | |
| 1999586 ~ 2000760 | H | Ar33 | D1~D1175 | |
| 2000761 ~ 2001935 | H | Ar34 | D1~D1175 | |

| No. | $R^2$ | $R^4$ | $R^5$ |
|---|---|---|---|
| 2001936 ~ 2003110 | H | Ar35 | D1~D1175 |
| 2003111 ~ 2004285 | H | Ar36 | D1~D1175 |
| 2004286 ~ 2005460 | H | Ar37 | D1~D1175 |
| 2005461 ~ 2006635 | H | Ar38 | D1~D1175 |
| 2006636 ~ 2007810 | H | Ar39 | D1~D1175 |
| 2007811 ~ 2008985 | H | Ar40 | D1~D1175 |
| 2008986 ~ 2010160 | H | Ar41 | D1~D1175 |
| 2010161 ~ 2011335 | H | Ar42 | D1~D1175 |
| 2011336 ~ 2012510 | H | Ar43 | D1~D1175 |
| 2012511 ~ 2013685 | H | Ar44 | D1~D1175 |
| 2013686 ~ 2014860 | H | Ar45 | D1~D1175 |
| 2014861 ~ 2016035 | H | Ar46 | D1~D1175 |
| 2016036 ~ 2017210 | H | Ar47 | D1~D1175 |
| 2017211 ~ 2018385 | H | Ar48 | D1~D1175 |
| 2018386 ~ 2019560 | H | Ar49 | D1~D1175 |
| 2019561 ~ 2020735 | H | Ar50 | D1~D1175 |
| 2020736 ~ 2021910 | H | Ar51 | D1~D1175 |
| 2021911 ~ 2023085 | H | Ar52 | D1~D1175 |
| 2023086 ~ 2024260 | H | Ar53 | D1~D1175 |
| 2024261 ~ 2025435 | H | Ar54 | D1~D1175 |
| 2025436 ~ 2026610 | H | Ar55 | D1~D1175 |
| 2026611 ~ 2027785 | H | Ar56 | D1~D1175 |
| 2027786 ~ 2028960 | H | Ar57 | D1~D1175 |
| 2028961 ~ 2030135 | H | Ar58 | D1~D1175 |
| 2030136 ~ 2031310 | H | Ar59 | D1~D1175 |

Next, specific examples of the compound having a structure represented by the following general formula (1c) are shown in Table 4. In Table 4, structures of the compounds are shown in the same manner as in Table 2.

General Formula (1c)

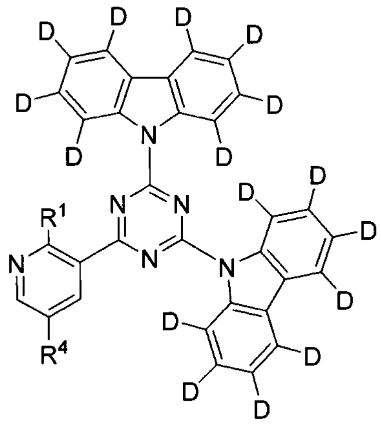

[Table 4]

| No. | $R^1$ | $R^4$ |
|---|---|---|
| 2031311 ~ 2032485 | D1~D1175 | Ar1 |
| 2032486 ~ 2033660 | D1~D1175 | Ar2 |
| 2033661 ~ 2034835 | D1~D1175 | Ar3 |
| 2034836 ~ 2036010 | D1~D1175 | Ar4 |
| 2036011 ~ 2037185 | D1~D1175 | Ar5 |
| 2037186 ~ 2038360 | D1~D1175 | Ar6 |
| 2038361 ~ 2039535 | D1~D1175 | Ar7 |
| 2039536 ~ 2040710 | D1~D1175 | Ar8 |
| 2040711 ~ 2041885 | D1~D1175 | Ar9 |
| 2041886 ~ 2043060 | D1~D1175 | Ar10 |
| 2043061 ~ 2044235 | D1~D1175 | Ar11 |
| 2044236 ~ 2045410 | D1~D1175 | Ar12 |
| 2045411 ~ 2046585 | D1~D1175 | Ar13 |
| 2046586 ~ 2047760 | D1~D1175 | Ar14 |
| 2047761 ~ 2048935 | D1~D1175 | Ar15 |
| 2048936 ~ 2050110 | D1~D1175 | Ar16 |
| 2050111 ~ 2051285 | D1~D1175 | Ar17 |
| 2051286 ~ 2052460 | D1~D1175 | Ar18 |
| 2052461 ~ 2053635 | D1~D1175 | Ar19 |
| 2053636 ~ 2054810 | D1~D1175 | Ar20 |
| 2054811 ~ 2055985 | D1~D1175 | Ar21 |
| 2055986 ~ 2057160 | D1~D1175 | Ar22 |
| 2057161 ~ 2058335 | D1~D1175 | Ar23 |
| 2058336 ~ 2059510 | D1~D1175 | Ar24 |
| 2059511 ~ 2060685 | D1~D1175 | Ar25 |
| 2060686 ~ 2061860 | D1~D1175 | Ar26 |
| 2061861 ~ 2063035 | D1~D1175 | Ar27 |

(continued)

| No. | $R^1$ | $R^4$ |
|---|---|---|
| 2063036 ~ 2064210 | D1~D1175 | Ar28 |
| 2064211 ~ 2065385 | D1~D1175 | Ar29 |
| 2065386 ~ 2066560 | D1~D1175 | Ar30 |
| 2066561 ~ 2067735 | D1~D1175 | Ar31 |
| 2067736 ~ 2068910 | D1~D1175 | Ar32 |
| 2068911 ~ 2070085 | D1~D1175 | Ar33 |
| 2070086 ~ 2071260 | D1~D1175 | Ar34 |
| 2071261 ~ 2072435 | D1~D1175 | Ar35 |
| 2072436 ~ 2073610 | D1~D1175 | Ar36 |
| 2073611 ~ 2074785 | D1~D1175 | Ar37 |
| 2074786 ~ 2075960 | D1~D1175 | Ar38 |
| 2075961 ~ 2077135 | D1~D1175 | Ar39 |
| 2077136 ~ 2078310 | D1~D1175 | Ar40 |
| 2078311 ~ 2079485 | D1~D1175 | Ar41 |
| 2079486 ~ 2080660 | D1~D1175 | Ar42 |
| 2080661 ~ 2081835 | D1~D1175 | Ar43 |
| 2081836 ~ 2083010 | D~D1175 | Ar44 |
| 2083011 ~ 2084185 | D1~D1175 | Ar45 |
| 2084186 ~ 2085360 | D1~D1175 | Ar46 |
| 2085361 ~ 2086535 | D1~D1175 | Ar47 |
| 2086536 ~ 2087710 | D1~D1175 | Ar48 |
| 2087711 ~ 2088885 | D1~D1175 | Ar49 |
| 2088886 ~ 2090060 | D1~D1175 | Ar50 |
| 2090061 ~ 2091235 | D1~D1175 | Ar51 |
| 2091236 ~ 2092410 | D1~D1175 | Ar52 |
| 2092411 ~ 2093585 | D1~D1175 | Ar53 |
| 2093586 ~ 2094760 | D1~D1175 | Ar54 |
| 2094761 ~ 2095935 | D1~D1175 | Ar55 |
| 2095936 ~ 2097110 | D1~D1175 | Ar56 |
| 2097111 ~ 2098285 | D1~D1175 | Ar57 |
| 2098286 ~ 2099460 | D1~D1175 | Ar58 |
| 2099461 ~ 2100635 | D1~D1175 | Ar59 |
| 2100636 ~ 2101810 | D1~D1175 | H |

In Tables 1 to 4, the structures where $Ar^1$ and $Ar^2$ in the general formula (1) represent a deuterated carbazolyl group (D717) are specified as the structures of Compounds 1 to 2101810. In Table 5, $Ar^1$ and $Ar^2$ in Compounds 1 to 2101810 were changed as in Table 5, and the resultant compounds were sequentially displayed as in the table format. In Table 5, Compounds 1 to 2101810 are also shown in the first row for clarifying the correspondence relationship. In the second row in Table 5, for example, Compound 1(1) shows a compound having a structure formed by replacing $Ar^2$ in Compound 1 to Ar1. Compound 2(1) indicates a compound having a structure in which $Ar^2$ of Compound 2 is changed to Ar1. Compound 2031310(1) indicates a compound having a structure in which $Ar^2$ of Compound 2101810 is changed to Ar1. Compounds 1(2) to 2101810(2) in the third row in Table 5 and the subsequent compounds are also specified in the

same manner. $X^1$ to $X^3$ of the compounds specified in Table 5 all represent a nitrogen atom (N), $L^1$ represents a single bond, and $R^1$ represents a hydrogen atom.

[Table 5]

| No. | $Ar^1$ | $Ar^2$ |
|---|---|---|
| 1 ~ 2101810 | D717 | D717 |
| 1(1) ~ 2101810(1) | D717 | Ar1 |
| 1(2) ~ 2101810(2) | D717 | Ar2 |
| 1(3) ~ 2101810(3) | D717 | Ar3 |
| 1(4) ~ 2101810(4) | D717 | Ar4 |
| 1(5) ~ 2101810(5) | D717 | Ar5 |
| 1(6) ~ 2101810(6) | D717 | Ar6 |
| 1(7) ~ 2101810(7) | D717 | Ar7 |
| 1(8) ~ 2101810(8) | D717 | Ar8 |
| 1(9) ~ 2101810(9) | D717 | Ar9 |
| 1(10) ~ 2101810(10) | D717 | Ar10 |
| 1(11) ~ 2101810(11) | D717 | Ar11 |
| 1(12) ~ 2101810(12) | D717 | Ar12 |
| 1(13) ~ 2101810(13) | D717 | Ar13 |
| 1(14) ~ 2101810(14) | D717 | Ar14 |
| 1(15) ~ 2101810(15) | D717 | Ar15 |
| 1(16) ~ 2101810(16) | D717 | Ar16 |
| 1(17) ~ 2101810(17) | D717 | Ar17 |
| 1(18) ~ 2101810(18) | D717 | Ar18 |
| 1(19) ~ 2101810(19) | D717 | Ar19 |
| 1(20) ~ 2101810(20) | D717 | Ar20 |
| 1(21) ~ 2101810(21) | D717 | Ar21 |
| 1(22) ~ 2101810(22) | D717 | Ar22 |
| 1(23) ~ 2101810(23) | D717 | Ar23 |
| 1(24) ~ 2101810(24) | D717 | Ar24 |
| 1(25) ~ 2101810(25) | D717 | Ar25 |
| 1(26) ~ 2101810(26) | D717 | Ar26 |
| 1(27) ~ 2101810(27) | D717 | Ar27 |
| 1(28) ~ 2101810(28) | D717 | Ar28 |
| 1(29) ~ 2101810(29) | D717 | Ar29 |
| 1(30) ~ 2101810(30) | D717 | Ar30 |
| 1(31) ~ 2101810(31) | D717 | Ar31 |
| 1(32) ~ 2101810(32) | D717 | Ar32 |
| 1(33) ~ 2101810(33) | D717 | Ar33 |
| 1(34) ~ 2101810(34) | D717 | Ar34 |
| 1(35) ~ 2101810(35) | D717 | Ar35 |
| 1(36) ~ 2101810(36) | D717 | Ar36 |

(continued)

| No. | $Ar^1$ | $Ar^2$ |
|---|---|---|
| 1(37) ~ 2101810(37) | D717 | Ar37 |
| 1(38) ~ 2101810(38) | D717 | Ar38 |
| 1(39) ~ 2101810(39) | D717 | Ar39 |
| 1(40) ~ 2101810(40) | D717 | Ar40 |
| 1(41) ~ 2101810(41) | D717 | Ar41 |
| 1(42) ~ 2101810(42) | D717 | Ar42 |
| 1(43) ~ 2101810(43) | D717 | Ar43 |
| 1(44) ~ 2101810(44) | D717 | Ar44 |
| 1(45) ~ 2101810(45) | D717 | Ar45 |
| 1(46) ~ 2101810(46) | D717 | Ar46 |
| 1(47) ~ 2101810(47) | D717 | Ar47 |
| 1(48) ~ 2101810(48) | D717 | Ar48 |
| 1(49) ~ 2101810(49) | D717 | Ar49 |
| 1(50) ~ 2101810(50) | D717 | Ar50 |
| 1(51) ~ 2101810(51) | D717 | Ar51 |
| 1(52) ~ 2101810(52) | D717 | Ar52 |
| 1(53) ~ 2101810(53) | D717 | Ar53 |
| 1(54) ~ 2101810(54) | D717 | Ar54 |
| 1(55) ~ 2101810(55) | D717 | Ar55 |
| 1(56) ~ 2101810(56) | D717 | Ar56 |
| 1(57) ~ 2101810(57) | D717 | Ar57 |
| 1(58) ~ 2101810(58) | D717 | Ar58 |
| 1(59) ~ 2101810(59) | D717 | Ar59 |
| 1(60) ~ 2101810(60) | D | Ar1 |
| 1(61) ~ 2101810(61) | D1 | Ar2 |
| 1(62) ~ 2101810(62) | D1 | Ar3 |
| 1(63) ~ 2101810(63) | D1 | Ar4 |
| 1(64) ~ 2101810(64) | D1 | Ar5 |
| 1(65) ~ 2101810(65) | D1 | Ar6 |
| 1(66) ~ 2101810(66) | D1 | Ar7 |
| 1(67) ~ 2101810(67) | D1 | Ar8 |
| 1(68) ~ 2101810(68) | D1 | Ar9 |
| 1(69) ~ 2101810(69) | D1 | Ar10 |
| 1(70) ~ 2101810(70) | D1 | Ar11 |
| 1(71) ~ 2101810(71) | D1 | Ar12 |
| 1(72) ~ 2101810(72) | D1 | Ar13 |
| 1(73) ~ 2101810(73) | D1 | Ar14 |
| 1(74) ~ 2101810(74) | D1 | Ar15 |
| 1(75) ~ 2101810(75) | D1 | Ar16 |

(continued)

| No. | $Ar^1$ | $Ar^2$ |
|---|---|---|
| 1(76) ~ 2101810(76) | D1 | Ar17 |
| 1(77) ~ 2101810(77) | D1 | Ar18 |
| 1(78) ~ 2101810(78) | D1 | Ar19 |
| 1(79) ~ 2101810(79) | D1 | Ar20 |
| 1(80) ~ 2101810(80) | D1 | Ar21 |
| 1(81) ~ 2101810(81) | D1 | Ar22 |
| 1(82) ~ 2101810(82) | D1 | Ar23 |
| 1(83) ~ 2101810(83) | D1 | Ar24 |
| 1(84) ~ 2101810(84) | D1 | Ar25 |
| 1(85) ~ 2101810(85) | D1 | Ar26 |
| 1(86) ~ 2101810(86) | D1 | Ar27 |
| 1(87) ~ 2101810(87) | D1 | Ar28 |
| 1(88) ~ 2101810(88) | D1 | Ar29 |
| 1(89) ~ 2101810(89) | D1 | Ar30 |
| 1(90) ~ 2101810(90) | D1 | Ar31 |
| 1(91) ~ 2101810(91) | D1 | Ar32 |
| 1(92) ~ 2101810(92) | D1 | Ar33 |
| 1(93) ~ 2101810(93) | D1 | Ar34 |
| 1(94) ~ 2101810(94) | D1 | Ar35 |
| 1(95) ~ 2101810(95) | D1 | Ar36 |
| 1(96) ~ 2101810(96) | D1 | Ar37 |
| 1(97) ~ 2101810(97) | D1 | Ar38 |
| 1(98) ~ 2101810(98) | D1 | Ar39 |
| 1(99) ~ 2101810(99) | D1 | Ar40 |
| 1(100) ~ 2101810(100) | D1 | Ar41 |
| 1(101) ~ 2101810(101) | D1 | Ar42 |
| 1(102) ~ 2101810(102) | D1 | Ar43 |
| 1(103) ~ 2101810(103) | D1 | Ar44 |
| 1(104) ~ 2101810(104) | D1 | Ar45 |
| 1(105) ~ 2101810(105) | D1 | Ar46 |
| 1(106) ~ 2101810(106) | D1 | Ar47 |
| 1(107) ~ 2101810(107) | D1 | Ar48 |
| 1(108) ~ 2101810(108) | D1 | Ar49 |
| 1(109) ~ 2101810(109) | D1 | Ar50 |
| 1(110) ~ 2101810(110) | D1 | Ar51 |
| 1(111) ~ 2101810(111) | D1 | Ar52 |
| 1(112) ~ 2101810(112) | D1 | Ar53 |
| 1(113) ~ 2101810(113) | D1 | Ar54 |
| 1(114) ~ 2101810(114) | D1 | Ar55 |

(continued)

| No. | $Ar^1$ | $Ar^2$ |
|---|---|---|
| 1(115) ~ 2101810(115) | D1 | Ar56 |
| 1(116) ~ 2101810(116) | D1 | Ar57 |
| 1(117) ~ 2101810(117) | D 1 | Ar58 |
| 1(118) ~ 2101810(118) | D1 | Ar59 |
| 1(119) ~ 2101810(119) | D7 | Ar1 |
| 1(120) ~ 2101810(120) | D7 | Ar2 |
| 1(121) ~ 2101810(121) | D7 | Ar3 |
| 1(122) ~ 2101810(122) | D7 | Ar4 |
| 1(123) ~ 2101810(123) | D7 | Ar5 |
| 1(124) ~ 2101810(124) | D7 | Ar6 |
| 1(125) ~ 2101810(125) | D7 | Ar7 |
| 1(126) ~ 2101810(126) | D7 | Ar8 |
| 1(127) ~ 2101810(127) | D7 | Ar9 |
| 1(128) ~ 2101810(128) | D7 | Ar10 |
| 1(129) ~ 2101810(129) | D7 | Ar11 |
| 1(130) ~ 2101810(130) | D7 | Ar12 |
| 1(131) ~ 2101810(131) | D7 | Ar13 |
| 1(132) ~ 2101810(132) | D7 | Ar14 |
| 1(133) ~ 2101810(133) | D7 | Ar15 |
| 1(134) ~ 2101810(134) | D7 | Ar16 |
| 1(135) ~ 2101810(135) | D7 | Ar17 |
| 1(136) ~ 2101810(136) | D7 | Ar18 |
| 1(137) ~ 2101810(137) | D7 | Ar19 |
| 1(138) ~ 2101810(138) | D7 | Ar20 |
| 1(139) ~ 2101810(139) | D7 | Ar21 |
| 1(140) ~ 2101810(140) | D7 | Ar22 |
| 1(141) ~ 2101810(141) | D7 | Ar23 |
| 1(142) ~ 2101810(142) | D7 | Ar24 |
| 1(143) ~ 2101810(143) | D7 | Ar25 |
| 1(144) ~ 2101810(144) | D7 | Ar26 |
| 1(145) ~ 2101810(145) | D7 | Ar27 |
| 1(146) ~ 2101810(146) | D7 | Ar28 |
| 1(147) ~ 2101810(147) | D7 | Ar29 |
| 1(148) ~ 2101810(148) | D7 | Ar30 |
| 1(149) ~ 2101810(149) | D7 | Ar31 |
| 1(150) ~ 2101810(150) | D7 | Ar32 |
| 1(151) ~ 2101810(151) | D7 | Ar33 |
| 1(152) ~ 2101810(152) | D7 | Ar34 |
| 1(153) ~ 2101810(153) | D7 | Ar35 |

(continued)

| No. | $Ar^1$ | $Ar^2$ |
|---|---|---|
| 1(154) ~ 2101810(154) | D7 | Ar36 |
| 1(155) ~ 2101810(155) | D7 | Ar37 |
| 1(156) ~ 2101810(156) | D7 | Ar38 |
| 1(157) ~ 2101810(157) | D7 | Ar39 |
| 1(158) ~ 2101810(158) | D7 | Ar40 |
| 1(159) ~ 2101810(159) | D7 | Ar41 |
| 1(160) ~ 2101810(160) | D7 | Ar42 |
| 1(161) ~ 2101810(161) | D7 | Ar43 |
| 1(162) ~ 2101810(162) | D7 | Ar44 |
| 1(163) ~ 2101810(163) | D7 | Ar45 |
| 1(164) ~ 2101810(164) | D7 | Ar46 |
| 1(165) ~ 2101810(165) | D7 | Ar47 |
| 1(166) ~ 2101810(166) | D7 | Ar48 |
| 1(167) ~ 2101810(167) | D7 | Ar49 |
| 1(168) ~ 2101810(168) | D7 | Ar50 |
| 1(169) ~ 2101810(169) | D7 | Ar51 |
| 1(170) ~ 2101810(170) | D7 | Ar52 |
| 1(171) ~ 2101810(171) | D7 | Ar53 |
| 1(172) ~ 2101810(172) | D7 | Ar54 |
| 1(173) ~ 2101810(173) | D7 | Ar55 |
| 1(174) ~ 2101810(174) | D7 | Ar56 |
| 1(175) ~ 2101810(175) | D7 | Ar57 |
| 1(176) ~ 2101810(176) | D7 | Ar58 |
| 1(177) ~ 2101810(177) | D7 | Ar59 |
| 1(178) ~ 2101810(178) | D8 | Ar1 |
| 1(179) ~ 2101810(179) | D8 | Ar2 |
| 1(180) ~ 2101810(180) | D8 | Ar3 |
| 1(181) ~ 2101810(181) | D8 | Ar4 |
| 1(182) ~ 2101810(182) | D8 | Ar5 |
| 1(183) ~ 2101810(183) | D8 | Ar6 |
| 1(184) ~ 2101810(184) | D8 | Ar7 |
| 1(185) ~ 2101810(185) | D8 | Ar8 |
| 1(186) ~ 2101810(186) | D8 | Ar9 |
| 1(187) ~ 2101810(187) | D8 | Ar10 |
| 1(188) ~ 2101810(188) | D8 | Ar11 |
| 1(189) ~ 2101810(189) | D8 | Ar12 |
| 1(190) ~ 2101810(190) | D8 | Ar13 |
| 1(191) ~ 2101810(191) | D8 | Ar14 |
| 1(192) ~ 2101810(192) | D8 | Ar15 |

(continued)

| No. | $Ar^1$ | $Ar^2$ |
|---|---|---|
| 1(193) ~ 2101810(193) | D8 | Ar16 |
| 1(194) ~ 2101810(194) | D8 | Ar17 |
| 1(195) ~ 2101810(195) | D8 | Ar18 |
| 1(196) ~ 2101810(196) | D8 | Ar19 |
| 1(197) ~ 2101810(197) | D8 | Ar20 |
| 1(198) ~ 2101810(198) | D8 | Ar21 |
| 1(199) ~ 2101810(199) | D8 | Ar22 |
| 1(200) ~ 2101810(200) | D8 | Ar23 |
| 1(201) ~ 2101810(201) | D8 | Ar24 |
| 1(202) ~ 2101810(202) | D8 | Ar25 |
| 1(203) ~ 2101810(203) | D8 | Ar26 |
| 1(204) ~ 2101810(204) | D8 | Ar27 |
| 1(205) ~ 2101810(205) | D8 | Ar28 |
| 1(206) ~ 2101810(206) | D8 | Ar29 |
| 1(207) ~ 2101810(207) | D8 | Ar30 |
| 1(208) ~ 2101810(208) | D8 | Ar31 |
| 1(209) ~ 2101810(209) | D8 | Ar32 |
| 1(210) ~ 2101810(210) | D8 | Ar33 |
| 1(211) ~ 2101810(211) | D8 | Ar34 |
| 1(212) ~ 2101810(212) | D8 | Ar35 |
| 1(213) ~ 2101810(213) | D8 | Ar36 |
| 1(214) ~ 2101810(214) | D8 | Ar37 |
| 1(215) ~ 2101810(215) | D8 | Ar38 |
| 1(216) ~ 2101810(216) | D8 | Ar39 |
| 1(217) ~ 2101810(217) | D8 | Ar40 |
| 1(218) ~ 2101810(218) | D8 | Ar41 |
| 1(219) ~ 2101810(219) | D8 | Ar42 |
| 1(220) ~ 2101810(220) | D8 | Ar43 |
| 1(221) ~ 2101810(221) | D8 | Ar44 |
| 1(222) ~ 2101810(222) | D8 | Ar45 |
| 1(223) ~ 2101810(223) | D8 | Ar46 |
| 1(224) ~ 2101810(224) | D8 | Ar47 |
| 1(225) ~ 2101810(225) | D8 | Ar48 |
| 1(226) ~ 2101810(226) | D8 | Ar49 |
| 1(227) ~ 2101810(227) | D8 | Ar50 |
| 1(228) ~ 2101810(228) | D8 | Ar51 |
| 1(229) ~ 2101810(229) | D8 | Ar52 |
| 1(230) ~ 2101810(230) | D8 | Ar53 |
| 1(231) ~ 2101810(231) | D8 | Ar54 |

(continued)

| No. | Ar1 | Ar2 |
|---|---|---|
| 1(232) ~ 2101810(232) | D8 | Ar55 |
| 1(233) ~ 2101810(233) | D8 | Ar56 |
| 1(234) ~ 2101810(234) | D8 | Ar57 |
| 1(235) ~ 2101810(235) | D8 | Ar58 |
| 1(236) ~ 2101810(236) | D8 | Ar59 |
| 1(237) ~ 2101810(237) | D9 | Ar1 |
| 1(238) ~ 2101810(238) | D9 | Ar2 |
| 1(239) ~ 2101810(239) | D9 | Ar3 |
| 1(240) ~ 2101810(240) | D9 | Ar4 |
| 1(241) ~ 2101810(241) | D9 | Ar5 |
| 1(242) ~ 2101810(242) | D9 | Ar6 |
| 1(243) ~ 2101810(243) | D9 | Ar7 |
| 1(244) ~ 2101810(244) | D9 | Ar8 |
| 1(245) ~ 2101810(245) | D9 | Ar9 |
| 1(246) ~ 2101810(246) | D9 | Ar10 |
| 1(247) ~ 2101810(247) | D9 | Ar11 |
| 1(248) ~ 2101810(248) | D9 | Ar12 |
| 1(249) ~ 2101810(249) | D9 | Ar13 |
| 1(250) ~ 2101810(250) | D9 | Ar14 |
| 1(251) ~ 2101810(251) | D9 | Ar15 |
| 1(252) ~ 2101810(252) | D9 | Ar16 |
| 1(253) ~ 2101810(253) | D9 | Ar17 |
| 1(254) ~ 2101810(254) | D9 | Ar18 |
| 1(255) ~ 2101810(255) | D9 | Ar19 |
| 1(256) ~ 2101810(256) | D9 | Ar20 |
| 1(257) ~ 2101810(257) | D9 | Ar21 |
| 1(258) ~ 2101810(258) | D9 | Ar22 |
| 1(259) ~ 2101810(259) | D9 | Ar23 |
| 1(260) ~ 2101810(260) | D9 | Ar24 |
| 1(261) ~ 2101810(261) | D9 | Ar25 |
| 1(262) ~ 2101810(262) | D9 | Ar26 |
| 1(263) ~ 2101810(263) | D9 | Ar27 |
| 1(264) ~ 2101810(264) | D9 | Ar28 |
| 1(265) ~ 2101810(265) | D9 | Ar29 |
| 1(266) ~ 2101810(266) | D9 | Ar30 |
| 1(267) ~ 2101810(267) | D9 | Ar31 |
| 1(268) ~ 2101810(268) | D9 | Ar32 |
| 1(269) ~ 2101810(269) | D9 | Ar33 |
| 1(270) ~ 2101810(270) | D9 | Ar34 |

(continued)

| No. | Ar1 | Ar2 |
|---|---|---|
| 1(271) ~ 2101810(271) | D9 | Ar35 |
| 1(272) ~ 2101810(272) | D9 | Ar36 |
| 1(273) ~ 2101810(273) | D9 | Ar37 |
| 1(274) ~ 2101810(274) | D9 | Ar38 |
| 1(275) ~ 2101810(275) | D9 | Ar39 |
| 1(276) ~ 2101810(276) | D9 | Ar40 |
| 1(277) ~ 2101810(277) | D9 | Ar41 |
| 1(278) ~ 2101810(278) | D9 | Ar42 |
| 1(279) ~ 2101810(279) | D9 | Ar43 |
| 1(280) ~ 2101810(280) | D9 | Ar44 |
| 1(281) ~ 2101810(281) | D9 | Ar45 |
| 1(282) ~ 2101810(282) | D9 | Ar46 |
| 1(283) ~ 2101810(283) | D9 | Ar47 |
| 1(284) ~ 2101810(284) | D9 | Ar48 |
| 1(285) ~ 2101810(285) | D9 | Ar49 |
| 1(286) ~ 2101810(286) | D9 | Ar50 |
| 1(287) ~ 2101810(287) | D9 | Ar51 |
| 1(288) ~ 2101810(288) | D9 | Ar52 |
| 1(289) ~ 2101810(289) | D9 | Ar53 |
| 1(290) ~ 2101810(290) | D9 | Ar54 |
| 1(291) ~ 2101810(291) | D9 | Ar55 |
| 1(292) ~ 2101810(292) | D9 | Ar56 |
| 1(293) ~ 2101810(293) | D9 | Ar57 |
| 1(294) ~ 2101810(294) | D9 | Ar58 |
| 1(295) ~ 2101810(295) | D9 | Ar59 |
| 1(296) ~ 2101810(296) | D19 | Ar1 |
| 1(297) ~ 2101810(297) | D19 | Ar2 |
| 1(298) ~ 2101810(298) | D19 | Ar3 |
| 1(299) ~ 2101810(299) | D19 | Ar4 |
| 1(300) ~ 2101810(300) | D19 | Ar5 |
| 1(301) ~ 2101810(301) | D19 | Ar6 |
| 1(302) ~ 2101810(302) | D19 | Ar7 |
| 1(303) ~ 2101810(303) | D19 | Ar8 |
| 1(304) ~ 2101810(304) | D19 | Ar9 |
| 1(305) ~ 2101810(305) | D19 | Ar10 |
| 1(306) ~ 2101810(306) | D19 | Ar11 |
| 1(307) - 2101810(307) | D19 | Ar12 |
| 1(308) ~ 2101810(308) | D19 | Ar13 |
| 1(309) ~ 2101810(309) | D19 | Ar14 |

(continued)

| No. | $Ar^1$ | $Ar^2$ |
|---|---|---|
| 1(310) ~ 2101810(310) | D19 | Ar15 |
| 1(311) ~ 2101810(311) | D19 | Ar16 |
| 1(312) ~ 2101810(312) | D19 | Ar17 |
| 1(313) ~ 2101810(313) | D19 | Ar18 |
| 1(314) ~ 2101810(314) | D19 | Ar19 |
| 1(315) ~ 2101810(315) | D19 | Ar20 |
| 1(316) ~ 2101810(316) | D19 | Ar21 |
| 1(317) ~ 2101810(317) | D19 | Ar22 |
| 1(318) ~ 2101810(318) | D19 | Ar23 |
| 1(319) ~ 2101810(319) | D19 | Ar24 |
| 1(320) ~ 2101810(320) | D19 | Ar25 |
| 1(321) ~ 2101810(321) | D19 | Ar26 |
| 1(322) ~ 2101810(322) | D19 | Ar27 |
| 1(323) ~ 2101810(323) | D19 | Ar28 |
| 1(324) ~ 2101810(324) | D19 | Ar29 |
| 1(325) ~ 2101810(325) | D19 | Ar30 |
| 1(326) ~ 2101810(326) | D19 | Ar31 |
| 1(327) ~ 2101810(327) | D19 | Ar32 |
| 1(328) ~ 2101810(328) | D19 | Ar33 |
| 1(329) ~ 2101810(329) | D19 | Ar34 |
| 1(330) ~ 2101810(330) | D19 | Ar35 |
| 1(331) ~ 2101810(331) | D19 | Ar36 |
| 1(332) ~ 2101810(332) | D19 | Ar37 |
| 1(333) ~ 2101810(333) | D19 | Ar38 |
| 1(334) ~ 2101810(334) | D19 | Ar39 |
| 1(335) ~ 2101810(335) | D19 | Ar40 |
| 1(336) ~ 2101810(336) | D19 | Ar41 |
| 1(337) ~ 2101810(337) | D19 | Ar42 |
| 1(338) ~ 2101810(338) | D19 | Ar43 |
| 1(339) ~ 2101810(339) | D19 | Ar44 |
| 1(340) ~ 2101810(340) | D19 | Ar45 |
| 1(341) ~ 2101810(341) | D19 | Ar46 |
| 1(342) ~ 2101810(342) | D19 | Ar47 |
| 1(343) ~ 2101810(343) | D19 | Ar48 |
| 1(344) ~ 2101810(344) | D19 | Ar49 |
| 1(345) ~ 2101810(345) | D19 | Ar50 |
| 1(346) ~ 2101810(346) | D19 | Ar51 |
| 1(341) ~ 2101810(347) | D19 | Ar52 |
| 1(348) ~ 2101810(348) | D19 | Ar53 |

(continued)

| No. | Ar¹ | Ar² |
|---|---|---|
| 1(349) ~ 2101810(349) | D19 | Ar54 |
| 1(350) ~ 2101810(350) | D19 | Ar55 |
| 1(351) ~ 2101810(351) | D19 | Ar56 |
| 1(352) ~ 2101810(352) | D19 | Ar57 |
| 1(353) ~ 2101810(353) | D19 | Ar58 |
| 1(354) ~ 2101810(354) | D19 | Ar59 |
| 1(355) ~ 2101810(355) | D37 | Ar1 |
| 1(356) ~ 2101810(356) | D37 | Ar2 |
| 1(357) ~ 2101810(357) | D37 | Ar3 |
| 1(358) ~ 2101810(358) | D37 | Ar4 |
| 1(359) ~ 2101810(359) | D37 | Ar5 |
| 1(360) ~ 2101810(360) | D37 | Ar6 |
| 1(361) ~ 2101810(361) | D37 | Ar7 |
| 1(362) ~ 2101810(362) | D37 | Ar8 |
| 1(363) ~ 2101810(363) | D37 | Ar9 |
| 1(364) ~ 2101810(364) | D37 | Ar10 |
| 1(365) ~ 2101810(365) | D37 | Ar11 |
| 1(366) ~ 2101810(366) | D37 | Ar12 |
| 1(367) ~ 2101810(367) | D37 | Ar13 |
| 1(368) ~ 2101810(368) | D37 | Ar14 |
| 1(369) ~ 2101810(369) | D37 | Ar15 |
| 1(370) ~ 2101810(370) | D37 | Ar16 |
| 1(371) ~ 2101810(371) | D37 | Ar17 |
| 1(372) ~ 2101810(372) | D37 | Ar18 |
| 1(373) ~ 2101810(373) | D37 | Ar19 |
| 1(374) ~ 2101810(374) | D37 | Ar20 |
| 1(375) ~ 2101810(375) | D37 | Ar21 |
| 1(376) ~ 2101810(376) | D37 | Ar22 |
| 1(377) ~ 2101810(377) | D37 | Ar23 |
| 1(378) ~ 2101810(378) | D37 | Ar24 |
| 1(379) ~ 2101810(379) | D37 | Ar25 |
| 1(380) ~ 2101810(380) | D37 | Ar26 |
| 1(381) ~ 2101810(381) | D37 | Ar27 |
| 1(382) ~ 2101810(382) | D37 | Ar28 |
| 1(383) ~ 2101810(383) | D37 | Ar29 |
| 1(384) ~ 2101810(384) | D37 | Ar30 |
| 1(385) ~ 2101810(385) | D37 | Ar31 |
| 1(386) ~ 2101810(386) | D37 | Ar32 |
| 1(387) ~ 2101810(387) | D37 | Ar33 |

(continued)

| No. | Ar1 | Ar2 |
|---|---|---|
| 1(388) ~ 2101810(388) | D37 | Ar34 |
| 1(389) ~ 2101810(389) | D37 | Ar35 |
| 1(390) ~ 2101810(390) | D37 | Ar36 |
| 1(391) ~ 2101810(391) | D37 | Ar37 |
| 1(392) ~ 2101810(392) | D37 | Ar38 |
| 1(393) ~ 2101810(393) | D37 | Ar39 |
| 1(394) ~ 2101810(394) | D37 | Ar40 |
| 1(395) ~ 2101810(395) | D37 | Ar41 |
| 1(396) ~ 2101810(396) | D37 | Ar42 |
| 1(397) ~ 2101810(397) | D37 | Ar43 |
| 1(398) ~ 2101810(398) | D37 | Ar44 |
| 1(399) ~ 2101810(399) | D37 | Ar45 |
| 1(400) ~ 2101810(400) | D37 | Ar46 |
| 1(401) ~ 2101810(401) | D37 | Ar47 |
| 1(402) ~ 2101810(402) | D37 | Ar48 |
| 1(403) ~ 2101810(403) | D37 | Ar49 |
| 1(404) ~ 2101810(404) | D37 | Ar50 |
| 1(405) ~ 2101810(405) | D37 | Ar51 |
| 1(406) ~ 2101810(406) | D37 | Ar52 |
| 1(407) ~ 2101810(407) | D37 | Ar53 |
| 1(408) ~ 2101810(408) | D37 | Ar54 |
| 1(409) ~ 2101810(409) | D37 | Ar55 |
| 1(410) ~ 2101810(410) | D37 | Ar56 |
| 1(411) ~ 2101810(411) | D37 | Ar57 |
| 1(412) ~ 2101810(412) | D37 | Ar58 |
| 1(413) ~ 2101810(413) | D37 | Ar59 |
| 1(414) ~ 2101810(414) | D50 | Ar1 |
| 1(415) ~ 2101810(415) | D50 | Ar2 |
| 1(416) ~ 2101810(416) | D50 | Ar3 |
| 1(417) ~ 2101810(417) | D50 | Ar4 |
| 1(418) ~ 2101810(418) | D50 | Ar5 |
| 1(419) ~ 2101810(419) | D50 | Ar6 |
| 1(420) ~ 2101810(420) | D50 | Ar7 |
| 1(421) ~ 2101810(421) | D50 | Ar8 |
| 1(422) ~ 2101810(422) | D50 | Ar9 |
| 1(423) ~ 2101810(423) | D50 | Ar10 |
| 1(424) ~ 2101810(424) | D50 | Ar11 |
| 1(425) ~ 2101810(425) | D50 | Ar12 |
| 1(426) ~ 2101810(426) | D50 | Ar13 |

(continued)

| No. | $Ar^1$ | $Ar^2$ |
|---|---|---|
| 1(427) ~ 2101810(427) | D50 | Ar14 |
| 1(428) ~ 2101810(428) | D50 | Ar15 |
| 1(429) ~ 2101810(429) | D50 | Ar16 |
| 1(430) ~ 2101810(430) | D50 | Ar17 |
| 1(431) ~ 2101810(431) | D50 | Ar18 |
| 1(432) ~ 2101810(432) | D50 | Ar19 |
| 1(433) ~ 2101810(433) | D50 | Ar20 |
| 1(434) ~ 2101810(434) | D50 | Ar21 |
| 1(435) ~ 2101810(435) | D50 | Ar22 |
| 1(436) ~ 2101810(436) | D50 | Ar23 |
| 1(437) ~ 2101810(437) | D50 | Ar24 |
| 1(438) ~ 2101810(438) | D50 | Ar25 |
| 1(439) ~ 2101810(439) | D50 | Ar26 |
| 1(440) ~ 2101810(440) | D50 | Ar27 |
| 1(441) ~ 2101810(441) | D50 | Ar28 |
| 1(442) ~ 2101810(442) | D50 | Ar29 |
| 1(443) ~ 2101810(443) | D50 | Ar30 |
| 1(444) ~ 2101810(444) | D50 | Ar31 |
| 1(445) ~ 2101810(445) | D50 | Ar32 |
| 1(446) ~ 2101810(446) | D50 | Ar33 |
| 1(447) ~ 2101810(447) | D50 | Ar34 |
| 1(448) ~ 2101810(448) | D50 | Ar35 |
| 1(449) ~ 2101810(449) | D50 | Ar36 |
| 1(450) ~ 2101810(450) | D50 | Ar37 |
| 1(451) ~ 2101810(451) | D50 | Ar38 |
| 1(452) ~ 2101810(452) | D50 | Ar39 |
| 1(453) ~ 2101810(453) | D50 | Ar40 |
| 1(454) ~ 2101810(454) | D50 | Ar41 |
| 1(455) ~ 2101810(455) | D50 | Ar42 |
| 1(456) ~ 2101810(456) | D50 | Ar43 |
| 1(457) ~ 2101810(457) | D50 | Ar44 |
| 1(458) ~ 2101810(458) | D50 | Ar45 |
| 1(459) ~ 2101810(459) | D50 | Ar46 |
| 1(460) ~ 2101810(460) | D50 | Ar47 |
| 1(461) ~ 2101810(461) | D50 | Ar48 |
| 1(462) ~ 2101810(462) | D50 | Ar49 |
| 1(463) ~ 2101810(463) | D50 | Ar50 |
| 1(464) ~ 2101810(464) | D50 | Ar51 |
| 1(465) ~ 2101810(465) | D50 | Ar52 |

(continued)

| No. | $Ar^1$ | $Ar^2$ |
|---|---|---|
| 1(466) ~ 2101810(466) | D50 | Ar53 |
| 1(467) ~ 2101810(467) | D50 | Ar54 |
| 1(468) ~ 2101810(468) | D50 | Ar55 |
| 1(469) ~ 2101810(469) | D50 | Ar56 |
| 1(470) ~ 2101810(470) | D50 | Ar57 |
| 1(471) ~ 2101810(471) | D50 | Ar58 |
| 1(472) ~ 2101810(472) | D50 | Ar59 |
| 1(473) ~ 2101810(473) | D77 | Ar1 |
| 1(474) ~ 2101810(474) | D77 | Ar2 |
| 1(475) ~ 2101810(475) | D77 | Ar3 |
| 1(476) ~ 2101810(476) | D77 | Ar4 |
| 1(477) ~ 2101810(477) | D77 | Ar5 |
| 1(478) ~ 2101810(478) | D77 | Ar6 |
| 1(479) ~ 2101810(479) | D77 | Ar7 |
| 1(480) ~ 2101810(480) | D77 | Ar8 |
| 1(481) ~ 2101810(481) | D77 | Ar9 |
| 1(482) ~ 2101810(482) | D77 | Ar10 |
| 1(483) ~ 2101810(483) | D77 | Ar11 |
| 1(484) ~ 2101810(484) | D77 | Ar12 |
| 1(485) ~ 2101810(485) | D77 | Ar13 |
| 1(486) ~ 2101810(486) | D77 | Ar14 |
| 1(487) ~ 2101810(487) | D77 | Ar15 |
| 1(488) ~ 2101810(488) | D77 | Ar16 |
| 1(489) ~ 2101810(489) | D77 | Ar17 |
| 1(490) ~ 2101810(490) | D77 | Ar18 |
| 1(491) ~ 2101810(491) | D77 | Ar19 |
| 1(492) ~ 2101810(492) | D77 | Ar20 |
| 1(493) ~ 2101810(493) | D77 | Ar21 |
| 1(494) ~ 2101810(494) | D77 | Ar22 |
| 1(495) ~ 2101810(495) | D77 | Ar23 |
| 1(496) ~ 2101810(496) | D77 | Ar24 |
| 1(497) ~ 2101810(497) | D77 | Ar25 |
| 1(498) ~ 2101810(498) | D77 | Ar26 |
| 1(499) ~ 2101810(499) | D77 | Ar27 |
| 1(500) ~ 2101810(500) | D77 | Ar28 |
| 1(501) ~ 2101810(501) | D77 | Ar29 |
| 1(502) ~ 2101810(502) | D77 | Ar30 |
| 1(503) ~ 2101810(503) | D77 | Ar31 |
| 1(504) ~ 2101810(504) | D77 | Ar32 |

(continued)

| No. | $Ar^1$ | $Ar^2$ |
|---|---|---|
| 1(505) ~ 2101810(505) | D77 | Ar33 |
| 1(506) ~ 2101810(506) | D77 | Ar34 |
| 1(507) ~ 2101810(507) | D77 | Ar35 |
| 1(508) ~ 2101810(508) | D77 | Ar36 |
| 1(509) ~ 2101810(509) | D77 | Ar37 |
| 1(510) ~ 2101810(510) | D77 | Ar38 |
| 1(511) ~ 2101810(511) | D77 | Ar39 |
| 1(512) ~ 2101810(512) | D77 | Ar40 |
| 1(513) ~ 2101810(513) | D77 | Ar41 |
| 1(514) ~ 2101810(514) | D77 | Ar42 |
| 1(515) ~ 2101810(515) | D77 | Ar43 |
| 1(516) ~ 2101810(516) | D77 | Ar44 |
| 1(517) ~ 2101810(517) | D77 | Ar45 |
| 1(518) ~ 2101810(518) | D77 | Ar46 |
| 1(519) ~ 2101810(519) | D77 | Ar47 |
| 1(520) ~ 2101810(520) | D77 | Ar48 |
| 1(521) ~ 2101810(521) | D77 | Ar49 |
| 1(522) ~ 2101810(522) | D77 | Ar50 |
| 1(523) ~ 2101810(523) | D77 | Ar51 |
| 1(524) ~ 2101810(524) | D77 | Ar52 |
| 1(525) ~ 2101810(525) | D77 | Ar53 |
| 1(526) ~ 2101810(526) | D77 | Ar54 |
| 1(527) ~ 2101810(527) | D77 | Ar55 |
| 1(528) ~ 2101810(528) | D77 | Ar56 |
| 1(529) ~ 2101810(529) | D77 | Ar57 |
| 1(530) ~ 2101810(530) | D77 | Ar58 |
| 1(531) ~ 2101810(531) | D77 | Ar59 |
| 1(532) ~ 2101810(532) | D281 | Ar1 |
| 1(533) ~ 2101810(533) | D281 | Ar2 |
| 1(534) ~ 2101810(534) | D281 | Ar3 |
| 1(535) ~ 2101810(535) | D281 | Ar4 |
| 1(536) ~ 2101810(536) | D281 | Ar5 |
| 1(537) ~ 2101810(537) | D281 | Ar6 |
| 1(538) ~ 2101810(538) | D281 | Ar7 |
| 1(539) ~ 2101810(539) | D281 | Ar8 |
| 1(540) ~ 2101810(540) | D281 | Ar9 |
| 1(541) ~ 2101810(541) | D281 | Ar10 |
| 1(542) ~ 2101810(542) | D281 | Ar11 |
| 1(543) ~ 2101810(543) | D281 | Ar12 |

(continued)

| No. | Ar$^1$ | Ar$^2$ |
|---|---|---|
| 1(544) ~ 2101810(544) | D281 | Ar13 |
| 1(545) ~ 2101810(545) | D281 | Ar14 |
| 1(546) ~ 2101810(546) | D281 | Ar15 |
| 1(547) ~ 2101810(547) | D281 | Ar16 |
| 1(548) ~ 2101810(548) | D281 | Ar17 |
| 1(549) ~ 2101810(549) | D281 | Ar18 |
| 1(550) ~ 2101810(550) | D281 | Ar19 |
| 1(551) ~ 2101810(551) | D281 | Ar20 |
| 1(552) ~ 2101810(552) | D281 | Ar21 |
| 1(553) ~ 2101810(553) | D281 | Ar22 |
| 1(554) ~ 2101810(554) | D281 | Ar23 |
| 1(555) ~ 2101810(555) | D281 | Ar24 |
| 1(556) ~ 2101810(556) | D281 | Ar25 |
| 1(557) ~ 2101810(557) | D281 | Ar26 |
| 1(558) ~ 2101810(558) | D281 | Ar27 |
| 1(559) ~ 2101810(559) | D281 | Ar28 |
| 1(560) ~ 2101810(560) | D281 | Ar29 |
| 1(561) ~ 2101810(561) | D281 | Ar30 |
| 1(562) ~ 2101810(562) | D281 | Ar31 |
| 1(563) ~ 2101810(563) | D281 | Ar32 |
| 1(564) ~ 2101810(564) | D281 | Ar33 |
| 1(565) ~ 2101810(565) | D281 | Ar34 |
| 1(566) ~ 2101810(566) | D281 | Ar35 |
| 1(567) ~ 2101810(567) | D281 | Ar36 |
| 1(568) ~ 2101810(568) | D281 | Ar37 |
| 1(569) ~ 2101810(569) | D281 | Ar38 |
| 1(570) ~ 2101810(570) | D281 | Ar39 |
| 1(571) ~ 2101810(571) | D281 | Ar40 |
| 1(572) ~ 2101810(572) | D281 | Ar41 |
| 1(573) ~ 2101810(573) | D281 | Ar42 |
| 1(574) ~ 2101810(574) | D281 | Ar43 |
| 1(575) ~ 2101810(575) | D281 | Ar44 |
| 1(576) ~ 2101810(576) | D281 | Ar45 |
| 1(577) ~ 2101810(577) | D281 | Ar46 |
| 1(578) ~ 2101810(578) | D281 | Ar47 |
| 1(579) ~ 2101810(579) | D281 | Ar48 |
| 1(580) ~ 2101810(580) | D281 | Ar49 |
| 1(581) ~ 2101810(581) | D281 | Ar50 |
| 1(582) ~ 2101810(582) | D281 | Ar51 |

(continued)

| No. | $Ar^1$ | $Ar^2$ |
|---|---|---|
| 1(583) ~ 2101810(583) | D281 | Ar52 |
| 1(584) ~ 2101810(584) | D281 | Ar53 |
| 1(585) ~ 2101810(585) | D281 | Ar54 |
| 1(586) ~ 2101810(586) | D281 | Ar55 |
| 1(587) ~ 2101810(587) | D281 | Ar56 |
| 1(588) ~ 2101810(588) | D281 | Ar57 |
| 1(589) ~ 2101810(589) | D281 | Ar58 |
| 1(590) ~ 2101810(590) | D281 | Ar59 |
| 1(591) ~ 2101810(591) | D459 | Ar1 |
| 1(592) ~ 2101810(592) | D459 | Ar2 |
| 1(593) ~ 2101810(593) | D459 | Ar3 |
| 1(594) ~ 2101810(594) | D459 | Ar4 |
| 1(595) ~ 2101810(595) | D459 | Ar5 |
| 1(596) ~ 2101810(596) | D459 | Ar6 |
| 1(597) ~ 2101810(597) | D459 | Ar7 |
| 1(598) ~ 2101810(598) | D459 | Ar8 |
| 1(599) ~ 2101810(599) | D459 | Ar9 |
| 1(600) ~ 2101810(600) | D459 | Ar10 |
| 1(601) ~ 2101810(601) | D459 | Ar11 |
| 1(602) ~ 2101810(602) | D459 | Ar12 |
| 1(603) ~ 2101810(603) | D459 | Ar13 |
| 1(604) ~ 2101810(604) | D459 | Ar14 |
| 1(605) ~ 2101810(605) | D459 | Ar15 |
| 1(606) ~ 2101810(606) | D459 | Ar16 |
| 1(607) ~ 2101810(607) | D459 | Ar17 |
| 1(608) ~ 2101810(608) | D459 | Ar18 |
| 1(609) ~ 2101810(609) | D459 | Ar19 |
| 1(610) ~ 2101810(610) | D459 | Ar20 |
| 1(611) ~ 2101810(611) | D459 | Ar21 |
| 1(612) ~ 2101810(612) | D459 | Ar22 |
| 1(613) ~ 2101810(613) | D459 | Ar23 |
| 1(614) ~ 2101810(614) | D459 | Ar24 |
| 1(615) ~ 2101810(615) | D459 | Ar25 |
| 1(616) ~ 2101810(616) | D459 | Ar26 |
| 1(617) ~ 2101810(617) | D459 | Ar27 |
| 1(618) ~ 2101810(618) | D459 | Ar28 |
| 1(619) ~ 2101810(619) | D459 | Ar29 |
| 1(620) ~ 2101810(620) | D459 | Ar30 |
| 1(621) ~ 2101810(621) | D459 | Ar31 |

(continued)

| No. | $Ar^1$ | $Ar^2$ |
|---|---|---|
| 1(622) ~ 2101810(622) | D459 | Ar32 |
| 1(623) ~ 2101810(623) | D459 | Ar33 |
| 1(624) ~ 2101810(624) | D459 | Ar34 |
| 1(625) ~ 2101810(625) | D459 | Ar35 |
| 1(626) ~ 2101810(626) | D459 | Ar36 |
| 1(627) ~ 2101810(627) | D459 | Ar37 |
| 1(628) ~ 2101810(628) | D459 | Ar38 |
| 1(629) ~ 2101810(629) | D459 | Ar39 |
| 1(630) ~ 2101810(630) | D459 | Ar40 |
| 1(631) ~ 2101810(631) | D459 | Ar41 |
| 1(632) ~ 2101810(632) | D459 | Ar42 |
| 1(633) ~ 2101810(633) | D459 | Ar43 |
| 1(634) ~ 2101810(634) | D459 | Ar44 |
| 1(635) ~ 2101810(635) | D459 | Ar45 |
| 1(636) ~ 2101810(636) | D459 | Ar46 |
| 1(637) ~ 2101810(637) | D459 | Ar47 |
| 1(638) ~ 2101810(638) | D459 | Ar48 |
| 1(639) ~ 2101810(639) | D459 | Ar49 |
| 1(640) ~ 2101810(640) | D459 | Ar50 |
| 1(641) ~ 2101810(641) | D459 | Ar51 |
| 1(642) ~ 2101810(642) | D459 | Ar52 |
| 1(643) ~ 2101810(643) | D459 | Ar53 |
| 1(644) ~ 2101810(644) | D459 | Ar54 |
| 1(645) ~ 2101810(645) | D459 | Ar55 |
| 1(646) ~ 2101810(646) | D459 | Ar56 |
| 1(647) ~ 2101810(647) | D459 | Ar57 |
| 1(648) ~ 2101810(648) | D459 | Ar58 |
| 1(649) ~ 2101810(649) | D459 | Ar59 |
| 1(650) ~ 2101810(650) | D465 | Ar1 |
| 1(651) ~ 2101810(651) | D465 | Ar2 |
| 1(652) ~ 2101810(652) | D465 | Ar3 |
| 1(653) ~ 2101810(653) | D465 | Ar4 |
| 1(654) ~ 2101810(654) | D465 | Ar5 |
| 1(655) ~ 2101810(655) | D465 | Ar6 |
| 1(656) ~ 2101810(656) | D465 | Ar7 |
| 1(657) ~ 2101810(657) | D465 | Ar8 |
| 1(658) ~ 2101810(658) | D465 | Ar9 |
| 1(659) ~ 2101810(659) | D465 | Ar10 |
| 1(660) ~ 2101810(660) | D465 | Ar11 |

(continued)

| No. | $Ar^1$ | $Ar^2$ |
|---|---|---|
| 1(661) ~ 2101810(661) | D465 | Ar12 |
| 1(662) ~ 2101810(662) | D465 | Ar13 |
| 1(663) ~ 2101810(663) | D465 | Ar14 |
| 1(664) ~ 2101810(664) | D465 | Ar15 |
| 1(665) ~ 2101810(665) | D465 | Ar16 |
| 1(666) ~ 2101810(666) | D465 | Ar17 |
| 1(667) ~ 2101810(667) | D465 | Ar18 |
| 1(668) ~ 2101810(668) | D465 | Ar19 |
| 1(669) ~ 2101810(669) | D465 | Ar20 |
| 1(670) ~ 2101810(670) | D465 | Ar21 |
| 1(671) ~ 2101810(671) | D465 | Ar22 |
| 1(672) ~ 2101810(672) | D465 | Ar23 |
| 1(673) ~ 2101810(673) | D465 | Ar24 |
| 1(674) ~ 2101810(674) | D465 | Ar25 |
| 1(675) ~ 2101810(675) | D465 | Ar26 |
| 1(676) ~ 2101810(676) | D465 | Ar27 |
| 1(677) ~ 2101810(677) | D465 | Ar28 |
| 1(678) ~ 2101810(678) | D465 | Ar29 |
| 1(679) ~ 2101810(679) | D465 | Ar30 |
| 1(680) ~ 2101810(680) | D465 | Ar31 |
| 1(681) ~ 2101810(681) | D465 | Ar32 |
| 1(682) ~ 2101810(682) | D465 | Ar33 |
| 1(683) ~ 2101810(683) | D465 | Ar34 |
| 1(684) ~ 2101810(684) | D465 | Ar35 |
| 1(685) ~ 2101810(685) | D465 | Ar36 |
| 1(686) ~ 2101810(686) | D465 | Ar37 |
| 1(687) ~ 2101810(687) | D465 | Ar38 |
| 1(688) ~ 2101810(688) | D465 | Ar39 |
| 1(689) ~ 2101810(689) | D465 | Ar40 |
| 1(690) ~ 2101810(690) | D465 | Ar41 |
| 1(691) ~ 2101810(691) | D465 | Ar42 |
| 1(692) ~ 2101810(692) | D465 | Ar43 |
| 1(693) ~ 2101810(693) | D465 | Ar44 |
| 1(694) ~ 2101810(694) | D465 | Ar45 |
| 1(695) ~ 2101810(695) | D465 | Ar46 |
| 1(696) ~ 2101810(696) | D465 | Ar47 |
| 1(697) ~ 2101810(697) | D465 | Ar48 |
| 1(698) ~ 2101810(698) | D465 | Ar49 |
| 1(699) ~ 2101810(699) | D465 | Ar50 |

(continued)

| No. | $Ar^1$ | $Ar^2$ |
|---|---|---|
| 1(700) ~ 2101810(700) | D465 | Ar51 |
| 1(701) ~ 2101810(701) | D465 | Ar52 |
| 1(702) ~ 2101810(702) | D465 | Ar53 |
| 1(703) ~ 2101810(703) | D465 | Ar54 |
| 1(704) ~ 2101810(704) | D465 | Ar55 |
| 1(705) ~ 2101810(705) | D465 | Ar56 |
| 1(706) ~ 2101810(706) | D465 | Ar57 |
| 1(707) ~ 2101810(707) | D465 | Ar58 |
| 1(708) ~ 2101810(708) | D465 | Ar59 |
| 1(709) ~ 2101810(709) | D466 | Ar1 |
| 1(710) ~ 2101810(710) | D466 | Ar2 |
| 1(711) ~ 2101810(711) | D466 | Ar3 |
| 1(712) ~ 2101810(712) | D466 | Ar4 |
| 1(713) ~ 2101810(713) | D466 | Ar5 |
| 1(714) ~ 2101810(714) | D466 | Ar6 |
| 1(715) ~ 2101810(715) | D466 | Ar7 |
| 1(716) ~ 2101810(716) | D466 | Ar8 |
| 1(717) ~ 2101810(717) | D466 | Ar9 |
| 1(718) ~ 2101810(718) | D466 | Ar10 |
| 1(719) ~ 2101810(719) | D466 | Ar11 |
| 1(720) ~ 2101810(720) | D466 | Ar12 |
| 1(721) ~ 2101810(721) | D466 | Ar13 |
| 1(722) ~ 2101810(722) | D466 | Ar14 |
| 1(723) ~ 2101810(723) | D466 | Ar15 |
| 1(724) ~ 2101810(724) | D466 | Ar16 |
| 1(725) ~ 2101810(725) | D466 | Ar17 |
| 1(726) ~ 2101810(726) | D466 | Ar18 |
| 1(727) ~ 2101810(727) | D466 | Ar19 |
| 1(728) ~ 2101810(728) | D466 | Ar20 |
| 1(729) ~ 2101810(729) | D466 | Ar21 |
| 1(730) ~ 2101810(730) | D466 | Ar22 |
| 1(731) ~ 2101810(731) | D466 | Ar23 |
| 1(732) ~ 2101810(732) | D466 | Ar24 |
| 1(733) ~ 2101810(733) | D466 | Ar25 |
| 1(734) ~ 2101810(734) | D466 | Ar26 |
| 1(735) ~ 2101810(735) | D466 | Ar27 |
| 1(736) ~ 2101810(736) | D466 | Ar28 |
| 1(737) ~ 2101810(737) | D466 | Ar29 |
| 1(738) ~ 2101810(738) | D466 | Ar30 |

(continued)

| No. | Ar1 | Ar2 |
|---|---|---|
| 1(739) ~ 2101810(739) | D466 | Ar31 |
| 1(740) ~ 2101810(740) | D466 | Ar32 |
| 1(741) ~ 2101810(741) | D466 | Ar33 |
| 1(742) ~ 2101810(742) | D466 | Ar34 |
| 1(743) ~ 2101810(743) | D466 | Ar35 |
| 1(744) ~ 2101810(744) | D466 | Ar36 |
| 1(745) ~ 2101810(745) | D466 | Ar37 |
| 1(746) ~ 2101810(746) | D466 | Ar38 |
| 1(747) ~ 2101810(747) | D466 | Ar39 |
| 1(748) ~ 2101810(748) | D466 | Ar40 |
| 1(749) ~ 2101810(749) | D466 | Ar41 |
| 1(750) ~ 2101810(750) | D466 | Ar42 |
| 1(751) ~ 2101810(751) | D466 | Ar43 |
| 1(752) ~ 2101810(752) | D466 | Ar44 |
| 1(753) ~ 2101810(753) | D466 | Ar45 |
| 1(754) ~ 2101810(754) | D466 | Ar46 |
| 1(755) ~ 2101810(755) | D466 | Ar47 |
| 1(756) ~ 2101810(756) | D466 | Ar48 |
| 1(757) ~ 2101810(757) | D466 | Ar49 |
| 1(758) ~ 2101810(758) | D466 | Ar50 |
| 1(759) ~ 2101810(759) | D466 | Ar51 |
| 1(760) ~ 2101810(760) | D466 | Ar52 |
| 1(761) ~ 2101810(761) | D466 | Ar53 |
| 1(762) ~ 2101810(762) | D466 | Ar54 |
| 1(763) ~ 2101810(763) | D466 | Ar55 |
| 1(764) ~ 2101810(764) | D466 | Ar56 |
| 1(765) ~ 2101810(765) | D466 | Ar57 |
| 1(766) ~ 2101810(766) | D466 | Ar58 |
| 1(767) ~ 2101810(767) | D466 | Ar59 |
| 1(768) ~ 2101810(768) | D467 | Ar1 |
| 1(769) ~ 2101810(769) | D467 | Ar2 |
| 1(770) ~ 2101810(770) | D467 | Ar3 |
| 1(771) ~ 2101810(771) | D467 | Ar4 |
| 1(772) ~ 2101810(772) | D467 | Ar5 |
| 1(773) ~ 2101810(773) | D467 | Ar6 |
| 1(774) ~ 2101810(774) | D467 | Ar7 |
| 1(775) ~ 2101810(775) | D467 | Ar8 |
| 1(776) ~ 2101810(776) | D467 | Ar9 |
| 1(777) ~ 2101810(777) | D467 | Ar10 |

(continued)

| No. | Ar1 | Ar2 |
|---|---|---|
| 1(778) ~ 2101810(778) | D467 | Ar11 |
| 1(779) ~ 2101810(779) | D467 | Ar12 |
| 1(780) ~ 2101810(780) | D467 | Ar13 |
| 1(781) ~ 2101810(781) | D467 | Ar14 |
| 1(782) ~ 2101810(782) | D467 | Ar15 |
| 1(783) ~ 2101810(783) | D467 | Ar16 |
| 1(784) ~ 2101810(784) | D467 | Ar17 |
| 1(785) ~ 2101810(785) | D467 | Ar18 |
| 1(786) ~ 2101810(786) | D467 | Ar19 |
| 1(787) ~ 2101810(787) | D467 | Ar20 |
| 1(788) ~ 2101810(788) | D467 | Ar21 |
| 1(789) ~ 2101810(789) | D467 | Ar22 |
| 1(790) ~ 2101810(790) | D467 | Ar23 |
| 1(791) ~ 2101810(791) | D467 | Ar24 |
| 1(792) ~ 2101810(792) | D467 | Ar25 |
| 1(793) ~ 2101810(793) | D467 | Ar26 |
| 1(794) ~ 2101810(794) | D467 | Ar27 |
| 1(795) ~ 2101810(795) | D467 | Ar28 |
| 1(796) ~ 2101810(796) | D467 | Ar29 |
| 1(797) ~ 2101810(797) | D467 | Ar30 |
| 1(798) ~ 2101810(798) | D467 | Ar31 |
| 1(799) ~ 2101810(799) | D467 | Ar32 |
| 1(800) ~ 2101810(800) | D467 | Ar33 |
| 1(801) ~ 2101810(801) | D467 | Ar34 |
| 1(802) ~ 2101810(802) | D467 | Ar35 |
| 1(803) ~ 2101810(803) | D467 | Ar36 |
| 1(804) ~ 2101810(804) | D467 | Ar37 |
| 1(805) ~ 2101810(805) | D467 | Ar38 |
| 1(806) ~ 2101810(806) | D467 | Ar39 |
| 1(807) ~ 2101810(807) | D467 | Ar40 |
| 1(808) ~ 2101810(808) | D467 | Ar41 |
| 1(809) ~ 2101810(809) | D467 | Ar42 |
| 1(810) ~ 2101810(810) | D467 | Ar43 |
| 1(811) ~ 2101810(811) | D467 | Ar44 |
| 1(812) ~ 2101810(812) | D467 | Ar45 |
| 1(813) ~ 2101810(813) | D467 | Ar46 |
| 1(814) ~ 2101810(814) | D467 | Ar47 |
| 1(815) ~ 2101810(815) | D467 | Ar48 |
| 1(816) ~ 2101810(816) | D467 | Ar49 |

(continued)

| No. | Ar1 | Ar2 |
|---|---|---|
| 1(817) ~ 2101810(817) | D467 | Ar50 |
| 1(818) ~ 2101810(818) | D467 | Ar51 |
| 1(819) ~ 2101810(819) | D467 | Ar52 |
| 1(820) ~ 2101810(820) | D467 | Ar53 |
| 1(821) ~ 2101810(821) | D467 | Ar54 |
| 1(822) ~ 2101810(822) | D467 | Ar55 |
| 1(823) ~ 2101810(823) | D467 | Ar56 |
| 1(824) ~ 2101810(824) | D467 | Ar57 |
| 1(825) ~ 2101810(825) | D467 | Ar58 |
| 1(826) ~ 2101810(826) | D467 | Ar59 |
| 1(827) ~ 2101810(827) | D471 | Ar1 |
| 1(828) ~ 2101810(828) | D471 | Ar2 |
| 1(829) ~ 2101810(829) | D471 | Ar3 |
| 1(830) ~ 2101810(830) | D471 | Ar4 |
| 1(831) ~ 2101810(831) | D471 | Ar5 |
| 1(832) ~ 2101810(832) | D471 | Ar6 |
| 1(833) ~ 2101810(833) | D471 | Ar7 |
| 1(834) ~ 2101810(834) | D471 | Ar8 |
| 1(835) ~ 2101810(835) | D471 | Ar9 |
| 1(836) ~ 2101810(836) | D471 | Ar10 |
| 1(837) ~ 2101810(837) | D471 | Ar11 |
| 1(838) ~ 2101810(838) | D471 | Ar12 |
| 1(839) ~ 2101810(839) | D471 | Ar13 |
| 1(840) ~ 2101810(840) | D471 | Ar14 |
| 1(841) ~ 2101810(841) | D471 | Ar15 |
| 1(842) ~ 2101810(842) | D471 | Ar16 |
| 1(843) ~ 2101810(843) | D471 | Ar17 |
| 1(844) ~ 2101810(844) | D471 | Ar18 |
| 1(845) ~ 2101810(845) | D471 | Ar19 |
| 1(846) ~ 2101810(846) | D471 | Ar20 |
| 1(847) ~ 2101810(847) | D471 | Ar21 |
| 1(848) ~ 2101810(848) | D471 | Ar22 |
| 1(849) ~ 2101810(849) | D471 | Ar23 |
| 1(850) ~ 2101810(850) | D471 | Ar24 |
| 1(851) ~ 2101810(851) | D471 | Ar25 |
| 1(852) ~ 2101810(852) | D471 | Ar26 |
| 1(853) ~ 2101810(853) | D471 | Ar27 |
| 1(854) ~ 2101810(854) | D471 | Ar28 |
| 1(855) ~ 2101810(855) | D471 | Ar29 |

(continued)

| No. | $Ar^1$ | $Ar^2$ |
|---|---|---|
| 1(856) ~ 2101810(856) | D471 | Ar30 |
| 1(857) ~ 2101810(857) | D471 | Ar31 |
| 1(858) ~ 2101810(858) | D471 | Ar32 |
| 1(859) ~ 2101810(859) | D471 | Ar33 |
| 1(860) ~ 2101810(860) | D471 | Ar34 |
| 1(861) ~ 2101810(861) | D471 | Ar35 |
| 1(862) ~ 2101810(862) | D471 | Ar36 |
| 1(863) ~ 2101810(863) | D471 | Ar37 |
| 1(864) ~ 2101810(864) | D471 | Ar38 |
| 1(865) ~ 2101810(865) | D471 | Ar39 |
| 1(866) ~ 2101810(866) | D471 | Ar40 |
| 1(867) ~ 2101810(867) | D471 | Ar41 |
| 1(868) ~ 2101810(868) | D471 | Ar42 |
| 1(869) ~ 2101810(869) | D471 | Ar43 |
| 1(870) ~ 2101810(870) | D471 | Ar44 |
| 1(871) ~ 2101810(871) | D471 | Ar45 |
| 1(872) ~ 2101810(872) | D471 | Ar46 |
| 1(873) ~ 2101810(873) | D471 | Ar47 |
| 1(874) ~ 2101810(874) | D471 | Ar48 |
| 1(875) ~ 2101810(875) | D471 | Ar49 |
| 1(876) ~ 2101810(876) | D471 | Ar50 |
| 1(877) ~ 2101810(877) | D471 | Ar51 |
| 1(878) ~ 2101810(878) | D471 | Ar52 |
| 1(879) ~ 2101810(879) | D471 | Ar53 |
| 1(880) ~ 2101810(880) | D471 | Ar54 |
| 1(881) ~ 2101810(881) | D471 | Ar55 |
| 1(882) ~ 2101810(882) | D471 | Ar56 |
| 1(883) ~ 2101810(883) | D471 | Ar57 |
| 1(884) ~ 2101810(884) | D471 | Ar58 |
| 1(885) ~ 2101810(885) | D471 | Ar59 |
| 1(886) ~ 2101810(886) | D486 | Ar1 |
| 1(887) ~ 2101810(887) | D486 | Ar2 |
| 1(888) ~ 2101810(888) | D486 | Ar3 |
| 1(889) ~ 2101810(889) | D486 | Ar4 |
| 1(890) ~ 2101810(890) | D486 | Ar5 |
| 1(891) ~ 2101810(891) | D486 | Ar6 |
| 1(892) ~ 2101810(892) | D486 | Ar7 |
| 1(893) ~ 2101810(893) | D486 | Ar8 |
| 1(894) ~ 2101810(894) | D486 | Ar9 |

(continued)

| No. | Ar1 | Ar2 |
|---|---|---|
| 1(895) ~ 2101810(895) | D486 | Ar10 |
| 1(896) ~ 2101810(896), | D486 | Ar11 |
| 1(897) ~ 2101810(897) | D486 | Ar12 |
| 1(898) ~ 2101810(898) | D486 | Ar13 |
| 1(899) ~ 2101810(899) | D486 | Ar14 |
| 1(900) ~ 2101810(900), | D486 | Ar15 |
| 1(901) ~ 2101810(301) | D486 | Ar16 |
| 1(902) ~ 2101810(902) | D486 | Ar17 |
| 1(903) ~ 2101810(903) | D486 | Ar18 |
| 1(904) ~ 2101810(904) | D486 | Ar19 |
| 1(905) ~ 2101810(905) | D486 | Ar20 |
| 1(906) ~ 2101810(906) | D486 | Ar21 |
| 1(907) ~ 2101810(907) | D486 | Ar22 |
| 1(908) ~ 2101810(908) | D486 | Ar23 |
| 1(909) ~ 2101810(909) | D486 | Ar24 |
| 1(910) ~ 2101810(910) | D486 | Ar25 |
| 1(911) ~ 2101810(911) | D486 | Ar26 |
| 1(912) ~ 2101810(912) | D486 | Ar27 |
| 1913) ~ 2101810(913) | D486 | Ar28 |
| 1(914) ~ 2101810(914) | D486 | Ar29 |
| 1(915) ~ 2101810(915) | D486 | Ar30 |
| 1(916) ~ 2101810(916) | D486 | Ar31 |
| 1(917) ~ 2101810(917) | D486 | Ar32 |
| 1(918) ~ 2101810(918) | D486 | Ar33 |
| 1(919) ~ 2101810(919) | D486 | Ar34 |
| 1(920) ~ 2101810(920) | D486 | Ar35 |
| 1(921) ~ 2101810(921) | D486 | Ar36 |
| 1(922) ~ 2101810(922) | D486 | Ar37 |
| 1(923) ~ 2101810(923) | D486 | Ar38 |
| 1(924) ~ 2101810(924) | D486 | Ar39 |
| 1(925) ~ 2101810(925) | D486 | Ar40 |
| 1(926) ~ 2101810(926) | D486 | Ar41 |
| 1(927) ~ 2101810(927) | D486 | Ar42 |
| 1(928) ~ 2101810(928) | D486 | Ar43 |
| 1(929) ~ 2101810(929) | D486 | Ar44 |
| 1(930) ~ 2101810(930) | D486 | Ar45 |
| 1(931) ~ 2101810(931) | D486 | Ar46 |
| 1(932) ~ 2101810(932) | D486 | Ar47 |
| 1(933) ~ 2101810(933) | D486 | Ar48 |

(continued)

| No. | $Ar^1$ | $Ar^2$ |
|---|---|---|
| 1(934) ~ 2101810(934) | D486 | Ar49 |
| 1(935) ~ 2101810(935) | D486 | Ar50 |
| 1(936) ~ 2101810(936) | D486 | Ar51 |
| 1(937) ~ 2101810(937) | D486 | Ar52 |
| 1(938) ~ 2101810(938) | D486 | Ar53 |
| 1(939) ~ 2101810(939) | D486 | Ar54 |
| 1(940) ~ 2101810(940) | D486 | Ar55 |
| 1(941) ~ 2101810(941) | D486 | Ar56 |
| 1(942) ~ 2101810(942) | D486 | Ar57 |
| 1(943) ~ 2101810(943) | D486 | Ar58 |
| 1(944) ~ 2101810(944) | D486 | Ar59 |
| 1(945) ~ 2101810(945) | D520 | Ar1 |
| 1(946) ~ 2101810(946) | D520 | Ar2 |
| 1(947) ~ 2101810(947) | D520 | Ar3 |
| 1(948) ~ 2101810(948) | D520 | Ar4 |
| 1(949) ~ 2101810(949) | D520 | Ar5 |
| 1(950) ~ 2101810(950) | D520 | Ar6 |
| 1(951) ~ 2101810(951) | D520 | Ar7 |
| 1(952) ~ 2101810(952) | D520 | Ar8 |
| 1(953) ~ 2101810(953) | D520 | Ar9 |
| 1(954) ~ 2101810(954) | D520 | Ar10 |
| 1(955) ~ 2101810(955) | D520 | Ar11 |
| 1(956) ~ 2101810(956) | D520 | Ar12 |
| 1(957) ~ 2101810(957) | D520 | Ar13 |
| 1(958) ~ 2101810(958) | D520 | Ar14 |
| 1(959) ~ 2101810(959) | D520 | Ar15 |
| 1(960) ~ 2101810(960) | D520 | Ar16 |
| 1(961) ~ 2101810(961) | D520 | Ar17 |
| 1(962) ~ 2101810(962) | D520 | Ar18 |
| 1(963) ~ 2101810(963) | D520 | Ar19 |
| 1(964) ~ 2101810(964) | D520 | Ar20 |
| 1(965) ~ 2101810(965) | D520 | Ar21 |
| 1(966) ~ 2101810(966) | D520 | Ar22 |
| 1(967) ~ 2101810(967) | D520 | Ar23 |
| 1(968) ~ 2101810(968) | D520 | Ar24 |
| 1(969) ~ 2101810(969) | D520 | Ar25 |
| 1(970) ~ 2101810(970) | D520 | Ar26 |
| 1(971) ~ 2101810(971) | D520 | Ar27 |
| 1(972) ~ 2101810(972) | D520 | Ar28 |

(continued)

| No. | $Ar^1$ | $Ar^2$ |
|---|---|---|
| 1(973) ~ 2101810(973) | D520 | Ar29 |
| 1(974) ~ 2101810(974) | D520 | Ar30 |
| 1(975) ~ 2101810(975) | D520 | Ar31 |
| 1(976) ~ 2101810(976) | D520 | Ar32 |
| 1(977) ~ 2101810(977) | D520 | Ar33 |
| 1(978) ~ 2101810(978) | D520 | Ar34 |
| 1(979) ~ 2101810(979) | D520 | Ar35 |
| 1980) ~ 2101810(980) | D520 | Ar36 |
| 1(981) ~ 2101810(981) | D520 | Ar37 |
| 1(982) ~ 2101810(982) | D520 | Ar38 |
| 1(983) ~ 2101810(983) | D520 | Ar39 |
| 1(984) ~ 2101810(984) | D520 | Ar40 |
| 1(985) ~ 2101810(985) | D520 | Ar41 |
| 1(986) ~ 2101810(986) | D520 | Ar42 |
| 1(987) ~ 2101810(987) | D520 | Ar43 |
| 1(988) ~ 2101810(988) | D520 | Ar44 |
| 1(989) ~ 2101810(989) | D520 | Ar45 |
| 1(990) ~ 2101810(990) | D520 | Ar46 |
| 1(991) ~ 2101810(991) | D520 | Ar47 |
| 1(992) ~ 2101810(992) | D520 | Ar48 |
| 1(993) ~ 2101810(993) | D520 | Ar49 |
| 1(994) ~ 2101810(994) | D520 | Ar50 |
| 1(995) ~ 2101810(995) | D520 | Ar51 |
| 1(996) ~ 2101810(996) | D520 | Ar52 |
| 1(997) ~ 2101810(997) | D520 | Ar53 |
| 1(998) ~ 2101810(998) | D520 | Ar54 |
| 1(999) ~ 2101810(999) | D520 | Ar55 |
| 1(1000) ~ 2101810(1000) | D520 | Ar56 |
| 1(1001) ~ 2101810(1001) | D520 | Ar57 |
| 1(1002) ~ 2101810(1002) | D520 | Ar58 |
| 1(1003) ~ 2101810(1003) | D520 | Ar59 |
| 1(1004) ~ 2101810(1004) | D621 | Ar1 |
| 1(1005) ~ 2101810(1005) | D621 | Ar2 |
| 1(1006) ~ 2101810(1006) | D621 | Ar3 |
| 1(1007) ~ 2101810(1007) | D621 | Ar4 |
| 1(1008) ~ 2101810(1008) | D621 | Ar5 |
| 1(1009) ~ 2101810(1009) | D621 | Ar6 |
| 1(1010) ~ 2101810(1010) | D621 | Ar7 |
| 1(1011) ~ 2101810(1011) | D621 | Ar8 |

(continued)

| No. | $Ar^1$ | $Ar^2$ |
|---|---|---|
| 1(1012) ~ 2101810(1012) | D621 | Ar9 |
| 1(1013) ~ 2101810(1013) | D621 | Ar10 |
| 1(1014) ~ 2101810(1014) | D621 | Ar11 |
| 1(1015) ~ 2101810(1015) | D621 | Ar12 |
| 1(1016) ~ 2101810(1016) | D621 | Ar13 |
| 1(1017) ~ 2101810(1017) | D621 | Ar14 |
| 1(1018) ~ 2101810(1018) | D621 | Ar15 |
| 1(1019) ~ 2101810(1019) | D621 | Ar16 |
| 1(1020) ~ 2101810(1020) | D621 | Ar17 |
| 1(1021) ~ 2101810(1021) | D621 | Ar18 |
| 1(1022) ~ 2101810(1022) | D621 | Ar19 |
| 1(1023) ~ 2101810(1023) | D621 | Ar20 |
| 1(1024) ~ 2101810(1024) | D621 | Ar21 |
| 1(1025) ~ 2101810(1025) | D621 | Ar22 |
| 1(1026) ~ 2101810(1026) | D621 | Ar23 |
| 1(1027) ~ 2101810(1027) | D621 | Ar24 |
| 1(1028) ~ 2101810(1028) | D621 | Ar25 |
| 1(1029) ~ 2101810(1029) | D621 | Ar26 |
| 1(1030) ~ 2101810(1030) | D621 | Ar27 |
| 1(1031) ~ 2101810(1031) | D621 | Ar28 |
| 1(1032) ~ 2101810(1032) | D621 | Ar29 |
| 1(1033) ~ 2101810(1033) | D621 | Ar30 |
| 1(1034) ~ 2101810(1034) | D621 | Ar31 |
| 1(1035) ~ 2101810(1035) | D621 | Ar32 |
| 1(1036) ~ 2101810(1036) | D621 | Ar33 |
| 1(1037) ~ 2101810(1037) | D621 | Ar34 |
| 1(1038) ~ 2101810(1038) | D621 | Ar35 |
| 1(1039) ~ 2101810(1039) | D621 | Ar36 |
| 1(1040) ~ 2101810(1040) | D621 | Ar37 |
| 1(1041) ~ 2101810(1041) | D621 | Ar38 |
| 1(1042) ~ 2101810(1042) | D621 | Ar39 |
| 1(1043) ~ 2101810(1043) | D621 | Ar40 |
| 1(1044) ~ 2101810(1044) | D621 | Ar41 |
| 1(1045) ~ 2101810(1045) | D621 | Ar42 |
| 1(1046) ~ 2101810(1046) | D621 | Ar43 |
| 1(1047) ~ 2101810(1047) | D621 | Ar44 |
| 1(1048) ~ 2101810(1048) | D621 | Ar45 |
| 1(1049) ~ 2101810(1049) | D621 | Ar46 |
| 1(1050) ~ 2101810(1050) | D621 | Ar47 |

(continued)

| No. | $Ar^1$ | $Ar^2$ |
|---|---|---|
| 1(1051) ~ 2101810(1051) | D621 | Ar48 |
| 1(1052) ~ 2101810(1052) | D621 | Ar49 |
| 1(1053) ~ 2101810(1053) | D621 | Ar50 |
| 1(1054) ~ 2101810(1054) | D621 | Ar51 |
| 1(1055) ~ 2101810(1055) | D621 | Ar52 |
| 1(1056) ~ 2101810(1056) | D621 | Ar53 |
| 1(1057) ~ 2101810(1057) | D621 | Ar54 |
| 1(1058) ~ 2101810(1058) | D621 | Ar55 |
| 1(1059) ~ 2101810(1059) | D621 | Ar56 |
| 1(1060) ~ 2101810(1060) | D621 | Ar57 |
| 1(1061) ~ 2101810(1061) | D621 | Ar58 |
| 1(1062) ~ 2101810(1062) | D621 | Ar59 |
| 1(1063) ~ 2101810(1063) | D711 | Ar1 |
| 1(1064) ~ 2101810(1064) | D711 | Ar2 |
| 1(1065) ~ 2101810(1065) | D711 | Ar3 |
| 1(1066) ~ 2101810(1066) | D711 | Ar4 |
| 1(1067) ~ 2101810(1067) | D711 | Ar5 |
| 1(1068) ~ 2101810(1068) | D711 | Ar6 |
| 1(1069) ~ 2101810(1069) | D711 | Ar7 |
| 1(1070) ~ 2101810(1070) | D711 | Ar8 |
| 1(1071) ~ 2101810(1071) | D711 | Ar9 |
| 1(1072) ~ 2101810(1072) | D711 | Ar10 |
| 1(1073) ~ 2101810(1073) | D711 | Ar11 |
| 1(1074) ~ 2101810(1074) | D711 | Ar12 |
| 1(1075) ~ 2101810(1075) | D711 | Ar13 |
| 1(1076) ~ 2101810(1076) | D711 | Ar14 |
| 1(1077) ~ 2101810(1077) | D711 | Ar15 |
| 1(1078) ~ 2101810(1078) | D711 | Ar16 |
| 1(1079) ~ 2101810(1079) | D711 | Ar17 |
| 1(1080) ~ 2101810(1080) | D711 | Ar18 |
| 1(1081) ~ 2101810(1081) | D711 | Ar19 |
| 1(1082) ~ 2101810(1082) | D711 | Ar20 |
| 1(1083) ~ 2101810(1083) | D711 | Ar21 |
| 1(1084) ~ 2101810(1084) | D711 | Ar22 |
| 1(1085) ~ 2101810(1085) | D711 | Ar23 |
| 1(1086) ~ 2101810(1086) | D711 | Ar24 |
| 1(1087) ~ 2101810(1087) | D711 | Ar25 |
| 1(1088) ~ 2101810(1088) | D711 | Ar26 |
| 1(1089) ~ 2101810(1089) | D711 | Ar27 |

(continued)

| No. | $Ar^1$ | $Ar^2$ |
|---|---|---|
| 1(1090) ~ 2101810(1090) | D711 | Ar28 |
| 1(1091) ~ 2101810(1091) | D711 | Ar29 |
| 1(1092) ~ 2101810(1092) | D711 | Ar30 |
| 1(1093) ~ 2101810(1093) | D711 | Ar31 |
| 1(1094) ~ 2101810(1094) | D711 | Ar32 |
| 1(1095) ~ 2101810(1095) | D711 | Ar33 |
| 1(1096) ~ 2101810(1096) | D711 | Ar34 |
| 1(1097) ~ 2101810(1097) | D711 | Ar35 |
| 1(1098) ~ 2101810(1098) | D711 | Ar36 |
| 1(1099) ~ 2101810(1099) | D711 | Ar37 |
| 1(1100) ~ 2101810(1100) | D711 | Ar38 |
| 1(1101) ~ 2101810(1101) | D711 | Ar39 |
| 1(1102) ~ 2101810(1102) | D711 | Ar40 |
| 1(1103) ~ 2101810(1103) | D711 | Ar41 |
| 1(1104) ~ 2101810(1104) | D711 | Ar42 |
| 1(1105) ~ 2101810(1105) | D711 | Ar43 |
| 1(1106) ~ 2101810(1106) | D711 | Ar44 |
| 1(1107) ~ 2101810(1107) | D711 | Ar45 |
| 1(1108) ~ 2101810(1108) | D711 | Ar46 |
| 1(1109) ~ 2101810(1109) | D711 | Ar47 |
| 1(1110) ~ 2101810(1110) | D711 | Ar48 |
| 1(1111) ~ 2101810(1111) | D711 | Ar49 |
| 1(1112) ~ 2101810(1112) | D711 | Ar50 |
| 1(1113) ~ 2101810(1113) | D711 | Ar51 |
| 1(1114) ~ 2101810(1114) | D711 | Ar52 |
| 1(1115) ~ 2101810(1115) | D711 | Ar53 |
| 1(1116) ~ 2101810(1116) | D711 | Ar54 |
| 1(1117) ~ 2101810(1117) | D711 | Ar55 |
| 1(1118) ~ 2101810(1118) | D711 | Ar56 |
| 1(1119) ~ 2101810(1119) | D711 | Ar57 |
| 1(1120) ~ 2101810(1120) | D711 | Ar58 |
| 1(1121) ~ 2101810(1121) | D711 | Ar59 |
| 1(1122) ~ 2101810(1122) | D712 | Ar1 |
| 1(1123) ~ 2101810(1123) | D712 | Ar2 |
| 1(1124) ~ 2101810(1124) | D712 | Ar3 |
| 1(1125) ~ 2101810(1125) | D712 | Ar4 |
| 1(1126) ~ 2101810(1126) | D712 | Ar5 |
| 1(1127) ~ 2101810(1127) | D712 | Ar6 |
| 1(1128) ~ 2101810(1128) | D712 | Ar7 |

(continued)

| No. | $Ar^1$ | $Ar^2$ |
|---|---|---|
| 1(1129) ~ 2101810(1129) | D712 | Ar8 |
| 1(1130) ~ 2101810(1130) | D712 | Ar9 |
| 1(1131) ~ 2101810(1131) | D712 | Ar10 |
| 1(1132) ~ 2101810(1132) | D712 | Ar11 |
| 1(1133) ~ 2101810(1133) | D712 | Ar12 |
| 1(1134) ~ 2101810(1134) | D712 | Ar13 |
| 1(1135) ~ 2101810(1135) | D712 | Ar14 |
| 1(1136) ~ 2101810(1136) | D712 | Ar15 |
| 1(1137) ~ 2101810(1137) | D712 | Ar16 |
| 1(1138) ~ 2101810(1138) | D712 | Ar17 |
| 1(1139) ~ 2101810(1139) | D712 | Ar18 |
| 1(1140) ~ 2101810(1140) | D712 | Ar19 |
| 1(1141) ~ 2101810(1141) | D712 | Ar20 |
| 1(1142) ~ 2101810(1142) | D712 | Ar21 |
| 1(1143) ~ 2101810(1143) | D712 | Ar22 |
| 1(1144) ~ 2101810(1144) | D712 | Ar23 |
| 1(1145) ~ 2101810(1145) | D712 | Ar24 |
| 1(1146) ~ 2101810(1146) | D712 | Ar25 |
| 1(1147) ~ 2101810(1147) | D712 | Ar26 |
| 1(1148) ~ 2101810(1148) | D712 | Ar27 |
| 1(1149) ~ 2101810(1149) | D712 | Ar28 |
| 1(1150) ~ 2101810(1150) | D712 | Ar29 |
| 1(1151) ~ 2101810(1151) | D712 | Ar30 |
| 1(1152) ~ 2101810(1152) | D712 | Ar31 |
| 1(1153) ~ 2101810(1153) | D712 | Ar32 |
| 1(1154) ~ 2101810(1154) | D712 | Ar33 |
| 1(1155) ~ 2101810(1155) | D712 | Ar34 |
| 1(1156) ~ 2101810(1156) | D712 | Ar35 |
| 1(1157) ~ 2101810(1157) | D712 | Ar36 |
| 1(1158) ~ 2101810(1158) | D712 | Ar37 |
| 1(1159) ~ 2101810(1159) | D712 | Ar38 |
| 1(1160) ~ 2101810(1160) | D712 | Ar39 |
| 1(1161) ~ 2101810(1161) | D712 | Ar40 |
| 1(1162) ~ 2101810(1162) | D712 | Ar41 |
| 1(1163) ~ 2101810(1163) | D712 | Ar42 |
| 1(1164) ~ 2101810(1164) | D712 | Ar43 |
| 1(1165) ~ 2101810(1165) | D712 | Ar44 |
| 1(1166) ~ 2101810(1166) | D712 | Ar45 |
| 1(1167) ~ 2101810(1167) | D712 | Ar46 |

(continued)

| No. | Ar$^1$ | Ar$^2$ |
|---|---|---|
| 1(1168) ~ 2101810(1168) | D712 | Ar47 |
| 1(1169) ~ 2101810(1169) | D712 | Ar48 |
| 1(1170) ~ 2101810(1170) | D712 | Ar49 |
| 1(1171) ~ 2101810(1171) | D712 | Ar50 |
| 1(1172) ~ 2101810(1172) | D712 | Ar51 |
| 1(1173) ~ 2101810(1173) | D712 | Ar52 |
| 1(1174) ~ 2101810(1174) | D712 | Ar53 |
| 1(1175) ~ 2101810(1175) | D712 | Ar54 |
| 1(1176) ~ 2101810(1176) | D712 | Ar55 |
| 1(1177) ~ 2101810(1177) | D712 | Ar56 |
| 1(1178) ~ 2101810(1178) | D712 | Ar57 |
| 1(1179) ~ 2101810(1179) | D712 | Ar58 |
| 1(1180) ~ 2101810(1180) | D712 | Ar59 |
| 1(1181) ~ 2101810(1181) | D713 | Ar1 |
| 1(1182) ~ 2101810(1182) | D713 | Ar2 |
| 1(1183) ~ 2101810(1183) | D713 | Ar3 |
| 1(1184) ~ 2101810(1184) | D713 | Ar4 |
| 1(1185) ~ 2101810(1185) | D713 | Ar5 |
| 1(1186) ~ 2101810(1186) | D713 | Ar6 |
| 1(1187) ~ 2101810(1187) | D713 | Ar7 |
| 1(1188) ~ 2101810(1188) | D713 | Ar8 |
| 1(1189) ~ 2101810(1189) | D713 | Ar9 |
| 1(1190) ~ 2101810(1190) | D713 | Ar10 |
| 1(1191) ~ 2101810(1191) | D713 | Ar11 |
| 1(1192) ~ 2101810(1192) | D713 | Ar12 |
| 1(1193) ~ 2101810(1193) | D713 | Ar13 |
| 1(1194) ~ 2101810(1194) | D713 | Ar14 |
| 1(1195) ~ 2101810(1195) | D713 | Ar15 |
| 1(1196) ~ 2101810(1196) | D713 | Ar16 |
| 1(1197) ~ 2101810(1197) | D713 | Ar17 |
| 1(1198) ~ 2101810(1198) | D713 | Ar18 |
| 1(1199) ~ 2101810(1199) | D713 | Ar19 |
| 1(1200) ~ 2101810(1200) | D713 | Ar20 |
| 1(1201) ~ 2101810(1201) | D713 | Ar21 |
| 1(1202) ~ 2101810(1202) | D713 | Ar22 |
| 1(1203) ~ 2101810(1203) | D713 | Ar23 |
| 1(1204) ~ 2101810(1204) | D713 | Ar24 |
| 1(1205) ~ 2101810(1205) | D713 | Ar25 |
| 1(1206) ~ 2101810(1206) | D713 | Ar26 |

(continued)

| No. | $Ar^1$ | $Ar^2$ |
|---|---|---|
| 1(1207) ~ 2101810(1207) | D713 | Ar27 |
| 1(1208) ~ 2101810(1208) | D713 | Ar28 |
| 1(1209) ~ 2101810(1209) | D713 | Ar29 |
| 1(1210) ~ 2101810(1210) | D713 | Ar30 |
| 1(1211) ~ 2101810(1211) | D713 | Ar31 |
| 1(1212) ~ 2101810(1212) | D713 | Ar32 |
| 1(1213) ~ 2101810(1213) | D713 | Ar33 |
| 1(1214) ~ 2101810(1214) | D713 | Ar34 |
| 1(1215) ~ 2101810(1215) | D713 | Ar35 |
| 1(1216) ~ 2101810(1216) | D713 | Ar36 |
| 1(1217) ~ 2101810(1217) | D713 | Ar37 |
| 1(1218) ~ 2101810(1218) | D713 | Ar38 |
| 1(1219) ~ 2101810(1219) | D713 | Ar39 |
| 1(1220) ~ 2101810(1220) | D713 | Ar40 |
| 1(1221) ~ 2101810(1221) | D713 | Ar41 |
| 1(1222) ~ 2101810(1222) | D713 | Ar42 |
| 1(1223) ~ 2101810(1223) | D713 | Ar43 |
| 1(1224) ~ 2101810(1224) | D713 | Ar44 |
| 1(1225) ~ 2101810(1225) | D713 | Ar45 |
| 1(1226) ~ 2101810(1226) | D713 | Ar46 |
| 1(1227) ~ 2101810(1227) | D713 | Ar47 |
| 1(1228) ~ 2101810(1228) | D713 | Ar48 |
| 1(1229) ~ 2101810(1229) | D713 | Ar49 |
| 1(1230) ~ 2101810(1230) | D713 | Ar50 |
| 1(1231) ~ 2101810(1231) | D713 | Ar51 |
| 1(1232) ~ 2101810(1232) | D713 | Ar52 |
| 1(1233) ~ 2101810(1233) | D713 | Ar53 |
| 1(1234) ~ 2101810(1234) | D713 | Ar54 |
| 1(1235) ~ 2101810(1235) | D713 | Ar55 |
| 1(1236) ~ 2101810(1236) | D713 | Ar56 |
| 1(1237) ~ 2101810(1237) | D713 | Ar57 |
| 1(1238) ~ 2101810(1238) | D713 | Ar58 |
| 1(1239) ~ 2101810(1239) | D713 | Ar59 |
| 1(1240) ~ 2101810(1240) | D714 | Ar1 |
| 1(1241) ~ 2101810(1241) | D714 | Ar2 |
| 1(1242) ~ 2101810(1242) | D714 | Ar3 |
| 1(1243) ~ 2101810(1243) | D714 | Ar4 |
| 1(1244) ~ 2101810(1244) | D714 | Ar5 |
| 1(1245) ~ 2101810(1245) | D714 | Ar6 |

(continued)

| No. | $Ar^1$ | $Ar^2$ |
|---|---|---|
| 1(1246) ~ 2101810(1246) | D714 | Ar7 |
| 1(1247) ~ 2101810(1247) | D714 | Ar8 |
| 1(1248) ~ 2101810(1248) | D714 | Ar9 |
| 1(1249) ~ 2101810(1249) | D714 | Ar10 |
| 1(1250) ~ 2101810(1250) | D714 | Ar11 |
| 1(1251) ~ 2101810(1251) | D714 | Ar12 |
| 1(1252) ~ 2101810(1252) | D714 | Ar13 |
| 1(1253) ~ 2101810(1253) | D714 | Ar14 |
| 1(1254) ~ 2101810(1254) | D714 | Ar15 |
| 1(1255) ~ 2101810(1255) | D714 | Ar16 |
| 1(1256) ~ 2101810(1256) | D714 | Ar17 |
| 1(1257) ~ 2101810(1257) | D714 | Ar18 |
| 1(1258) ~ 2101810(1258) | D714 | Ar19 |
| 1(1259) ~ 2101810(1259) | D714 | Ar20 |
| 1(1260) ~ 2101810(1260) | D714 | Ar21 |
| 1(1261) ~ 2101810(1261) | D714 | Ar22 |
| 1(1262) ~ 2101810(1262) | D714 | Ar23 |
| 1(1263) ~ 2101810(1263) | D714 | Ar24 |
| 1(1264) ~ 2101810(1264) | D714 | Ar25 |
| 1(1265) ~ 2101810(1265) | D714 | Ar26 |
| 1(1266) ~ 2101810(1266) | D714 | Ar27 |
| 1(1267) ~ 2101810(1267) | D714 | Ar28 |
| 1(1268) ~ 2101810(1268) | D714 | Ar29 |
| 1(1269) ~ 2101810(1269) | D714 | Ar30 |
| 1(1270) ~ 2101810(1270) | D714 | Ar31 |
| 1(1271) ~ 2101810(1271) | D714 | Ar32 |
| 1(1272) ~ 2101810(1272) | D714 | Ar33 |
| 1(1273) ~ 2101810(1273) | D714 | Ar34 |
| 1(1274) ~ 2101810(1274) | D714 | Ar35 |
| 1(1275) ~ 2101810(1275) | D714 | Ar36 |
| 1(1276) ~ 2101810(1276) | D714 | Ar37 |
| 1(1277) ~ 2101810(1277) | D714 | Ar38 |
| 1(1278) ~ 2101810(1278) | D714 | Ar39 |
| 1(1279) ~ 2101810(1279) | D714 | Ar40 |
| 1(1280) ~ 2101810(1280) | D714 | Ar41 |
| 1(1281) ~ 2101810(1281) | D714 | Ar42 |
| 1(1282) ~ 2101810(1282) | D714 | Ar43 |
| 1(1283) ~ 2101810(1283) | D714 | Ar44 |
| 1(1284) ~ 2101810(1284) | D714 | Ar45 |

(continued)

| No. | $Ar^1$ | $Ar^2$ |
|---|---|---|
| 1(1285) ~ 2101810(1285) | D714 | Ar46 |
| 1(1286) ~ 2101810(1286) | D714 | Ar47 |
| 1(1287) ~ 2101810(1287) | D714 | Ar48 |
| 1(1288) ~ 2101810(1288) | D714 | Ar49 |
| 1(1289) ~ 2101810(1289) | D714 | Ar50 |
| 1(1290) ~ 2101810(1290) | D714 | Ar51 |
| 1(1291) ~ 2101810(1291) | D714 | Ar52 |
| 1(1292) ~ 2101810(1292) | D714 | Ar53 |
| 1(1293) ~ 2101810(1293) | D714 | Ar54 |
| 1(1294) ~ 2101810(1294) | D714 | Ar55 |
| 1(1295) ~ 2101810(1295) | D714 | Ar56 |
| 1(1296) ~ 2101810(1296) | D714 | Ar57 |
| 1(1297) ~ 2101810(1297) | D714 | Ar58 |
| 1(1298) ~ 2101810(1298) | D714 | Ar59 |
| 1(1299) ~ 2101810(1299) | D715 | Ar1 |
| 1(1300) ~ 2101810(1300) | D715 | Ar2 |
| 1(1301) ~ 2101810(1301) | D715 | Ar3 |
| 1(1302) ~ 2101810(1302) | D715 | Ar4 |
| 1(1303) ~ 2101810(1303) | D715 | Ar5 |
| 1(1304) ~ 2101810(1304) | D715 | Ar6 |
| 1(1305) ~ 2101810(1305) | D715 | Ar7 |
| 1(1306) ~ 2101810(1306) | D715 | Ar8 |
| 1(1307) ~ 2101810(1307) | D715 | Ar9 |
| 1(1308) ~ 2101810(1308) | D715 | Ar10 |
| 1(1309) ~ 2101810(1309) | D715 | Ar11 |
| 1(1310) ~ 2101810(1310) | D715 | Ar12 |
| 1(1311) ~ 2101810(1311) | D715 | Ar13 |
| 1(1312) ~ 2101810(1312) | D715 | Ar14 |
| 1(1313) ~ 2101810(1313) | D715 | Ar15 |
| 1(1314) ~ 2101810(1314) | D715 | Ar16 |
| 1(1315) ~ 2101810(1315) | D715 | Ar17 |
| 1(1316) ~ 2101810(1316) | D715 | Ar18 |
| 1(1317) ~ 2101810(1317) | D715 | Ar19 |
| 1(1318) ~ 2101810(1318) | D715 | Ar20 |
| 1(1319) ~ 2101810(1319) | D715 | Ar21 |
| 1(1320) ~ 2101810(1320) | D715 | Ar22 |
| 1(1321) ~ 2101810(1321) | D715 | Ar23 |
| 1(1322) ~ 2101810(1322) | D715 | Ar24 |
| 1(1323) ~ 2101810(1323) | D715 | Ar25 |

(continued)

| No. | $Ar^1$ | $Ar^2$ |
|---|---|---|
| 1(1324) ~ 2101810(1324) | D715 | Ar26 |
| 1(1325) ~ 2101810(1325) | D715 | Ar27 |
| 1(1326) ~ 2101810(1326) | D715 | Ar28 |
| 1(1327) ~ 2101810(1327) | D715 | Ar29 |
| 1(1328) ~ 2101810(1328) | D715 | Ar30 |
| 1(1329) ~ 2101810(1329) | D715 | Ar31 |
| 1(1330) ~ 2101810(1330) | D715 | Ar32 |
| 1(1331) ~ 2101810(1331) | D715 | Ar33 |
| 1(1332) ~ 2101810(1332) | D715 | Ar34 |
| 1(1333) ~ 2101810(1333) | D715 | Ar35 |
| 1(1334) ~ 2101810(1334) | D715 | Ar36 |
| 1(1335) ~ 2101810(1335) | D715 | Ar37 |
| 1(1336) ~ 2101810(1336) | D715 | Ar38 |
| 1(1337) ~ 2101810(1337) | D715 | Ar39 |
| 1(1338) ~ 2101810(1338) | D715 | Ar40 |
| 1(1339) ~ 2101810(1339) | D715 | Ar41 |
| 1(1340) ~ 2101810(1340) | D715 | Ar42 |
| 1(1341) ~ 2101810(1341) | D715 | Ar43 |
| 1(1342) ~ 2101810(1342) | D715 | Ar44 |
| 1(1343) ~ 2101810(1343) | D715 | Ar45 |
| 1(1344) ~ 2101810(1344) | D715 | Ar46 |
| 1(1345) ~ 2101810(1345) | D715 | Ar47 |
| 1(1346) ~ 2101810(1346) | D715 | Ar48 |
| 1(1347) ~ 2101810(1347) | D715 | Ar49 |
| 1(1348) ~ 2101810(1348) | D715 | Ar50 |
| 1(1349) ~ 2101810(1349) | D715 | Ar51 |
| 1(1350) ~ 2101810(1350) | D715 | Ar52 |
| 1(1351) ~ 2101810(1351) | D715 | Ar53 |
| 1(1352) ~ 2101810(1352) | D715 | Ar54 |
| 1(1353) ~ 2101810(1353) | D715 | Ar55 |
| 1(1354) ~ 2101810(1354) | D715 | Ar56 |
| 1(1355) ~ 2101810(1355) | D715 | Ar57 |
| 1(1356) ~ 2101810(1356) | D715 | Ar58 |
| 1(1357) ~ 2101810(1357) | D715 | Ar59 |
| 1(1358) ~ 2101810(1358) | D716 | Ar1 |
| 1(1359) ~ 2101810(1359) | D716 | Ar2 |
| 1(1360) ~ 2101810(1360) | D716 | Ar3 |
| 1(1361) ~ 2101810(1361) | D716 | Ar4 |
| 1(1362) ~ 2101810(1362) | D716 | Ar5 |

(continued)

| No. | $Ar^1$ | $Ar^2$ |
|---|---|---|
| 1(1363) ~ 2101810(1363) | D716 | Ar6 |
| 1(1364) ~ 2101810(1364) | D716 | Ar7 |
| 1(1365) ~ 2101810(1365) | D716 | Ar8 |
| 1(1366) ~ 2101810(1366) | D716 | Ar9 |
| 1(1367) ~ 2101810(1367) | D716 | Ar10 |
| 1(1368) ~ 2101810(1368) | D716 | Ar11 |
| 1(1369) ~ 2101810(1369) | D716 | Ar12 |
| 1(1370) ~ 2101810(1370) | D716 | Ar13 |
| 1(1371) ~ 2101810(1371) | D716 | Ar14 |
| 1(1372) ~ 2101810(1372) | D716 | Ar15 |
| 1(1373) ~ 2101810(1373) | D716 | Ar16 |
| 1(1374) ~ 2101810(1374) | D716 | Ar17 |
| 1(1375) ~ 2101810(1375) | D716 | Ar18 |
| 1(1376) ~ 2101810(1376) | D716 | Ar19 |
| 1(1377) ~ 2101810(1377) | D716 | Ar20 |
| 1(1378) ~ 2101810(1378) | D716 | Ar21 |
| 1(1379) ~ 2101810(1379) | D716 | Ar22 |
| 1(1380) ~ 2101810(1380) | D716 | Ar23 |
| 1(1381) ~ 2101810(1381) | D716 | Ar24 |
| 1(1382) ~ 2101810(1382) | D716 | Ar25 |
| 1(1383) ~ 2101810(1383) | D716 | Ar26 |
| 1(1384) ~ 2101810(1384) | D716 | Ar27 |
| 1(1385) ~ 2101810(1385) | D716 | Ar28 |
| 1(1386) ~ 2101810(1386) | D716 | Ar29 |
| 1(1387) ~ 2101810(1387) | D716 | Ar30 |
| 1(1388) ~ 2101810(1388) | D716 | Ar31 |
| 1(1389) ~ 2101810(1389) | D716 | Ar32 |
| 1(1390) ~ 2101810(1390) | D716 | Ar33 |
| 1(1391) ~ 2101810(1391) | D716 | Ar34 |
| 1(1392) ~ 2101810(1392) | D716 | Ar35 |
| 1(1393) ~ 2101810(1393) | D716 | Ar36 |
| 1(1394) ~ 2101810(1394) | D716 | Ar37 |
| 1(1395) ~ 2101810(1395) | D716 | Ar38 |
| 1(1396) ~ 2101810(1396) | D716 | Ar39 |
| 1(1397) ~ 2101810(1397) | D716 | Ar40 |
| 1(1398) ~ 2101810(1398) | D716 | Ar41 |
| 1(1399) ~ 2101810(1399) | D716 | Ar42 |
| 1(1400) ~ 2101810(1400) | D716 | Ar43 |
| 1(1401) ~ 2101810(1401) | D716 | Ar44 |

(continued)

| No. | $Ar^1$ | $Ar^2$ |
|---|---|---|
| 1(1402) ~ 2101810(1402) | D716 | Ar45 |
| 1(1403) ~ 2101810(1403) | D716 | Ar46 |
| 1(1404) ~ 2101810(1404) | D716 | Ar47 |
| 1(1405) ~ 2101810(1405) | D716 | Ar48 |
| 1(1406) ~ 2101810(1406) | D716 | Ar49 |
| 1(1407) ~ 2101810(1407) | D716 | Ar50 |
| 1(1408) ~ 2101810(1408) | D716 | Ar51 |
| 1(1409) ~ 2101810(1409) | D716 | Ar52 |
| 1(1410) ~ 2101810(1410) | D716 | Ar53 |
| 1(1411) ~ 2101810(1411) | D716 | Ar54 |
| 1(1412) ~ 2101810(1412) | D716 | Ar55 |
| 1(1413) ~ 2101810(1413) | D716 | Ar56 |
| 1(1414) ~ 2101810(1414) | D716 | Ar57 |
| 1(1415) ~ 2101810(1415) | D716 | Ar58 |
| 1(1416) ~ 2101810(1416) | D716 | Ar59 |
| 1(1417) ~ 2101810(1417) | D724 | Ar1 |
| 1(1418) ~ 2101810(1418) | D724 | Ar2 |
| 1(1419) ~ 2101810(1419) | D724 | Ar3 |
| 1(1420) ~ 2101810(1420) | D724 | Ar4 |
| 1(1421) ~ 2101810(1421) | D724 | Ar5 |
| 1(1422) ~ 2101810(1422) | D724 | Ar6 |
| 1(1423) ~ 2101810(1423) | D724 | Ar7 |
| 1(1424) ~ 2101810(1424) | D724 | Ar8 |
| 1(1425) ~ 2101810(1425) | D724 | Ar9 |
| 1(1426) ~ 2101810(1426) | D724 | Ar10 |
| 1(1427) ~ 2101810(1427) | D724 | Ar11 |
| 1(1428) ~ 2101810(1428) | D724 | Ar12 |
| 1(1429) ~ 2101810(1429) | D724 | Ar13 |
| 1(1430) ~ 2101810(1430) | D724 | Ar14 |
| 1(1431) ~ 2101810(1431) | D724 | Ar15 |
| 1(1432) ~ 2101810(1432) | D724 | Ar16 |
| 1(1433) ~ 2101810(1433) | D724 | Ar17 |
| 1(1434) ~ 2101810(1434) | D724 | Ar18 |
| 1(1435) ~ 2101810(1435) | D724 | Ar19 |
| 1(1436) ~ 2101810(1436) | D724 | Ar20 |
| 1(1437) ~ 2101810(1437) | D724 | Ar21 |
| 1(1438) ~ 2101810(1438) | D724 | Ar22 |
| 1(1439) ~ 2101810(1439) | D724 | Ar23 |
| 1(1440) ~ 2101810(1440) | D724 | Ar24 |

(continued)

| No. | $Ar^1$ | $Ar^2$ |
|---|---|---|
| 1(1441) ~ 2101810(1441) | D724 | Ar25 |
| 1(1442) ~ 2101810(1442) | D724 | Ar26 |
| 1(1443) ~ 2101810(1443) | D724 | Ar27 |
| 1(1444) ~ 2101810(1444) | D724 | Ar28 |
| 1(1445) ~ 2101810(1445) | D724 | Ar29 |
| 1(1446) ~ 2101810(1446) | D724 | Ar30 |
| 1(1447) ~ 2101810(1447) | D724 | Ar31 |
| 1(1448) ~ 2101810(1448) | D724 | Ar32 |
| 1(1449) ~ 2101810(1449) | D724 | Ar33 |
| 1(1450) ~ 2101810(1450) | D724 | Ar34 |
| 1(1451) ~ 2101810(1451) | D724 | Ar35 |
| 1(1452) ~ 2101810(1452) | D724 | Ar36 |
| 1(1453) ~ 2101810(1453) | D724 | Ar37 |
| 1(1454) ~ 2101810(1454) | D724 | Ar38 |
| 1(1455) ~ 2101810(1455) | D724 | Ar39 |
| 1(1456) ~ 2101810(1456) | D724 | Ar40 |
| 1(1457) ~ 2101810(1457) | D724 | Ar41 |
| 1(1458) ~ 2101810(1458) | D724 | Ar42 |
| 1(1459) ~ 2101810(1459) | D724 | Ar43 |
| 1(1460) ~ 2101810(1460) | D724 | Ar44 |
| 1(1461) ~ 2101810(1461) | D724 | Ar45 |
| 1(1462) ~ 2101810(1462) | D724 | Ar46 |
| 1(1463) ~ 2101810(1463) | D724 | Ar47 |
| 1(1464) ~ 2101810(1464) | D724 | Ar48 |
| 1(1465) ~ 2101810(1465) | D724 | Ar49 |
| 1(1466) ~ 2101810(1466) | D724 | Ar50 |
| 1(1467) ~ 2101810(1467) | D724 | Ar51 |
| 1(1468) ~ 2101810(1468) | D724 | Ar52 |
| 1(1469) ~ 2101810(1469) | D724 | Ar53 |
| 1(1470) ~ 2101810(1470) | D724 | Ar54 |
| 1(1471) ~ 2101810(1471) | D724 | Ar55 |
| 1(1472) ~ 2101810(1472) | D724 | Ar56 |
| 1(1473) ~ 2101810(1473) | D724 | Ar57 |
| 1(1474) ~ 2101810(1474) | D724 | Ar58 |
| 1(1475) ~ 2101810(1475) | D724 | Ar59 |
| 1(1476) ~ 2101810(1476) | D725 | Ar1 |
| 1(1477) ~ 2101810(1477) | D725 | Ar2 |
| 1(1478) ~ 2101810(1478) | D725 | Ar3 |
| 1(1479) ~ 2101810(1479) | D725 | Ar4 |

(continued)

| No. | $Ar^1$ | $Ar^2$ |
|---|---|---|
| 1(1480) ~ 2101810(1480) | D725 | Ar5 |
| 1(1481) ~ 2101810(1481) | D725 | Ar6 |
| 1(1482) ~ 2101810(1482) | D725 | Ar7 |
| 1(1483) ~ 2101810(1483) | D725 | Ar8 |
| 1(1484) ~ 2101810(1484) | D725 | Ar9 |
| 1(1485) ~ 2101810(1485) | D725 | Ar10 |
| 1(1486) ~ 2101810(1486) | D725 | Ar11 |
| 1(1487) ~ 2101810(1487) | D725 | Ar12 |
| 1(1488) ~ 2101810(1488) | D725 | Ar13 |
| 1(1489) ~ 2101810(1489) | D725 | Ar14 |
| 1(1490) ~ 2101810(1490) | D725 | Ar15 |
| 1(1491) ~ 2101810(1491) | D725 | Ar16 |
| 1(1492) ~ 2101810(1492) | D725 | Ar17 |
| 1(1493) ~ 2101810(1493) | D725 | Ar18 |
| 1(1494) ~ 2101810(1494) | D725 | Ar19 |
| 1(1495) ~ 2101810(1495) | D725 | Ar20 |
| 1(1496) ~ 2101810(1496) | D725 | Ar21 |
| 1(1497) ~ 2101810(1497) | D725 | Ar22 |
| 1(1498) ~ 2101810(1498) | D725 | Ar23 |
| 1(1499) ~ 2101810(1499) | D725 | Ar24 |
| 1(1500) ~ 2101810(1500) | D725 | Ar25 |
| 1(1501) ~ 2101810(1501) | D725 | Ar26 |
| 1(1502) ~ 2101810(1502) | D725 | Ar27 |
| 1(1503) ~ 2101810(1503) | D725 | Ar28 |
| 1(1504) ~ 2101810(1504) | D725 | Ar29 |
| 1(1505) ~ 2101810(1505) | D725 | Ar30 |
| 1(1506) ~ 2101810(1506) | D725 | Ar31 |
| 1(1507) ~ 2101810(1507) | D725 | Ar32 |
| 1(1508) ~ 2101810(1508) | D725 | Ar33 |
| 1(1509) ~ 2101810(1509) | D725 | Ar34 |
| 1(1510) ~ 2101810(1510) | D725 | Ar35 |
| 1(1511) ~ 2101810(1511) | D725 | Ar36 |
| 1(1512) ~ 2101810(1512) | D725 | Ar37 |
| 1(1513) ~ 2101810(1513) | D725 | Ar38 |
| 1(1514) ~ 2101810(1514) | D725 | Ar39 |
| 1(1515) ~ 2101810(1515) | D725 | Ar40 |
| 1(1516) ~ 2101810(1516) | D725 | Ar41 |
| 1(1517) ~ 2101810(1517) | D725 | Ar42 |
| 1(1518) ~ 2101810(1518) | D725 | Ar43 |

(continued)

| No. | $Ar^1$ | $Ar^2$ |
|---|---|---|
| 1(1519) ~ 2101810(1519) | D725 | Ar44 |
| 1(1520) ~ 2101810(1520) | D725 | Ar45 |
| 1(1521) ~ 2101810(1521) | D725 | Ar46 |
| 1(1522) ~ 2101810(1522) | D725 | Ar47 |
| 1(1523) ~ 2101810(1523) | D725 | Ar48 |
| 1(1524) ~ 2101810(1524) | D725 | Ar49 |
| 1(1525) ~ 2101810(1525) | D725 | Ar50 |
| 1(1526) ~ 2101810(1526) | D725 | Ar51 |
| 1(1527) ~ 2101810(1527) | D725 | Ar52 |
| 1(1528) ~ 2101810(1528) | D725 | Ar53 |
| 1(1529) ~ 2101810(1529) | D725 | Ar54 |
| 1(1530) ~ 2101810(1530) | D725 | Ar55 |
| 1(1531) ~ 2101810(1531) | D725 | Ar56 |
| 1(1532) ~ 2101810(1532) | D725 | Ar57 |
| 1(1533) ~ 2101810(1533) | D725 | Ar58 |
| 1(1534) ~ 2101810(1534) | D725 | Ar59 |
| 1(1535) ~ 2101810(1535) | D735 | Ar1 |
| 1(1536) ~ 2101810(1536) | D735 | Ar2 |
| 1(1537) ~ 2101810(1537) | D735 | Ar3 |
| 1(1538) ~ 2101810(1538) | D735 | Ar4 |
| 1(1539) ~ 2101810(1539) | D735 | Ar5 |
| 1(1540) ~ 2101810(1540) | D735 | Ar6 |
| 1(1541) ~ 2101810(1541) | D735 | Ar7 |
| 1(1542) ~ 2101810(1542) | D735 | Ar8 |
| 1(1543) ~ 2101810(1543) | D735 | Ar9 |
| 1(1548) ~ 2101810(1544) | D735 | Ar10 |
| 1(1545) ~ 2101810(1545) | D735 | Ar11 |
| 1(1546) ~ 2101810(1546) | D735 | Ar12 |
| 1(1547) ~ 2101810(1547) | D735 | Ar13 |
| 1(1548) ~ 2101810(1548) | D735 | Ar14 |
| 1(1549) ~ 2101810(1549) | D735 | Ar15 |
| 1(1550) ~ 2101810(1550) | D735 | Ar16 |
| 1(1551) ~ 2101810(1551) | D735 | Ar17 |
| 1(1552) ~ 2101810(1552) | D735 | Ar18 |
| 1(1553) ~ 2101810(1553) | D735 | Ar19 |
| 1(1554) ~ 2101810(1554) | D735 | Ar20 |
| 1(1555) ~ 2101810(1555) | D735 | Ar21 |
| 1(1556) ~ 2101810(1556) | D735 | Ar22 |
| 1(1557) ~ 2101810(1557) | D735 | Ar23 |

(continued)

| No. | $Ar^1$ | $Ar^2$ |
|---|---|---|
| 1(1558) ~ 2101810(1558) | D735 | Ar24 |
| 1(1559) ~ 2101810(1559) | D735 | Ar25 |
| 1(1560) ~ 2101810(1560) | D735 | Ar26 |
| 1(1561) ~ 2101810(1561) | D735 | Ar27 |
| 1(1562) ~ 2101810(1562) | D735 | Ar28 |
| 1(1563) ~ 2101810(1563) | D735 | Ar29 |
| 1(1564) ~ 2101810(1564) | D735 | Ar30 |
| 1(1565) ~ 2101810(1565) | D735 | Ar31 |
| 1(1566) ~ 2101810(1566) | D735 | Ar32 |
| 1(1567) ~ 2101810(1567) | D735 | Ar33 |
| 1(1568) ~ 2101810(1568) | D735 | Ar34 |
| 1(1569) ~ 2101810(1569) | D735 | Ar35 |
| 1(1570) ~ 2101810(1570) | D735 | Ar36 |
| 1(1571) ~ 2101810(1571) | D735 | Ar37 |
| 1(1572) ~ 2101810(1572) | D735 | Ar38 |
| 1(1573) ~ 2101810(1573) | D735 | Ar39 |
| 1(1574) ~ 2101810(1574) | D735 | Ar40 |
| 1(1575) ~ 2101810(1575) | D735 | Ar41 |
| 1(1576) ~ 2101810(1576) | D735 | Ar42 |
| 1(1577) ~ 2101810(1577) | D735 | Ar43 |
| 1(1578) ~ 2101810(1578) | D735 | Ar44 |
| 1(1579) ~ 2101810(1579) | D735 | Ar45 |
| 1(1580) ~ 2101810(1580) | D735 | Ar46 |
| 1(1581) ~ 2101810(1581) | D735 | Ar47 |
| 1(1582) ~ 2101810(1582) | D735 | Ar48 |
| 1(1583) ~ 2101810(1583) | D735 | Ar49 |
| 1(1584) ~ 2101810(1584) | D735 | Ar50 |
| 1(1585) ~ 2101810(1585) | D735 | Ar51 |
| 1(1586) ~ 2101810(1586) | D735 | Ar52 |
| 1(1587) ~ 2101810(1587) | D735 | Ar53 |
| 1(1588) ~ 2101810(1588) | D735 | Ar54 |
| 1(1589) ~ 2101810(1589) | D735 | Ar55 |
| 1(1590) ~ 2101810(1590) | D735 | Ar56 |
| 1(1591) ~ 2101810(1591) | D735 | Ar57 |
| 1(1592) ~ 2101810(1592) | D735 | Ar58 |
| 1(1593) ~ 2101810(1593) | D735 | Ar59 |
| 1(1594) ~ 2101810(1594) | D843 | Ar1 |
| 1(1595) ~ 2101810(1595) | D843 | Ar2 |
| 1(1596) ~ 2101810(1596) | D843 | Ar3 |

(continued)

| No. | Ar1 | Ar2 |
|---|---|---|
| 1(1597) ~ 2101810(1597) | D843 | Ar4 |
| 1(1598) ~ 2101810(1598) | D843 | Ar5 |
| 1(1599) ~ 2101810(1599) | D843 | Ar6 |
| 1(1600) ~ 2101810(1600) | D843 | Ar7 |
| 1(1601) ~ 2101810(1601) | D843 | Ar8 |
| 1(1602) ~ 2101810(1602) | D843 | Ar9 |
| 1(1603) ~ 2101810(1603) | D843 | Ar10 |
| 1(1604) ~ 2101810(1604) | D843 | Ar11 |
| 1(1605) ~ 2101810(1605) | D843 | Ar12 |
| 1(1606) ~ 2101810(1606) | D843 | Ar13 |
| 1(1607) ~ 2101810(1607) | D843 | Ar14 |
| 1(1608) ~ 2101810(1608) | D843 | Ar15 |
| 1(1609) ~ 2101810(1609) | D843 | Ar16 |
| 1(1610) ~ 2101810(1610) | D843 | Ar17 |
| 1(1611) ~ 2101810(1611) | D843 | Ar18 |
| 1(1612) ~ 2101810(1612) | D843 | Ar19 |
| 1(1613) ~ 2101810(1613) | D843 | Ar20 |
| 1(1614) ~ 2101810(1614) | D843 | Ar21 |
| 1(1615) ~ 2101810(1615) | D843 | Ar22 |
| 1(1616) ~ 2101810(1616) | D843 | Ar23 |
| 1(1617) ~ 2101810(1617) | D843 | Ar24 |
| 1(1618) ~ 2101810(1618) | D843 | Ar25 |
| 1(1619) ~ 2101810(1619) | D843 | Ar26 |
| 1(1620) ~ 2101810(1620) | D843 | Ar27 |
| 1(1621) ~ 2101810(1621) | D843 | Ar28 |
| 1(1622) ~ 2101810(1622) | D843 | Ar29 |
| 1(1623) ~ 2101810(1623) | D843 | Ar30 |
| 1(1624) ~ 2101810(1624) | D843 | Ar31 |
| 1(1625) ~ 2101810(1625) | D843 | Ar32 |
| 1(1626) ~ 2101810(1626) | D843 | Ar33 |
| 1(1627) ~ 2101810(1627) | D843 | Ar34 |
| 1(1628) ~ 2101810(1628) | D843 | Ar35 |
| 1(1629) ~ 2101810(1629) | D843 | Ar36 |
| 1(1630) ~ 2101810(1630) | D843 | Ar37 |
| 1(1631) ~ 2101810(1631) | D843 | Ar38 |
| 1(1632) ~ 2101810(1632) | D843 | Ar39 |
| 1(1633) ~ 2101810(1633) | D843 | Ar40 |
| 1(1634) ~ 2101810(1634) | D843 | Ar41 |
| 1(1635) ~ 2101810(1635) | D843 | Ar42 |

(continued)

| No. | Ar1 | Ar2 |
|---|---|---|
| 1(1636) ~ 2101810(1636) | D843 | Ar43 |
| 1(1637) ~ 2101810(1637) | D843 | Ar44 |
| 1(1638) ~ 2101810(1638) | D843 | Ar45 |
| 1(1639) ~ 2101810(1639) | D843 | Ar46 |
| 1(1640) ~ 2101810(1640) | D843 | Ar47 |
| 1(1641) ~ 2101810(1641) | D843 | Ar48 |
| 1(1642) ~ 2101810(1642) | D843 | Ar49 |
| 1(1643) ~ 2101810(1643) | D843 | Ar50 |
| 1(1644) ~ 2101810(1644) | D843 | Ar51 |
| 1(1645) ~ 2101810(1645) | D843 | Ar52 |
| 1(1646) ~ 2101810(1646) | D843 | Ar53 |
| 1(1647) ~ 2101810(1647) | D843 | Ar54 |
| 1(1648) ~ 2101810(1648) | D843 | Ar55 |
| 1(1649) ~ 2101810(1649) | D843 | Ar56 |
| 1(1650) ~ 2101810(1650) | D843 | Ar57 |
| 1(1651) ~ 2101810(1651) | D843 | Ar58 |
| 1(1652) ~ 2101810(1652) | D843 | Ar59 |
| 1(1653) ~ 2101810(1653) | D717 | D1 |
| 1(1654) ~ 2101810(1654) | D717 | D7 |
| 1(1655) ~ 2101810(1655) | D717 | D8 |
| 1(1656) ~ 2101810(1656) | D717 | D9 |
| 1(1657) ~ 2101810(1657) | D717 | D19 |
| 1(1658) ~ 2101810(1658) | D717 | D37 |
| 1(1659) ~ 2101810(1659) | D717 | D50 |
| 1(1660) ~ 2101810(1660) | D717 | D77 |
| 1(1661) ~ 2101810(1661) | D717 | D281 |
| 1(1662) ~ 2101810(1662) | D717 | D459 |
| 1(1663) ~ 2101810(1663) | D717 | D465 |
| 1(1664) ~ 2101810(1664) | D717 | D466 |
| 1(1665) ~ 2101810(1665) | D717 | D467 |
| 1(1666)~ 2101810(1666) | D717 | D471 |
| 1(1667) ~ 2101810(1667) | D717 | D486 |
| 1(1668) ~ 2101810(1668) | D717 | D520 |
| 1(1669) ~ 2101810(1669) | D717 | D621 |
| 1(1670) ~ 2101810(1676) | D717 | D711 |
| 1(1671) ~ 2101810(1671) | D717 | D712 |
| 1(1672) ~ 2101810(1672) | D717 | D713 |
| 1(1673) ~ 2101810(1673) | D717 | D714 |
| 1(1674) ~ 2101810(1674) | D717 | D715 |

(continued)

| No. | $Ar^1$ | $Ar^2$ |
|---|---|---|
| 1(1675) ~ 2101810(1675) | D717 | D716 |
| 1(1676) ~ 2101810(1676) | D717 | D725 |
| 1(1677) ~ 2101810(1677) | D717 | D735 |
| 1(1678) ~ 2101810(1678) | D717 | D843 |
| 1(1679) ~ 2101810(1679) | D1 | D7 |
| 1(1680) ~ 2101810(1680) | D1 | D8 |
| 1(1681) ~ 2101810(1681) | D1 | D9 |
| 1(1682) ~ 2101810(1682) | D1 | D19 |
| 1(1683) ~ 2101810(1683) | D1 | D37 |
| 1(1684) ~ 2101810(1684) | D1 | D50 |
| 1(1685) ~ 2101810(1685) | D1 | D77 |
| 1(1686) ~ 2101810(1686) | D1 | D281 |
| 1(1687) ~ 2101810(1687) | D1 | D459 |
| 1(1688) ~ 2101810(1688) | D1 | D465 |
| 1(1689) ~ 2101810(1689) | D1 | D466 |
| 1(1690) ~ 2101810(1690) | D1 | D467 |
| 1(1691) ~ 2101810(1691) | D1 | D471 |
| 1(1692) ~ 2101810(1692) | D1 | D486 |
| 1(1693) ~ 2101810(1693) | D1 | D520 |
| 1(1694) ~ 2101810(1694) | D1 | D621 |
| 1(1695) ~ 2101810(1695) | D1 | D711 |
| 1(1696) ~ 2101810(1696) | D1 | D712 |
| 1(1697) ~ 2101810(1697) | D1 | D713 |
| 1(1698) ~ 2101810(1698) | D1 | D714 |
| 1(1699) ~ 2101810(1699) | D1 | D715 |
| 1(1700) ~ 2101810(1700) | D1 | D716 |
| 1(1701) ~ 2101810(1701) | D1 | D725 |
| 1(1702) ~ 2101810(1702) | D1 | D735 |
| 1(1703) ~ 2101810(1703) | D1 | D843 |
| 1(1704) ~ 2101810(1704) | D7 | D8 |
| 1(1705) ~ 2101810(1705) | D7 | D9 |
| 1(1706) ~ 2101810(1706) | D7 | D19 |
| 1(1707) ~ 2101810(1707) | D7 | D37 |
| 1(1708) ~ 2101810(1708) | D7 | D50 |
| 1(1709) ~ 2101810(1709) | D7 | D77 |
| 1(1710) ~ 2101810(1710) | D7 | D281 |
| 1(1711) ~ 2101810(1711) | D7 | D459 |
| 1(1712) ~ 2101810(1712) | D7 | D465 |
| 1(1713) ~ 2101810(1713) | D7 | D466 |

(continued)

| No. | Ar1 | Ar2 |
|---|---|---|
| 1(1714) ~ 2101810(1714) | D7 | D467 |
| 1(1715) ~ 2101810(1715) | D7 | D471 |
| 1(1716) ~ 2101810(1716) | D7 | D486 |
| 1(1717) ~ 2101810(1717) | D7 | D520 |
| 1(1718) ~ 2101810(1718) | D7 | D621 |
| 1(1719) ~ 2101810(1719) | D7 | D711 |
| 1(1720) ~ 2101810(1720) | D7 | D712 |
| 1(1721) ~ 2101810(1721) | D7 | D713 |
| 1(1722) ~ 2101810(1722) | D7 | D714 |
| 1(1723) ~ 2101810(1723) | D7 | D715 |
| 1(1724) ~ 2101810(1724) | D7 | D716 |
| 1(1725) ~ 2101810(1725) | D7 | D725 |
| 1(1726) ~ 2101810(1726) | D7 | D735 |
| 1(1727) ~ 2101810(1727) | D7 | D843 |
| 1(1728) ~ 2101810(1728) | D8 | D9 |
| 1(1729) ~ 2101810(1729) | D8 | D19 |
| 1(1730) ~ 2101810(1730) | D8 | D37 |
| 1(1731) ~ 2101810(1731) | D8 | D50 |
| 1(1732) ~ 2101810(1732) | D8 | D77 |
| 1(1733) ~ 2101810(1733) | D8 | D281 |
| 1(1734) ~ 2101810(1734) | D8 | D459 |
| 1(1735) ~ 2101810(1735) | D8 | D465 |
| 1(1736) ~ 2101810(1736) | D8 | D466 |
| 1(1737) ~ 2101810(1737) | D8 | D467 |
| 1(1738) ~ 2101810(1738) | D8 | D471 |
| 1(1739) ~ 2101810(1739) | D8 | D486 |
| 1(1740) ~ 2101810(1740) | D8 | D520 |
| 1(1741) ~ 2101810(1741) | D8 | D621 |
| 1(1742) ~ 2101810(1742) | D8 | D711 |
| 1(1743) ~ 2101810(1743) | D8 | D712 |
| 1(1744) ~ 2101810(1744) | D8 | D713 |
| 1(1745) ~ 2101810(1745) | D8 | D714 |
| 1(1746) ~ 2101810(1746) | D8 | D715 |
| 1(1747) ~ 2101810(1747) | D8 | D716 |
| 1(1748) ~ 2101810(1748) | D8 | D725 |
| 1(1749) ~ 2101810(1749) | D8 | D735 |
| 1(1750) ~ 2101810(1750) | D8 | D843 |
| 1(1751) ~ 2101810(1751) | D9 | D19 |
| 1(1752) ~ 2101810(1752) | D9 | D37 |

(continued)

| No. | $Ar^1$ | $Ar^2$ |
|---|---|---|
| 1(1753) ~ 2101810(1753) | D9 | D50 |
| 1(1754) ~ 2101810(1754) | D9 | D77 |
| 1(1755) ~ 2101810(1755) | D9 | D281 |
| 1(1756) ~ 2101810(1756) | D9 | D459 |
| 1(1757) ~ 2101810(1757) | D9 | D465 |
| 1(1758) ~ 2101810(1758) | D9 | D466 |
| 1(1759) ~ 2101810(1759) | D9 | D467 |
| 1(1760) ~ 2101810(1760) | D9 | D471 |
| 1(1761) ~ 2101810(1761) | D9 | D486 |
| 1(1762) ~ 2101810(1762) | D9 | D520 |
| 1(1763) ~ 2101810(1763) | D9 | D621 |
| 1(1764) ~ 2101810(1764) | D9 | D711 |
| 1(1765) ~ 2101810(1765) | D9 | D712 |
| 1(1766) ~ 2101810(1766) | D9 | D713 |
| 1(1767) ~ 2101810(1767) | D9 | D714 |
| 1(1768) ~ 2101810(1768) | D9 | D715 |
| 1(1769) ~ 2101810(1769) | D9 | D716 |
| 1(1770) ~ 2101810(1770) | D9 | D725 |
| 1(1771) ~ 2101810(1771) | D9 | D735 |
| 1(1772) ~ 2101810(1772) | D9 | D843 |
| 1(1773) ~ 2101810(1773) | D19 | D37 |
| 1(1774) ~ 2101810(1774) | D19 | D50 |
| 1(1775) ~ 2101810(1775) | D19 | D77 |
| 1(1776) ~ 2101810(1776) | D19 | D281 |
| 1(1777) ~ 2101810(1777) | D19 | D459 |
| 1(1778) ~ 2101810(1778) | D19 | D465 |
| 1(1779) ~ 2101810(1779) | D19 | D466 |
| 1(1780) ~ 2101810(1780) | D19 | D467 |
| 1(1781) ~ 2101810(1781) | D19 | D471 |
| 1(1782) ~ 2101810(1782) | D19 | D486 |
| 1(1783) ~ 2101810(1783) | D19 | D520 |
| 1(1784) ~ 2101810(1784) | D19 | D621 |
| 1(1785) ~ 2101810(1785) | D19 | D711 |
| 1(1786) ~ 2101810(1786) | D19 | D712 |
| 1(1787) ~ 2101810(1787) | D19 | D713 |
| 1(1788) ~ 2101810(1788) | D19 | D714 |
| 1(1789) ~ 2101810(1789) | D19 | D715 |
| 1(1790) ~ 2101810(1790) | D19 | D716 |
| 1(1791) ~ 2101810(1791) | D19 | D725 |

(continued)

| No. | $Ar^1$ | $Ar^2$ |
|---|---|---|
| 1(1792) ~ 2101810(1792) | D19 | D735 |
| 1(1793) ~ 2101810(1793) | D19 | D843 |
| 1(1794) ~ 2101810(1794) | D37 | D50 |
| 1(1795) ~ 2101810(1795) | D37 | D77 |
| 1(1796) ~ 2101810(1796) | D37 | D281 |
| 1(1797) ~ 2101810(1797) | D37 | D459 |
| 1(1798) ~ 2101810(1798) | D37 | D465 |
| 1(1799) ~ 2101810(1799) | D37 | D466 |
| 1(1800) ~ 2101810(1800) | D37 | D467 |
| 1(1801) ~ 2101810(1801) | D37 | D471 |
| 1(1802) ~ 2101810(1802) | D37 | D486 |
| 1(1803) ~ 2101810(1803) | D37 | D520 |
| 1(1804) ~ 2101810(1804) | D37 | D621 |
| 1(1805) ~ 2101810(1805) | D37 | D711 |
| 1(1806) ~ 2101810(1806) | D37 | D712 |
| 1(1807) ~ 2101810(1807) | D37 | D713 |
| 1(1808) ~ 2101810(1808) | D37 | D714 |
| 1(1809) ~ 2101810(1809) | D37 | D715 |
| 1(1810) ~ 2101810(1810) | D37 | D716 |
| 1(1811) ~ 2101810(1811) | D37 | D725 |
| 1(1812) ~ 2101810(1812) | D37 | D735 |
| 1(1813) ~ 2101810(1813) | D37 | D843 |

The compounds specified by the above numbers are all individually disclosed. In addition, among the specific examples of the compounds, in the case where a rotamer is present, a mixture of rotamers and each separated rotamer are also disclosed in the description herein.

In one aspect of the present invention, compounds are selected from Compound Group α consisting of Compounds 1 to 2101810 and Compounds 1(n) to 2101810(n), where n is 1 to 1813. In one aspect of the present invention, compounds are selected from those of Compound Group α satisfying the above [4]. In one aspect of the present invention, compounds are selected from those of Compound Group α satisfying the above [4]. In one aspect of the present invention, compounds are selected from those of Compound Group α satisfying the above [5]. In one aspect of the present invention, compounds are selected from those of Compound Group α satisfying the above [6]. In one aspect of the present invention, compounds are selected from those of Compound Group α satisfying the above [7]. In one aspect of the present invention, compounds are selected from those of Compound Group α satisfying the above [8]. In one aspect of the present invention, compounds are selected from those of Compound Group α satisfying the above [9]. In one aspect of the present invention, compounds are selected from those of Compound Group α satisfying the above [10]. In one aspect of the present invention, compounds are selected from those of Compound Group α satisfying the above [11]. In one aspect of the present invention, compounds are selected from those of Compound Group α satisfying the above [12]. In one aspect of the present invention, compounds are selected from those of Compound Group α satisfying the above [13]. In one aspect of the present invention, compounds are selected from those of Compound Group α satisfying the above [17].

In one aspect of the present invention, compounds are selected from Compound Group β consisting of Compounds 1 to 1015655 and Compounds 1(n) to 1015655(n), where n is 1 to 1813. In one aspect of the present invention, compounds are selected from those of Compound Group β satisfying the above [4]. In one aspect of the present invention, compounds are selected from those of Compound Group β satisfying the above [5]. In one aspect of the present invention, compounds are selected from those of Compound Group β satisfying the above [6]. In one aspect of the present invention, compounds are selected from those of Compound Group β satisfying the above [7]. In one aspect of the present

invention, compounds are selected from those of Compound Group β satisfying the above [8]. In one aspect of the present invention, compounds are selected from those of Compound Group β satisfying the above [9]. In one aspect of the present invention, compounds are selected from those of Compound Group β satisfying the above [10]. In one aspect of the present invention, compounds are selected from those of Compound Group β satisfying the above [11]. In one aspect of the present invention, compounds are selected from those of Compound Group α satisfying the above [12]. In one aspect of the present invention, compounds are selected from those of Compound Group α satisfying the above [13]. In one aspect of the present invention, compounds are selected from those of Compound Group β satisfying the above [17].

In one aspect of the present invention, compounds are selected from Compound Group γ consisting of Compounds 1015656 to 2031310 and Compounds 1015656(n) to 2031310(n), where n is 1 to 1813. In one aspect of the present invention, compounds are selected from those of Compound Group γ satisfying the above [4]. In one aspect of the present invention, compounds are selected from those of Compound Group γ satisfying the above [5]. In one aspect of the present invention, compounds are selected from those of Compound Group γ satisfying the above [6]. In one aspect of the present invention, compounds are selected from those of Compound Group γ satisfying the above [7]. In one aspect of the present invention, compounds are selected from those of Compound Group γ satisfying the above [8]. In one aspect of the present invention, compounds are selected from those of Compound Group γ satisfying the above [10]. In one aspect of the present invention, compounds are selected from those of Compound Group γ satisfying the above [11]. In one aspect of the present invention, compounds are selected from those of Compound Group α satisfying the above [12]. In one aspect of the present invention, compounds are selected from those of Compound Group α satisfying the above [13]. In one aspect of the present invention, compounds are selected from those of Compound Group γ satisfying the above [17]. In one aspect of the present invention, compounds are selected from Compound Group δ consisting of Compounds 2031311 to 2101810 and Compounds 2031311(n) to 2101810(n), where n is 1 to 1813. In one aspect of the present invention, compounds are selected from those of Compound Group δ satisfying the above [5]. In one aspect of the present invention, compounds are selected from those of Compound Group δ satisfying the above [9]. In one aspect of the present invention, compounds are selected from those of Compound Group δ satisfying the above [13]. In one aspect of the present invention, compounds are selected from those of Compound Group δ satisfying the above [17].

In one aspect of the present invention, the compound represented by the general formula (1) is selected from the following group of compounds.

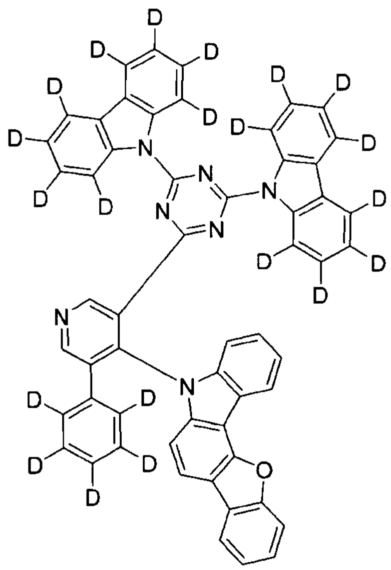

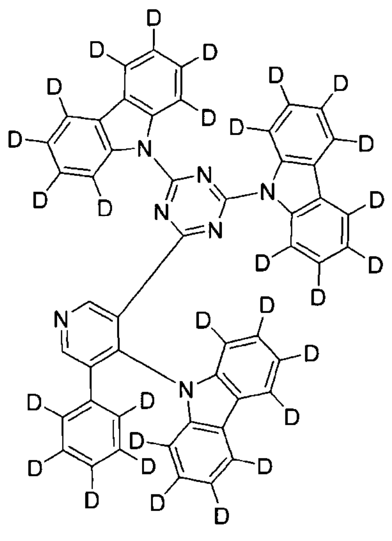

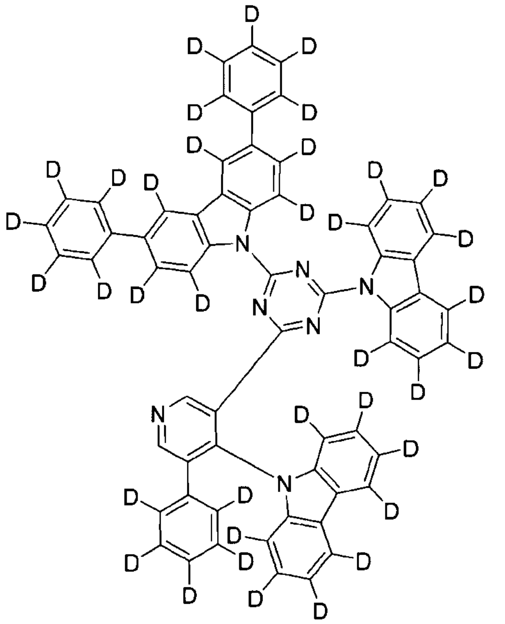

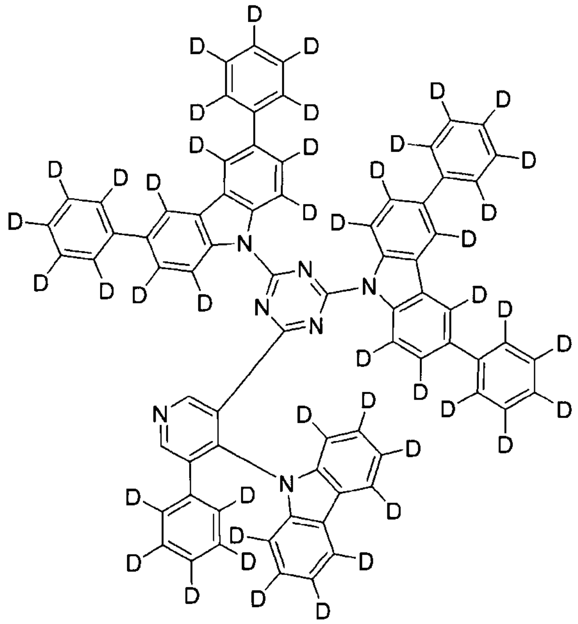

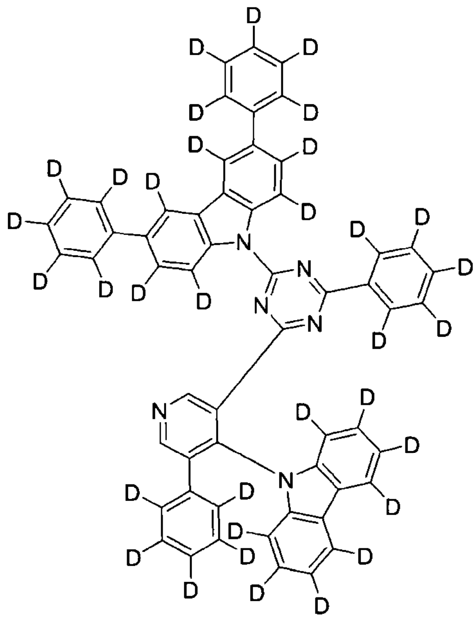

In one aspect of the present invention, the compound represented by the general formula (1) is selected from the following group of compounds.

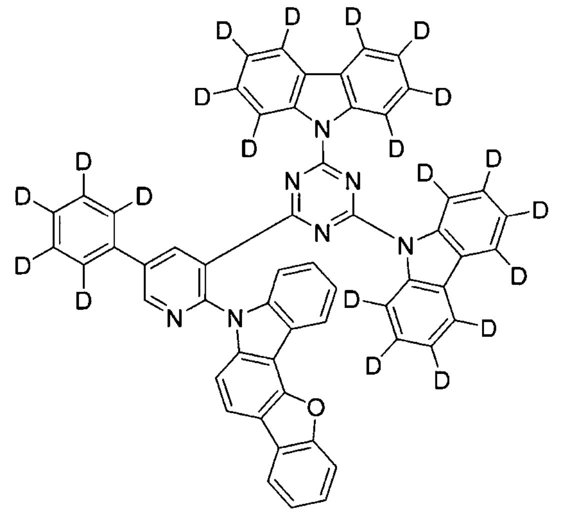

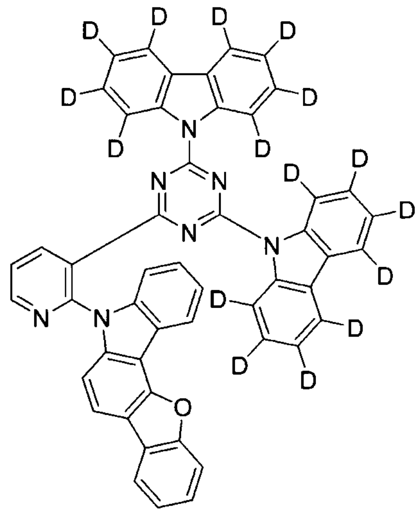

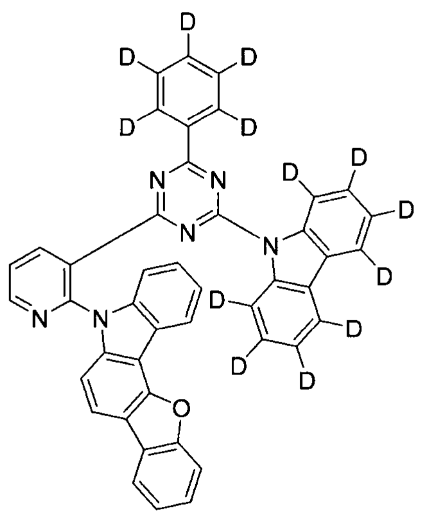

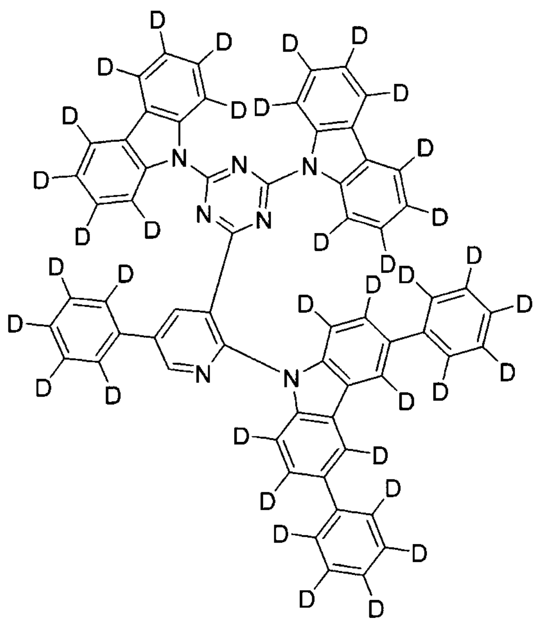

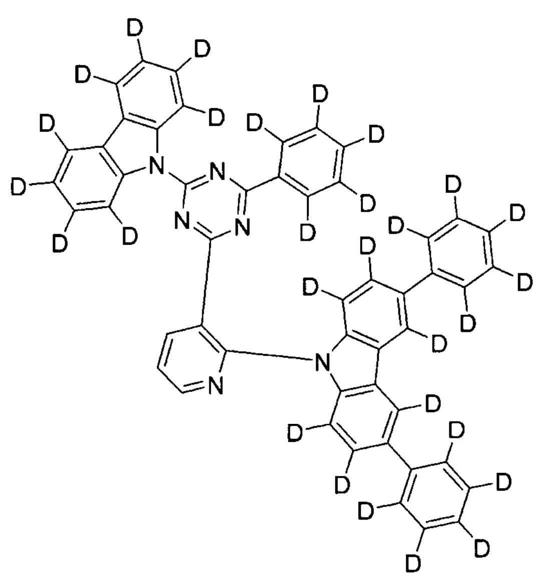

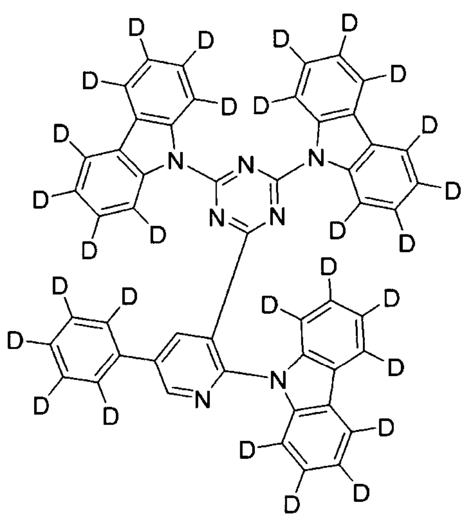

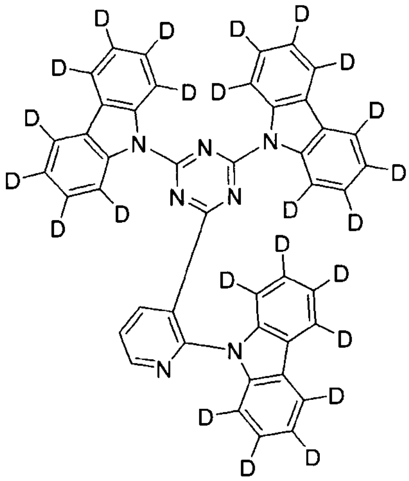

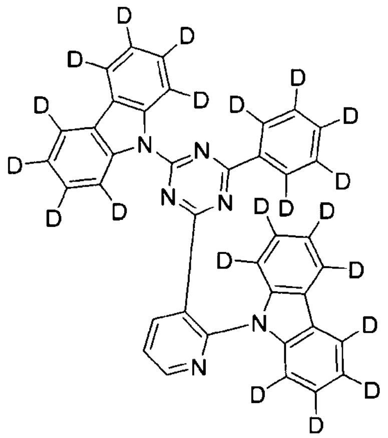

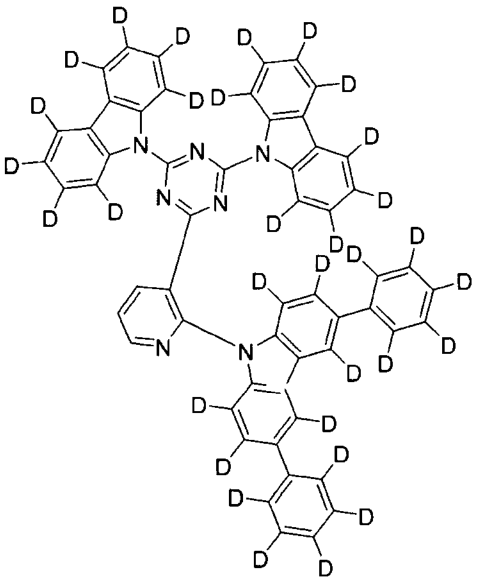

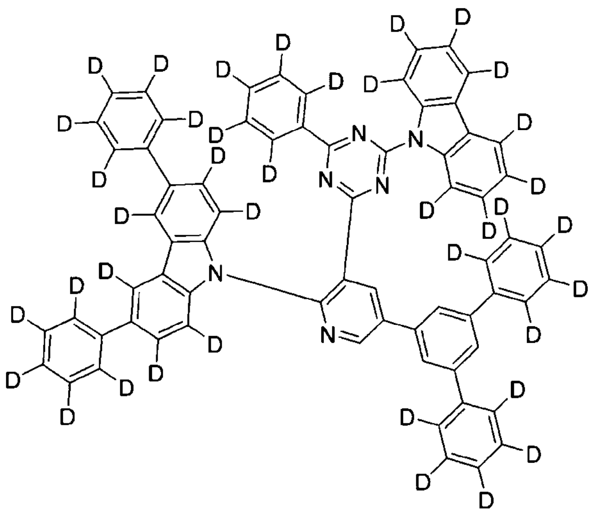

In one preferred aspect of the present invention, the compound represented by the general formula (1) is selected from the following group of compounds.

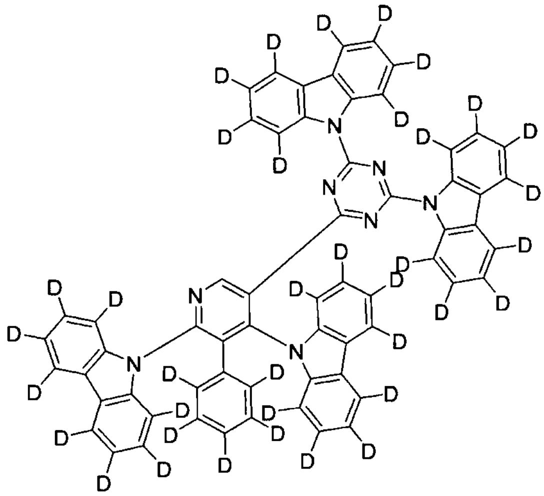

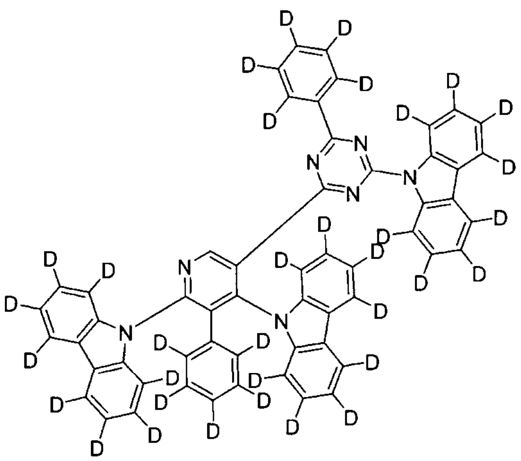

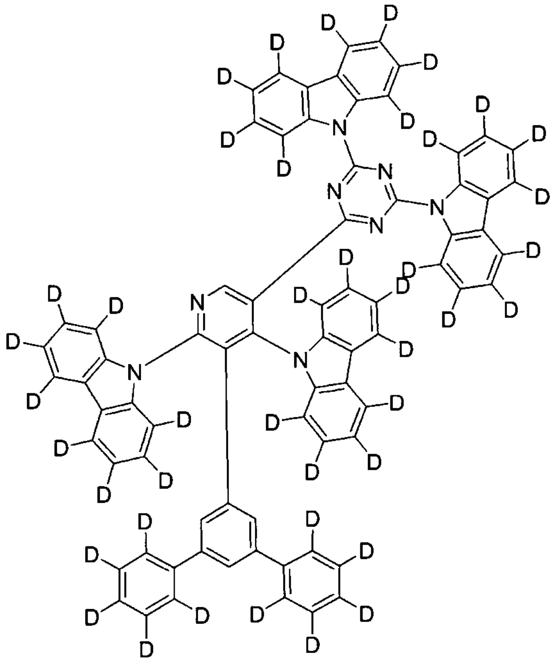

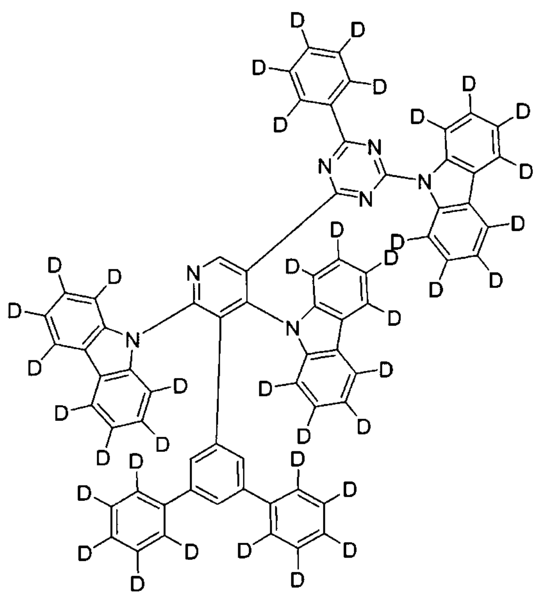

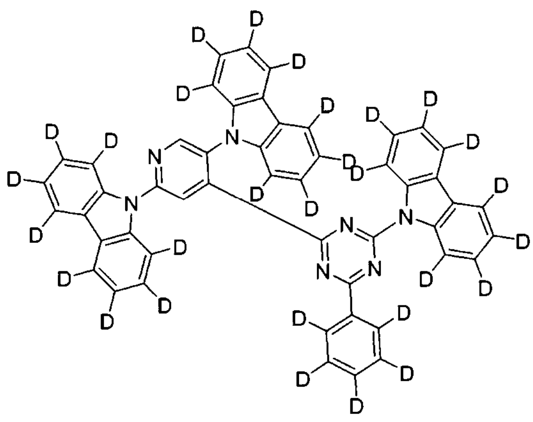

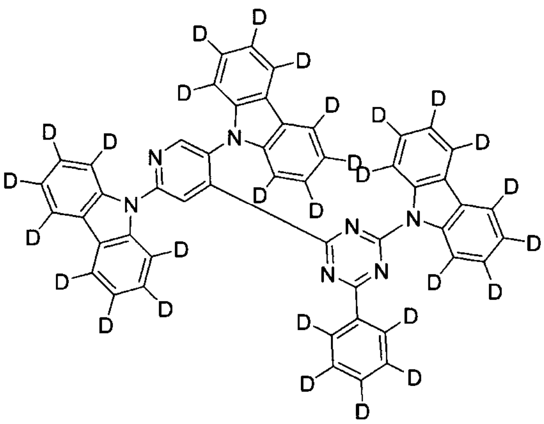

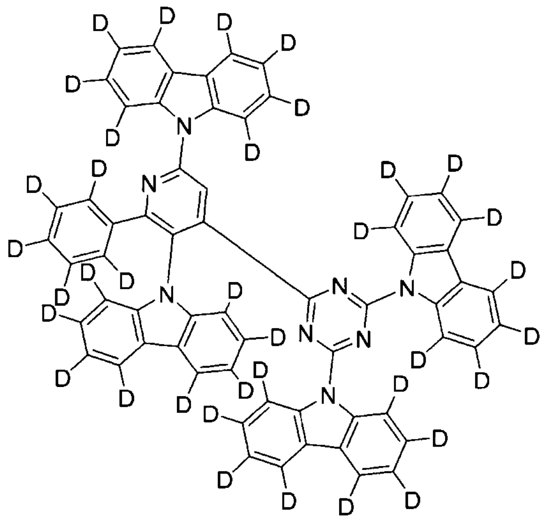

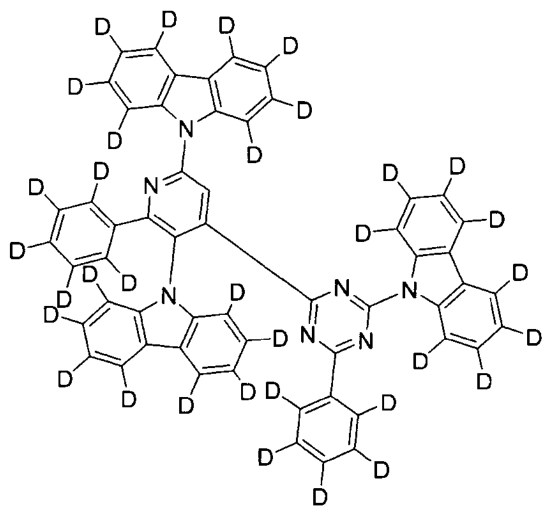

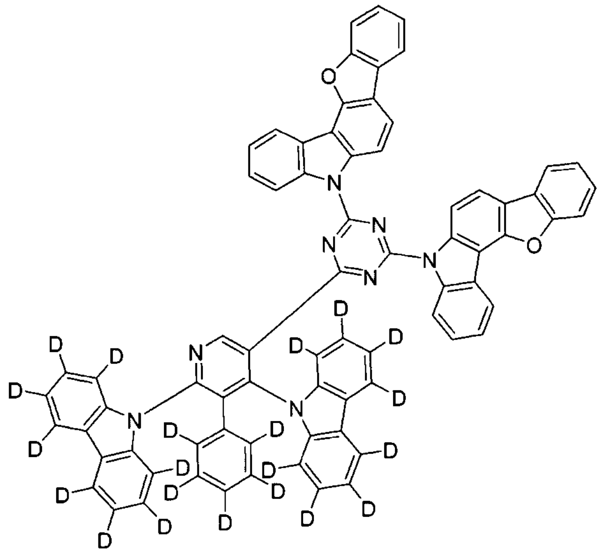

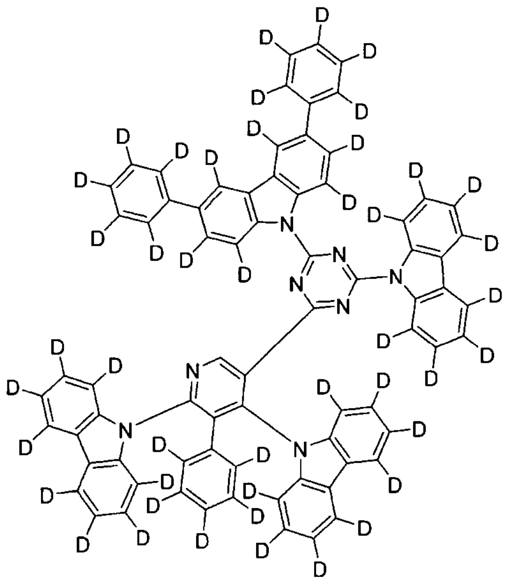

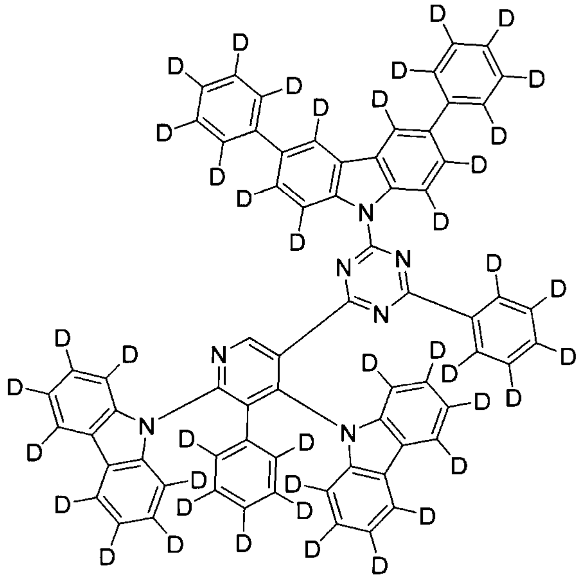

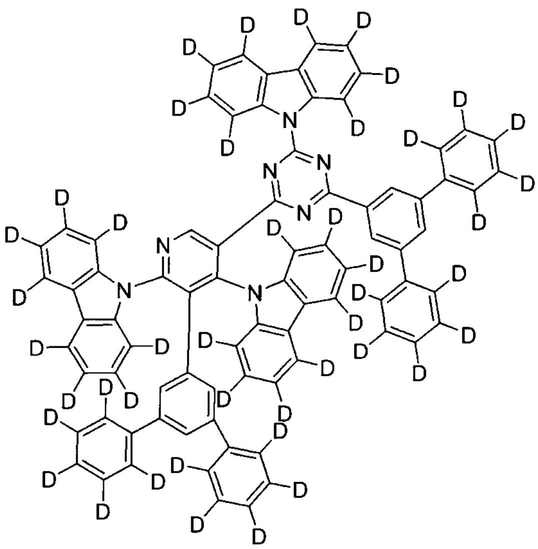

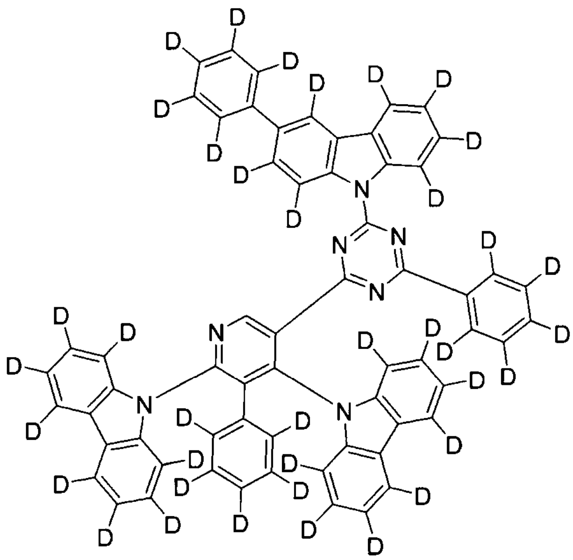

In one aspect of the present invention, the compound represented by the general formula (1) is selected from the following group of compounds.

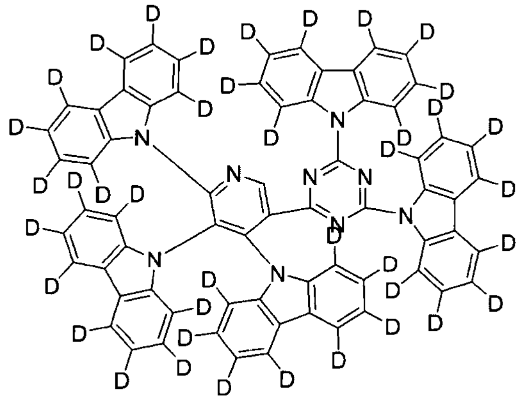

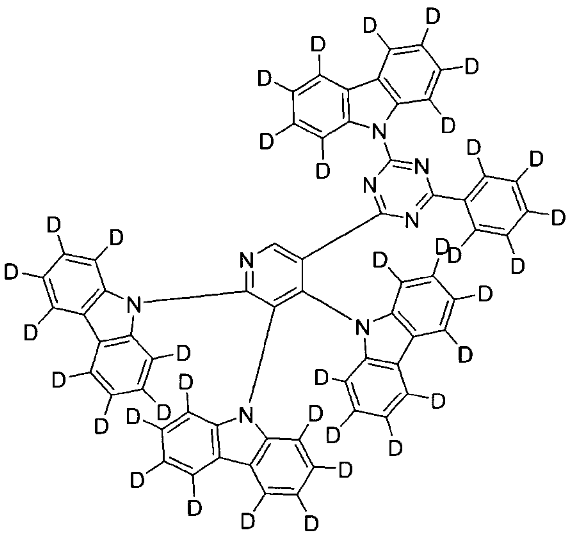

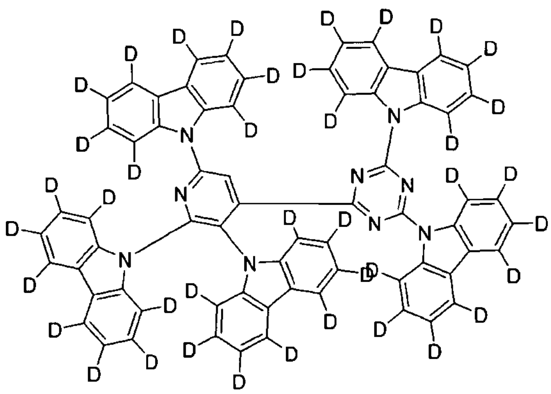

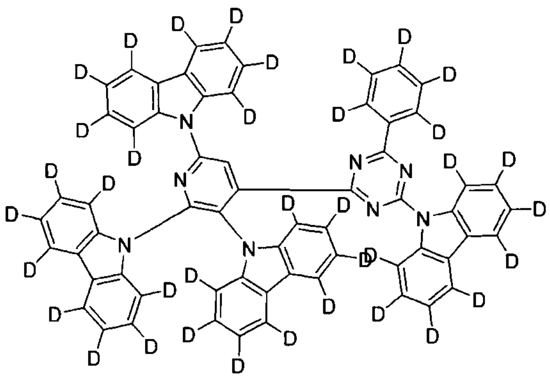

A molecular weight of the compound represented by the general formula (1) is preferably 1500 or less, more preferably 1200 or less, still more preferably 1000 or less, and even more preferably 900 or less, for example, in the case where an organic layer containing the compound represented by the general formula (1) is intended to be film-formed and used by a vapor deposition method. A lower limit of the molecular weight is the molecular weight of the minimum compound represented by the general formula (1).

The compound represented by the general formula (1) can be formed into a film by a coating method regardless of the molecular weight. When the coating method is used, even a compound having a relatively large molecular weight can be formed into a film. The compound represented by the general formula (1) has an advantage of being easily dissolved in an organic solvent. For this reason, the compound represented by the general formula (1) is easily applicable to a coating method and is easily purified to increase its purity.

It is also conceivable to use a compound containing a plurality of structures represented by the general formula (1) in a molecule as a light emitting material by applying the present invention.

For example, it is conceivable that a polymer obtained by allowing a polymerizable group to be present in the structure represented by the general formula (1) in advance and polymerizing the polymerizable group is used as the light emitting material. For example, it is conceivable that a polymer having a repeating unit is obtained by preparing a monomer containing a polymerizable functional group at any site of the general formula (1) and polymerizing the monomer alone or copolymerizing the monomer with another monomer, and the polymer is used as the light emitting material. Alternatively, it is also conceivable to obtain a dimer or a trimer by coupling compounds having a structure represented by the general formula (1) to each other and to use the dimer or the trimer as a light emitting material.

Examples of the polymer having a repeating unit containing a structure represented by the general formula (1) include polymers containing a structure represented by any one of the following two general formulae.

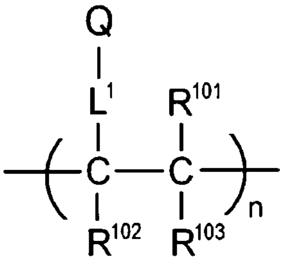

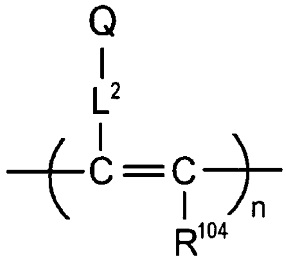

In the above general formulae, Q represents a group containing the structure represented by the general formula (1), and $L^1$ and $L^2$ represent a linking group. The linking group preferably has 0 to 20 carbon atoms, more preferably 1 to 15 carbon atoms, and still more preferably 2 to 10 carbon atoms. The linking group preferably has a structure represented by $-X^{11}-L^{11}-$. Here, $X^{11}$ represents an oxygen atom or a sulfur atom, and is preferably an oxygen atom. $L^{11}$ represents a linking group, and is preferably a substituted or unsubstituted alkylene group or a substituted or unsubstituted arylene group, and more preferably a substituted or unsubstituted alkylene group having 1 to 10 carbon atoms or a substituted or unsubstituted phenylene group.

In the above general formulae, $R^{101}$, $R^{102}$, $R^{103}$ and $R^{104}$ each independently represent a substituent. It is preferably a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom, or a chlorine atom, and still more preferably an unsubstituted alkyl group having 1 to 3 carbon atoms or an unsubstituted alkoxy group having 1 to 3 carbon atoms.

The linking group represented by $L^1$ and $L^2$ can bond to any site of the general formula (1) constituting Q. Two or more linking groups can be linked to one Q to form a cross-linked structure or a network structure.

Specific structural examples of the repeating unit include structures represented by the following formulae.

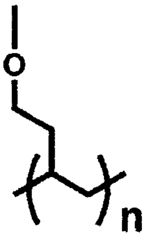

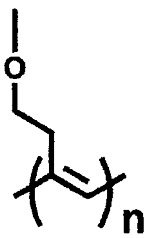

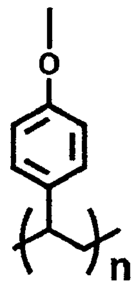

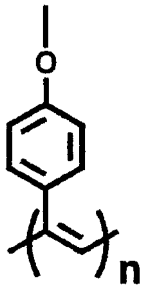

The polymer having a repeating unit including these formulae can be synthesized by introducing a hydroxy group into any site of the general formula (1), reacting the following compound using the hydroxy group as a linker to introduce a polymerizable group, and polymerizing the polymerizable group.

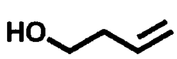

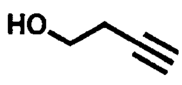

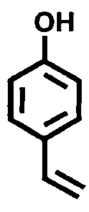

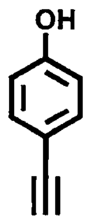

The polymer having a structure represented by the general formula (1) in the molecule can be a polymer having only a repeating unit that has the structure represented by the general formula (1), or can be a polymer containing a repeating unit that has any other structure. The repeating unit having the structure represented by the general formula (1) to be contained in the polymer can be a single kind or two or more kinds. The repeating unit not having the structure represented by the general formula (1) includes those derived from monomers used in general copolymerization. For example, the repeating unit includes repeating units derived from monomers having an ethylenically unsaturated bond, such as ethylene or styrene.

In some embodiments, the compound represented by the general formula (1) is a light emitting material.

In some embodiments, the compound represented by the general formula (1) is a compound capable of emitting

delayed fluorescence.

In some embodiments of the present disclosure, the compound represented by the general formula (1) is, when excited thermally or by an electronic means, able to emit light in a UV region, emit light of blue, green, yellow, orange, or red in a visible spectral region (e.g., about 420 nm to about 500 nm, about 500 nm to about 600 nm, or about 600 nm to about 700 nm) or emit light in a near IR region.

In some embodiments of the present disclosure, the compound represented by the general formula (1) is, when excited thermally or by an electronic means, able to emit light of red or orange in a visible spectral region (e.g., about 620 nm to about 780 nm, about 650 nm).

In some embodiments of the present disclosure, the compound represented by the general formula (1) is, when excited thermally or by an electronic means, able to emit light of orange or yellow in a visible spectral region (e.g., about 570 nm to about 620 nm, about 590 nm, about 570 nm).

In some embodiments of the present disclosure, the compound represented by the general formula (1) is, when excited thermally or by an electronic means, able to emit light of green in a visible spectral region (e.g., about 490 nm to about 575 nm, about 510 nm).

In some embodiments of the present disclosure, the compound represented by the general formula (1) is, when excited thermally or by an electronic means, able to emit light of blue in a visible spectral region (e.g., about 400 nm to about 490 nm, about 475 nm).

In some embodiments of the present disclosure, the compound represented by the general formula (1) is, when excited thermally or by an electronic means, able to emit light in a UV spectral region (e.g., about 280 to 400 nm).

In some embodiments of the present disclosure, the compound represented by the general formula (1) is, when excited thermally or by an electronic means, able to emit light in an IR spectral region (e.g., about 780 nm to 2 $\mu$m).

In some embodiments of the present disclosure, an organic semiconductor device using the compound represented by the general formula (1) can be produced. The organic semiconductor device referred to herein can be an organic optical device in which light is interposed or an organic device in which light is not interposed. The organic optical device can be an organic light emitting device in which the device emits light, an organic light receiving device in which the device receives light, or a device in which energy transfer by light occurs in the device. In some embodiments of the present disclosure, an organic optical device such as an organic electroluminescent device or a solid-state imaging device (for example, a CMOS image sensor) can be produced by using the compound represented by the general formula (1). In some embodiments of the present disclosure, a complementary metal-oxide film semiconductor (CMOS) or the like using the compound represented by the general formula (1) can be produced.

Electronic characteristics of small-molecule chemical substance libraries can be calculated by known *ab initio* quantum chemistry calculation. For example, according to time-dependent density functional theory calculation using 6-31G* as a basis, and a functional group known as Becke's three parameters, Lee-Yang-Parr hybrid functionals, the Hartree-Fock equation (TD-DFT/B3LYP/6-31G*) is analyzed and molecular fractions (parts) having HOMO not lower than a specific threshold value and LUMO not higher than a specific threshold value can be screened.

With that, for example, in the presence of a HOMO energy (for example, ionization potential) of -6.5 eV or more, a donor part ("D") can be selected. Also, for example, in the presence of a LUMO energy (for example, electron affinity) of -0.5 eV or less, an acceptor part ("A") can be selected. A bridge part ("B") is a strong conjugated system, for example, capable of strictly limiting the acceptor part and the donor part in a specific three-dimensional configuration, and therefore prevents the donor part and the acceptor part from overlapping in the $\pi$-conjugated system.

In some embodiments, a compound library is screened using one or more of the following characteristics.

1. Light emission around a specific wavelength
2. A triplet state over a calculated specific energy level
3. $\Delta E_{ST}$ value lower than a specific value
4. Quantum yield more than a specific value
5. HOMO level
6. LUMO level

In some embodiments, the difference ($\Delta E_{ST}$) between the lowest singlet excited state and the lowest triplet excited state at 77 K is less than about 0.5 eV, less than about 0.4 eV, less than about 0.3 eV, less than about 0.2 eV, or less than about 0.1 eV. In some embodiments, $\Delta E_{ST}$ value is less than about 0.09 eV, less than about 0.08 eV, less than about 0.07 eV, less than about 0.06 eV, less than about 0.05 eV, less than about 0.04 eV, less than about 0.03 eV, less than about 0.02 eV, or less than about 0.01 eV.

In some embodiments, the compound represented by the general formula (1) shows a quantum yield of more than 25%, for example, about 30%, about 35%, about 40%, about 45%, about 50%, about 55%, about 60%, about 65%, about 70%, about 75%, about 80%, about 85%, about 90%, about 95% or more.

[Method for Synthesizing Compound Represented by General Formula (1)]

The compound represented by the general formula (1) includes a novel compound.

The compound represented by the general formula (1) can be synthesized by combining known reactions. For example, among the compounds represented by the general formula (1), a compound having a structure where a substituted or unsubstituted carbazolyl group, a substituted or unsubstituted aryl group (for example, a phenyl group), and a triazyl group substituted with two substituted or unsubstituted carbazolyl groups are bonded to a pyridine ring can be synthesized by introducing a triazyl group having two substituted or unsubstituted carbazolyl groups into a pyridine derivative having a substituted or unsubstituted aryl group and a halogen atom, and then reacting the resulting compound with a substituted or unsubstituted carbazole. For details of the reaction conditions, Synthesis Examples described later can be referred to.

[Structure Using Compound Represented by General Formula (1)]

In some embodiments, the compound represented by the general formula (1) is used along with one or more materials (e.g., small molecules, polymers, metals, metal complexes), by combining them, or by dispersing the compound, or by covalent-bonding with the compound, or by coating with the compound, or by carrying the compound, or by associating with the compound, and solid films or layers are formed. For example, by combining the compound represented by the general formula (1) with an electroactive material, a film can be formed. In some cases, the compound represented by the general formula (1) can be combined with a hole transporting polymer. In some cases, the compound represented by the general formula (1) can be combined with an electron transporting polymer. In some cases, the compound represented by the general formula (1) can be combined with a hole transporting polymer and an electron transporting polymer. In some cases, the compound represented by the general formula (1) can be combined with a copolymer having both a hole transporting moiety and an electron transporting moiety. In the embodiments mentioned above, the electrons and/or the holes formed in a solid film or layer can be interacted with the compound represented by the general formula (1).

[Film Formation]

In some embodiments, a film containing the compound represented by the general formula (1) can be formed in a wet process. In a wet process, a solution prepared by dissolving a composition containing the compound of the present invention is applied onto a surface, and then the solvent is removed to form a film. The wet process includes a spin coating method, a slit coating method, an inkjet method (a spraying method), a gravure printing method, an offset printing method and flexographic printing method, which, however are not limitative. In the wet process, an appropriate organic solvent capable of dissolving a composition containing the compound of the present invention is selected and used. In some embodiments, a substituent (e.g., an alkyl group) capable of increasing the solubility in an organic solvent can be introduced into the compound contained in the composition.

In some embodiments, a film containing the compound of the present invention can be formed in a dry process. In some embodiments, a vacuum deposition method is employable as a dry process, which, however, is not limitative. In the case where a vacuum deposition method is employed, compounds to constitute a film can be co-deposited from individual vapor deposition sources, or can be co-deposited from a single vapor deposition source formed by mixing the compounds. In the case where a single vapor deposition source is used, a mixed powder prepared by mixing compound powders can be used, or a compression molded body prepared by compression-molding the mixed powder can be used, or a mixture prepared by heating and melting the compounds and cooling the resulting melt can be used. In some embodiments, by co-deposition under the condition where the vapor deposition rate (weight reduction rate) of the plurality of compounds contained in a single vapor deposition source is the same or is nearly the same, a film having a compositional ratio corresponding to the compositional ratio of the plurality of compounds contained in the vapor deposition source can be formed. When the plurality of compounds are mixed in the same compositional ratio as the compositional ratio of the film to be formed to prepare a vapor deposition source, a film having a desired compositional ratio can be formed in a simplified manner. In some embodiments, the temperature at which the compounds to be co-deposited have the same weight reduction ratio is specifically defined, and the temperature can be employed as the temperature of co-deposition.

[Use Examples of Compound Represented by General Formula (1)]

The compound represented by the general formula (1) is useful as a material for an organic light emitting device. In particular, the compound is preferably used for an organic light emitting diode or the like.

Organic light emitting Diode:

One embodiment of the present invention relates to use of the compound represented by the general formula (1) of the present invention as a light emitting material for organic light emitting devices. In some embodiments, the compound represented by the general formula (1) of the present invention can be effectively used as a light emitting material in a light emitting layer in an organic light emitting device. In some embodiments, the compound represented by the general formula (1) includes a delayed fluorescent substance that emits delayed fluorescence. In some embodiments, the present invention provides a delayed fluorescent substance having a structure represented by the general formula (1). In some embodiments, the present invention relates to use of the compound represented by the general formula (1) as a delayed fluorescent substance. In some embodiments, the compound represented by the general formula (1) of the present invention can be used as a host material, and can be used along with one or more light emitting materials, and the light emitting material can be a fluorescent material, a phosphorescent material or a TADF. In some embodiments, the compound represented by the general formula (1) can be used as a hole transporting material. In some embodiments, the compound represented by the general formula (1) can be used as an electron transporting material. In some embodiments, the present invention relates to a method of generating delayed fluorescence from the compound represented by the general formula (1). In some embodiments, the organic light emitting device containing the compound as a light emitting material emits delayed fluorescence and shows high light emission efficiency.

In some embodiments, the light emitting layer contains the compound represented by the general formula (1), and the compound represented by the general formula (1) is aligned in parallel to a substrate. In some embodiments, the substrate is a film-forming surface. In some embodiment, the alignment of the compound represented by the general formula (1) relative to the film-forming surface can have some influence on the propagation direction of light emitted by the aligned compounds, or can determine the direction. In some embodiments, by aligning the propagation direction of light emitted by the compound represented by the general formula (1), the light extraction efficiency from the light emitting layer can be improved.

One aspect of the present invention relates to an organic light emitting device. In some embodiments, the organic light emitting device includes a light emitting layer. In some embodiments, the light emitting layer contains, as a light emitting material, the compound represented by the general formula (1). In some embodiments, the organic light emitting device is an organic photoluminescent device (organic PL device). In some embodiments, the organic light emitting device is an organic electroluminescent device (organic EL device). In some embodiments, the compound represented by the general formula (1) assists light irradiation from the other light emitting materials contained in the light emitting layer (as a so-called assist dopant). In some embodiments, the compound represented by the general formula (1) contained in the light emitting layer is in a lowest excited singlet energy level, and is contained between the lowest excited single energy level of the host material contained in the light emitting layer and the lowest excited singlet energy level of the other light emitting materials contained in the light emitting layer.

In some embodiments, the organic photoluminescent device includes at least one light emitting layer. In some embodiments, the organic electroluminescent device includes at least an anode, a cathode, and an organic layer between the anode and the cathode. In some embodiments, the organic layer includes at least a light emitting layer. In some embodiments, the organic layer includes only a light emitting layer. In some embodiments, the organic layer includes one or more organic layers in addition to the light emitting layer. Examples of the organic layer include a hole transporting layer, a hole injection layer, an electron barrier layer, a hole barrier layer, an electron injection layer, an electron transporting layer and an exciton barrier layer. In some embodiments, the hole transporting layer can be a hole injection and transporting layer having a hole injection function, and the electron transporting layer can be an electron injection and transporting layer having an electron injection function.

Light Emitting Layer:

In some embodiments, the light emitting layer is a layer where holes and electrons injected from the anode and the cathode, respectively, are recombined to form excitons. In some embodiments, the layer emits light.

In some embodiments, only a light emitting material is used as the light emitting layer. In some embodiments, the light emitting layer contains a light emitting material and a host material. In some embodiments, the light emitting material is one or more compounds represented by the general formula (1). In some embodiments, for improving light emission efficiency of an organic electroluminescent device and an organic photoluminescent device, the singlet exciton and the triplet exciton generated in a light emitting material are confined inside the light emitting material. In some embodiments, a host material is used in the light emitting layer in addition to a light emitting material. In some embodiments, the host material is an organic compound. In some embodiments, the organic compound has an excited singlet energy and an excited triplet energy, and at least one of them is higher than those in the light emitting material of the present invention. In some embodiments, the singlet exciton and the triplet exciton generated in the light emitting material of the present invention are confined in the molecules of the light emitting material of the present invention. In some embodiments, the

singlet and triplet excitons are fully confined for improving light emission efficiency. In some embodiments, although high luminous radiation efficiency is still attained, singlet excitons and triplet excitons are not fully confined, that is, a host material capable of attaining high light emission efficiency can be used in the present invention with no specific limitation. In some embodiments, in the light emitting material in the light emitting layer of the device of the present invention, luminous radiation occurs. In some embodiments, radiated light includes both fluorescence and delayed fluorescence. In some embodiments, radiated light includes radiated light from a host material. In some embodiments, radiated light is composed of radiated light from a host material. In some embodiments, radiated light includes radiated light from the compound represented by the general formula (1) and radiated light from a host material. In some embodiment, a TADF molecule and a host material are used. In some embodiments, TADF is an assist dopant and has a lower excited singlet energy than the host material in the light emitting layer and a higher excited singlet energy than the light emitting material in the light emitting layer.

In the case where the compound represented by the general formula (1) is used as an assist dopant, various compounds can be employed as a light emitting material (preferably a fluorescent material). As such light emitting materials, employable are an anthracene derivative, a tetracene derivative, a naphthacene derivative, a pyrene derivative, a perylene derivative, a chrysene derivative, a rubrene derivative, a coumarin derivative, a pyran derivative, a stilbene derivative, a fluorene derivative, an anthryl derivative, a pyrromethene derivative, a terphenyl derivative, a terphenylene derivative, a fluoranthene derivative, an amine derivative, a quinacridone derivative, an oxadiazole derivative, a malononitrile derivative, a pyran derivative, a carbazole derivative, a julolidine derivative, a thiazole derivative, and a derivative having a metal (Al, Zn). These exemplified skeletons can have a substituent, or may not have a substituent. These exemplified skeletons can be combined.

Light emitting materials that can be used in combination with the assist dopant having a structure represented by the general formula (1) are shown below.

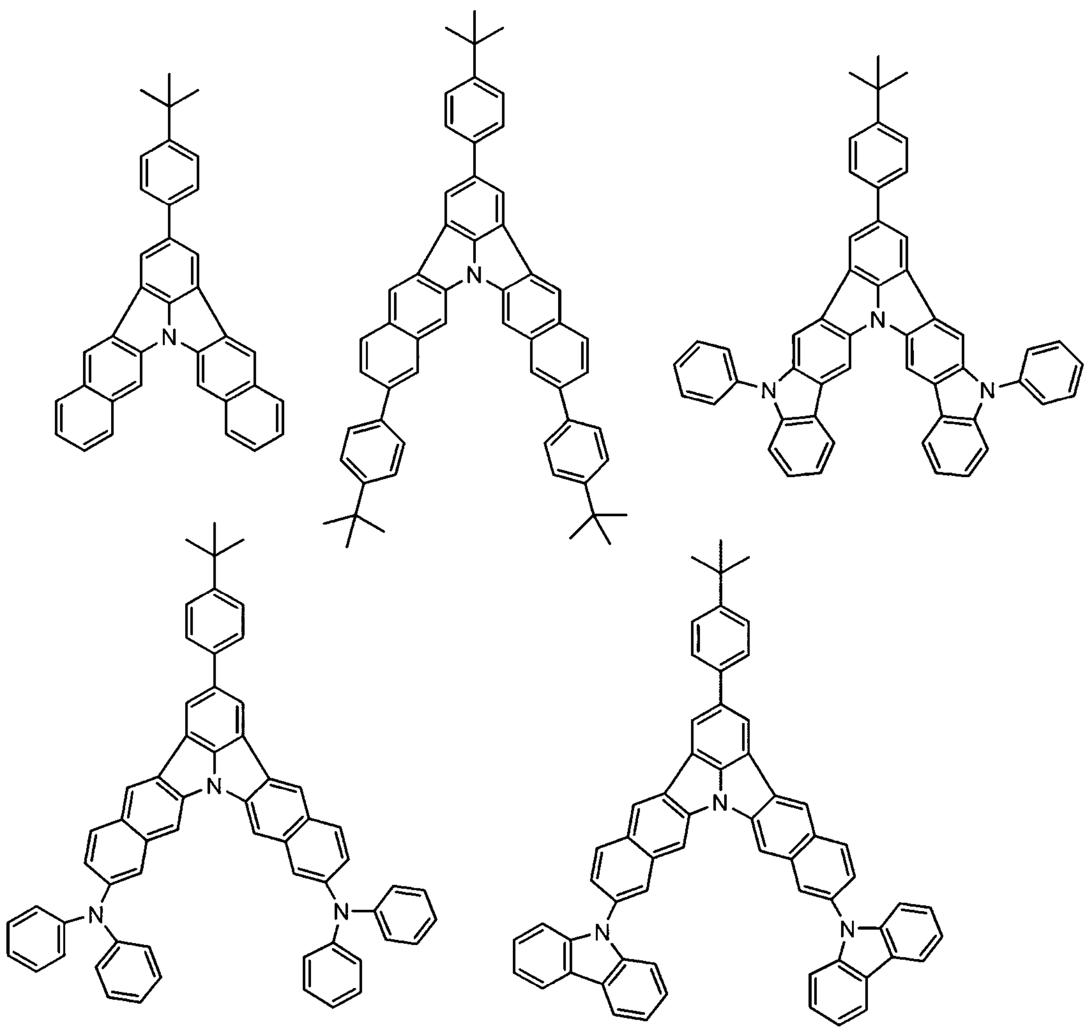

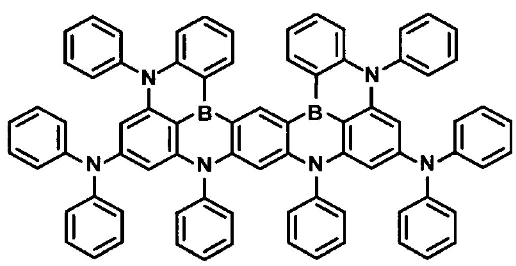

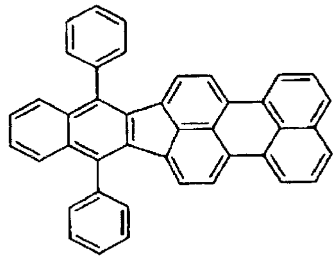

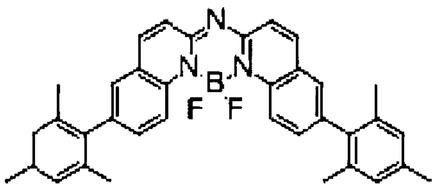

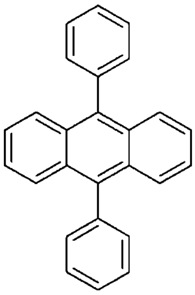

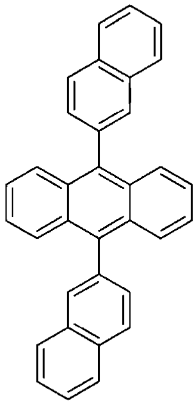

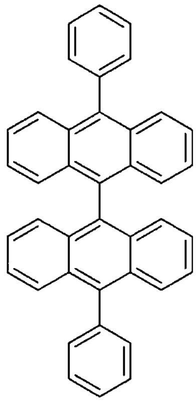

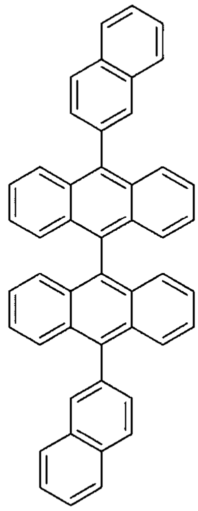

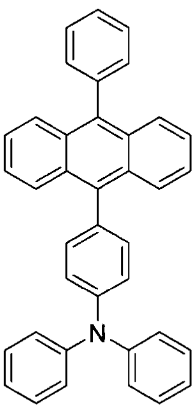

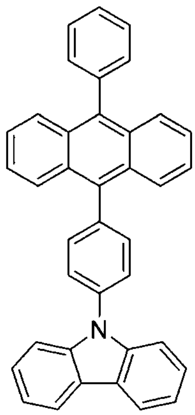

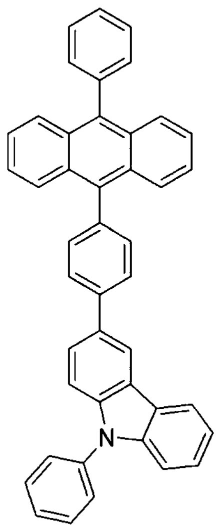

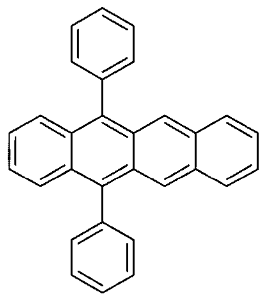

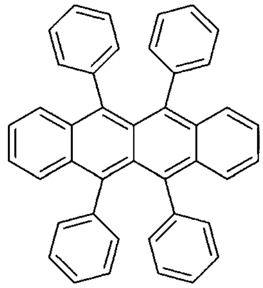
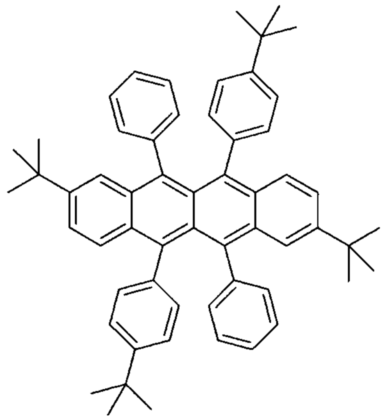
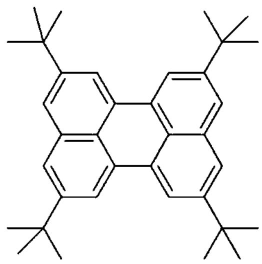
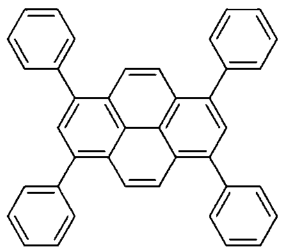
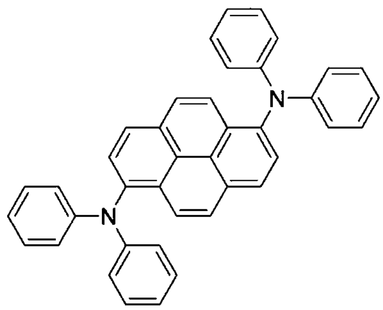
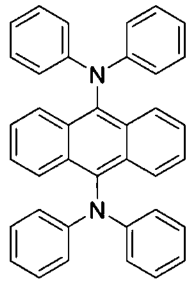
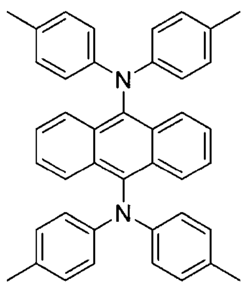
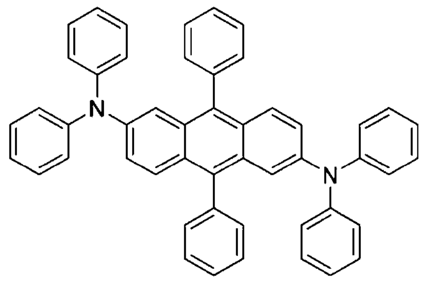
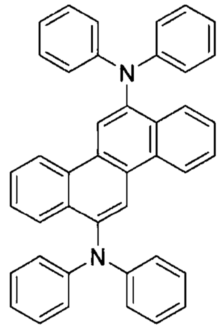
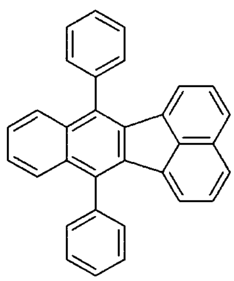
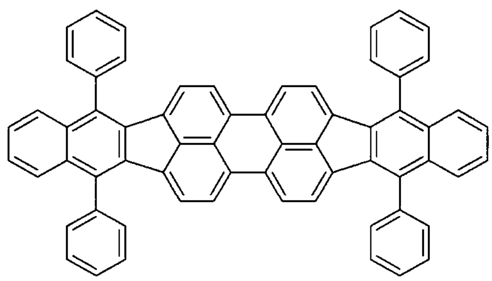
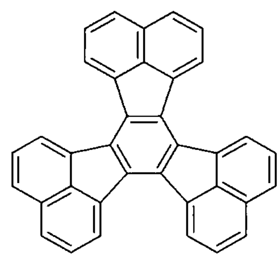
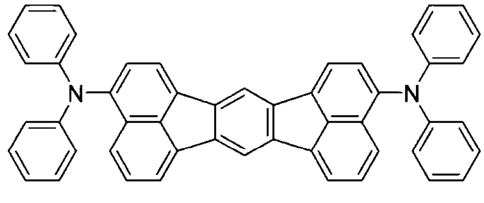
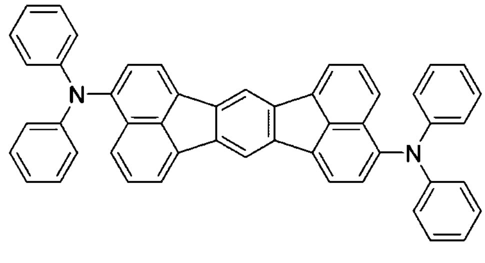

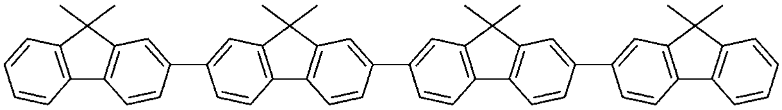

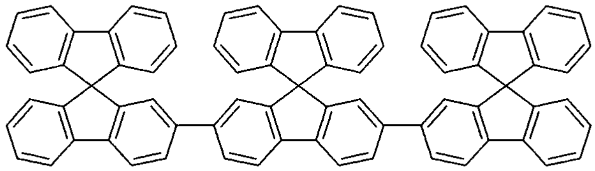

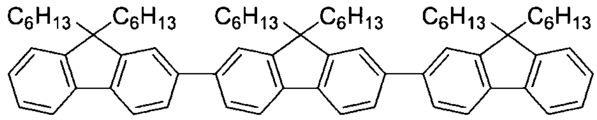

In addition, the compounds described in WO2015/022974, paragraphs 0220 to 0239 are also especially favorably employable as a light emitting material for use along with the assist dopant having a structure represented by the general formula (1).

Compounds represented by the following general formula (2) are further preferred light emitting materials.

General Formula (2)

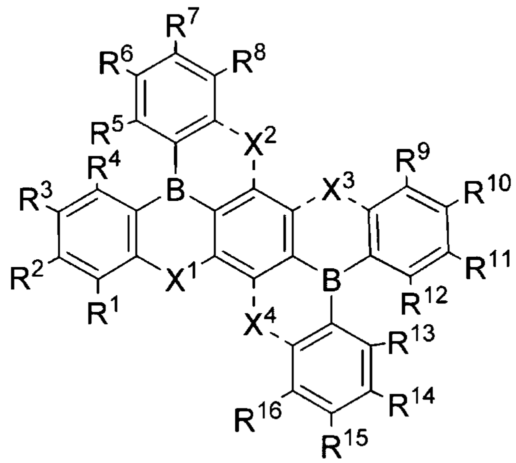

In the general formula (2), $R^1$ and $R^3$ to $R^{16}$ each independently represent a hydrogen atom, a deuterium atom, or a substituent. $R^2$ represents an acceptor group, or $R^1$ and $R^2$ bond to each other to form an acceptor group, or $R^2$ and $R^3$ bond to each other to form an acceptor group. $R^3$ and $R^4$, $R^4$ and $R^5$, $R^5$ and $R^6$, $R^6$ and $R^7$, $R^7$ and $R^8$, $R^9$ and $R^{10}$, $R^{10}$ and $R^{11}$, $R^{11}$ and $R^{12}$, $R^{12}$ and $R^{13}$, $R^{13}$ and $R^{14}$, $R^{14}$ and $R^{15}$, and $R^{15}$ and $R^{16}$ each can bond to each other to form a cyclic structure. $X^1$ represents O or NR, and R represents a substituent. Of $X^2$ to $X^4$, at least one of $X^3$ and $X^4$ represents O or NR, and the remainder can be O or NR, or unlinked. When not linked, both ends each independently represent a hydrogen atom, a deuterium atom or a substituent. In the general formula (2), C-$R^1$, C-$R^3$, C-$R^4$, C-$R^5$, C-$R^6$, C-$R^7$, C-$R^8$, C-$R^9$, C-$R^{10}$, C-$R^{11}$, C-$R^{12}$, C-$R^{13}$, C-$R^{14}$, C-$R^{15}$, and C-$R^{16}$ can be substituted with N.

In one aspect of the present invention, when $X^2$ represents O or NR, $R^7$ represents an acceptor group, $R^6$ and $R^7$ bond to each other to form an acceptor group, or $R^7$ and $R^8$ bond to each other to form an acceptor group. In one aspect of the present invention, when $X^3$ represents O or NR, $R^{10}$ represents an acceptor group, $R^9$ and $R^{10}$ bond to each other to form an acceptor group, or $R^{10}$ and $R^{11}$ bond to each other to form an acceptor group. In one aspect of the present invention, when $X^4$ represents O or NR, $R^{15}$ represents an acceptor group, $R^{14}$ and $R^{15}$ bond to each other to form an acceptor group, or $R^{15}$ and $R^{16}$ bond to each other to form an acceptor group. In one aspect of the present invention, when $X^2$ represents NR and when R represents a substituted or unsubstituted phenyl group and forms a carbazole ring by directly bonding to the carbon atom to which $R^8$ bonds, at least one of the 3-position and the 6-position of the carbazole ring is substituted with an acceptor group. In one aspect of the present invention, when $X^3$ represents NR and when R represents a substituted or unsubstituted phenyl group and forms a carbazole ring by directly bonding to the carbon atom to which $R^9$ bonds, at least one of the 3-position and the 6-position of the carbazole ring is substituted with an acceptor

group. In one aspect of the present invention, when $X^4$ represents NR and when R represents a substituted or unsubstituted phenyl group and forms a carbazole ring by directly bonding to the carbon atom to which $R^{16}$ bonds, at least one of the 3-position and the 6-position of the carbazole ring is substituted with an acceptor group. In one aspect of the present invention, when $X^1$ represents NR and when R represents a substituted or unsubstituted phenyl group and forms a carbazole ring by directly bonding to the carbon atom to which $R^1$ bonds, the 3-position of the carbazole ring is substituted with an acceptor group (here, the 3-position is on the phenyl group). One aspect of the present invention is a compound represented by the following general formula (2a).

General Formula (2a)

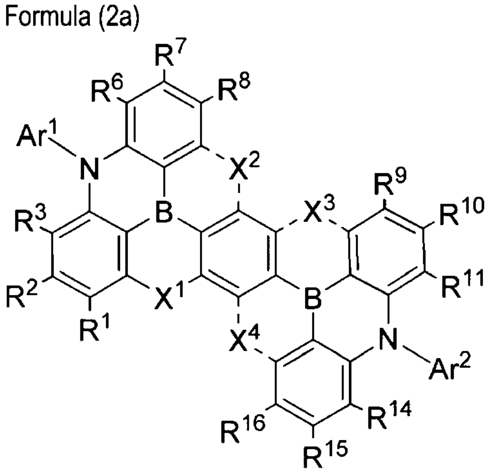

In the general formula (2a), $R^1$, $R^3$, $R^6$ to $R^{11}$, and $R^{14}$ to $R^{16}$ each independently represent a hydrogen atom, a deuterium atom, or a substituent. $R^2$ represents an acceptor group, or $R^1$ and $R^2$ bond to each other to form an acceptor group, or $R^2$ and $R^3$ bond to each other to form an acceptor group.

$R^6$ and $R^7$, $R^7$ and $R^8$, $R^9$ and $R^{10}$, $R^{10}$ and $R^{11}$, $R^{14}$ and $R^{15}$, and $R^{15}$ and $R^{16}$ each can bond to each other to form a cyclic structure. $X^1$ represents O or NR, and R represents a substituent. Of $X^2$ to $X^4$, at least one of $X^3$ and $X^4$ represents O or NR, and the remainder can be O or NR, or unlinked. When not linked, both ends each independently represent a hydrogen atom, a deuterium atom or a substituent. $Ar^1$ and $Ar^2$ each independently represent a substituted or unsubstituted aryl group, or a substituted or unsubstituted heteroaryl group. In the general formula (2a), C-$R^1$, C-$R^3$, C-$R^6$, C-$R^7$, C-$R^8$, C-$R^9$, C-$R^{10}$, C-$R^{11}$, C-$R^{14}$, C-$R^{15}$, and C-$R^{16}$ can be substituted with N.

Compounds represented by the following general formula (3) are further preferred light emitting materials.

General Formula (3)

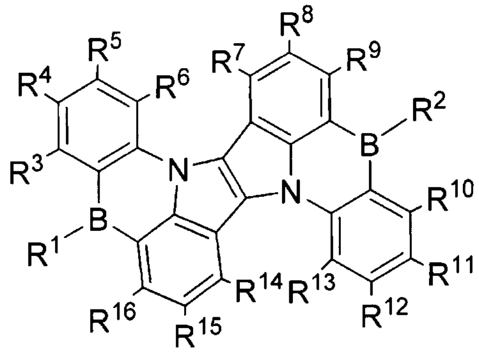

In the general formula (3), $R^1$ and $R^2$ each independently represent a substituted or unsubstituted alkyl group, a substituted or unsubstituted aryl group, or a substituted or unsubstituted heteroaryl group, and $R^3$ to $R^{16}$ each independently represent a hydrogen atom, a deuterium atom or a substituent. $R^1$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^2$, $R^2$ and $R^{10}$, $R^{10}$ and $R^{11}$, $R^{11}$ and $R^{12}$, $R^{12}$ and $R^{13}$, $R^{13}$ and $R^{14}$, $R^{14}$ and $R^{15}$, $R^{15}$ and $R^{16}$, and $R^{16}$ and $R^1$ each can bond to each other to form a cyclic structure. In the general formula (3), C-$R^3$, C-$R^4$, C-$R^5$, C-$R^6$, C-$R^7$, C-$R^8$, C-$R^9$, C-$R^{10}$, C-$R^{11}$, C-$R^{12}$, C-$R^{13}$, C-$R^{14}$, C-$R^{15}$, and C-$R^{16}$ can be substituted with N.

In one aspect of the present invention, $R^1$ and $R^2$ each independently represent a substituted or unsubstituted phenyl group optionally fused with any other ring. In one aspect of the present invention, $R^3$ and $R^{10}$ each independently represent a substituted amino group. In one aspect of the present invention, at least one pair of $R^1$ and $R^3$, and $R^2$ and $R^{10}$

bonds to each other to form a cyclic structure. In one aspect of the present invention, the cyclic structure includes a benzazaborine ring.

Compounds represented by the following general formula (4) are further preferred light emitting materials.

General Formula (4)

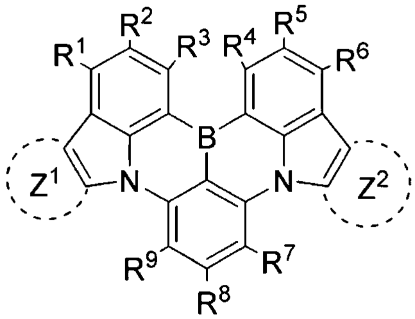

In the general formula (4), $Z^1$ and $Z^2$ each independently represent a substituted or unsubstituted aromatic ring, or a substituted or unsubstituted heteroaromatic ring, $R^1$ to $R^9$ each independently represent a hydrogen atom, a deuterium atom or a substituent. $R^1$ and $R^2$, $R^2$ and $R^3$, $R^3$ and $R^4$, $R^4$ and $R^5$, $R^5$ and $R^6$, $R^7$ and $R^8$, and $R^8$ and $R^9$ each can bond to each other to form a cyclic structure. At least one of the ring formed by $Z^1$, $Z^2$, $R^1$ and $R^2$ bonding to each other, the ring formed by $R^2$ and $R^3$ bonding to each other, the ring formed by $R^4$ and $R^5$ bonding to each other, and the ring formed by $R^5$ and $R^6$ bonding to each other is a furan ring of a substituted or unsubstituted benzofuran, a thiophene ring of a substituted or unsubstituted benzothiophene, or a pyrrole ring of a substituted or unsubstituted indole, and at least one of $R^1$ to $R^9$ is a substituted or unsubstituted aryl group or an acceptor group, or at least one of $Z^1$ and $Z^2$ is a ring having an aryl group or an acceptor group as a substituent. Of the benzene ring skeleton-constituting carbon atoms to constitute the benzofuran ring, the benzothiophene ring, and the indole ring, a substitutable carbon atom can be substituted with a nitrogen atom. In the general formula (4), C-$R^1$, C-$R^2$, C-$R^3$, C-$R^4$, C-$R^5$, C-$R^6$, C-$R^7$, C-$R^8$, and C-$R^9$ can be substituted with N.

In one aspect of the present invention, $Z^1$ and $Z^2$ each independently represent a substituted or unsubstituted non-fused benzene ring, a furan ring fused with a substituted or unsubstituted benzene ring, a thiophene ring fused with a substituted or unsubstituted benzene ring, or a pyrrole ring fused with a substituted or unsubstituted benzene ring. In one aspect of the present invention, $R^1$ to $R^9$ each independently represent a substituted or unsubstituted aryl group or an acceptor group, or at least one ring selected from the group consisting of the ring formed by $R^1$ and $R^2$ bonding to each other, the ring formed by $R^2$ and $R^3$ bonding to each other, the ring formed by $R^4$ and $R^5$ bonding to each other, and the ring formed by $R^5$ and $R^6$ bonding to each other is a furan ring fused with a substituted or unsubstituted benzene ring, a thiophene ring fused with a substituted or unsubstituted benzene ring, or a pyrrole ring fused with a substituted or unsubstituted benzene ring. In one aspect of the present invention, $R^8$ represents a substituted or unsubstituted aryl group, or an acceptor group. One aspect of the present invention contains two or more rings selected from the group consisting of the benzofuran ring, the benzothiophene ring, and the indole ring.

Further more preferred light emitting materials include compounds having a ring-fused structure A, in which the carbon-carbon bond a in the following structure $\alpha$ is fused with a furan ring constituting a substituted or unsubstituted benzofuran ring, a thiophene ring constituting a substituted or unsubstituted benzothiophene ring, or a pyrrole ring constituting a substituted or unsubstituted indole ring, or the carbon-carbon bond b is fused with a benzene ring constituting a substituted or unsubstituted dibenzofuran ring, a benzene ring constituting a substituted or unsubstituted dibenzothiophene ring, a benzene ring constituting a substituted or unsubstituted carbazole ring, or a benzene ring constituting a substituted or unsubstituted dibenzodioxane ring (the hydrogen atom in the structure can be substituted with a deuterium atom or a substituent).

Structure α

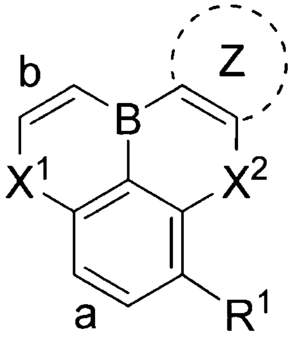

In the structure α, $X^1$ and $X^2$ each independently represent a nitrogen atom to which a substituted or unsubstituted aryl group or a substituted or unsubstituted aryl group bonds, or an oxygen atom, Z represents a substituted or unsubstituted aromatic ring, or a substituted or unsubstituted heteroaromatic ring, $R^1$ represents a hydrogen atom, a deuterium atom or a substituent, and Z and $X^2$ can bond to each other to form a cyclic structure.

In the ring-fused structure A, the structure fused to b and $X^1$, the structure fused to b and Z, and Z and $X^2$ each can bond to each other to form a cyclic structure.

Compounds represented by the following general formula (5) are further preferred light emitting materials.

General Formula (5)

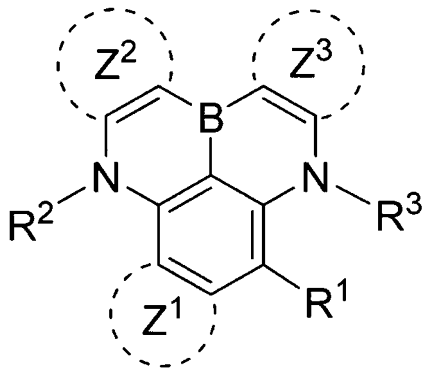

In the general formula (5), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ and $Z^3$ each independently represent a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group. $Z^1$ and $R^1$, $R^2$ and $Z^2$, $Z^2$ and $Z^3$, and $Z^3$ and $R^3$ each can bond to each other to form a cyclic structure. At least one pair of $R^2$ and $Z^2$, $Z^2$ and $Z^3$, and $Z^3$ and $R^3$ bonds to each other to form a cyclic structure.

Compounds represented by the following general formula (6) are further preferred light emitting materials.

General Formula (6)

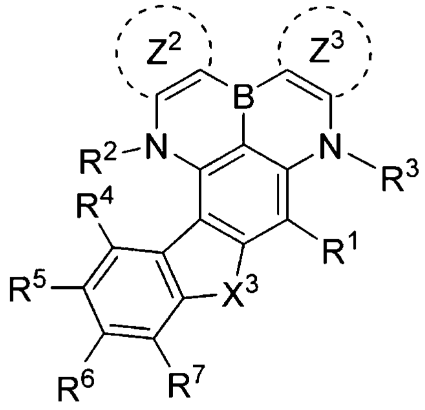

In the general formula (6), $X^3$ represents an oxygen atom or a sulfur atom, $Z^2$ and $Z^3$ each independently represent a substituted or unsubstituted aromatic ring, or a substituted or unsubstituted heteroaromatic ring, $R^1$ and $R^4$ to $R^7$ each represent a hydrogen atom, a deuterium atom or a substituent, $R^2$ and $R^3$ each independently represent a substituted or unsubstituted aryl group, or a substituted or unsubstituted heteroaryl group. $R^2$ and $Z^2$, $Z^2$ and $Z^3$, $Z^3$ and $R^3$, $R^4$ and $R^5$, $R^5$ and $R^6$, and $R^6$ and $R^7$ each can bond to each other to form a cyclic structure. At least one pair of $R^2$ and $Z^2$, $Z^2$ and $Z^3$, and $Z^3$ and $R^3$ bonds to each other to form a cyclic structure.

Compounds represented by the following general formula (7) are further preferred light emitting materials.

General Formula (7)

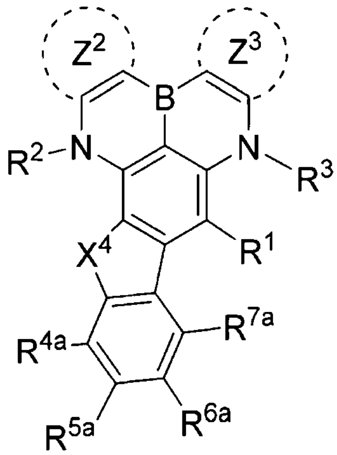

In the general formula (7), $X^4$ represents an oxygen atom or a sulfur atom, $Z^2$ and $Z^3$ each independently represent a substituted or unsubstituted aromatic ring, or a substituted or unsubstituted heteroaromatic ring, $R^1$ and $R^{4a}$ to $R^{7a}$ each represent a hydrogen atom, a deuterium atom or a substituent, and $R^2$ and $R^3$ each independently represent a substituted or unsubstituted aryl group, or a substituted or unsubstituted heteroaryl group. $R^2$ and $Z^2$, $Z^2$ and $Z^3$, $Z^3$ and $R^3$, $R^{4a}$ and $R^{5a}$, $R^{5a}$ and $R^{6a}$, $R^{6a}$ and $R^{7a}$, and $R^{7a}$ and $R^1$ each can bond to each other to form a cyclic structure. At least one pair of $R^2$ and $Z^2$, $Z^2$ and $Z^3$, and $Z^3$ and $R^3$ bonds to each other to form a cyclic structure.

Compounds represented by the following general formula (8) are further preferred light emitting materials.

General Formula (8)

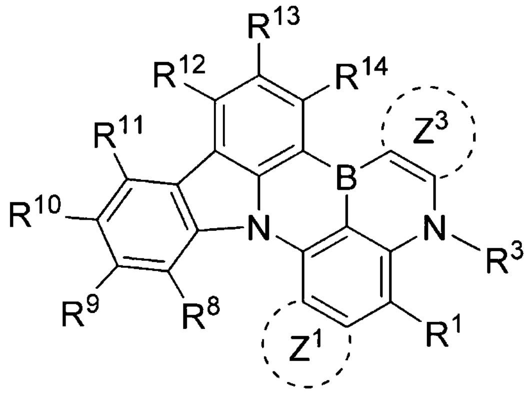

In the general formula (8), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^3$ represents a substituted or unsubstituted aromatic ring, or a substituted or unsubstituted heteroaromatic ring, $R^1$ and $R^8$ to $R^{14}$ each independently represent a hydrogen atom, a deuterium atom, or a substituent, and $R^3$ represents a substituted or unsubstituted aryl group, or a substituted or unsubstituted heteroaryl group. $Z^1$ and $R^1$, $R^8$ and $R^9$, $R^9$ and $R^{10}$, $R^{10}$ and $R^{11}$, $R^{11}$ and $R^{12}$, $R^{12}$ and $R^{13}$, $R^{13}$ and $R^{14}$, $R^{14}$ and $Z^3$, and $Z^3$ and $R^3$ each can bond to each other to form a cyclic structure.

Compounds represented by the following general formula (9) are further preferred light emitting materials.

General Formula (9)

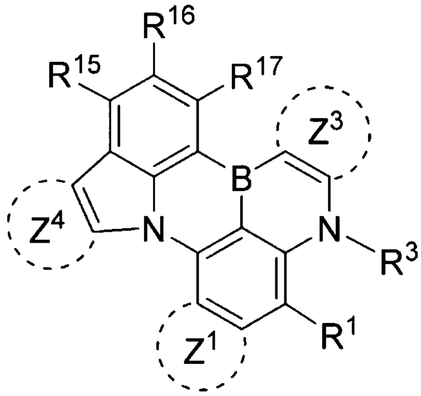

In the general formula (9), $Z^1$ and $Z^4$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^3$ represents a substituted or unsubstituted aromatic ring, or a substituted or unsubstituted heteroaromatic ring, $R^1$ and $R^{15}$ to $R^{17}$ each independently represent a hydrogen atom, a deuterium atom, or a substituent, and $R^3$ represents a substituted or unsubstituted aryl group, or a substituted or unsubstituted heteroaryl group. $Z^1$ and $R^1$, $Z^4$ and $R^{15}$, $R^{15}$ and $R^{16}$, $R^{16}$ and $R^{17}$, $R^{17}$ and $Z^3$, and $Z^3$ and $R^3$ each can bond to each other to form a cyclic structure.

Compounds represented by the following general formula (10) are further preferred light emitting materials.

General Formula (10)

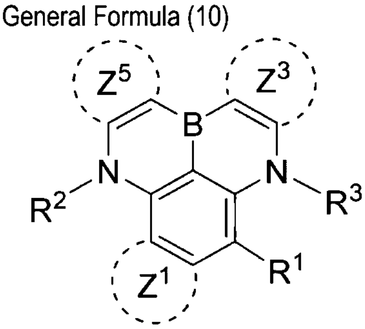

In the general formula (10), $Z^1$ and $Z^5$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^3$ represents a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group. $Z^1$ and $R^1$, $R^2$ and $Z^5$, $Z^5$ and $Z^3$, $Z^3$ and $R^3$ each can bond to each other to form a cyclic structure. At least one pair of $R^2$ and $Z^2$, $Z^2$ and $Z^3$, and $Z^3$ and $R^3$ bonds to each other to form a cyclic structure.

Compounds represented by the following general formula (11) are further preferred light emitting materials.

General Formula (11)

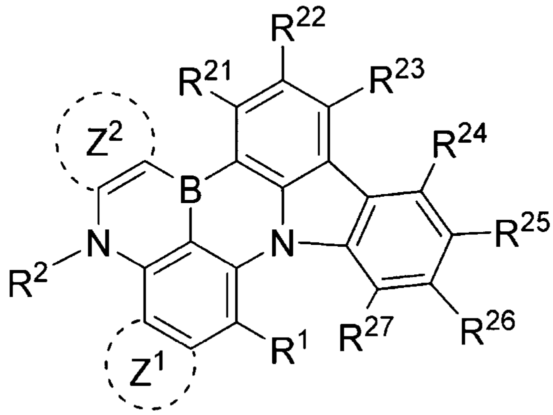

In the general formula (11), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ represents a substituted or unsubstituted aromatic ring, or a substituted or unsubstituted heteroaromatic ring, $R^1$ and $R^{21}$ to $R^{27}$ each independently represent a hydrogen atom, a deuterium atom, or a substituent, and $R^2$ represents a substituted or unsubstituted aryl group, or a substituted or unsubstituted heteroaryl group. $R^1$ and $Z^1$, $R^2$ and $Z^2$, $Z^2$ and $R^{21}$, $R^{21}$ and $R^{22}$, $R^{22}$ and $R^{23}$, $R^{23}$ and $R^{24}$, $R^{24}$ and $R^{25}$, $R^{25}$ and $R^{26}$, and $R^{26}$ and $R^{27}$ each can bond to each other to form a cyclic structure.

Compounds represented by the following general formula (12) are further preferred light emitting materials.

General Formula (12)

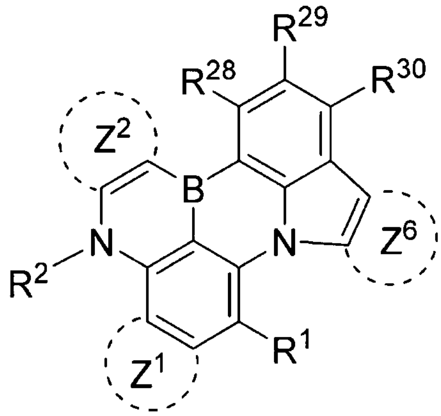

In the general formula (12), $Z^1$ and $Z^6$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ represents a substituted or unsubstituted aromatic ring, or a substituted or unsubstituted heteroaromatic ring, $R^1$ and $R^{28}$ to $R^{30}$ each independently represent a hydrogen atom, a deuterium atom, or a substituent, and $R^2$ represents a substituted or unsubstituted aryl group, or a substituted or unsubstituted heteroaryl group. $R^1$ and $Z^1$, $R^2$ and $Z^2$, $Z^2$ and $R^{28}$, $R^{28}$ and $R^{29}$, $R^{29}$ and $R^{30}$, and $R^{30}$ and $Z^6$ each can bond to each other to form a cyclic structure.

Compounds represented by the following general formula (13) are further preferred light emitting materials.

General Formula (13)

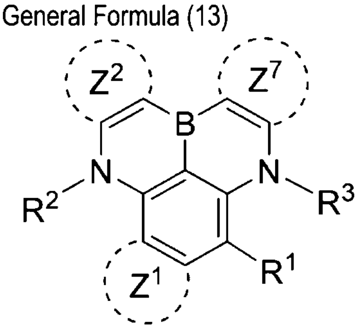

In the general formula (13), $Z^1$ and $Z^7$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ represents a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group. $R^1$ and $Z^1$, $R^2$ and $Z^2$, $Z^2$ and $Z^7$, and $Z^7$ and $R^3$ each can bond to each other to form a cyclic structure. At least one pair of $R^2$ and $Z^2$, $Z^2$ and $Z^7$, and $Z^7$ and $R^3$ bonds to each other to form a cyclic structure.

Compounds represented by the following general formula (14) are further preferred light emitting materials.

General Formula (14)

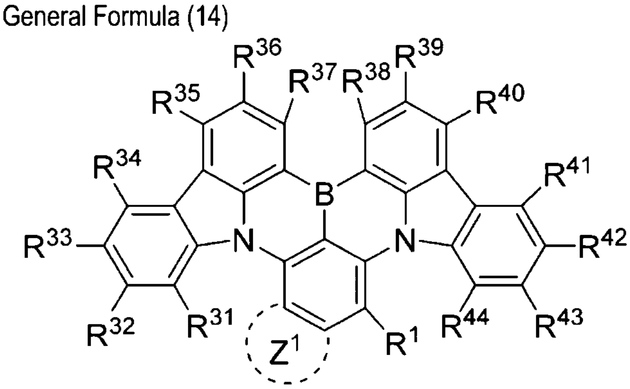

In the general formula (14), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{31}$ to $R^{44}$ each independently represent a substituted or unsubstituted aryl group, or a substituted or unsubstituted heteroaryl group. $R^1$ and $Z^1$, $R^{31}$ and $R^{32}$, $R^{32}$ and $R^{33}$, $R^{33}$ and $R^{34}$, $R^{34}$ and $R^{35}$, $R^{35}$ and $R^{36}$, $R^{36}$ and $R^{37}$, $R^{37}$ and $R^{38}$, $R^{38}$ and $R^{39}$, $R^{39}$ and $R^{40}$, $R^{40}$ and $R^{41}$, $R^{41}$ and $R^{42}$, $R^{42}$ and $R^{43}$, and $R^{43}$ and $R^{44}$ each can bond to each other to form a cyclic structure.

Compounds represented by the following general formula (15) are further preferred light emitting materials.

General Formula (15)

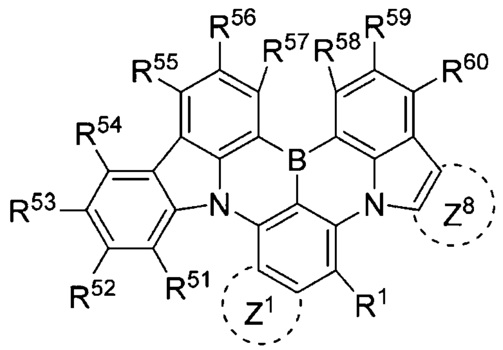

In the general formula (15), $Z^1$ and $Z^8$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{51}$ to $R^{60}$ each independently represent a hydrogen atom, a deuterium atom, or a substituent. $R^1$ and $Z^1$, $R^{51}$ and $R^{52}$, $R^{52}$ and $R^{53}$, $R^{53}$ and $R^{54}$, $R^{54}$ and $R^{55}$, $R^{55}$ and $R^{56}$, $R^{56}$ and $R^{57}$, $R^{57}$ and $R^{58}$, $R^{58}$ and $R^{59}$, $R^{59}$ and $R^{60}$, and $R^{60}$ and $Z^2$ each can bond to each other to form a cyclic structure.

Compounds represented by the following general formula (16) are further preferred light emitting materials.

General Formula (16)

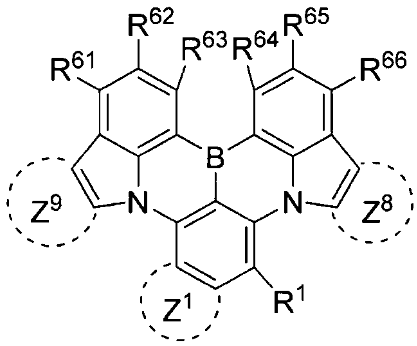

In the general formula (16), $Z^1$, $Z^8$ and $Z^9$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, and $R^1$ and $R^{61}$ to $R^{66}$ each independently represent a hydrogen atom, a deuterium atom, or a substituent. $R^1$ and $Z^1$, $Z^9$ and $R^{61}$, $R^{61}$ and $R^{62}$, $R^{62}$ and $R^{63}$, $R^{63}$ and $R^{64}$, $R^{64}$ and $R^{65}$, $R^{65}$ and $R^{66}$, and $R^{66}$ and $Z^8$ each can bond to each other to form a cyclic structure.

Compounds represented by the following general formula (17) are further preferred light emitting materials.

General Formula (17)

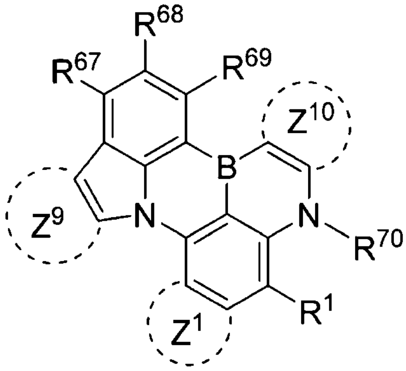

In the general formula (17), $Z^1$, $Z^9$ and $Z^{10}$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{67}$ to $R^{69}$ each independently represent a hydrogen atom, a deuterium atom, or a substituent, and $R^{70}$ represents a substituted or unsubstituted aryl group, or a substituted or unsubstituted heteroaryl group. $R^1$ and $Z^1$, $Z^9$ and $R^{67}$, $R^{67}$ and $R^{68}$, $R^{68}$ and $R^{69}$, $R^{69}$ and $Z^{10}$, and $Z^{10}$ and $R^{70}$ each can bond to each other to form a cyclic structure.

Compounds represented by the following general formula (18) are further preferred light emitting materials.

General Formula (18)

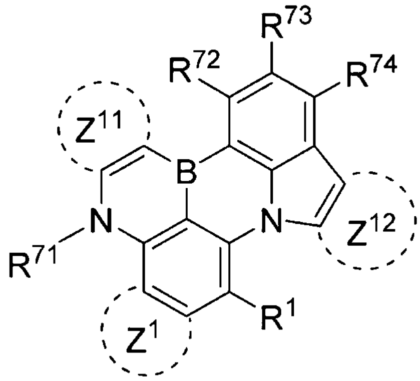

In the general formula (18), $Z^1$, $Z^{11}$ and $Z^{12}$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{72}$ to $R^{74}$ each independently represent a hydrogen atom, a deuterium atom, or a substituent, and $R^{71}$ represents a substituted or unsubstituted aryl group, or a substituted or unsubstituted heteroaryl group. $R^1$ and $Z^1$, $R^{71}$ and $Z^{11}$, $Z^{11}$ and $R^{72}$, $R^{72}$ and $R^{73}$, $R^{73}$ and $Z^{74}$, and $R^{74}$ and $Z^{12}$ each can bond to each other to form a cyclic structure.

Compounds represented by the following general formula (19) are further preferred light emitting materials.

General Formula (19)

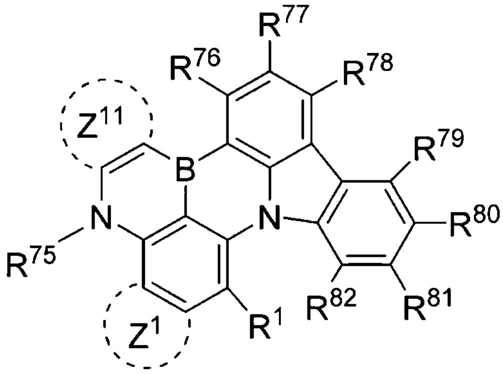

In the general formula (19), $Z^1$ and $Z^{11}$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{76}$ to $R^{82}$ each independently represent a substituted or unsubstituted aryl group, or a substituted or unsubstituted heteroaryl group, and $R^{75}$ represents a substituted or unsubstituted aryl group, or a substituted or unsubstituted heteroaryl group. $R^1$ and $Z^1$, $R^{75}$ and $Z^{11}$, $Z^{11}$ and $R^{76}$, $R^{76}$ and $R^{77}$, $R^{77}$ and $R^{78}$, $R^{78}$ and $R^{79}$, $R^{79}$ and $R^{80}$, $R^{80}$ and $R^{81}$, and $R^{81}$ and $R^{82}$ each can bond to each other to form a cyclic structure.

Compounds represented by the following general formula (20) are further preferred light emitting materials.

General Formula (20)

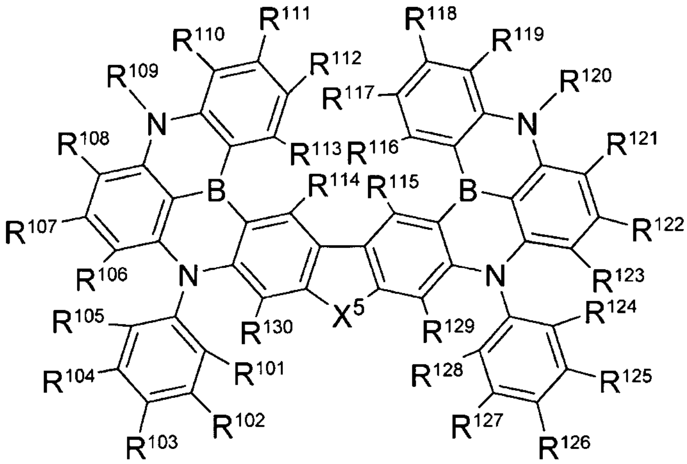

In the general formula (20), $X^5$ represents an oxygen atom, a sulfur atom, or a nitrogen atom to which a substituted or unsubstituted aryl group or a substituted or unsubstituted heteroaryl group bonds, $R^{101}$ to $R^{130}$ each independently represent a hydrogen atom, a deuterium atom or a substituent, and $R^{101}$ and $R^{102}$, $R^{102}$ and $R^{103}$, $R^{103}$ and $R^{104}$, $R^{104}$ and $R^{105}$, $R^{105}$ and $R^{106}$, $R^{106}$ and $R^{107}$, $R^{107}$ and $R^{108}$, $R^{198}$ and $R^{109}$, $R^{109}$ and $R^{110}$, $R^{110}$ and $R^{111}$, $R^{111}$ and $R^{112}$ , $R^{112}$ and $R^{113}$, $R^{113}$ and $R^{114}$ , $R^{114}$ and $R^{115}$, $R^{115}$ and $R^{116}$, $R^{116}$ and $R^{117}$, $R^{117}$ and $R^{118}$, $R^{118}$ and $R^{119}$, $R^{119}$ and $R^{120}$, $R^{120}$ and $R^{121}$, $R^{121}$ and $R^{122}$, $R^{122}$ and $R^{123}$, $R^{123}$ and $R^{124}$, $R^{124}$ and $R^{125}$, $R^{125}$ and $R^{126}$, $R^{126}$ and $R^{127}$, $R^{127}$ and $R^{128}$, $R^{128}$ and $R^{129}$, $R^{129}$ and $R^{130}$, and $R^{130}$ and $R^{101}$ each can bond to each other to form a cyclic structure.

Compounds represented by the following general formula (21) are further preferred light emitting materials.

General Formula (21)

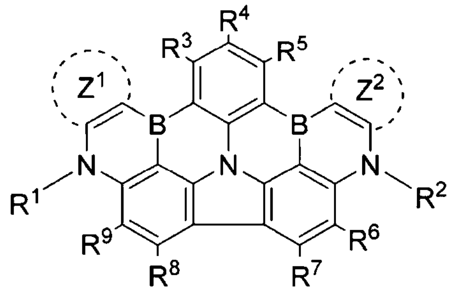

In the general formula (21), $R^1$ and $R^2$ each independently represent a substituted or unsubstituted alkyl group, a substituted or unsubstituted aryl group, or a substituted or unsubstituted heteroaryl group, $Z^1$ and $Z^2$ each independently represent a substituted or unsubstituted aromatic ring, or a substituted or unsubstituted heteroaromatic ring, and $R^3$ to $R^9$ each independently represent a hydrogen atom, a deuterium atom or a substituent. At least one of $R^1$, $R^2$, $Z^1$ and $Z^2$ includes a substituted or unsubstituted benzofuran ring, a substituted or unsubstituted benzothiophene ring, or a substituted or unsubstituted indole ring. $R^1$ and $Z^1$, $Z^1$ and $R^3$, $R^3$ and $R^4$, $R^4$ and $R^5$, $R^5$ and $Z^2$, $Z^2$ and $R^2$, $R^2$ and $R^6$, $R^6$ and $R^7$, $R^7$ and $R^8$, $R^8$ and $R^9$, and $R^9$ and $R^1$ each can bond to each other to form a cyclic structure. Of the benzene ring skeleton-constituting carbon atoms to constitute the benzofuran ring, the benzothiophene ring, and the indole ring, a substitutable carbon atom can be substituted with a nitrogen atom. In the general formula (21), C-$R^3$, C-$R^4$, C-$R^5$, C-$R^6$, C-$R^7$, C-$R^8$, and C-$R^9$ can be substituted with N.

In one aspect of the present invention, $R^1$ and $R^2$ each independently represent a substituted or unsubstituted alkyl group, a substituted or unsubstituted phenyl group, or a group containing one or more ring structures selected from the group consisting of a substituted or unsubstituted benzofuran ring, a substituted or unsubstituted benzothiophene ring and a substituted or unsubstituted indole ring. In one aspect of the present invention, $Z^1$ and $Z^2$ each independently represent a substituted or unsubstituted non-fused benzene ring, a furan ring fused with a substituted or unsubstituted benzene ring, a thiophene ring fused with a substituted or unsubstituted benzene ring, a pyrrole ring fused with a substituted or unsubstituted benzene ring, a benzene ring fused with a substituted or unsubstituted benzofuran ring, a benzene ring fused with a substituted or unsubstituted benzothiophene ring, or a benzene ring fused with a substituted or unsubstituted indole ring. In one aspect of the present invention, $R^1$ and $Z^1$ bond to each other to form a cyclic structure. In one aspect of the present invention, $R^1$ and $Z^1$ bond to each other to form a pyrrole ring.

Compounds represented by the following general formula (22) are further preferred light emitting materials.

General Formula (22)

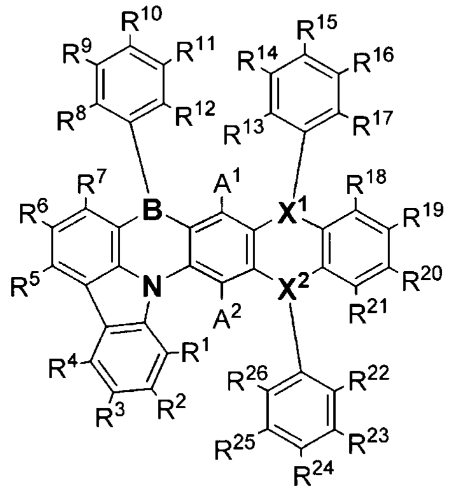

In the general formula (22), one of $X^1$ and $X^2$ represents a nitrogen atom, and the other represents a boron atom. $R^1$ to $R^{26}$, $A^1$ and $A^2$ each independently represent a hydrogen atom, a deuterium atom, or a substituent. $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ each can bond to each other to form a cyclic structure. When $X^1$ represents a

nitrogen atom, $R^{17}$ and $R^{18}$ bond to each other to be a single bond to form a pyrrole ring, and when $X^2$ represents a nitrogen atom, $R^{21}$ and $R^{22}$ bond to each other to be a single bond to form a pyrrole ring. In the case where $X^1$ represents a nitrogen atom, and where $R^7$ and $R^8$ and $R^{21}$ and $R^{22}$ each bond to each other via a nitrogen atom to form a 6-membered ring, and $R^{17}$ and $R^{18}$ bond to each other to form a single bond, at least one of $R^1$ to $R^6$ represents a substituted or unsubstituted aryl group, or any of $R^1$ and $R^2$, $R^2$ and $R^3$, $R^3$ and $R^4$, $R^4$ and $R^5$, and $R^5$ and $R^6$ bond to each other to form an aromatic ring or a heteroaromatic ring.

In one aspect of the present invention, at least one of $R^3$ and $R^6$ represents a substituent. In one aspect of the present invention, both $R^3$ and $R^6$ represent a substituent. In one aspect of the present invention, the substituent represented by $R^3$ and $R^6$ is one group selected from the group consisting of an alkyl group and an aryl group, or a group obtained by combining two or more of the groups. In one aspect of the present invention, both $R^8$ and $R^{12}$ represent a substituent. In one aspect of the present invention, the compounds are represented by the following general formula (1a).

General Formula (22a)

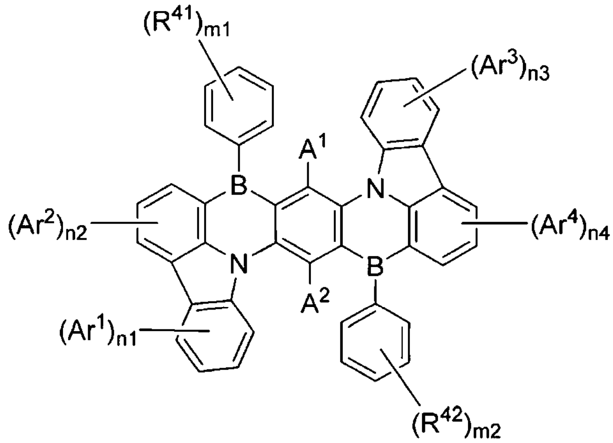

In the general formula (22a), $Ar^1$ to $Ar^4$ each independently represent a substituted or unsubstituted aryl group, or a substituted or unsubstituted heteroaryl group. $R^{41}$ and $R^{42}$ each independently represent a substituted or unsubstituted alkyl group. m1 and m2 each independently represent an integer of 0 to 5, n1 and n3 each independently represent an integer of 0 to 4, and n2 and n4 each independently represent an integer of 0 to 3. $A^1$ and $A^2$ each independently represent a hydrogen atom, a deuterium atom, or a substituent.

With respect to the detailed description, preferred ranges and specific examples of the compound represented by the general formula (22) or the general formula (22a), [0010] to [0119] in WO2022/270354A1 hereby incorporated as a part of this description by reference can be referred to. For example, the following compounds can be mentioned.

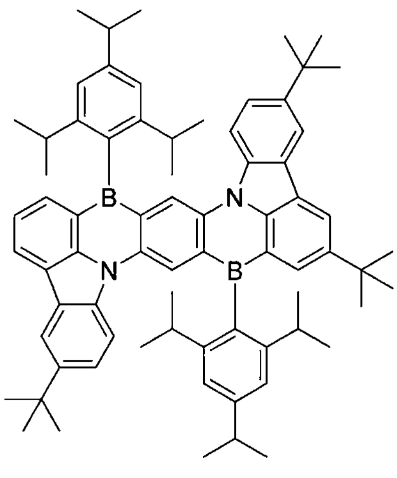

In some embodiments where a host material is used, the amount of the compound of the present invention contained in a light emitting layer as a light emitting material is 0.1% by weight or more. In some embodiments where a host material is used, the amount of the compound of the present invention contained in a light emitting layer as a light emitting material is 1% by weight or more. In some embodiments where a host material is used, the amount of the compound of the present invention contained in a light emitting layer as a light emitting material is 50% by weight or less. In some

embodiments where a host material is used, the amount of the compound of the present invention contained in a light emitting layer as a light emitting material is 20% by weight or less. In some embodiments where a host material is used, the amount of the compound of the present invention contained in a light emitting layer as a light emitting material is 10% by weight or less.

In some embodiments, the host material in a light emitting layer is an organic compound having a hole transporting capability and an electron transporting capability. In some embodiments, the host material in a light emitting layer is an organic compound that prevents increase in the wavelength of emitted light. In some embodiments, the host material in a light emitting layer is an organic compound having a high glass transition temperature.

In some embodiments, the host material is selected from the group consisting of the followings:

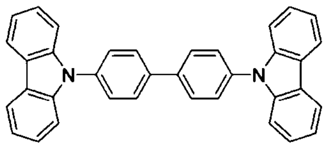
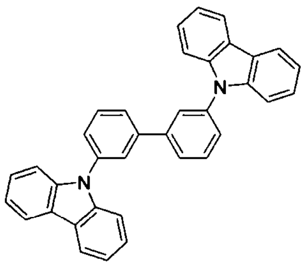
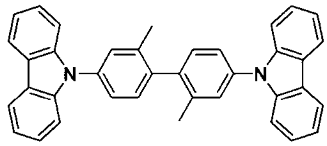
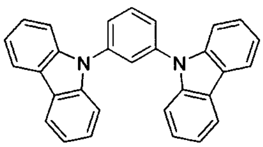
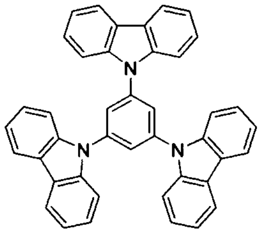
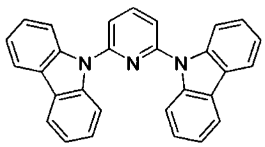
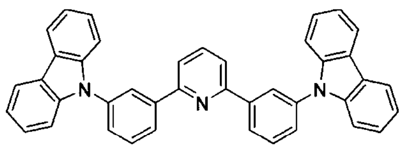
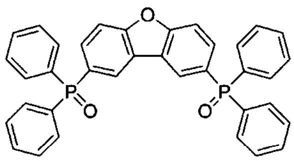
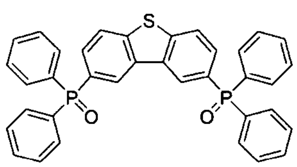
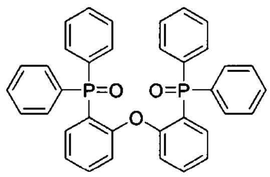
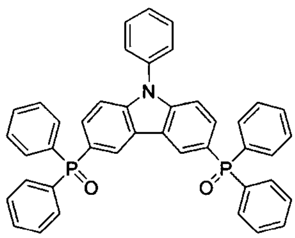
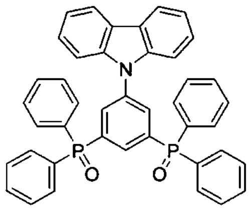
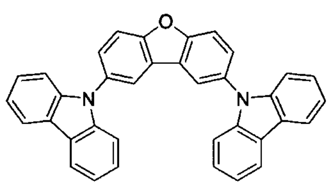
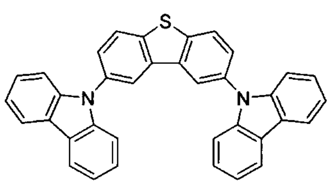
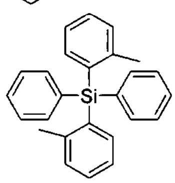
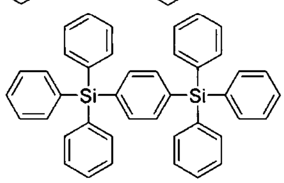
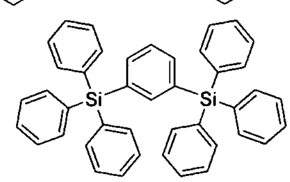
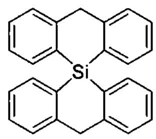

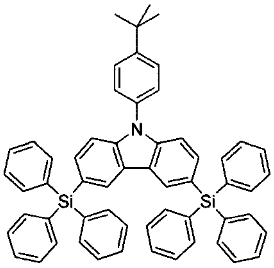
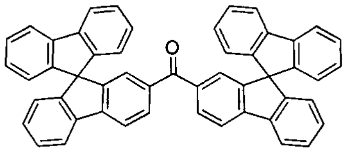
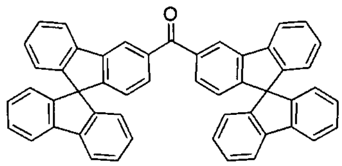

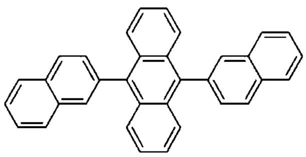
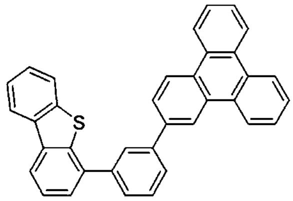
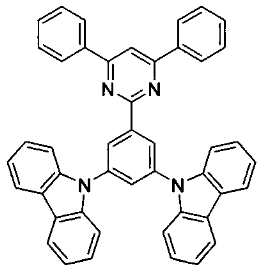

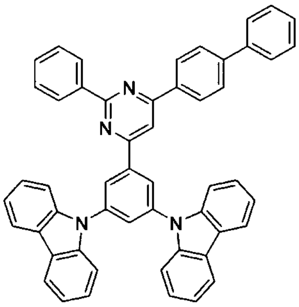
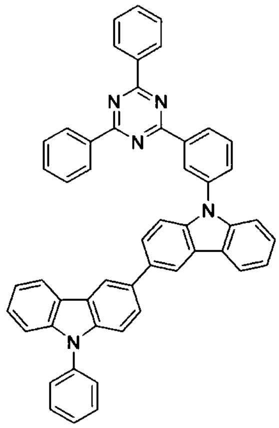
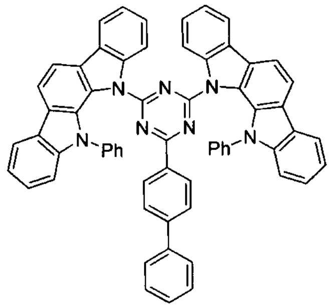

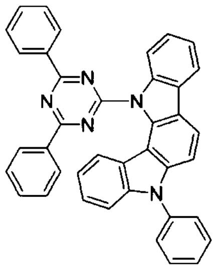
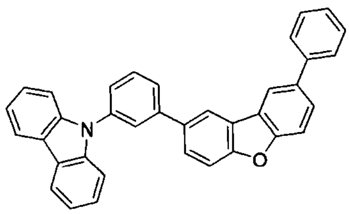
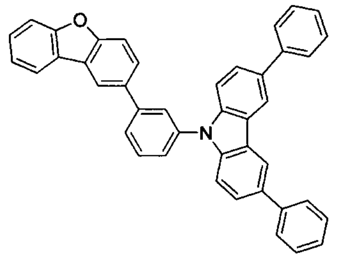

In some embodiments, the light emitting layer contains two or more kinds of TADF molecules differing in the structure. For example, the light emitting layer can contain three kinds of materials of a host material, a first TADF molecule and a second TADF molecule whose excited singlet energy level is higher in that order. In that case, both the first TADF molecule and the second TADF molecule are preferably such that the difference $\Delta E_{ST}$ between the lowest excited singlet energy level and the lowest excited triplet energy level at 77 K is 0.3 eV or less, more preferably 0.25 eV or less, still more preferably 0.2 eV or less, even more preferably 0.15 eV or less, further preferably 0.1 eV or less, further more preferably 0.07 eV or less, further more preferably 0.05 eV or less, further more preferably 0.03 eV or less, and particularly preferably 0.01 eV or less. The content of the first TADF molecule in the light emitting layer is preferably larger than the content of the second TADF molecule therein. The content of the host material in the light emitting layer is preferably larger than the content of the second TADF molecule therein. The content of the first TADF molecule in the light emitting layer can be larger than or can be smaller than or can be the same as the content of the host material therein. In some embodiments, the composition in the light emitting layer can be 10% by weight to 70% by weight of a host material, 10% by weight to 80% by weight of a first TADF molecule, and 0.1% by weight to 30% by weighty of a second TADF molecule. In some embodiments,

the composition in the light emitting layer can be 20% by weight to 45% by weight of a host material, 50% by weight to 75% by weight of a first TADF molecule, and 5% by weight to 20% by weighty of a second TADF molecule. In some embodiments, the photoluminescence quantum yield φPL1(A) by photo-excitation of a co-deposited film of a first TADF molecule and a host material (the content of the first TADF molecule in the co-deposited film = A% by weight) and the photoluminescence quantum yield φPL2(A) by photo-excitation of a co-deposited film of a second TADF molecule and a host material (the content of the second TADF molecule in the co-deposited film = A% by weight) satisfy a relational formula φPL1(A) > φPL2(A). In some embodiments, the photoluminescence quantum yield φPL2(B) by photo-excitation of a co-deposited film of a second TADF molecule and a host material (the content of the second TADF molecule in the co-deposited film = B% by weight) and the photoluminescence quantum yield φPL2(100) by photo-excitation of a single film of a second TADF molecule satisfy a relational formula φPL2(B) > φPL2(100). In some embodiments, the light emitting layer can contain three kinds of TADF molecules differing in the structure. The compound of the present invention can be any of the plurality of TADF compounds contained in the light emitting layer.

In some embodiments, the light emitting layer can be composed of materials selected from the group consisting of a host material, an assist dopant and a light emitting material. In some embodiments, the light emitting layer does not contain a metal element. In some embodiments, the light emitting layer can be formed of a material composed of atoms alone selected from the group consisting of a carbon atom, a hydrogen atom, a deuterium atom, a nitrogen atom, an oxygen atom and a sulfur atom. Alternatively, the light emitting layer can be formed of a material composed of atoms alone selected from the group consisting of a carbon atom, a hydrogen atom, a deuterium atom, a nitrogen atom and an oxygen atom. Alternatively, the light emitting layer can be formed of a material composed of atoms alone selected from the group consisting of a carbon atom, a hydrogen atom, a nitrogen atom and an oxygen atom.

In the case where the light emitting layer contains any other TADF material than the compound of the present invention, the TADF material can be a known delayed fluorescent material. As preferred delayed fluorescent materials, there can be mentioned compounds included in the general formulae described in WO2013/154064, paragraphs 0008 to 0048 and 0095 to 0133; WO2013/011954, paragraphs 0007 to 0047 and 0073 to 0085; WO2013/011955, paragraphs 0007 to 0033 and 0059 to 0066; WO2013/081088, paragraphs 0008 to 0071 and 0118 to 0133; JP 2013-256490 A, paragraphs 0009 to 0046 and 0093 to 0134; JP 2013-116975 A, paragraphs 0008 to 0020 and 0038 to 0040; WO2013/133359, paragraphs 0007 to 0032 and 0079 to 0084; WO2013/161437, paragraphs 0008 to 0054 and 0101 to 0121; JP 2014-9352 A, paragraphs 0007 to 0041 and 0060 to 0069; JP 2014-9224 A, paragraphs 0008 to 0048 and 0067 to 0076; JP 2017-119663 A, paragraphs 0013 to 0025; JP 2017-119664 A, paragraphs 0013 to 0026; JP 2017-222623 A, paragraphs 0012 to 0025; JP 2017-226838 A, paragraphs 0010 to 0050; JP 2018-100411 A, paragraphs 0012 to 0043; WO2018/047853, paragraphs 0016 to 0044; and especially, exemplary compounds therein capable of emitting delayed fluorescence. In addition, also preferably employable here are light emitting materials capable of emitting delayed fluorescence, as described in JP 2013-253121 A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP 2015-129240 A, WO2015/129714, WO2015/129715, WO2015/133501, WO2015/136880, WO2015/137244, WO2015/137202, WO2015/137136, WO2015/146541, and WO2015/159541. These patent publications described in this paragraph are hereby incorporated as a part of this description by reference.

In the following, the constituent members and the other layers than the light emitting layer of the organic electroluminescent device are described.

Substrate:

In some embodiments, the organic electroluminescent device of the present invention is supported by a substrate, in which the substrate is not particularly limited and can be any of those that have been commonly used in an organic electroluminescent device, for example those formed of glass, transparent plastics, quartz and silicon.

Anode:

In some embodiments, the anode of the organic electroluminescent device is made of a metal, an alloy, a conductive compound, or a combination thereof. In some embodiments, the metal, alloy, or electroconductive compound has a large work function (4 eV or more). In some embodiments, the metal is Au. In some embodiments, the conductive transparent material is selected from CuI, indium tin oxide (ITO), $SnO_2$, and ZnO. In some embodiments, an amorphous material capable of forming a transparent conductive film, such as IDIXO ($In_2O_3$-ZnO), is used. In some embodiments, the anode is a thin film. In some embodiments, the thin film is made by vapor deposition or sputtering. In some embodiments, the film is patterned by a photolithography method. In some embodiments, when the pattern may not require high accuracy (for example, approximately 100 μm or more), the pattern can be formed with a mask having a desired shape on vapor

deposition or sputtering of the electrode material. In some embodiments, when a material can be applied as a coating, such as an organic electroconductive compound, a wet film forming method, such as a printing method or a coating method is used. In some embodiments, when the emitted light goes through the anode, the anode has a transmittance of more than 10%, and the anode has a sheet resistance of several hundred Ohm per unit area or less. In some embodiments, the thickness of the anode is 10 nm to 1,000 nm. In some embodiments, the thickness of the anode is 10 nm to 200 nm. In some embodiments, the thickness of the anode varies depending on the material to be used.

Cathode:

In some embodiments, the cathode is made of an electrode material such as a metal having a small work function (4 eV or less) (referred to as an electron injection metal), an alloy, a conductive compound, or a combination thereof. In some embodiments, the electrode material is selected from sodium, a sodium-potassium alloy, magnesium, lithium, a magnesium-copper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth element. In some embodiments, a mixture of an electron injection metal and a second metal that is a stable metal having a work function larger than that of the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per unit area or less. In some embodiments, the thickness of the cathode is 10 nm to 5 $\mu$m. In some embodiments, the thickness of the cathode is 50 nm to 200 nm. In some embodiments, for transmitting the emitted light, any one of the anode and the cathode of the organic electroluminescent device is transparent or translucent. In some embodiments, the transparent or translucent electroluminescent device enhances the light emission luminance.

In some embodiments, the cathode is formed with a conductive transparent material, as described for the anode, to form a transparent or translucent cathode. In some embodiments, a device includes an anode and a cathode, both being transparent or translucent.

Injection Layer:

An injection layer is a layer between the electrode and the organic layer. In some embodiments, the injection layer decreases the drive voltage and enhances the light emission luminance. In some embodiments, the injection layer includes a hole injection layer and an electron injection layer. The injection layer can be positioned between the anode and the light emitting layer or the hole transporting layer, and between the cathode and the light emitting layer or the electron transporting layer. In some embodiments, an injection layer is present. In some embodiments, no injection layer is present.

Preferred compound examples for use as a hole injection material are shown below.

$MoO_3$,

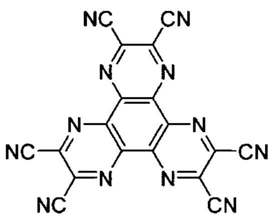
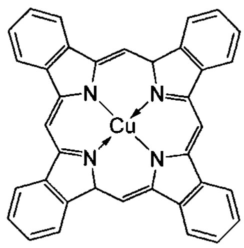
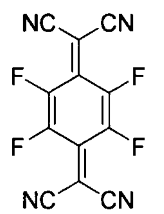
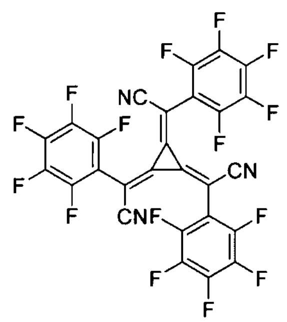
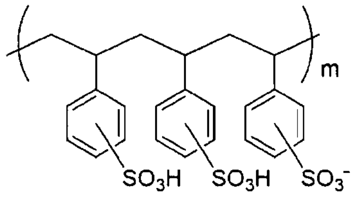
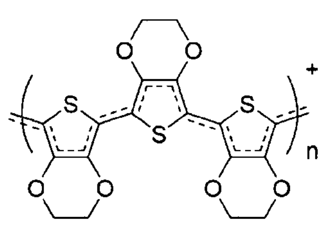

Next, preferred compound examples for use as an electron injection material are shown below.

L i F, C s F,

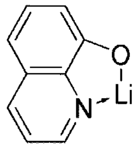

Barrier Layer:

A barrier layer is a layer capable of inhibiting charges (electrons or holes) and/or excitons present in the light emitting layer from being diffused outside the light emitting layer. In some embodiments, the electron barrier layer is between the light emitting layer and the hole transporting layer, and inhibits electrons from passing through the light emitting layer toward the hole transporting layer. In some embodiments, the hole barrier layer is between the light emitting layer and the electron transporting layer, and inhibits holes from passing through the light emitting layer toward the electron transporting layer. In some embodiments, the barrier layer inhibits excitons from being diffused outside the light emitting layer. In some embodiments, the electron barrier layer and the hole barrier layer are exciton barrier layers. As used herein, the term "electron barrier layer" or "exciton barrier layer" includes a layer that has both the function of an electron barrier layer and the function of an exciton barrier layer.

Hole Barrier Layer:

A hole barrier layer functions as an electron transporting layer. In some embodiments, the hole barrier layer inhibits holes from reaching the electron transporting layer while transporting electrons. In some embodiments, the hole barrier layer enhances the recombination probability of electrons and holes in the light emitting layer. The material used for the hole barrier layer can be the same materials as the ones described for the electron transporting layer.

Preferred compound examples for use for the hole barrier layer are shown below.

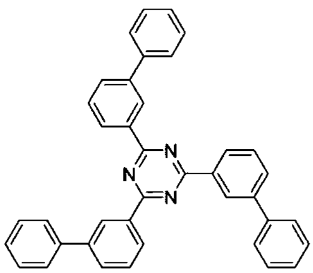
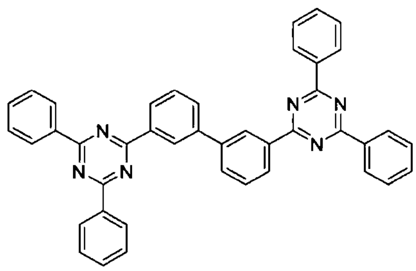
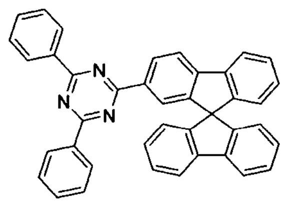

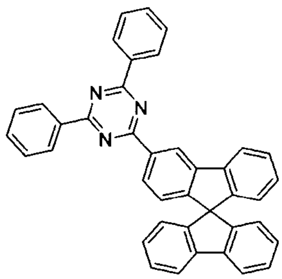
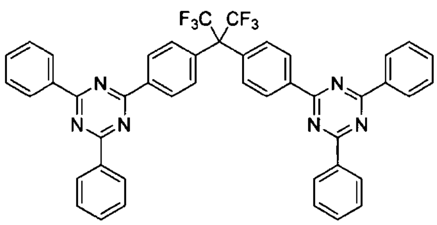
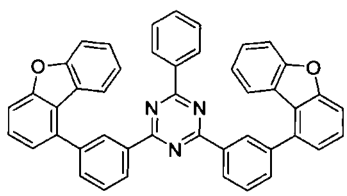

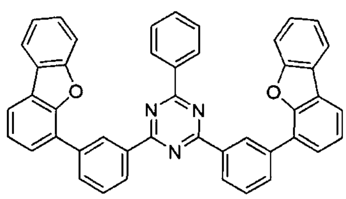
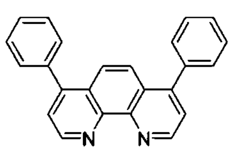
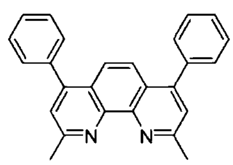

Electron Barrier Layer:

An electron barrier layer transports holes. In some embodiments, the electron barrier layer inhibits electrons from reaching the hole transporting layer while transporting holes. In some embodiments, the electron barrier layer enhances the recombination probability of electrons and holes in the light emitting layer. The material used for the electron barrier layer can be the same material as the ones described above for the hole transporting layer.

Specific examples of preferred compounds for use as the electron barrier material are shown below.

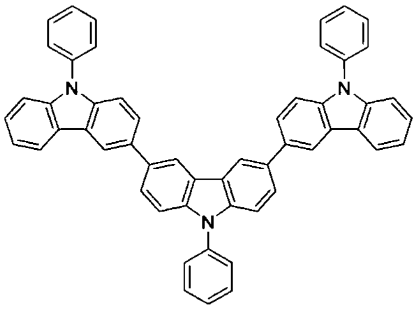

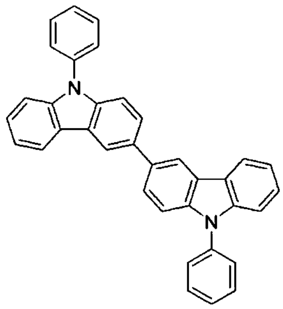

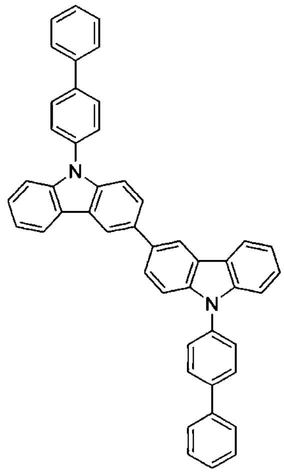

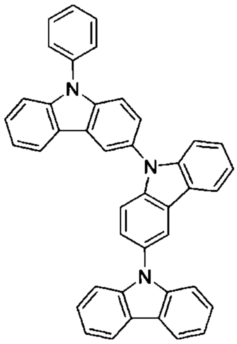

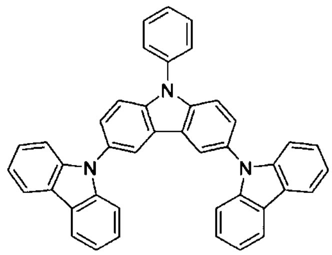

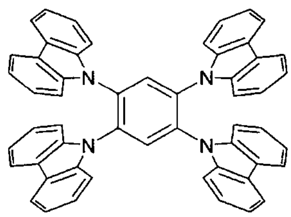

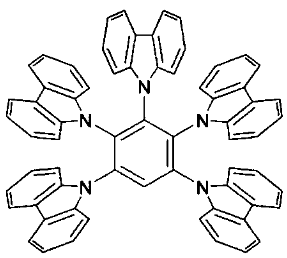

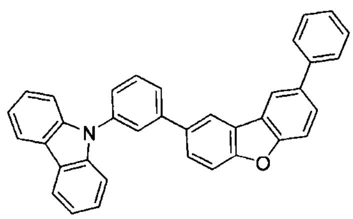

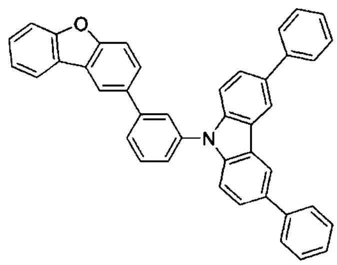

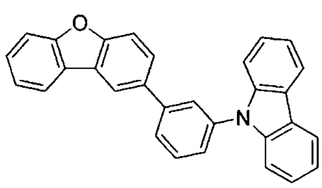

Exciton Barrier Layer:

An exciton barrier layer inhibits excitons generated through recombination of holes and electrons in the light emitting layer from being diffused to the charge transporting layer. In some embodiments, the exciton barrier layer enables

effective confinement of excitons in the light emitting layer. In some embodiments, the light emission efficiency of the device is enhanced. In some embodiments, the exciton barrier layer is adjacent to the light emitting layer on any of the side of the anode and the side of the cathode, and on both the sides. In some embodiments, when the exciton barrier layer is on the side of the anode, the layer can be between the hole transporting layer and the light emitting layer and adjacent to the light emitting layer. In some embodiments, when the exciton barrier layer is on the side of the cathode, the layer can be between the light emitting layer and the cathode and adjacent to the light emitting layer. In some embodiments, a hole injection layer, an electron barrier layer, or a similar layer is between the anode and the exciton barrier layer that is adjacent to the light emitting layer on the side of the anode. In some embodiments, a hole injection layer, an electron barrier layer, a hole barrier layer, or a similar layer is between the cathode and the exciton barrier layer that is adjacent to the light emitting layer on the side of the cathode. In some embodiments, the exciton barrier layer contains excited singlet energy and excited triplet energy, at least one of which is higher than the excited singlet energy and the excited triplet energy of the light emitting material, respectively.

Hole Transporting Layer:

The hole transporting layer includes a hole transporting material. In some embodiments, the hole transporting layer is a single layer. In some embodiments, the hole transporting layer includes a plurality of layers.

In some embodiments, the hole transporting material has one of injection or transporting property of holes and barrier property of electrons. In some embodiments, the hole transporting material is an organic material. In some embodiments, the hole transporting material is an inorganic material. Examples of known hole transporting materials that can be used in the present invention include, but are not limited to, a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an allylamine derivative, an amino-substituted chalcone derivative, an oxazole derivative, a styrylanthracene derivative, a fluorenone derivative, a hydrazone derivative, a stilbene derivative, a silazane derivative, an aniline copolymer and a conductive polymer oligomer (particularly, a thiophene oligomer), or a combination thereof. In some embodiments, the hole transporting material is selected from a porphyrin compound, an aromatic tertiary amine compound, and a styrylamine compound. In some embodiments, the hole transporting material is an aromatic tertiary amine compound. Specific examples of preferred compounds for use as the hole transporting material are shown below.

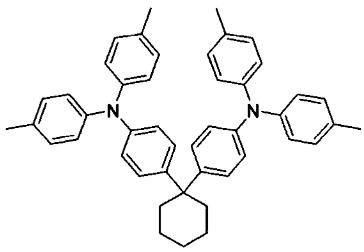
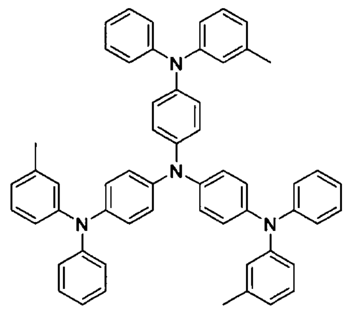
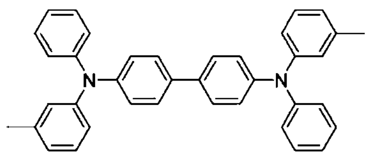
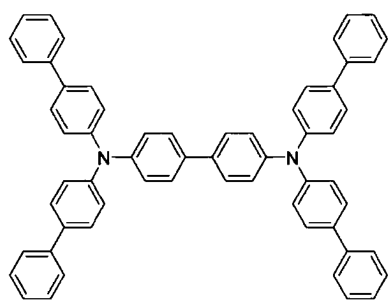
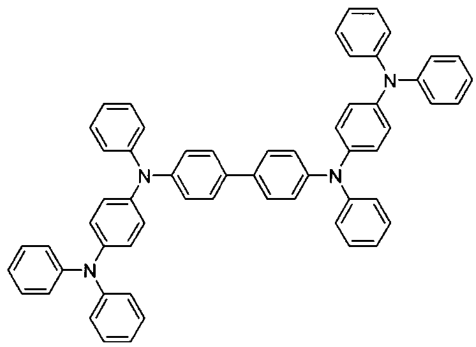

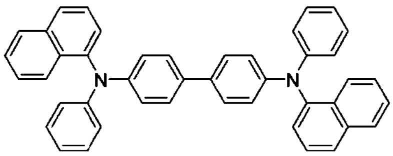
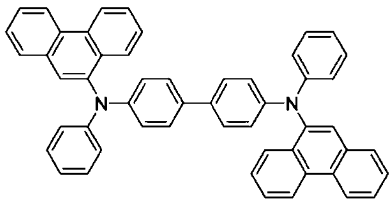

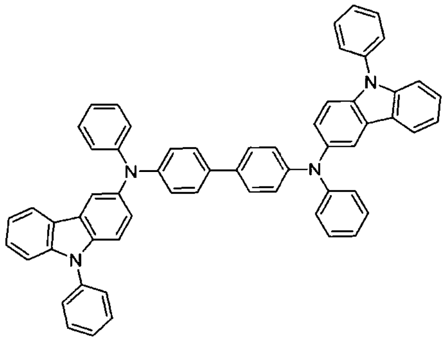
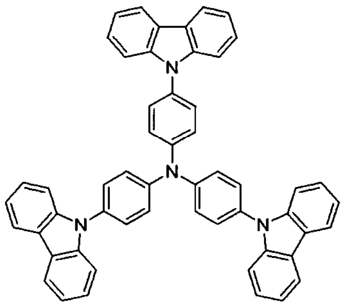
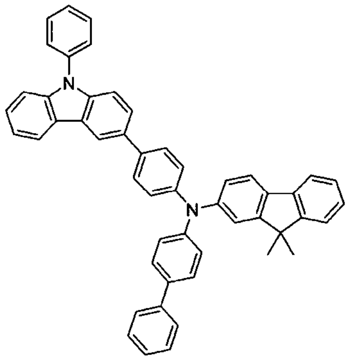

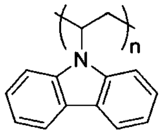
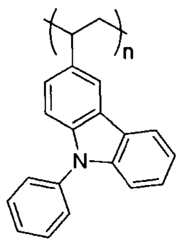
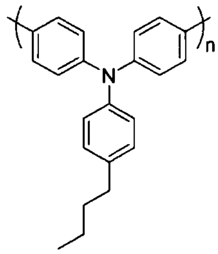

Electron Transporting Layer:

The electron transporting layer includes an electron transporting material. In some embodiments, the electron transporting layer is a single layer. In some embodiments, the electron transporting layer includes a plurality of layers.

In some embodiments, the electron transporting material needs only to have a function of transporting electrons, which are injected from the cathode, to the light emitting layer. In some embodiments, the electron transporting material also functions as a hole barrier material. Examples of the electron transporting layer that can be used in the present invention include but are not limited to a nitro-substituted fluorene derivative, a diphenylquinone derivative, a thiopyran dioxide derivative, carbodiimide, a fluorenylidene methane derivative, anthraquinodimethane, an anthrone derivatives, an oxadiazole derivative, an azole derivative, an azine derivative, or a combination thereof, or a polymer thereof. In some embodiments, the electron transporting material is a thiadiazole derivative, or a quinoxaline derivative. In some embodiments, the electron transporting material is a polymer material. Specific examples of preferred compounds for use as the electron transporting material are shown below.

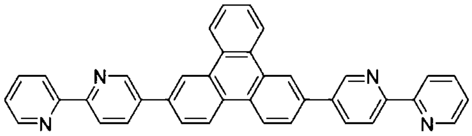
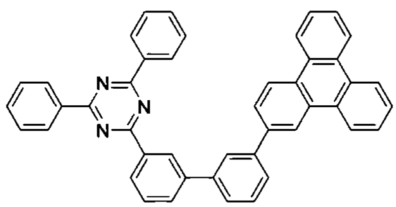

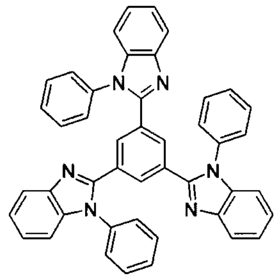
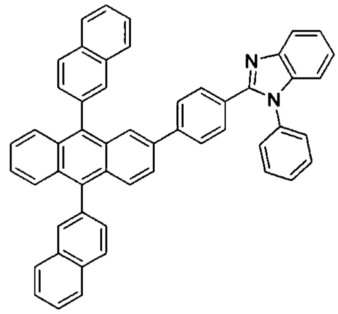
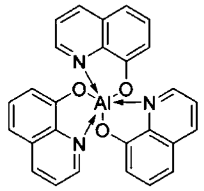

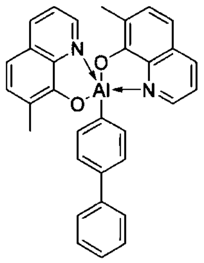
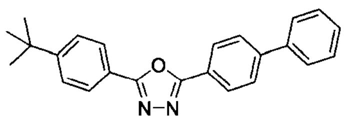
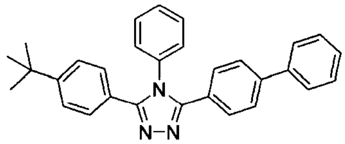

Further, compound examples preferred as a material that can be added to the organic layers are shown. For example, it is conceivable to add these as a stabilization material.

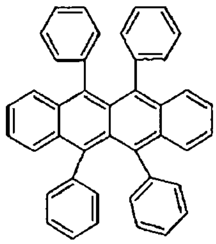
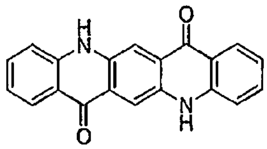
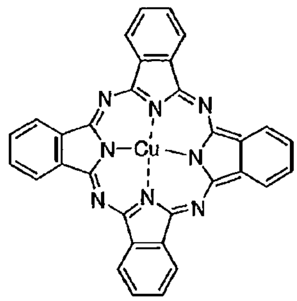

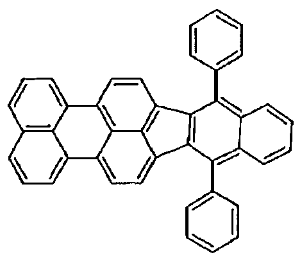
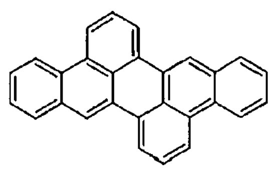

Preferred materials for use in the organic electroluminescent device are specifically shown. However, the materials usable in the present invention should not be limitatively interpreted by the following exemplary compounds. Compounds that are exemplified as materials having a specific function can also be used as materials having any other function.

Devices:

In some embodiments, a light emitting layer is incorporated into a device. For example, the device includes, but is not limited to, an OLED bulb, an OLED lamp, a television screen, a computer monitor, a mobile phone, and a tablet.

In some embodiments, an electronic device includes an OLED including an anode, a cathode, and at least one organic layer including a light emitting layer between the anode and the cathode.

In some embodiments, compositions described herein can be incorporated into various light-sensitive or light-activated devices, such as OLEDs or opto-electronic devices. In some embodiments, the composition can be useful in facilitating charge transfer or energy transfer within a device and/or as a hole transporting material. The device can be, for

example, an organic light emitting diode (OLED), an organic integrated circuit (OIC), an organic field-effect transistor (O-FET), an organic thin-film transistor (O-TFT), an organic light emitting transistor (O-LET), an organic solar cell (O-SC), an organic optical detector, an organic photoreceptor, an organic field-quench device (O-FQD), a light-emitting electrochemical cell (LEC) or an organic laser diode (O-laser).

Bulbs or Lamps:

In some embodiments, an electronic device includes an OLED including an anode, a cathode, and at least one organic layer including a light emitting layer between the anode and the cathode.

In some embodiments, a device includes OLEDs that differ in color. In some embodiments, a device includes an array including a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.

In some embodiments, a device is an OLED light including:

a circuit board having a first surface with a mounting surface and an opposing second surface, and defining at least one opening,
at least one OLED on the mounting surface, the at least one OLED configured to emit light, the OLED including an anode, a cathode, and at least one organic layer including a light emitting layer between the anode and the cathode,
a housing for the circuit board, and
at least one connector arranged at an end of the housing, the housing and the connector defining a package adapted for installation in a light fixture.

In some embodiments, the OLED light includes a plurality of OLEDs mounted on a circuit board such that light emits in a plurality of directions. In some embodiments, a portion of the light emitted in a first direction is deflected to emit in a second direction. In some embodiments, a reflector is used to deflect the light emitted in a first direction.

Displays or Screens:

In some embodiments, the light emitting layer of the present invention can be used in a screen or a display. In some embodiments, the compounds of the present invention are deposited onto a substrate using a process including, but not limited to, vacuum evaporation, deposition, vapor deposition, or chemical vapor deposition (CVD). In some embodiments, the substrate is a photoplate structure useful in a two-sided etching that provides a unique aspect ratio pixel. The screen (which can also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the fine patterning of pixels needed for high resolution displays while optimizing the chemical vapor deposition onto a TFT backplane.

The internal patterning of the pixel allows the construction of a three-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point, the entire pixel area is subjected to a similar etching rate but the depths are varying depending on the halftone pattern. Varying the size and spacing of the halftone pattern allows etching to be inhibited at different rates within the pixel and allow a localized deeper etch needed to create steep vertical bevels.

A preferred material for the deposition mask is invar. Invar is a metal alloy that is cold rolled into a long thin sheet in a steel mill. Invar cannot be electrodeposited onto a rotating mandrel as the nickel mask. A preferred and more cost feasible method for forming the opening areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etching. In further embodiments, a screen or display pattern is fabricated using plasma etching.

Methods of Manufacturing Devices:

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light emitting layer, a counter electrode, and an encapsulation layer, and then is cut

from the mother panel.

In another aspect of the present invention, provided herein is a method of manufacturing an organic light emitting diode (OLED) display, the method including:

a step of forming a barrier layer on a base substrate of a mother panel,
a step of forming a plurality of display units in units of cell panels on the barrier layer,
a step of forming an encapsulation layer on each of the display units of the cell panels, and
a step of applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer includes a light emitting layer, a gate electrode, and a source/drain electrode. Each of the plurality of display units may include a thin film transistor (TFT) layer, a planarization film formed on the TFT layer, and a light emitting unit formed on the planarization film, in which the organic film applied to the interface portion is formed of a material same as a material of the planarization film and is formed at a same time as the planarization film is formed. In some embodiments, a light emitting unit is connected to the TFT layer with a passivation layer and a planarization film therebetween and an encapsulation layer that covers and protects the light emitting unit. In some embodiments of the method of manufacturing, the organic film contacts neither the display units nor the encapsulation layer.

Each of the organic film and the planarization film can include any one of polyimide and acryl. In some embodiments, the barrier layer can be an inorganic film. In some embodiments, the base substrate can be formed of polyimide. The method can further include a step of, before the forming of the barrier layer on one surface of the base substrate formed of polyimide, attaching a carrier substrate formed of a glass material to another surface of the base substrate, and a step of, before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is manufactured. In some embodiments, the organic film can be formed on the edge portion of the barrier layer such that a portion of the organic film directly contacts the base substrate and a remaining portion of the organic film contacts the barrier layer while surrounding the edge portion of the barrier layer.

In some embodiments, the light emitting layer includes a pixel electrode, a counter electrode, and an organic light emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light emitting layer emits light, thereby forming an image. Hereinafter, an image forming unit including the TFT layer and the light emitting unit is referred to as a display unit.

In some embodiments, the encapsulation layer that covers the display unit and prevents penetration of external moisture can be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further includes a step of cutting along the interface portion, a groove is formed in the barrier layer, at least a portion of the organic film is formed in the groove, and the groove does not penetrate into the base substrate. In some embodiments, the TFT layer of each of the cell panels is formed, and the

passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, when the entire barrier layer is entirely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels can be softly cut and cracks can be prevented from occurring in the barrier layer. In one embodiment, the organic film covering the groove at the interface portion and the planarization film are spaced apart from each other. For example, when the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light emitting unit, and the encapsulation layer is disposed on the display unit to cover the display unit. Accordingly, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks can be prevented from occurring in the barrier layer during the cutting.

In some embodiments, the methods reduce a defect rate of a product and stabilize a quality thereof.

Another aspect is an OLED display including a barrier layer that is formed on a base substrate, a display unit that is formed on the barrier layer, an encapsulation layer that is formed on the display unit, and an organic film that is applied to an edge portion of the barrier layer.

Examples

The features of the present invention will be described more specifically with reference to Synthesis Examples and Examples given below. The materials, processes, procedures and the like shown below can be appropriately modified unless they deviate from the substance of the present invention. Accordingly, the scope of the present invention is not construed as being limited to the specific examples shown below. The light emission characteristics were evaluated using a source meter (available from Keithley Instruments, Inc.: 2400 series), a semiconductor parameter analyzer (available from Agilent Technologies, Inc., E5273A), an optical power meter device (available from Newport Corporation, 1930C), an optical spectroscope (available from Ocean Optics Corporation, USB2000), a spectroradiometer (available from Topcon Corporation, SR-3), and a streak camera (available from Hamamatsu Photonics K.K., Model C4334). The energies of HOMO and LUMO were measured by photoelectron spectroscopy in air (such as AC-3 available from Riken Keiki Co., Ltd.).

In the following Synthesis Examples, compounds included in the general formula (1) were synthesized.

(Synthesis Example 1) Synthesis of Compound 46542

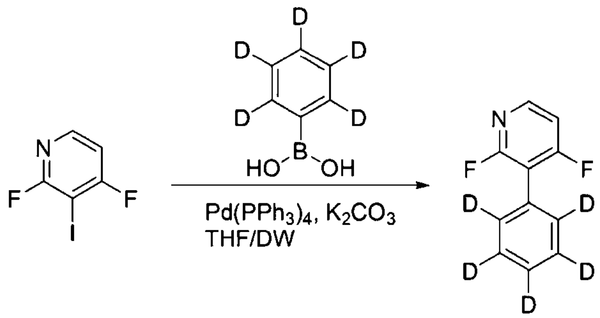


**a**

Compound a

Under a nitrogen atmosphere, a solution of distilled water and tetrahydrofuran (200 mL) in which (phenyl-d5) boronic acid (3.16 g, 24.90 mmol), 2,4-difluoro-3-iodopyridine (5.00 g, 20.75 mmol), potassium carbonate (8.60 g, 62.25 mmol, 2M aqueous solution), and tetrakis(triphenylphosphine)palladium(0) (3 mol%) were dissolved was refluxed for 12 hours. The reaction solution was cooled to room temperature and extracted with methylene chloride and distilled water, and the solvent of the organic layer was then distilled off with an evaporator. The obtained residue was purified by silica gel column chromatography using, as an eluent, a mixed solvent of chloroform:hexane=1:1 to obtain white solid Compound a (2.80 g, 14.27 mmol, yield 68.8%).

$^1$H NMR (400 $MH_Z$, $CDCl_3$) $\delta$ 8.1675 (dd, J = 8.0 $H_Z$, 6.0 $H_Z$, 1H), 7.0725 (dd, J = 8.2 $H_Z$, 5.2 Hz, 1H)
ASAP MS Spectral Analysis: C11H2DSF2N: theoretical value 196, observed value 197.

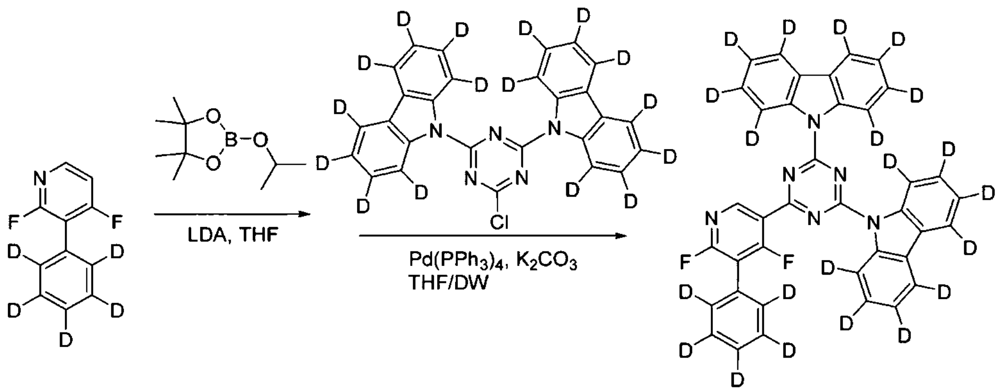


**a**

**b**

Compound b

Under a nitrogen stream at -85°C, a 1M lithium diisopropylamide solution (3.57 mL, 3.57 mmol) was gradually added to a tetrahydrofuran solution (15 mL) of Compound a (0.70 g, 3.57 mmol) and 2-isopropoxy-4,4,5,5-tetramethyl-1,3,2-dioxaborane (0.66 g, 3.57 mmol). After 10 minutes, the mixture was heated to -75°C, stirred for 1 hour, and then slowly returned to room temperature. To the mixture, 9,9'-(6-chloro-1,3,5-triazine-2,4-diyl)bis(9H-carbazole-1,2,3,4,5,6,7,8-d8) (1.98 g, 4.28 mmol), a 2M aqueous potassium carbonate solution (2.68 mL, 5.35 mmol), tetrahydrofuran (100 mL), and tetrakis(triphenylphosphine)palladium(0) (0.12 g, 0.11 mmol) were added, followed by heating to reflux for 12 hours. The reaction solution was returned to room temperature, water was added to terminate the reaction, extraction was then performed with chloroform, and the solvent was distilled off with an evaporator. The obtained residue was purified by silica gel column chromatography using, as an eluent, a mixed solvent of hexane:toluene = 1:1 to obtain white solid Compound b (1.00 g, 1.61 mmol, yield 45.1%).

$^1$H NMR (400 $MH_Z$, $CDCl_3$) $\delta$ 9.3635 (d, J = 9.2 $H_Z$, 1H).

ASAP MS Spectral Analysis: C38HD21F2N6: theoretical value 621, observed value 622.

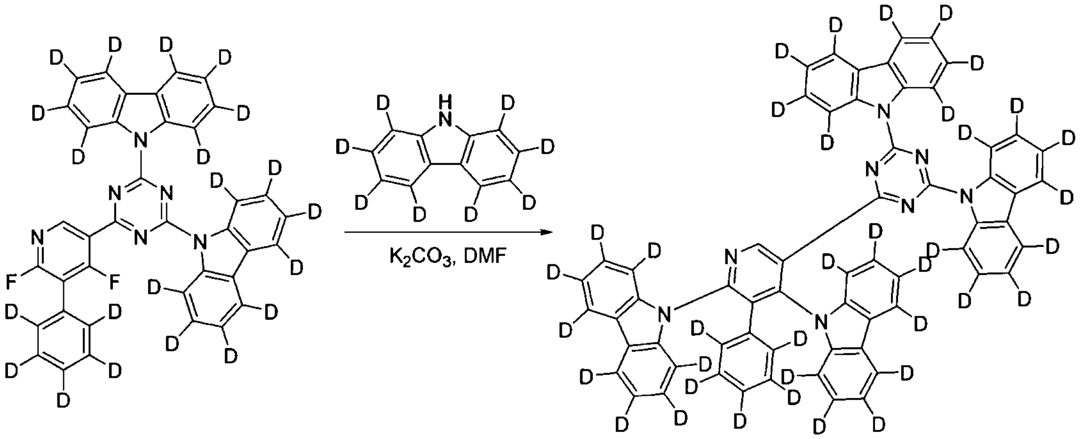


b

46542

Compound 46542

Under a nitrogen stream, a dimethylformamide solution (50 mL) in which 9H-carbazole-1,2,3,4,5,6,7,8-d8 (0.76 g, 4.34 mmol), potassium carbonate (0.80 g, 5.79 mmol), and Compound b (0.9 g, 1.45 mmol) were dissolved was stirred at 120°C overnight. The reaction product was returned to room temperature, a saturated ammonium chloride solution was added to terminate the reaction, extraction was then performed with chloroform, and the solvent was distilled off with an evaporator. The obtained residue was purified by silica gel column chromatography using, as an eluent, a mixed solvent of toluene and hexane to obtain Compound 46542 (0.83 g, 0.89 mmol, yield 61.5%).

$^1H$ NMR (400 $MH_Z$, $CDCl_3$) $\delta$ 9.632 (s, 1H).

ASAP MS Spectral Analysis: C62HD37N8: theoretical value 931, observed value 932.

(Synthesis Example 2) Synthesis of Compound 46542(40)

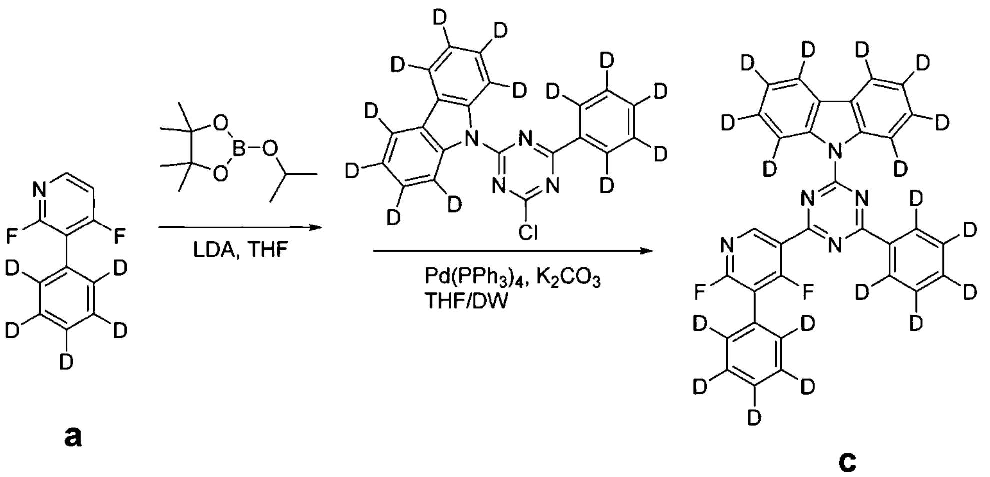


a

c

Compound c

Under a nitrogen stream at -85°C, a 1M diisopropylamide lithium solution (5.10 mL, 5.10 mmol) was gradually added to a tetrahydrofuran solution (15 mL) of Compound a (1.00 g, 5.09 mmol) and 2-isopropoxy-4,4,5,5-tetramethyl-1,3,2-dioxaborane (0.95 g, 5.10 mmol). After 10 minutes, the mixture was heated to -75°C, stirred for 1 hour, and then slowly returned to room temperature. To the mixture, 9-(4-chloro-6-(phenyl-d5)-1,3,5-triazin-2-yl)-9H-carbazole-1,2,3,4,5,6,7,8-d (2.26 g, 6.11 mmol), a 2M aqueous potassium carbonate solution (3.82 mL, 7.64 mmol), tetrahydrofuran (60 mL), and tetrakis(triphenylphosphine)palladium(0) (0.18 g, 0.15 mmol) were added, followed by heating to

reflux for 12 hours. The reaction solution was returned to room temperature, water was added to terminate the reaction, extraction was then performed with chloroform, and the solvent was distilled off with an evaporator. The obtained residue was purified by silica gel column chromatography using, as an eluent, a mixed solvent of hexane:toluene = 1:1 to obtain white solid Compound c (2.40 g, 4.53 mmol, yield 89.0%).

$^{1}$H NMR (400 $MH_Z$, $CDCl_3$) δ 9.34 (d, J = 9.2 $H_Z$, 1H).

ASAP MS Spectral Analysis: C32HD18F2N: theoretical value 529, observed value 530.

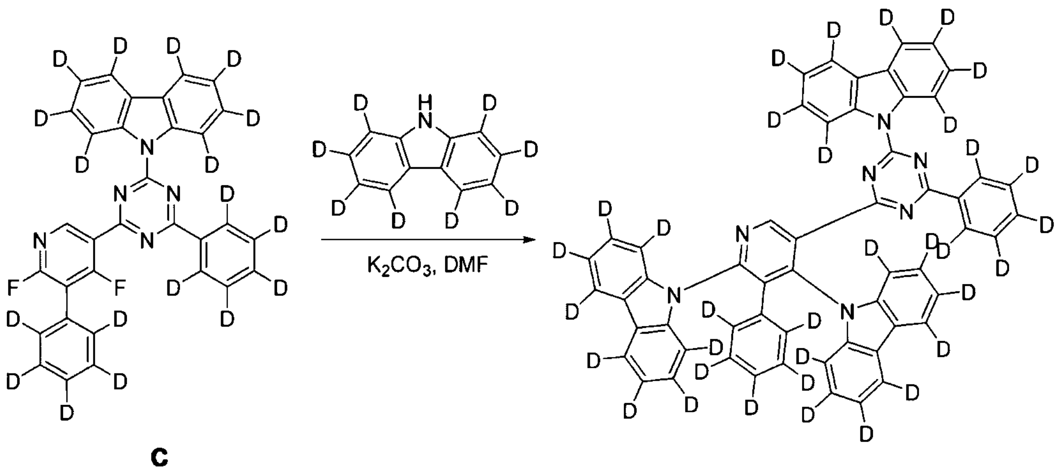


c

**46542(40)**

Compound 46542(40)

Under a nitrogen stream, a dimethylformamide solution (50 mL) in which 9H-carbazole-1,2,3,4,5,6,7,8-d8 (1.50 g, 8.50 mmol), potassium carbonate (1.57 g, 11.33 mmol) and Compound c (1.5 g, 2.83 mmol) were dissolved was stirred at 120°C overnight. The reaction product was returned to room temperature, a saturated ammonium chloride solution was added to terminate the reaction, extraction was then performed with chloroform, and the solvent was distilled off with an evaporator. The obtained residue was purified by silica gel column chromatography using, as an eluent, a mixed solvent of toluene and hexane to obtain Compound 46542(40) (2.20 g, 2.62 mmol, yield 92.5%).

$^{1}$H NMR (400 $MH_Z$, $CDCl_3$) δ 9.725 (s, 1H).

ASAP MS Spectral Analysis: C56HD34N7: theoretical value 839, observed value 840.

(Comparative Synthesis Example 1) Synthesis of Comparative Compound 2

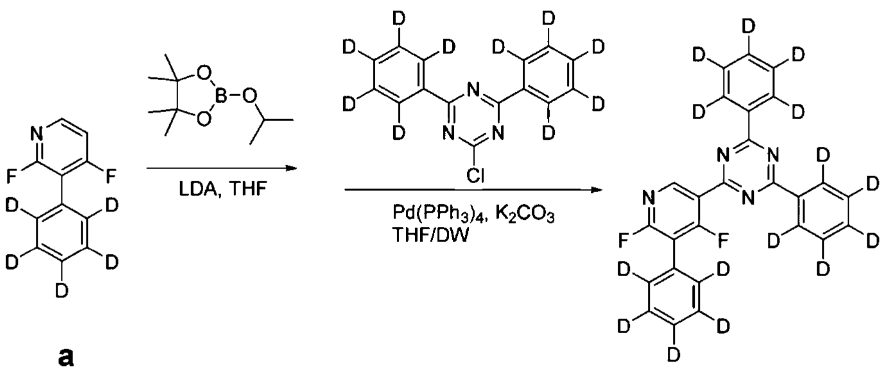


a

d

Compound d

Under a nitrogen stream at -85°C, a 1M diisopropylamide lithium solution (5.10 mL, 5.10 mmol) was gradually

added to a tetrahydrofuran solution (15 mL) of Compound a (1.0 g, 5.10 mmol) and 2-isopropoxy-4,4,5,5-tetramethyl-1,3,2-dioxaborane (0.95 g, 5.10 mmol). After 10 minutes, the mixture was heated to -75°C, stirred for 1 hour, and then slowly returned to room temperature. To the mixture, 2-chloro-4,6-bis(phenyl-d5)-1,3,5-triazine (1.56 g, 5.60 mmol), a 2 M aqueous potassium carbonate solution (3.82 mL, 7.64 mmol), tetrahydrofuran (20 mL), and tetrakis(triphenylphosphine)palladium(0) (0.18 g, 0.15 mmol) were added, followed by heating to reflux for 12 hours. The reaction solution was returned to room temperature, water was added to terminate the reaction, extraction was then performed with chloroform, and the solvent was distilled off with an evaporator. The obtained residue was purified by silica gel column chromatography using, as a solution, a mixed solvent of hexane:toluene=1:1 to obtain white solid Compound d (2.00 g, 4.57 mmol, yield 89.8%).

$^1H$ NMR (400 $MH_Z$, $CDCl_3$) $\delta$ 9.34 (d, J = 9.2 $H_Z$, 1H).

ASAP MS Spectral Analysis: C26HD15F2N: theoretical value 437, observed value 438.

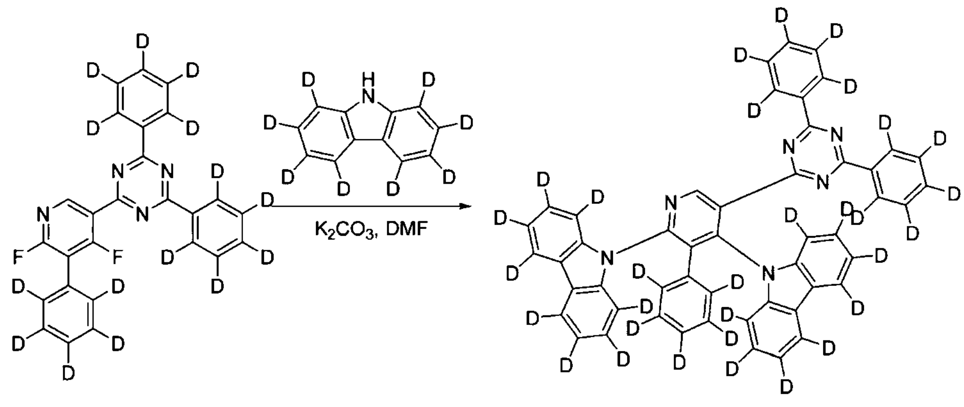


d

Comparative Compound 2

Comparative Compound 2

Under a nitrogen stream, a dimethylformamide solution (50 mL) in which 9H-carbazole-1,2,3,4,5,6,7,8-d8 (1.80 g, 10.29 mmol), potassium carbonate (1.90 g, 13.71 mmol) and compound d (1.5 g, 3.43 mmol) were dissolved was stirred at 120°C overnight. The reaction product was returned to room temperature, a saturated ammonium chloride solution was added to terminate the reaction, extraction was then performed with chloroform, and the solvent was distilled off with an evaporator. The obtained residue was purified by silica gel column chromatography using, as an eluent, a mixed solvent of toluene and hexane to obtain Comparative Compound 2 (2.45 g, 3.28 mmol, yield 95.5%).

$^1H$ NMR (400 $MH_Z$, $CDCl_3$) $\delta$ 9.705 (s, 1H).

ASAP MS Spectral Analysis: C50HD31N6: theoretical value 747, observed value 748.

(Synthesis Example 3) Synthesis of Compound 38317(40)

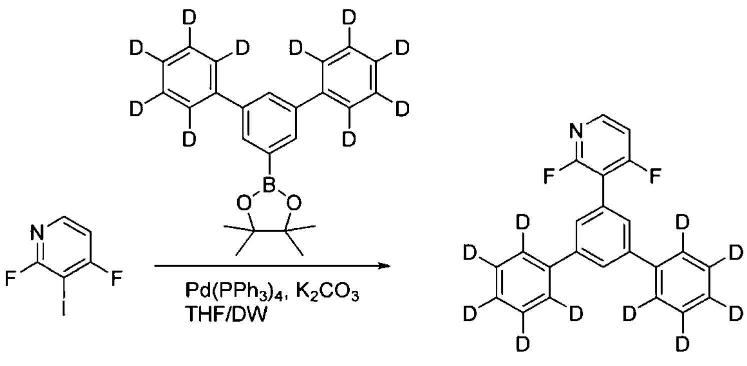


e

Compound e

In tetrahydrofuran (20 mL) and distilled water (depending on the amount of potassium carbonate), 2-([1,1':3',1"-terphenyl]-5'-yl-2,2",3,3",4,4",5,5",6,6"-d10)-4,4,5,5-tetramethyl-1,3,2-dioxaborane (2.52 g, 6.88 mmol), 2,4-difluoro-3-iodopyridine (1.66 g, 6.88 mmol), potassium carbonate (1.43 g, 10.32 mmol, 2 M aqueous solution), and $Pd(PPh_3)_4$ (3 mol%) were dissolved. The solution was refluxed under a nitrogen atmosphere for 12 hours and then cooled to room temperature. The solution was extracted with methylene chloride and distilled water. The solution of the organic layer was evaporated under vacuum and purified with chloroform:hexane (1:1). White solid Compound e (2.30 g, 6.507 mmol, yield 94.6%) was obtained.

$^1$H NMR (400 $MH_Z$, $CDCl_3$) $\delta$ 8.213 (dd, J = 8.0 $H_Z$, 5.6 $H_Z$, 1H), 7.868 (t, J = 2.0 $H_Z$, 1H), 7.676 (q, J = 1.6 $H_Z$, 2H), 7.12 (dd, J = 8.0 $H_Z$, 5.6 $H_Z$, 1H)
ASAP MS Spectral Analysis: C23H5D10F2N: theoretical value 353, observed value 354.

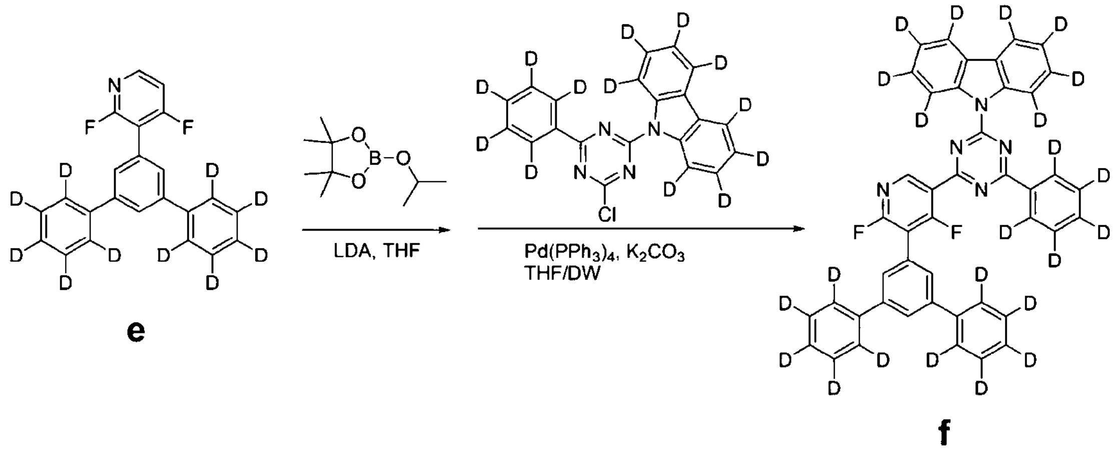


Compound f

Under a nitrogen stream, a 1M lithium diisopropylamide solution (2.83 mL, 2.83 mmol) was gradually added to a tetrahydrofuran solution (15 mL) of Compound e (1.0 g, 2.83 mmol) and 2-isopropoxy-4,4,5,5-tetramethyl-1,3,2-dioxaborane (0.53 g, 2.83 mmol) that was cooled to -85°C. After 10 minutes, the mixture was heated to -75°C and stirred for 1 hour. After the mixture was slowly returned to room temperature, 9-(4-chloro-6-(phenyl-d5)-1,3,5-triazin-2-yl)-9H-carbazole-1,2,3,4,5,6,7,8-d8 (1.15 g, 3.12 mmol), a 2M aqueous potassium carbonate solution (2.13 mL, 4.26 mmol), tetrahydrofuran (100 mL), and tetrakis(triphenylphosphine)palladium(0) (0.10 g, 0.09 mmol) were added, followed by heating to reflux for 12 hours. The reaction solution was returned to room temperature, then quenched by adding water, and extracted with chloroform. The solvent was distilled off with an evaporator and the residue was purified by silica gel column chromatography (hexane: toluene = 1: 1) to obtain white solid Compound f(1.28 g, 1.864 mmol, yield 65.7%).
$^1$H NMR (400 $MH_Z$, $CDCl_3$) $\delta$ 9.4025 (d, J = 9.2 $H_Z$, 1H), 7.953 (t, J = 1.6 $H_Z$, 1H), 7.812 (m, 2H).
ASAP MS Spectral Analysis: C44H4D23F2N5: theoretical value 686, observed value 687.

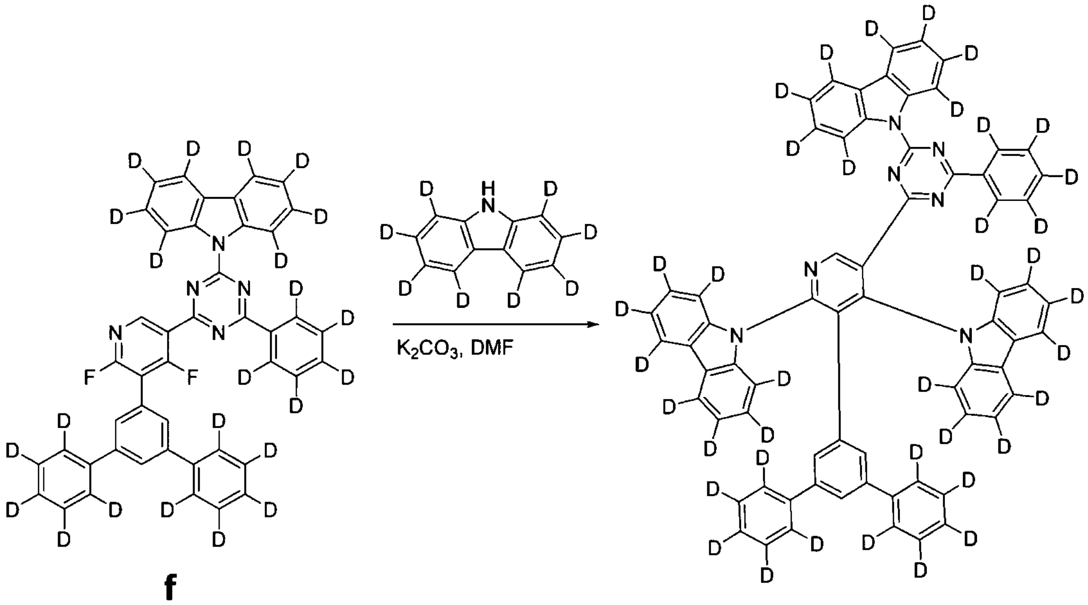


f

38317(40)

Compound 38317(40)

Under a nitrogen stream, a dimethylformamide solution (50 mL) of 9H-carbazole-1,2,3,4,5,6,7,8-d8 (0.77 g, 4.37 mmol), potassium carbonate (0.72 g, 5.24 mmol), and Compound f (1.2 g, 1.75 mmol) was stirred at 120°C overnight. The mixture was returned to room temperature, then quenched with a saturated ammonium chloride solution, and extracted with chloroform. The solvent was distilled off with an evaporator, and the residue was purified by silica gel column chromatography (toluene:hexane) to obtain Compound 38317(40) (1.70 g, 1.704 mmol, yield 97.6%).

$^1$H NMR (400 $MH_Z$, $CDCl_3$) $\delta$ 9.860 (s, 1H), 6.989 (t, J = 1.6 $H_Z$, 1H), 6.833 (d, J= 1.6 $H_Z$, 2H)
ASAP MS Spectral Analysis: C50HD31N6: theoretical value 997, observed value 998.

(Synthesis Example 4) Synthesis of Compound 46542 (1456)

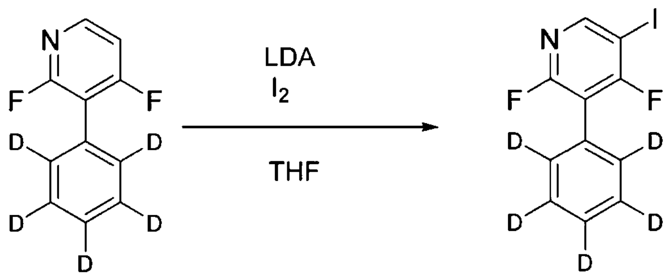


a

g

Compound g

Under a nitrogen atmosphere at -85°C, a tetrahydrofuran/hexane solution (9.68 mL, 9.68 mmol) of 1.0M lithium diisopropylamide was gradually dropwise added to a tetrahydrofuran solution (12.1 mL) of Compound a (1.9 g, 9.68 mmol). After one hour, tetrahydrofuran (4.8 ml) in which iodine (2.46g, 9.68 mmol) was dissolved was gradually added, and the mixture was returned to room temperature, and then quenched by addition of an aqueous sodium thiosulfate solution (10% by weight). Thereafter, the mixture was extracted with ethyl acetate and washed with saturated saline. The extract was

dried over anhydrous magnesium sulfate and filtered, and the obtained filtrate was concentrated. By recrystallization from methylene chloride, white solid Compound g (1.72g, 5.3 mmol, yield 55.1%) was obtained.

$^1$H NMR (400 $MH_Z$, $CDCl_3$) δ 8.43 (dd, J = 8.4 $H_Z$,7.2, 1H)
ASAP MS Spectral Analysis: C11HD5F2IN: theoretical value 321, observed value 323.

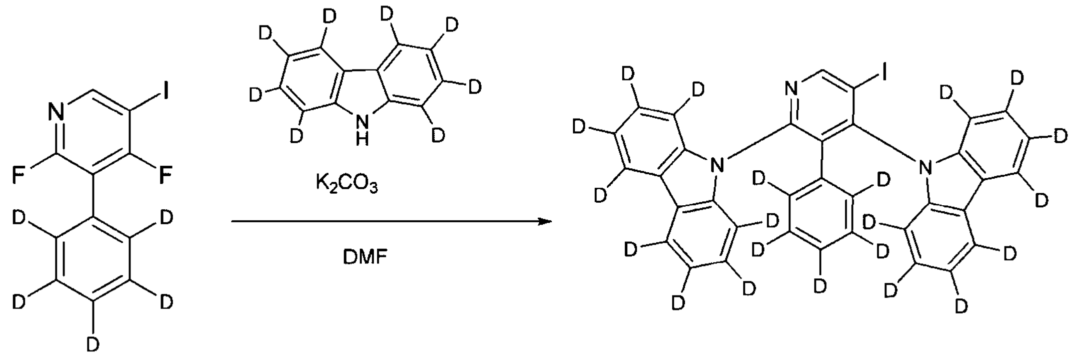


g h

Compound h

Under a nitrogen atmosphere, carbazole-1,2,3,4,5,6,7,8-d8 (1.95 g, 11.1 mmol) and potassium carbonate (1.93 g, 13.9 mmol) were added to a mixture of Compound g (1.5 g, 4.65 mmol) and dimethylformamide (46 mL), and the mixture was stirred at 100°C for 15 hours. The reaction solution was cooled to room temperature, ion-exchanged water was added, methanol was added, and the mixture was filtered. The obtained solid was reprecipitated with chloroform/methanol, washed with ethyl acetate under heating, and filtered. The obtained solid was reprecipitated with chloroform/hexane to obtain white solid Compound h (1.5 g, 2.3 mmol, yield 50.8%).

$^1$H NMR (400 $MH_Z$, $CDCl_3$) δ 9.26 (d, J = 6.4 $H_Z$, 1H)
ASAP MS Spectral Analysis: C35HD21IN3: theoretical value 632, observed value 633.

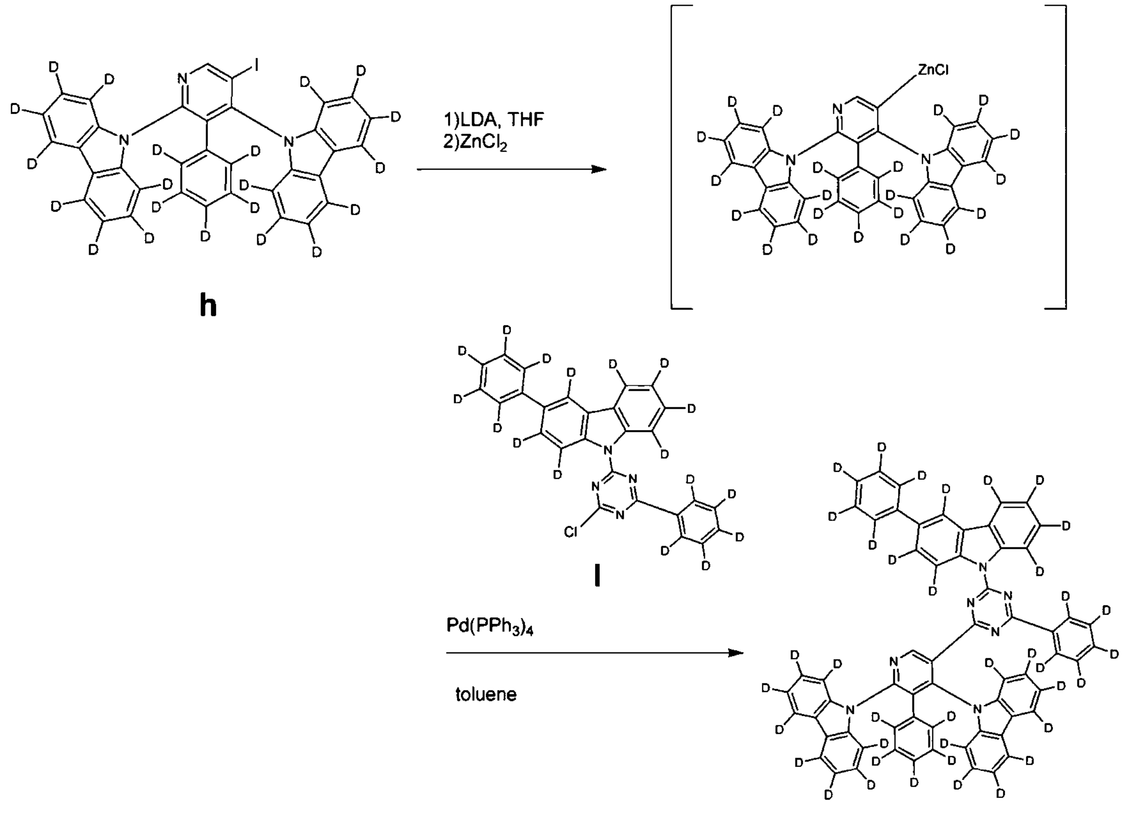


h

l

**46542(1456)**

Compound 46542(1456)

Under a nitrogen atmosphere at -78°C, a tetrahydrofuran/hexane solution (2.6 mL, 2.6 mmol) of 1.0M lithium diisopropylamide was slowly added dropwise to a tetrahydrofuran solution (9.5 mL) of Compound h (1.5 g, 2.3 mmol). After stirring for 30 minutes, a tetrahydrofuran solution (7.1 mL, 7.1 mmol) of 1M zinc chloride was added, and the mixture was heated to room temperature. After stirring for 1 hour, Compound l (1.54 g, 2.3 mmol), tetrakis(triphenylphosphine) palladium(0) (0.14 g, 0.11 mmol) and toluene (47 ml) were added, followed by refluxing for 16 hours. After that, the mixture was cooled to room temperature, ion-exchanged water and methanol were added, and the obtained solid was filtered and washed with ethyl acetate, toluene, and methylene chloride to obtain yellow solid Compound 46542(1456) (1.16 g, 1.26 mmol, yield 46%).

$^1$H NMR (400 $MH_Z$, $CDCl_3$) $\delta$ 9.72 (s, 1H)
ASAP MS Spectral Analysis: C62HD38N7: theoretical value 919, observed value 920.

(Example 1) Preparation and Evaluation of Thin Film

Compound 46542 was vapor-deposited on a quartz substrate by a vacuum deposition method under conditions of a vacuum degree of less than $1 \times 10^{-3}$ Pa to form a neat thin film of Compound 46542 having a thickness of 100 nm.

Separately, Compound 46542 and PyD2Cz having the following structure were vapor-deposited from different vapor deposition sources on a quartz substrate by a vacuum deposition method under conditions of a vacuum degree of less than $1 \times 10^{-3}$ Pa to form a doped thin film having a content of Compound 46542 of 20% by weight and a thickness of 100 nm.

In the same manner but using Compound 46542(40), Compound 38317(40), Compound 46542(1456), Comparative Compound 1, and Comparative Compound 2 in place of Compound 46542, neat thin films and doped thin films were formed.

The percentage of the delayed fluorescent component and the lifetime ($\tau$2) of the delayed fluorescent component were measured by analyzing the photoluminescence of each doped thin film formed when irradiated with 300 nm excitation light. Also, using the formed neat thin films, the HOMO energy and the LUMO energy were measured. The results are shown in the following Table. As shown in the following Table, it was confirmed that the compound represented by the

general formula (1) has a delayed fluorescence lifetime ($\tau 2$) shorter than those of Comparative Compounds 1 and 2.

[Table 6]

| | Percentage (%) of Delayed Fluorescent Component | $\tau 2$ (nanosecond) | HOMO (eV) | LUMO (eV) | Emission Maximum Wavelength (nm) |
|---|---|---|---|---|---|
| Compound 46542 | 79.4 | 2008 | 6.07 | 3.19 | 491 |
| Compound 46542(40) | 90.7 | 2980 | 6.01 | 3.20 | 485 |
| Compound 38317(40) | 91.9 | 3156 | 6.02 | 3.23 | 486 |
| Compound 46542(1456) | 89.8 | 2652 | 6.03 | 3.16 | 487 |
| Comparative Compound 1 | 78.5 | 4224 | 5.95 | 3.05 | 482 |
| Comparative Compound 2 | 91.8 | 3607 | 5.92 | 3.04 | 482 |

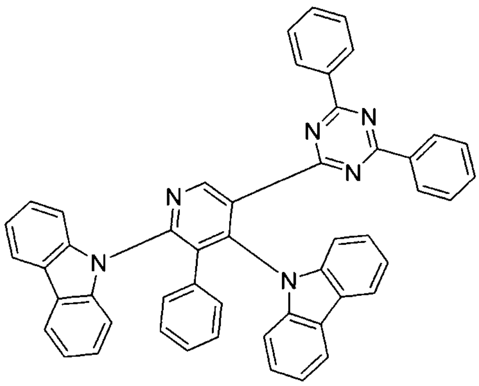

Comparative Compound 1

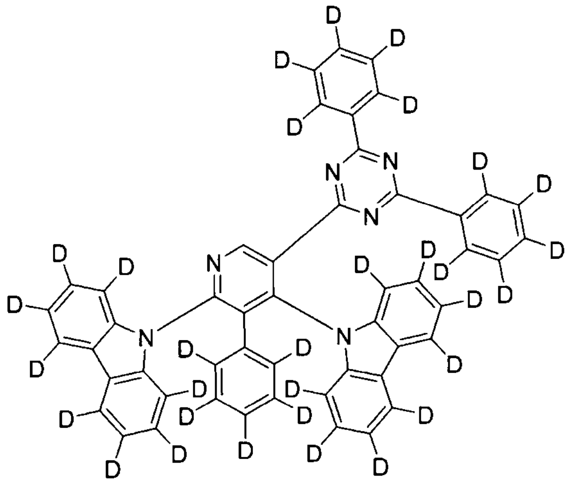

Comparative Compound 2

(Example 2) Production and Evaluation of Organic Electroluminescent Device

On a glass substrate on which an anode made of indium-tin oxide (ITO) having a film thickness of 50 nm was formed, each thin film was laminated by a vacuum deposition method at a vacuum degree of $5.0 \times 10^{-5}$ Pa. First, HATCN was formed to a thickness of 10 nm on the ITO, NPD was formed to a thickness of 30 nm thereon, further TrisPCz was formed to a thickness of 10 nm, and further DFCz was formed to a thickness of 5 nm. Next, DFCz and Compound 46542 were co-deposited from different vapor deposition sources to form a layer with a thickness of 40 nm as a light emitting layer. The content of Compound 46542 in the light emitting layer was 30% by weight. Next, after SF3TRZ was formed at a thickness of 10 nm, Liq and SF3TRZ were co-deposited from different vapor deposition sources to form a layer with a thickness of 30 nm. The contents of Liq and SF3TRZ in this layer were 30% by weight and 70% by weight, respectively. Further, Liq was formed to a thickness of 2 nm, and aluminum (Al) was vapor-deposited to a thickness of 100 nm to form a cathode, thereby obtaining an organic electroluminescent device.

Organic electroluminescent devices were produced in the same manner except that Compound 46542(40), Compound 38317(40), Compound 46542(1456), Comparative Compound 1, and Comparative Compound 2 were used instead of Compound 46542.

The lapse time (LT95) until the luminescence intensity reached 95% of that at the start of the test when each organic electroluminescent device was driven at 2.0 mA/$cm^2$ was measured. The results are shown in the following Table. LT95 in the Table is expressed as a relative value, when LT95 of Comparative Compound 2 is defined as a standard (1). LT95 of each organic electroluminescent device using the compound represented by the general formula (1) was confirmed to be long and the device lifetime was improved.

[Table 7]

| | LT95 |
|---|---|
| Compound 46542 | 3.3 |
| Compound 46542(40) | 2.6 |
| Compound 38317(40) | 3.3 |
| Compound 46542(1456) | 5.8 |
| Comparative Compound 1 | 0.78 |
| Comparative Compound 2 | 1 |

(Example 3) Production and Evaluation of Organic Electroluminescent Device Using Assist Dopant

An organic electroluminescent device was produced in the same manner as in Example 2 only except that a light emitting layer having a thickness of 40 nm was formed by depositing DFCz, Compound 46542 and EM1 as a light emitting material in order of 69.5% by weight, 30.0% by weight and 0.5% by weight from different evaporation sources in place of the light emitting layer in Example 2.

Organic electroluminescent devices were produced in the same manner except that Compound 46542(40), Compound 38317(40), Compound 46542(1456), Comparative Compound 1, and Comparative Compound 2 were used instead of Compound 46542.

Device lifetime is also improved when the compound represented by the general formula (1) is used as an assist dopant.

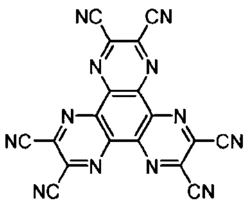
HATCN

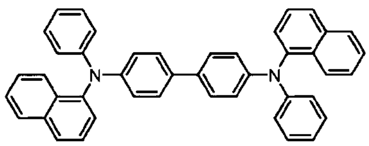
NPD

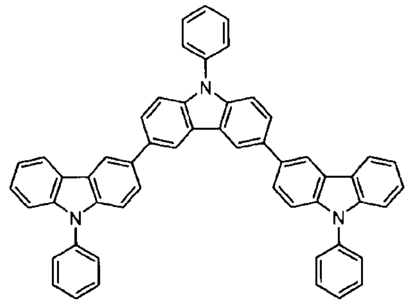
TrisPCz

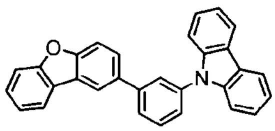
DFCz

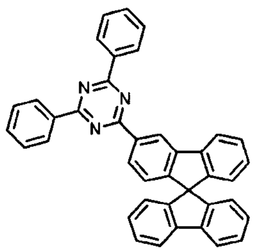
SF3TRZ

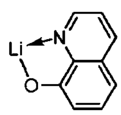
Liq

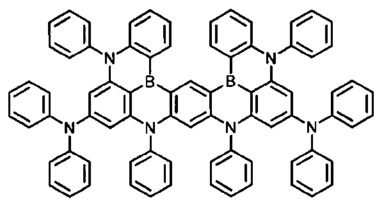
EM1

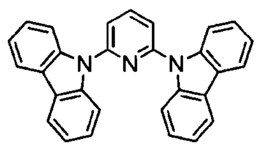
PyD2Cz

Industrial Applicability

By using a compound represented by the general formula (1), there can be provided an organic light emitting device having good light emission characteristics. Accordingly, the industrial applicability of the present invention is great.

## Claims

1. A compound represented by the following general formula (1):

General Formula (1)

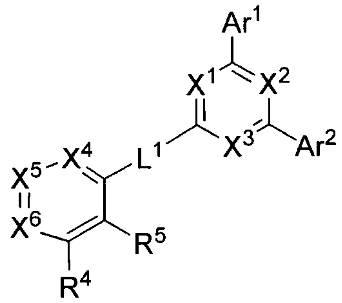

where $X^1$ to $X^3$ each independently represent N or C(R), and at least one of $X^1$ to $X^3$ represents N, R represents a hydrogen atom, a deuterium atom or a substituent, $Ar^1$ and $Ar^2$ each independently represent a substituted or unsubstituted aryl group or a substituted or unsubstituted heteroaryl group containing a nitrogen atom as a ring skeleton-constituting atom, and at least one of $Ar^1$ and $Ar^2$ represents a substituted or unsubstituted heteroaryl group bonding via the nitrogen atom, $L^1$ represents a single bond or a divalent linking group, and $X^4$ represents N or $C(R^1)$, $X^5$ represents N or $C(R^2)$, and $X^6$ represents N or $C(R^3)$, but only one of $X^4$ to $X^6$ represents N, $R^1$ to $R^5$ each independently represent a hydrogen atom, a deuterium atom, a cyano group, a substituted or unsubstituted alkyl group, a substituted or unsubstituted aryl group, or a donor group, one or more of $R^1$ to $R^5$ represent a donor group, zero to two of $R^1$ to $R^5$ represent a hydrogen atom or a deuterium atom, and zero or one of $R^1$ to $R^5$ represents a substituted or unsubstituted aryl group.

2. The compound according to claim 1, wherein
$X^5$ represents N.

3. The compound according to claim 1, wherein
$X^6$ represents N.

4. The compound according to claim 1, wherein
only one of $R^1$ to $R^5$ represents a substituted or unsubstituted aryl group.

5. The compound according to claim 4, wherein
$R^4$ represents a substituted or unsubstituted aryl group.

6. The compound according to claim 1, wherein
one of $R^1$ to $R^5$ represents a donor group.

7. The compound according to claim 1, wherein
two of $R^1$ to $R^5$ represent a donor group.

8. The compound according to claim 1, wherein
three of $R^1$ to $R^5$ represent a donor group.

9. The compound according to claim 1, wherein
the donor group is a substituted or unsubstituted carbazol-9-yl group.

10. The compound according to claim 2, wherein
$R^3$ to $R^5$ each independently represent a substituted or unsubstituted aryl group, or a donor group.

11. The compound according to claim 3, wherein
$R^2$, $R^4$ and $R^5$ each independently represent a substituted or unsubstituted aryl group, or a donor group.

12. The compound according to claim 1, wherein

$X^1$ to $X^3$ represent N.

**13.** The compound according to claim 1, wherein
$Ar^1$ represents a substituted or unsubstituted carbazol-9-yl group, and $Ar^2$ represents a substituted or unsubstituted aryl group.

**14.** The compound according to claim 1, wherein
$Ar^1$ and $Ar^2$ each independently represent a substituted or unsubstituted carbazol-9-yl group.

**15.** The compound according to claim 1, wherein
$L^1$ represents a single bond.

**16.** The compound according to claim 2, wherein
$R^1$ represents a hydrogen atom.

**17.** The compound according to claim 1, wherein
the compound has at least one deuterium atom.

**18.** A light emitting material comprising:
the compound according to any one of claims 1 to 17.

**19.** A delayed fluorescent substance comprising:
the compound according to any one of claims 1 to 17.

**20.** A film comprising:
the compound according to any one of claims 1 to 17.

**21.** An organic semiconductor device comprising:
the compound according to any one of claims 1 to 17.

**22.** An organic light emitting device comprising:
the compound according to any one of claims 1 to 17.

**23.** The organic light emitting device according to claim 22, wherein

the organic light emitting device includes a layer containing the compound, and
the layer also contains a host material.

**24.** The organic light emitting device according to claim 23, wherein

the layer containing the compound further contains a delayed fluorescent material in addition to the compound and the host material, and
the delayed fluorescent material has a lowest excited singlet energy lower than a lowest excited singlet energy of the host material and higher than a lowest excited singlet energy of the compound.

**25.** The organic light emitting device according to claim 23, wherein

the organic light emitting device includes the layer containing the compound, and
the layer also contains a light emitting material having a structure different from a structure of the compound.

**26.** The organic light emitting device according to claim 23, wherein
an amount of light emitted from the compound is largest among the materials contained in the organic light emitting device.

**27.** The organic light emitting device according to claim 25, wherein
an amount of light emitted from the light emitting material is larger than an amount of light emitted from the compound.

**28.** The organic light emitting device according to claim 22, which is an organic electroluminescent device.

**29.** The organic light emitting device according to claim 22, which emits delayed fluorescence.

## INTERNATIONAL SEARCH REPORT

International application No.
**PCT/JP2024/003317**

**A. CLASSIFICATION OF SUBJECT MATTER**

***C07D 401/14***(2006.01)i; ***C09K 11/06***(2006.01)i; ***H10K 50/00***(2023.01)i; ***H10K 50/10***(2023.01)i; ***H10K 50/11***(2023.01)i; ***H10K 59/00***(2023.01)i; ***H10K 59/10***(2023.01)i; ***H10K 85/60***(2023.01)i; *H10K 101/20*(2023.01)n
FI: C07D401/14 CSP; C09K11/06; C09K11/06 640; H05B33/14 B; H10K50/00; H10K50/10; H10K50/11; H10K59/00; H10K59/10; H10K85/60; H10K101:20

According to International Patent Classification (IPC) or to both national classification and IPC

**B. FIELDS SEARCHED**

Minimum documentation searched (classification system followed by classification symbols)

C07D401/14; C09K11/06; H10K50/00; H10K50/10; H10K50/11; H10K59/00; H10K59/10; H10K85/60; H10K101/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | CN 115433165 A (JIANGSU SUNERA TECHNOLOGY CO., LTD.) 06 December 2022 (2022-12-06)<br>claims, examples | 1-29 |
| X | WO 2020/218187 A1 (NIPPON STEEL CHEMICAL & MATERIAL CO., LTD.) 29 October 2020 (2020-10-29)<br>claims, paragraph [0045], examples | 1-7, 9, 12-29 |
| X | JP 2020-107868 A (SAMSUNG ELECTRONICS CO., LTD.) 09 July 2020 (2020-07-09)<br>claims, paragraph [0059], examples | 1-2, 4, 6, 9, 12-13, 15-29 |
| X | CN 110563705 A (HANGZHOU HONGWU TECHNOLOGY CO., LTD.) 13 December 2019 (2019-12-13)<br>claims, examples | 1, 3, 7, 9, 14-29 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 April 2024** | **23 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/003317**

**C. DOCUMENTS CONSIDERED TO BE RELEVANT**

| Category* | Citation of document, with indication, where appropriate, of the relevant passages | Relevant to claim No. |
|---|---|---|
| X | JP 2019-119723 A (SAMSUNG ELECTRONICS CO., LTD.) 22 July 2019 (2019-07-22) claims, paragraph [0112], examples | 1-2, 4, 6, 9, 12-13, 15-29 |
| X | CN 109851609 A (AAC TECHNOLOGIES (NANJING) INC.) 07 June 2019 (2019-06-07) claims, examples | 1-5, 7, 9, 11, 14-29 |
| X | US 2022/0098220 A1 (UNIVERSAL DISPLAY CORPORATION) 31 March 2022 (2022-03-31) claims, p. 135, examples | 1, 3, 7, 9, 14, 16-29 |
| X | CN 109651340 A (AAC TECHNOLOGIES (NANJING) INC.) 19 April 2019 (2019-04-19) claims, examples | 1, 3, 7, 9, 14, 16-29 |
| X | US 2019/0058130 A1 (KYULUX, INC.) 21 February 2019 (2019-02-21) claims, p. 83, examples | 1, 3, 7, 9, 14, 16-29 |
| P, X | US 2024/0025927 A1 (UNIVERSAL DISPLAY CORPORATION) 25 January 2024 (2024-01-25) p. 254, upper right, compound | 1, 3, 7, 9, 14-16, 18-29 |
| P, X | US 2023/0389421 A1 (UNIVERSAL DISPLAY CORPORATION) 30 November 2023 (2023-11-30) claim 11 | 1-3, 7, 9, 12, 14-15, 18-29 |

Form PCT/ISA/210 (second sheet) (July 2022)

**INTERNATIONAL SEARCH REPORT**
**Information on patent family members**

International application No.
**PCT/JP2024/003317**

| Patent document cited in search report | Publication date (day/month/year) | Patent family member(s) | Publication date (day/month/year) |
|---|---|---|---|
| CN 115433165 A | 06 December 2022 | (Family: none) | |
| WO 2020/218187 A1 | 29 October 2020 | US 2022/0216427 A1<br>claims, paragraph [0071], examples<br>EP 3967685 A1<br>CN 113727978 A<br>KR 10-2022-0004655 A | |
| JP 2020-107868 A | 09 July 2020 | US 2020/0212314 A1<br>claims, pp. 59, 60, examples<br>EP 3675194 A1<br>KR 10-2020-0083171 A<br>CN 111384300 A | |
| CN 110563705 A | 13 December 2019 | (Family: none) | |
| JP 2019-119723 A | 22 July 2019 | US 2019/0214570 A1<br>claims, pp. 208, 209, examples<br>KR 10-2019-0086347 A | |
| CN 109851609 A | 07 June 2019 | (Family: none) | |
| US 2022/0098220 A1 | 31 March 2022 | KR 10-2022-0043893 A | |
| CN 109651340 A | 19 April 2019 | (Family: none) | |
| US 2019/0058130 A1 | 21 February 2019 | (Family: none) | |
| US 2024/0025927 A1 | 25 January 2024 | (Family: none) | |
| US 2023/0389421 A1 | 30 November 2023 | EP 4282863 A1<br>CN 117105911 A<br>KR 10-2023-0163945 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021157600 A
- WO 2015022974 A
- WO 2022270354 A1
- WO 2013154064 A
- WO 2013011954 A
- WO 2013011955 A
- WO 2013081088 A
- JP 2013256490 A
- JP 2013116975 A
- WO 2013133359 A
- WO 2013161437 A
- JP 2014009352 A
- JP 2014009224 A
- JP 2017119663 A
- JP 2017119664 A
- JP 2017222623 A
- JP 2017226838 A
- JP 2018100411 A
- WO 2018047853 A
- JP 2013253121 A
- WO 2014034535 A
- WO 2014115743 A
- WO 2014122895 A
- WO 2014126200 A
- WO 2014136758 A
- WO 2014133121 A
- WO 2014136860 A
- WO 2014196585 A
- WO 2014189122 A
- WO 2014168101 A
- WO 2015008580 A
- WO 2014203840 A
- WO 2015002213 A
- WO 2015016200 A
- WO 2015019725 A
- WO 2015072470 A
- WO 2015108049 A
- WO 2015080182 A
- WO 2015072537 A
- WO 2015080183 A
- JP 2015129240 A
- WO 2015129714 A
- WO 2015129715 A
- WO 2015133501 A
- WO 2015136880 A
- WO 2015137244 A
- WO 2015137202 A
- WO 2015137136 A
- WO 2015146541 A
- WO 2015159541 A

### Non-patent literature cited in the description

- **HANSCH, C.** *Chem. Rev.*, 1991, vol. 91, 165-195